(19) Europäisches Patentamt
European Patent Office
Office européen des brevets

(11) EP 4 269 422 A1

(12) **EUROPEAN PATENT APPLICATION**
published in accordance with Art. 153(4) EPC

(43) Date of publication:
**01.11.2023 Bulletin 2023/44**

(21) Application number: **21911028.5**

(22) Date of filing: **24.12.2021**

(51) International Patent Classification (IPC):
***C07K 1/04*** (2006.01) ***C07C 269/06*** (2006.01)
***C07C 271/22*** (2006.01) ***C07K 5/06*** (2006.01)
***C07K 5/08*** (2006.01) ***C07K 5/10*** (2006.01)
***C07K 7/06*** (2006.01) ***C07K 7/64*** (2006.01)

(52) Cooperative Patent Classification (CPC):
**C07C 269/06; C07C 271/22; C07K 1/04; C07K 5/06; C07K 5/08; C07K 5/10; C07K 7/06; C07K 7/64**

(86) International application number:
**PCT/JP2021/048093**

(87) International publication number:
**WO 2022/138891 (30.06.2022 Gazette 2022/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.12.2020 JP 2020217157**
**06.05.2021 JP 2021078395**
**29.10.2021 JP 2021177528**

(71) Applicant: **CHUGAI SEIYAKU KABUSHIKI KAISHA**
**Tokyo 115-8543 (JP)**

(72) Inventors:
- **MORITA, Yuya**
**Kamakura-shi, Kanagawa 247-8530 (JP)**
- **NOMURA, Kenichi**
**Gotemba-shi, Shizuoka 412-8513 (JP)**

(74) Representative: **Vossius & Partner Patentanwälte Rechtsanwälte mbB**
**Siebertstraße 3**
**81675 München (DE)**

Remarks:
The complete document including Reference Table(s) and the Sequence Listing(s) can be downloaded from the EPO website

(54) **METHOD FOR PRODUCING PEPTIDE COMPOUND CONTAINING N-SUBSTITUTED-AMINO ACID RESIDUE**

(57) An object of the present invention is to provide a method for efficiently producing a high-purity peptide compound with a high yield. It has been found that the object can be achieved by supporting a peptide on a solid phase synthesis resin prior to a first elongation reaction in a solid phase process.

EP 4 269 422 A1

## Description

Technical Field

The present invention relates to a method for producing a peptide compound containing N-substituted-amino acid residue.

Background Art

Middle-molecule compounds (molecular weight: 500 to 2000) have attracted attention as a modality capable of achieving development of a drug against a tough target which is typified by protein-protein interaction inhibition or the like (Non Patent Literature 1).

It has been considered difficult to develop a peptide itself as a medicament because peptides are generally poor in druglikeness (metabolic stability and membrane permeability). In recent years, it has been found that by cyclization of a peptide or use of a non-natural amino acid such as a N-methylamino acid in a peptide, metabolic stability and membrane permeability are improved (Non Patent Literatures 2 and 3).

It has come to be known that among cyclic peptides containing a non-natural amino acid, particularly, cyclic peptides containing a N-substituted amino acid have druglikeness (Patent Literature 1).

It has been also suggested that library compounds of cyclic peptides containing a non-natural amino acid are useful for construction of a protein-protein interaction inhibitor (Non Patent Literature 4).

Conditions for ensuring that a cyclic peptide containing a non-natural amino acid is a druglike molecule having membrane permeability and metabolic stability allowing the use of the cyclic peptide as a medicament have been made clear, and cyclic peptides as a medicament modality have attracted increasing attention (Patent Literatures 2 and 3).

On the other hand, production of a peptide containing a N-methylamino acid in a sequence thereof has been considered to have the problem of low reactivity in a condensation reaction due to steric hindrance of a N-methyl group and reduction of the yield of a intended product due to racemization of an amino acid residue at the $\alpha$-position. Many problems with a N-methylamino residue site have been reported, e.g. an amide bond is likely to undergo a cleavage reaction under acidic conditions, and deficiency caused by a detachment reaction of two amino acid residues at the N-terminal due to formation of diketopiperazine easily occurs, and it is widely recognized that there is a higher degree of difficulty as compared to synthesis of a natural peptide (Non Patent Literature 5).

Synthesis of a peptide is achieved by elongation into a desired sequence by forming an amide bond. More specific methods include a liquid phase process and a solid phase process (Non Patent Literature 6).

Of these, the solid phase process comprises a step of preparing a solid phase synthesis resin in which an amino acid or a C-terminal of a peptide is supported on the solid phase synthesis resin using an atomic group linked to a polymer resin (solid phase synthesis resin) as a linker (supporting step); a step of deprotecting the amino acid supported on the solid phase synthesis resin, or a N-terminal amino group of the peptide; a condensation step of introducing an amino acid protected at the N-terminal through a condensation reaction as a subsequent sequence; an elongation step of repeating the deprotection step and the condensation step up to a desired sequence to link amino acid residues together to a peptide chain having an intended sequence; and cutting a peptide having an intended sequence from the solid phase synthesis resin (resin detachment step). As the amino acid protected at the N-terminal which is used in the elongation step, amino acids are commonly used in which an amino group mainly at the N-terminal is protected with a Fmoc group or a Boc group (Non Patent Literatures 7 and 8).

The solid phase synthesis resin is roughly classified according to an atomic group which is bound to a polymer to be used for the resin and which serves as a linker, and solid phase synthesis resins to which a linker atomic group containing a trityl skeleton or a benzyl skeleton is bound are commonly used. More specifically, the solid phase synthesis resin is typically CTS resin, Wang resin, SASRIN resin or Rink Amide resin (Non Patent Literature 8).

The resin detachment step is carried out mainly under acidic conditions, and the ease of resin detachment is determined depending on the stability of the linker atomic group to an acid. For example, a resin detachment reaction of a peptide from CTC resin on which a peptide residue can be supported using a trityl skeleton as a linker can be carried out even with a weakly acidic reagent. On the other hand, strong acid conditions are applied to a resin detachment reaction of a peptide from Wang resin to which a peptide can be bound using a benzyl skeleton as a linker (Non Patent Literature 8).

When CTC resin is used, it is possible to carry out a resin detachment reaction of a peptide under milder acidic conditions, and therefore in production of a peptide using CTC resin, a peptide having a protective group that is easily removed under acidic conditions can be selectively removed from the resin without elimination of the protective group. Thus, CTC resin is useful for production of a peptide protected with such a protection group (Non Patent Literature 9). On the other hand, it has been reported that in solid phase synthesis of a peptide using CTC resin, a peptide can be removed from CTC resin under mild conditions, and therefore a covalent bond between an amino acid or a peptide

supported on CTC resin and a linker of the resin is cleaved under condensation reaction conditions, so that the yield of an intended peptide decreases (sometimes referred to as premature cleavage, premature peptide release or premature acidolytic cleavage) (Non Patent Literatures 10 and 11).

## Citation List

### Patent Literature

Patent Literature 1: International Publication No. WO 2013/100132
Patent Literature 2: International Publication No. WO 2018/115864
Patent Literature 3: International Publication No. WO 2020/122182

### Non Patent Literature

Non Patent Literature 1: Future Med. Chem., 2009, 1, 1289-1310.
Non Patent Literature 2: Acc. Chem. Res., 2008, 41, 1331-1342.
Non Patent Literature 3: Angew. Chem. Int. Ed., 2013, 52, 254-269.
Non Patent Literature 4: Chem. Rev., 2019, 119, 10360-10391.
Non Patent Literature 5: J. Peptide Res., 2005, 65, 153-166.
Non Patent Literature 6: Amino Acids, Peptides and Proteins in Organic Chemistry: Building Blocks, Catalysis and Coupling Chemistry, Volume 3, 2011
Non Patent Literature 7: Amino Acids, 2018, 50, 39-68.
Non Patent Literature 8: Solid phase peptide synthesis (issued by Bachem) [Search on November 6, 2020], Internet <URL:https://www.bachem.com/fileadmin/user_upload/pdf/Catalogs_Brochures/Solid_Phase_Peptide_Synthesis.p df>
Non Patent Literature 9: QSAR Comb. Sci., 2007, 26, 1027-1035.
Non Patent Literature 10: Biopolymers, 2012, 98, 89-97.
Non Patent Literature 11: ACS Comb. Sci., 2013, 15, 229-234.

## Summary of Invention

### Technical Problem

An object of the present invention is to provide a method for efficiently producing a high-purity peptide compound with a high yield.

Non Patent Literatures 10 and 11 indicate that when an acidic additive such as oxyma, HOBt or HOAt is used in a condensation reaction for formation of an amide bond by condensation of a carboxyl group of a Fmoc-protected natural amino acid with an amino group of a natural amino acid supported on CTC resin, the yield decreases due to premature cleavage, whereas the yield may increase in a condensation reaction using HBTU and DIPEA as basic conditions. However, since the inhibitory effect on premature cleavage is limited, and under basic conditions, racemization of amino acids may occur, the reaction conditions cannot be said to be preferred. There are no known reports on problems of premature cleavage particularly in synthesis of a peptide containing a non-natural amino acid with significant steric hindrance, such as a N-methylamino acid.

The present inventors have tried to identify amino acids which may be associated with premature cleavage in a solid phase synthesis method using CTC resin. Further, the present inventors have conducted studies on synthesis of a peptide containing an amino acid residue with significant steric hindrance, such as a N-substituted-amino acid in a solid phase synthesis method using CTC resin, and resultantly found that in a step of condensation of a C-terminal amino acid (sometimes referred to as a "first-residue amino acid") supported on a solid phase synthesis resin with a second residue amino acid from the C-terminal (sometimes referred to simply as a "second residue amino acid"), there occur (i) a reaction of detachment of the first residue amino acid residue from CTC resin, i.e. a decrease in yield due to premature cleavage, and (ii) a decrease in purity due to generation of an excessively elongated form as a by-product in which detached amino acids are caught superfluously in an intended amino acid sequence.

As described above, detachment of an amino acid residue or a peptide residue supported on a solid phase synthesis resin from a solid phase synthesis resin linker, more specifically, detachment of a first residue amino acid

residue directly bound to the solid phase synthesis resin from the solid phase synthesis resin linker may occur for various amino acid residues. Nevertheless, an efficient peptide synthesis method which is less likely to have the above-described problem and less likely to cause generation of by-products has not been known so far. An object of the present invention is to provide a method for producing a high-purity peptide compound with a high yield using, as a peptide supported on a solid phase synthesis resin, which method is also applicable to production of a peptide containing a non-natural amino acid residue.

Solution to Problem

The present inventors have conducted studies for achieving the above-described object, and resultantly found a method in which an oligopeptide is directly supported on a resin in solid phase synthesis of a peptide compound containing a non-natural amino acid with significant steric hindrance. This enables avoidance of a step of condensation of first residue and second residue amino acids in a solid phase synthesis method which is likely to cause premature cleavage. It has been also confirmed that an oligopeptide is hardly detached from the resin, so that premature cleavage is suppressed when an oligopeptide residue supported on the solid phase synthesis resin is subjected to an elongation step with the addition of amino acids.

The present invention encompasses the following in one non-limiting specific aspect.

[1] A method for producing a peptide compound containing at least one N-substituted amino acid residue, a salt thereof or a solvate thereof by a solid phase process, wherein a peptide is supported on a solid phase synthesis resin before a first elongation reaction in the solid phase process.

[2] A method for producing a peptide compound containing at least one N-substituted amino acid residue, a salt thereof or a solvate thereof by a solid phase process, the method comprising a step of supporting a peptide on a solid phase synthesis resin.

[3] The method according to [1] or [2], wherein the peptide is an oligopeptide containing two or more amino acid residues.

[4] The method according to any of [1] to [3], wherein the peptide is a dipeptide or a tripeptide.

[5] The method according to any of [1] to [4], wherein an amino acid residue at the C-terminal of the peptide and/or an amino acid residue adjacent to the amino acid residue at the C-terminal are non-natural amino acid residues.

[6] The method according to any of [1] to [5], wherein the amino acid residue at the C-terminal of the peptide is a non-natural amino acid residue.

[7] The method according to [5] or [6], wherein the non-natural amino acid residue is a N-substituted amino acid residue.

[8] The method according to any of [1] to [7], wherein the amino acid residue at the C-terminal of the peptide is supported on the solid phase synthesis resin by a carboxyl group bonded to a carbon atom at the β-position or a carbon atom at the γ-position on the amino group.

[9] The method according to any of [1] to [8], wherein an amino acid residue at the C-terminal of the peptide and/or an amino acid residue adjacent to the amino acid residue at the C-terminal have a bulky side chain.

[10] The method according to [9], wherein the bulky side chain is an optionally substituted branched-chain alkyl group.

[11] The method according to [10], wherein the branched-chain alkyl group is bonded to a carbon atom at the α-position on the carboxyl group.

[12] The method according to [11], wherein the branched-chain alkyl group has a branch on a carbon atom at the β-position or a carbon atom at the γ-position on the carboxyl group.

[13] The method according to any of [1] to [12], wherein at least one N-substituted amino acid residue present in the peptide compound is a non-natural N-substituted amino acid residue.

[14] The method according to any of [1] to [13], wherein the peptide compound contains at least two N-substituted amino acid residues.

[15] The method according to any of [1] to [14], wherein the N-substituted amino acid residue constitutes 30% or more of all amino acid residues forming the peptide compound.

[16] The method according to any of [1] to [15], wherein the amino acid residue at the C-terminal of the peptide is aspartic acid, 2-aminobutanoic acid, glycine, alanine, valine, proline, tyrosine or 2-aminoisobutyric acid, or a N-substituted form or a derivative thereof, where aspartic acid or a N-substituted form or a derivative thereof is supported on the solid phase synthesis resin by the carboxyl group at the β-position on the amino group.

[17] The method according to any of [1] to [16], wherein the amino acid residue at the C-terminal of the peptide is represented by the following formula (A):

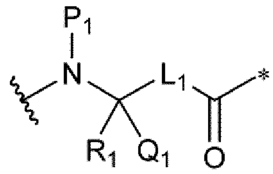

(A)

wherein

$L_1$ is a single bond, or $-CHM_1-$, $-CH_2CHM_1-$, $-CHM_1CH_2-$, $-(CH_2)_nS(CH_2)_m-$, $-(CH_2)_nSO(CH_2)_m-$, or $-(CH_2)_nSO_2(CH_2)_m-$, where n and m are each independently 1 or 2,
$R_1$ is hydrogen, $C_1$-$C_6$ alkyl, $C_2$-$C_6$ alkenyl, $C_2$-$C_6$ alkynyl, $C_1$-$C_6$ alkoxy $C_1$-$C_6$ alkyl, $C_7$-$C_{14}$ aralkyl, or aminocarbonyl (the amino is $-NH_2$, mono-$C_1$-$C_6$ alkylamino, di-$C_1$-$C_6$ alkylamino, or 4- to 8-membered cyclic amino), which is optionally substituted with one or more groups independently selected from the group consisting of halogen, oxo, hydroxy, $C_1$-$C_6$ alkyl, 4- to 7-membered heterocyclyl, aminocarbonyl (the amino is $-NH_2$, mono-$C_1$-$C_6$ alkylamino, di-$C_1$-$C_6$ alkylamino, or 4- to 8-membered cyclic amino), $C_1$-$C_6$ alkylsulfonyl, and $C_1$-$C_6$ alkoxy $C_1$-$C_6$ alkyl, or $R_1$ is a peptide chain containing one to four amino acid residues, or
$R_1$ and $P_1$ form a 4- to 7-membered saturated heterocyclic ring together with a carbon atom bonded to $R_1$ and a nitrogen atom bonded to $P_1$, or
$R_1$ and $Q_1$ form a 3- to 8-membered alicyclic ring or a 4- to 7-membered saturated heterocyclic ring together with a carbon atom bonded thereto, or
$R_1$ and $M_1$ form a 3- to 8-membered alicyclic ring together with a carbon atom bonded to $R_1$ and a carbon atom bonded to $M_1$,
$P_1$ is hydrogen, or $C_1$-$C_6$ alkyl, where the $C_1$-$C_6$ alkyl is optionally substituted with one or more groups independently selected from the group consisting of halogen, hydroxy, $C_1$-$C_6$ alkoxy, amino (the amino is $-NH_2$, mono-$C_1$-$C_6$ alkylamino, di-$C_1$-$C_6$ alkylamino, or 4- to 8-membered cyclic amino, which is optionally substituted with halogen), and aminocarbonyl (the amino is $-NH_2$, mono-$C_1$-$C_6$ alkylamino, di-$C_1$-$C_6$ alkylamino, or 4- to 8-membered cyclic amino), except when $R_1$ and $P_1$ form a 4- to 7-membered saturated heterocyclic ring,
$Q_1$ is hydrogen or $C_1$-$C_6$ alkyl except when $R_1$ and $Q_1$ form a 3- to 8-membered alicyclic ring or 4- to 7-membered saturated heterocyclic ring,
$M_1$ is hydrogen except when $R_1$ and $M_1$ form a 3- to 8-membered alicyclic ring,
* represents a site of binding to the solid phase synthesis resin, and
the wavy line represents a site of binding to the adjacent amino acid residue.

[18] The method according to [17], wherein $L_1$ is $-CHM_1-$,

$R_1$ is hydrogen, $C_1$-$C_6$ alkyl, halo $C_1$-$C_6$ alkyl, $C_2$-$C_6$ alkenyl, $C_2$-$C_6$ alkynyl, $C_1$-$C_6$ alkoxy $C_1$-$C_6$ alkyl (the $C_1$-$C_6$ alkoxy $C_1$-$C_6$ alkyl is optionally substituted with hydroxy, or aminocarbonyl (the amino is - $NH_2$, mono-$C_1$-$C_6$ alkylamino, di-$C_1$-$C_6$ alkylamino, or 4- to 8-membered cyclic amino)), $C_7$-$C_{14}$ aralkyl optionally substituted with one or more halogens, or aminocarbonyl (the amino is $-NH_2$, mono-$C_1$-$C_6$ alkylamino, di-$C_1$-$C_6$ alkylamino, or 4- to 8-membered cyclic amino, and the cyclic amino is optionally substituted with one or more halogens, one or more oxos, one or more $C_1$-$C_6$ alkyls, or 4- to 7-membered heterocyclyl), or
$R_1$ and $M_1$ form a 3- to 8-membered alicyclic ring together with a carbon atom bonded to $R_1$ and a carbon atom bonded to $M_1$, or
$R_1$ and $P_1$ form a 4- to 7-membered saturated heterocyclic ring together with a nitrogen atom bonded to $P_1$ and a carbon atom bonded to $R_1$,
$M_1$ is hydrogen except when $R_1$ and $M_1$ form a 3- to 8-membered alicyclic ring,
$P_1$ is hydrogen or $C_1$-$C_6$ alkyl except when $R_1$ and $P_1$ form a 4- to 7-membered saturated heterocyclic ring, and
$Q_1$ is hydrogen.

[19] The method according to [17], wherein the amino acid residue at the C-terminal of the peptide is bAla, bMeAla, 2-ACHxC, 2-ACPnC, 3-CF3-bAla, Asp-mor, Asp-mor(26-bicyc), Asp-mor(SO2), Asp-NMe2, Asp-oxz, Asp-pip, Asp-pip(345-F6), Asp-pip(4-Me), Asp-pip-tBu, Asp-piz(oxe), Asp-pyrro, Asp-pyrro(34-F4), Asp-pyrro(3-Me2), D-(Propargyl)Gly-(C#CH2), D-3-Abu, D-3-MeAbu, D-Gly(Allyl)-(C#CH2), D-Hph-(C#CH2), D-Leu-(C#CH2), D-MeAsp-pyrro, D-MeLeu-(C#CH2), D-Pic(2)-(C#CH2), D-Pro-(C#CH2), D-Ser(iPen)-(C#CH2), D-Ser(NtBu-Aca)-(C#CH2), EtAsp-pip, MeAsp-aze, MeAsp-mor, MeAsp-mor(26-bicyc), MeAsp-mor(SO2), MeAsp-NMe2, MeAsp-oxz, MeAsp-pip, MeAsp-pip(345-F6), MeAsp-pip(3-F2), MeAsp-pip(4-F2), MeAsp-pip(4-Me), MeAsp-piz(oxe), MeAsp-pyrro, MeAsp-pyrro(34-F4), MeAsp-pyrro(3-Me2), nPrAsp-pip, MeGly, MeVal, Pro, Aib, Ala, Gly, Tyr(tBu), Val, D-MeAsp-NMe2, Glu-mor, Glu-pip, MeGlu-pip, Glu-NMe2, MeGlu-NMe2, or MeCys(AcOH)-NMe2.

[20] The method according to any of [1] to [19], wherein the amino acid residue adjacent to the amino acid residue at the C-terminal of the peptide is represented by the following formula (B):

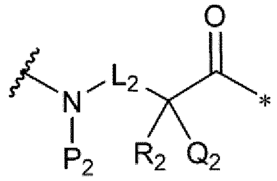

(B)

wherein

$L_2$ is a single bond, or $-CH_2-$,

$R_2$ is hydrogen, $C_1$-$C_6$ alkyl, $C_2$-$C_6$ alkynyl, $C_1$-$C_6$ alkoxy $C_1$-$C_6$ alkyl, $C_3$-$C_8$ cycloalkyl, $C_3$-$C_8$ cycloalkyl $C_1$-$C_6$ alkyl, $C_3$-$C_8$ cycloalkoxy $C_1$-$C_6$ alkyl, or $C_7$-$C_{14}$ aralkyl, which is optionally substituted with one or more groups independently selected from the group consisting of halogen, hydroxy, amino (the amino is $-NH_2$, protected amino, mono-$C_1$-$C_6$ alkylamino, di-$C_1$-$C_6$ alkylamino, or 4- to 8-membered cyclic amino, which is optionally substituted with halogen), aminocarbonyl (the amino is $-NH_2$, protected amino, mono-$C_1$-$C_6$ alkylamino, di-$C_1$-$C_6$ alkylamino, or 4- to 8-membered cyclic amino), and $C_1$-$C_6$ alkylsulfonyl, or

$R_2$ and $P_2$ form a 4- to 7-membered saturated heterocyclic ring together with a carbon atom bonded to $R_2$ and a nitrogen atom bonded to $P_2$, or

$R_2$ and $Q_2$ form a 3- to 8-membered alicyclic ring or a 4- to 7-membered saturated heterocyclic ring together with a carbon atom bonded thereto, or

$P_2$ is hydrogen, or $C_1$-$C_6$ alkyl, where the $C_1$-$C_6$ alkyl is optionally substituted with one or more groups independently selected from the group consisting of halogen, hydroxy, $C_1$-$C_6$ alkoxy, amino (the amino is $-NH_2$, mono-$C_1$-$C_6$ alkylamino, di-$C_1$-$C_6$ alkylamino, or 4- to 8-membered cyclic amino, which is optionally substituted with halogen), and aminocarbonyl (the amino is $-NH_2$, mono-$C_1$-$C_6$ alkylamino, di-$C_1$-$C_6$ alkylamino, or 4- to 8-membered cyclic amino), except when $R_2$ and $P_2$ form a 4- to 7-membered saturated heterocyclic ring,

$Q_2$ is hydrogen or $C_1$-$C_6$ alkyl except when $R_2$ and $Q_2$ form a 3- to 8-membered alicyclic ring or 4- to 7-membered saturated heterocyclic ring,

* represents a site of binding to the amino acid residue at the C-terminal, and

the wavy line represents a site of binding to the adjacent amino acid residue or a protective group for the amino group.

[21] The method according to [20], wherein $R_2$ is $C_1$-$C_6$ alkyl, halo $C_1$-$C_6$ alkyl, hydroxy $C_1$-$C_6$ alkyl, $C_1$-$C_6$ alkylsulfonyl $C_1$-$C_6$ alkyl, $C_2$-$C_6$ alkynyl, $C_1$-$C_6$ alkoxy $C_1$-$C_6$ alkyl optionally substituted with one or more halogens, $C_3$-$C_8$ cycloalkyl, $C_3$-$C_8$ cycloalkyl $C_1$-$C_6$ alkyl, $C_3$-$C_8$ cycloalkoxy $C_1$-$C_6$ alkyl, or $C_7$-$C_{14}$ aralkyl, or

$R_2$ and $P_2$ form a 4- to 7-membered saturated heterocyclic ring together with a nitrogen atom bonded to $P_2$ and a carbon atom bonded to $R_2$, and

$P_2$ is hydrogen or $C_1$-$C_6$ alkyl except when $R_2$ and $P_2$ form a 4- to 7-membered saturated heterocyclic ring.

[22] The method according to [21], wherein the amino acid residue adjacent to the amino acid residue at the C-terminal of the peptide is MeAla, MeLeu, MeCha, MeVal, MeAla(cPent), MeAla(cBu), MeAla(cPr), MeChg, MeGly(cPent), MeGly(cBu), MeGly(cPr), MeAbu, MeNva, MeNle, Val, Leu, MeNva(5-F2), MeHle, MeIle, MeSer(nPr), MeSer(cPr), MeHnl, MeHnl(7-F2), MePRA, MeSer(Me), MeThr, MeSer(cBu), MeSer(Tfe), MeThr(Me), MeHse(Me), MeMet(O2), Ile, Nle, Chg, Ala(cBu), Gly(cPent), Hle, Nva, Phe, Hph, Gly, Aib, Lys(Boc), Ala, D-MeVal, Asn(Trt), Ser(tBu), or bAla(2-Me2).

[23] The method according to any of [1] to [22], wherein the peptide is a dipeptide represented by the following formula (1):

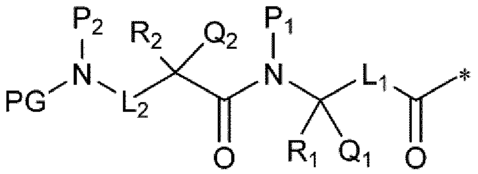

(1)

wherein

$L_1$ is a single bond, or $-CHM_1-$, $-CH_2CHM_1-$, $-CHM_1CH_2-$, $-(CH_2)_nS(CH_2)_m-$, $-(CH_2)_nSO(CH_2)_m-$, or $-(CH_2)_nSO_2(CH_2)_m-$, where n and m are each independently 1 or 2,
$R_1$ is hydrogen, $C_1$-$C_6$ alkyl, $C_2$-$C_6$ alkenyl, $C_2$-$C_6$ alkynyl, $C_1$-$C_6$ alkoxy $C_1$-$C_6$ alkyl, $C_7$-$C_{14}$ aralkyl, or aminocarbonyl (the amino is $-NH_2$, mono-$C_1$-$C_6$ alkylamino, di-$C_1$-$C_6$ alkylamino, or 4- to 8-membered cyclic amino), which is optionally substituted with one or more groups independently selected from the group consisting of halogen, oxo, hydroxy, $C_1$-$C_6$ alkyl, 4- to 7-membered heterocyclyl, aminocarbonyl (the amino is $-NH_2$, mono-$C_1$-$C_6$ alkylamino, di-$C_1$-$C_6$ alkylamino, or 4- to 8-membered cyclic amino), and $C_1$-$C_6$ alkylsulfonyl, or $R_1$ is a peptide chain containing one to four amino acid residues, or
$R_1$ and $P_1$ form a 4- to 7-membered saturated heterocyclic ring together with a carbon atom bonded to $R_1$ and a nitrogen atom bonded to $P_1$, or
$R_1$ and $Q_1$ form a 3- to 8-membered alicyclic ring or a 4- to 7-membered saturated heterocyclic ring together with a carbon atom bonded thereto, or
$R_1$ and $M_1$ form a 3- to 8-membered alicyclic ring together with a carbon atom bonded to $R_1$ and a carbon atom bonded to $M_1$,
$P_1$ is hydrogen, or $C_1$-$C_6$ alkyl, where the $C_1$-$C_6$ alkyl is optionally substituted with one or more groups independently selected from the group consisting of halogen, hydroxy, $C_1$-$C_6$ alkoxy, amino (the amino is $-NH_2$, mono-$C_1$-$C_6$ alkylamino, di-$C_1$-$C_6$ alkylamino, or 4- to 8-membered cyclic amino, which is optionally substituted with halogen), and aminocarbonyl (the amino is $-NH_2$, mono-$C_1$-$C_6$ alkylamino, di-$C_1$-$C_6$ alkylamino, or 4- to 8-membered cyclic amino), except when $R_1$ and $P_1$ form a 4- to 7-membered saturated heterocyclic ring,
$Q_1$ is hydrogen or $C_1$-$C_6$ alkyl except when $R_1$ and $Q_1$ form a 3- to 8-membered alicyclic ring or 4- to 7-membered saturated heterocyclic ring,
$M_1$ is hydrogen except when $R_1$ and $M_1$ form a 3- to 8-membered alicyclic ring,
$L_2$ is a single bond, or $-CH_2-$,
$R_2$ is hydrogen, $C_1$-$C_6$ alkyl, $C_2$-$C_6$ alkynyl, $C_1$-$C_6$ alkoxy $C_1$-$C_6$ alkyl, $C_3$-$C_8$ cycloalkyl, $C_3$-$C_8$ cycloalkyl $C_1$-$C_6$ alkyl, $C_3$-$C_8$ cycloalkoxy $C_1$-$C_6$ alkyl, or $C_7$-$C_{14}$ aralkyl, which is optionally substituted with one or more groups independently selected from the group consisting of halogen, hydroxy, amino (the amino is $-NH_2$, protected amino, mono-$C_1$-$C_6$ alkylamino, di-$C_1$-$C_6$ alkylamino, or 4- to 8-membered cyclic amino, which is optionally substituted with halogen), aminocarbonyl (the amino is $-NH_2$, protected amino, mono-$C_1$-$C_6$ alkylamino, di-$C_1$-$C_6$ alkylamino, or 4- to 8-membered cyclic amino), and $C_1$-$C_6$ alkylsulfonyl, or
$R_2$ and $P_2$ form a 4- to 7-membered saturated heterocyclic ring together with a carbon atom bonded to $R_2$ and a nitrogen atom bonded to $P_2$, or
$R_2$ and $Q_2$ form a 3- to 8-membered alicyclic ring or a 4- to 7-membered saturated heterocyclic ring together with a carbon atom bonded thereto, or
$P_2$ is hydrogen, or $C_1$-$C_6$ alkyl, where the $C_1$-$C_6$ alkyl is optionally substituted with one or more groups independently selected from the group consisting of halogen, hydroxy, $C_1$-$C_6$ alkoxy, amino (the amino is $-NH_2$, mono-$C_1$-$C_6$ alkylamino, di-$C_1$-$C_6$ alkylamino, or 4- to 8-membered cyclic amino, which is optionally substituted with halogen), and aminocarbonyl (the amino is $-NH_2$, mono-$C_1$-$C_6$ alkylamino, di-$C_1$-$C_6$ alkylamino, or 4- to 8-membered cyclic amino), except when $R_2$ and $P_2$ form a 4- to 7-membered saturated heterocyclic ring,
$Q_2$ is hydrogen or $C_1$-$C_6$ alkyl except when $R_2$ and $Q_2$ form a 3- to 8-membered alicyclic ring or 4- to 7-membered saturated heterocyclic ring,
* represents a site of binding to the solid phase synthesis resin, and
PG is a protective group for the amino group, provided that both $P_1$ and $P_2$ are not hydrogen.

[24] The method according to [23], wherein the peptide is a dipeptide represented by the following formula (2):

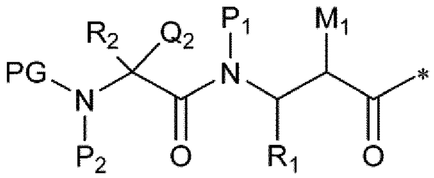
(2)

wherein

$R_1$ is hydrogen, $C_1$-$C_6$ alkyl, halo $C_1$-$C_6$ alkyl, $C_2$-$C_6$ alkenyl, $C_2$-$C_6$ alkynyl, $C_1$-$C_6$ alkoxy $C_1$-$C_6$ alkyl (the $C_1$-$C_6$ alkoxy $C_1$-$C_6$ alkyl is optionally substituted with hydroxy, or aminocarbonyl (the amino is - $NH_2$, mono-$C_1$-$C_6$ alkylamino, di-$C_1$-$C_6$ alkylamino, or 4- to 8-membered cyclic amino)), $C_7$-$C_{14}$ aralkyl optionally substituted with one or more halogens, or aminocarbonyl (the amino is -$NH_2$, mono-$C_1$-$C_6$ alkylamino, di-$C_1$-$C_6$ alkylamino, or 4- to 8-membered cyclic amino, and the cyclic amino is optionally substituted with one or more halogens, one or more oxos, one or more $C_1$-$C_6$ alkyls, or 4- to 7-membered heterocyclyl), or
$R_1$ and $M_1$ form a 3- to 8-membered alicyclic ring together with a carbon atom bonded to $R_1$ and a carbon atom bonded to $M_1$, or
$R_1$ and $P_1$ form a 4- to 7-membered saturated heterocyclic ring together with a nitrogen atom bonded to $P_1$ and a carbon atom bonded to $R_1$,
$M_1$ is hydrogen except when $R_1$ and $M_1$ form a 3- to 8-membered alicyclic ring,
$P_1$ is hydrogen or $C_1$-$C_6$ alkyl except when $R_1$ and $P_1$ form a 4- to 7-membered saturated heterocyclic ring,
$R_2$ is $C_1$-$C_6$ alkyl, halo $C_1$-$C_6$ alkyl, hydroxy $C_1$-$C_6$ alkyl, $C_1$-$C_6$ alkylsulfonyl $C_1$-$C_6$ alkyl, $C_2$-$C_6$ alkynyl, $C_1$-$C_6$ alkoxy $C_1$-$C_6$ alkyl optionally substituted with one or more halogens, $C_3$-$C_8$ cycloalkyl, $C_3$-$C_8$ cycloalkyl $C_1$-$C_6$ alkyl, $C_3$-$C_8$ cycloalkoxy $C_1$-$C_6$ alkyl, or $C_7$-$C_{14}$ aralkyl, or
$R_2$ and $P_2$ form a 4- to 7-membered saturated heterocyclic ring together with a nitrogen atom bonded to $P_2$ and a carbon atom bonded to $R_2$,
$P_2$ is hydrogen or $C_1$-$C_6$ alkyl except when $R_2$ and $P_2$ form a 4- to 7-membered saturated heterocyclic ring,
$Q_2$ is hydrogen,
* represents a site of binding to the solid phase synthesis resin, and
PG is a protective group for the amino group,
provided that both $P_1$ and $P_2$ are not hydrogen.

[25] The method according to any of [1] to [24], wherein the solid phase synthesis resin is a resin which can be removed under mild acidic conditions.
[26] The method according to [25], wherein the mild acidic conditions are conditions under which protective groups on side chains of one or more amino acids present in the peptide compound supported on the solid phase synthesis resin are not removed.
[27] The method according to [25] or [26], wherein the mild acidic conditions include conditions at a temperature around room temperature.
[28] The method according to any of [25] to [27], wherein the mild conditions include conditions of using a dilute acid solution, and the dilute condition of an acid is obtained by diluting the acid with a non-acidic solvent.
[29] The method according to any of [25] to [28], wherein the mild acidic conditions are acidic conditions with a pH of 2 or more.
[30] The method according to [28] or [29], wherein the acid is an acid having a pKa in water of -1 or more, 1 or more, 2 or more, 3 or more, 4 or more, 5 or more, 6 or more, 7 or more, 8 or more, 9 or more, 10 or more, 11 or more, or 12 or more.
[31] The method according to [30], wherein the acid is TFA, 2,2,2-trifluoroethanol, 1,1,1,3,3,3-hexafluoroisopropyl alcohol, trichloroacetic acid, acetic acid, formic acid or oxalic acid, or a mixture thereof.
[32] The method according to any of [29] to [31], wherein a volume % of the acid in the dilute solution is 60% or less, 50% or less, 40% or less, 30% or less, 20% or less, 10% or less, 5% or less, 4% or less, 3% or less, 2% or less, or 1% or less.
[33] The method according to any of [29] to [32], wherein the non-acidic solvent is DCM, dichloroethane, water or 2-MeTHF, or a mixed solvent thereof.
[34] The method according to any of [26] to [33], wherein the protective group is selected from the group consisting of Boc, Trt, THP, and tBu.
[35] The method according to any of [26] to [34], wherein the amino acid having a protective group on the side chain is Tyr(tBu), Ser(tBu), Thr(tBu), Asp(tBu), Glu(tBu), Trp(Boc), Lys(Boc), His(Boc), Ser(Trt), Thr(Trt), Trp(Trt), Lys(Trt), His(Trt), Asn(Trt), Gln(Trt), Ser(THP) or Thr(THP), or a N-alkyl form thereof.

[36] The method according to any of [1] to [35], wherein the solid phase synthesis resin is CTC resin, Wang resin, SASRIN resin, Trt resin, Mtt resin, Mmt resin, or Sieber resin.
[37] The method according to [36], wherein the solid phase synthesis resin is CTC resin, or Sieber resin.
[38] The method according to any of [1] and [3] to [37], comprising a step of supporting a peptide on the solid phase synthesis resin.
[39] The method according to any of [1] to [38], further comprising a step of elongating a peptide by one or more amino acid residues.
[40] A method for producing a cyclic peptide, a salt thereof, or a solvate thereof, the method comprising the steps of:

obtaining a peptide compound containing at least one N-substituted amino acid residue, a salt thereof or a solvate thereof in accordance with the method according to any of [1] to [39];
removing a solid phase synthesis resin; and
cyclizing a C-terminal-side group and a N-terminal-side group of the peptide compound, a salt thereof or a solvate thereof to form a cyclic portion.

[41] A method for improving a recovery ratio of a peptide compound as compared to elongation with amino acid residues one by one, wherein a peptide is supported on a solid phase synthesis resin before a first elongation reaction in production of a peptide compound containing at least one N-substituted amino acid residue, a salt thereof or a solvate thereof by a solid phase process.
[42] A method for suppressing generation of impurities as compared to elongation with amino acid residues one by one, wherein a peptide is supported on a solid phase synthesis resin before a first elongation reaction in production of a peptide compound containing at least one N-substituted amino acid residue, a salt thereof or a solvate thereof by a solid phase process.
[43] A method for suppressing premature cleavage as compared to elongation with amino acid residues one by one, wherein a peptide is supported on a solid phase synthesis resin before a first elongation reaction in production of a peptide compound containing at least one N-substituted amino acid residue, a salt thereof or a solvate thereof by a solid phase process.

Advantageous Effects of Invention

The present invention provides a useful method which can adapt to production of a peptide compound containing any number and type of amino acid residues and which is capable of producing the peptide compound with a high yield and a high purity. The present invention enables improvement of the yield by suppression of premature cleavage and improvement of the purity by avoidance of production of an excessively elongated form as a by-product, and accordingly, the efficiency of purification of an intended peptide compound is dramatically improved, resulting in significant improvement of the productivity of the method for solid phase synthesis of a peptide.

Description of Embodiments

(Abbreviations)

The abbreviations as used herein are listed below.

AA: ammonium acetate
Al: allyl
Alloc: allyloxycarbonyl
Boc: t-butoxycarbonyl
Cbz: benzyloxycarbonyl
COMU:(1-cyano-2-ethoxy-2-oxoethylideneaminooxy)dimethylamino-morpholino-carbenium hexafluorophosphate
DBU: 1,8-diazabicyclo[5.4.0]-7-undecene
DCM: dichloromethane
DIC: N,N'-diisopropylcarbodiimide
DIPEA: N,N-diisopropylethylamine
DMF: N,N-dimethylformamide
DMSO: dimethylsulfoxide
EDCI: 1-ethyl-3-(3-dimethylaminopropyl)carbodiimide
FA: formic acid
Fmoc: 9-fluorenylmethyloxycarbonyl

NMP: N-methyl-2-pyrrolidone
HATU: O-(7-aza-1H-benzotriazol-1-yl)-N,N,N',N'-tetramethyluronium hexafluorophosphate
HBTU: O-(1H-benzotriazol-1-yl)-N,N,N',N'-tetramethyluronium hexafluorophosphate
HFIP: 1,1,1,3,3,3-hexafluoroisopropyl alcohol
HOAt: 1-hydroxy-7-azabenzotriazole
HOBt: 1-hydroxybenzotriazole
oxyma: ethyl cyano(hydroxyimino)acetate
TBME: t-butyl methyl ether
Teoc: 2-(trimethylsilyl)ethoxycarbonyl
TFA: trifluoroacetic acid
TFE: 2,2,2-trifluoroethanol
THF: tetrahydrofuran
Trt: triphenylmethyl

Relationships between the abbreviations of amino acids as used herein and the structures thereof are shown below. The amino acids listed in the table below have the amino group protected with a Fmoc group, and relationships between the abbreviations of the amino acids having a free amino group resulting from removal of the Fmoc group and the structures thereof can also be known from the table below. Specifically, for example, it is obvious to those skilled in the art that MeAsp-pip is an amino acid having the following structure in which the Fmoc group is removed from Fmoc-MeAsp-pip, and the structure of MeAsp-pip as an amino acid residue thereof is also obvious to those skilled in the art.

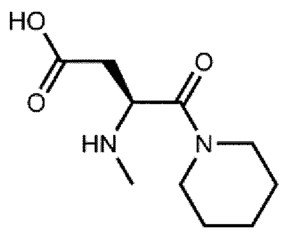

[Table A]

| Abbreviation of Fmoc amino acid | Structure of FmocOH amino acid |
|---|---|
| Fmoc-MeSer(tBuOH)-OH | 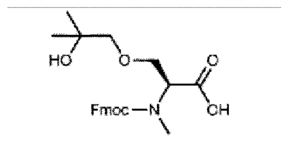 |
| Fmoc-MeGly-OH | 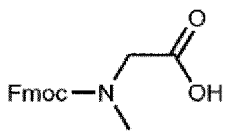 |
| Fmoc-MePhe-OH | 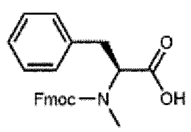 |
| Fmoc-MePhe(3-F)-OH | 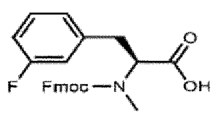 |

(continued)

| Abbreviation of Fmoc amino acid | Structure of FmocOH amino acid |
|---|---|
| Fmoc-MePhe(4-F)-OH | 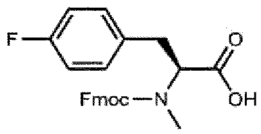 |
| Fmoc-D-MePhe-OH | 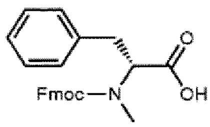 |
| Fmoc-MeVal-OH | 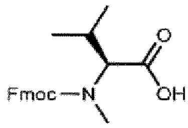 |
| Fmoc-D-MeVal-OH | 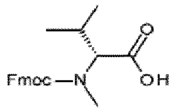 |
| Fmoc-Pro-OH | 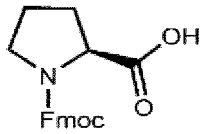 |
| Fmoc-Aib-OH | 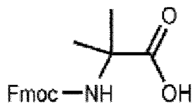 |
| Fmoc-Ala-OH | 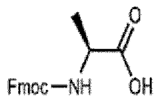 |
| Fmoc-Gly-OH | 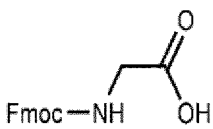 |
| Fmoc-Tyr(tBu)-OH | 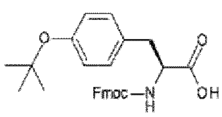 |
| Fmoc-bAla-OH | 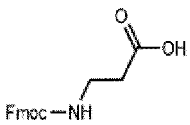 |

(continued)

| Abbreviation of Fmoc amino acid | Structure of FmocOH amino acid |
|---|---|
| Fmoc-bMeAla-OH | 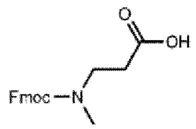 |
| Fmoc-2-ACHxC-OH | 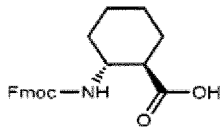 |
| Fmoc-2-ACPnC-OH | 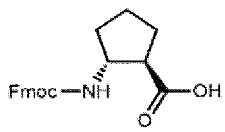 |
| Fmoc-3-CF3-bAla-OH | 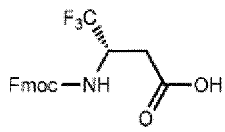 |
| Fmoc-Asp-mor | 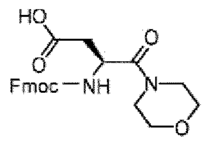 |
| Fmoc-Asp-mor(26-bicyc) | 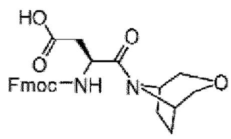 |
| Fmoc-Asp-mor(SO2) | 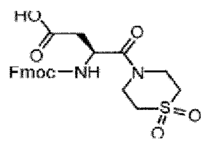 |
| Fmoc-Asp-NMe2 | 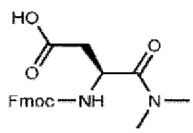 |
| Fmoc-Asp-oxz | 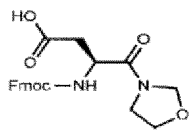 |
| Fmoc-Asp-pip | 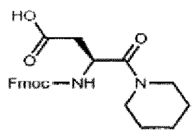 |

(continued)

| Abbreviation of Fmoc amino acid | Structure of FmocOH amino acid |
|---|---|
| Fmoc-Asp-pip(345-F6) | 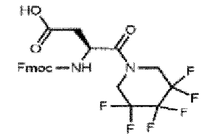 |
| Fmoc-Asp-pip(4-Me) | 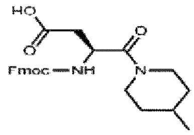 |
| Fmoc-Asp-pip-tBu | 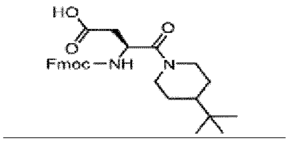 |
| Fmoc-Asp-piz(oxe) | 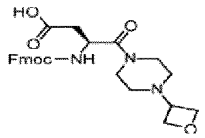 |
| Fmoc-Asp-pyrro | 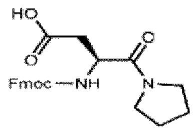 |
| Fmoc-Asp-pyrro(34-F4) | 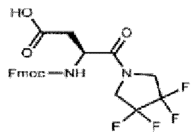 |
| Fmoc-Asp-pyrro(3-Me2) | 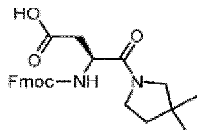 |
| Fmoc-D-(Propargyl)Gly-(C#CH2)-OH | 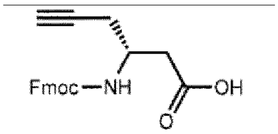 |
| Fmoc-D-3-Abu-OH | 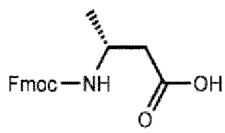 |
| Fmoc-D-3-MeAbu-OH | 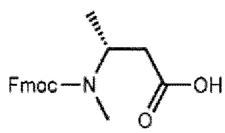 |

(continued)

| Abbreviation of Fmoc amino acid | Structure of FmocOH amino acid |
|---|---|
| Fmoc-D-Gly(Allyl)-(C#CH2)-OH | 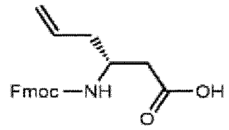 |
| Fmoc-D-Hph-(C#CH2)-OH | 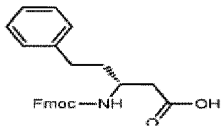 |
| Fmoc-D-Leu-(C#CH2)-OH | 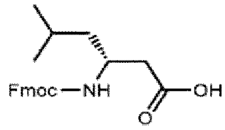 |
| Fmoc-D-MeAsp-pyrro | 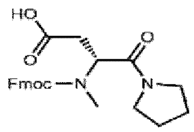 |
| Fmoc-D-MeLeu-(C#CH2)-OH | 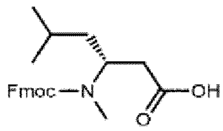 |
| Fmoc-D-Pic(2)-(C#CH2)-OH | 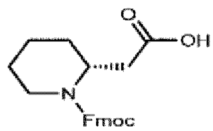 |
| Fmoc-D-Pro-(C#CH2)-OH | 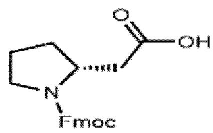 |
| Fmoc-D-Ser(iPen)-(C#CH2)-OH | 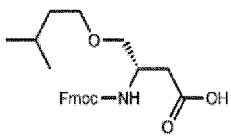 |
| Fmoc-D-Ser(NtBu-Aca)-(C#CH2)-OH | 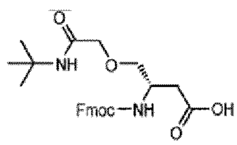 |
| Fmoc-EtAsp-pip | 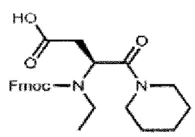 |

(continued)

| Abbreviation of Fmoc amino acid | Structure of FmocOH amino acid |
|---|---|
| Fmoc-MeAsp-aze | 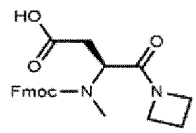 |
| Fmoc-MeAsp-mor | 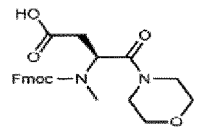 |
| Fmoc-MeAsp-mor(26-bicyc) | 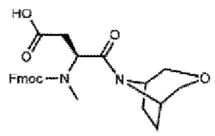 |
| Fmoc-MeAsp-mor(SO2) | 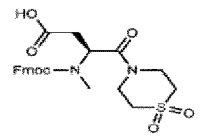 |
| Fmoc-MeAsp-NMe2 | 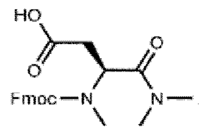 |
| Fmoc-MeAsp-oxz | 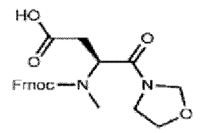 |
| Fmoc-MeAsp-pip | 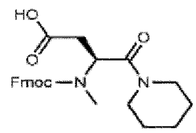 |
| Fmoc-MeAsp-pip(345-F6) | 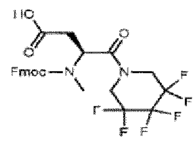 |
| Fmoc-MeAsp-pip(3-F2) | 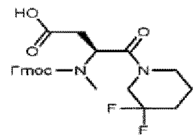 |
| Fmoc-MeAsp-pip(4-F2) | 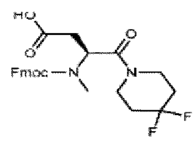 |

(continued)

| Abbreviation of Fmoc amino acid | Structure of FmocOH amino acid |
|---|---|
| Fmoc-MeAsp-pip(4-Me) | 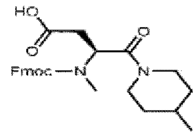 |
| Fmoc-MeAsp-piz(oxe) | 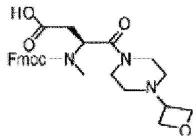 |
| Fmoc-MeAsp-pyrro | 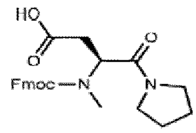 |
| Fmoc-MeAsp-pyrro(34-F4) | 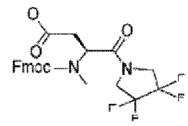 |
| Fmoc-MeAsp-pyrro(3-Me2) | 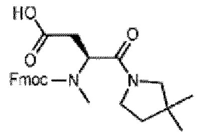 |
| Fmoc-nPrAsp-pip | 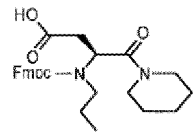 |
| Fmoc-D-MeAsp-NMe2 | 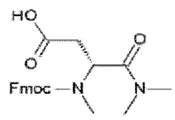 |
| Fmoc-MeAla-OH | 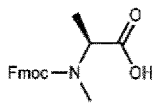 |
| Fmoc-MeLeu-OH | 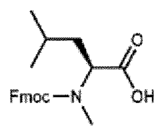 |
| Fmoc-MeCha-OH | 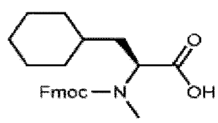 |

(continued)

| Abbreviation of Fmoc amino acid | Structure of FmocOH amino acid |
|---|---|
| Fmoc-MeAla(cPent)-OH | 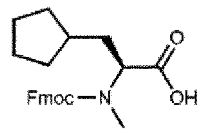 |
| Fmoc-MeAla(cBu)-OH | 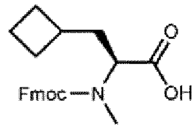 |
| Fmoc-MeAla(cPr)-OH | 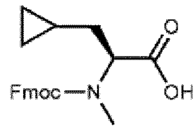 |
| Fmoc-MeChg-OH | 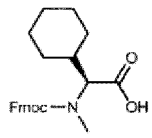 |
| Fmoc-MeGly(cPent)-OH | 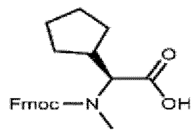 |
| Fmoc-MeGly(cBu)-OH | 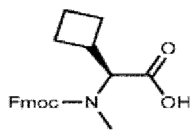 |
| Fmoc-MeGly(cPr)-OH | 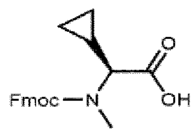 |
| Fmoc-MeAbu-OH | 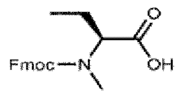 |
| Fmoc-MeNva-OH | 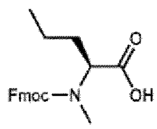 |
| Fmoc-MeN!e-OH | 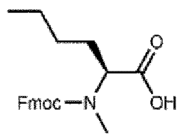 |
| Fmoc-Val-OH | 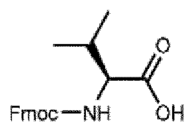 |

(continued)

| Abbreviation of Fmoc amino acid | Structure of FmocOH amino acid |
|---|---|
| Fmoc-Leu-OH | 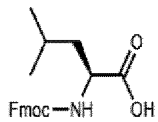 |
| Fmoc-MeNva(5-F2)-OH | 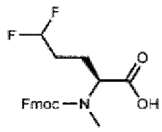 |
| Fmoc-MeHle-OH | 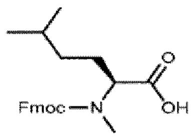 |
| Fmoc-Melle-OH | 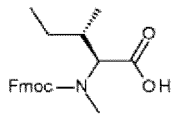 |
| Fmoc-MeSer(nPr)-OH | 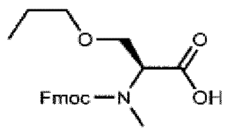 |
| Fmoc-MeSer(cPr)-OH | 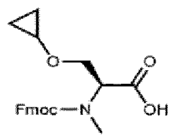 |
| Fmoc-MeHnl-OH | 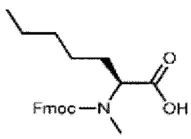 |
| Fmoc-MeHnl(7-F2)-OH | 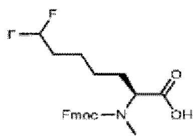 |
| Fmoc-MePRA-OH | 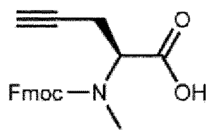 |
| Fmoc-MeSer(Me)-OH | 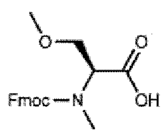 |

(continued)

| Abbreviation of Fmoc amino acid | Structure of FmocOH amino acid |
|---|---|
| Fmoc-MeThr-OH | 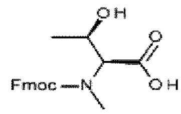 |
| Fmoc-MeSer(cBu)-OH | 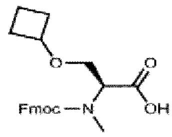 |
| Fmoc-MeSer(Tfe)-OH | 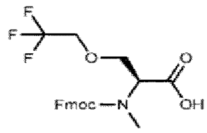 |
| Fmoc-MeThr(Me)-OH | 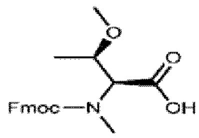 |
| Fmoc-MeHse(Me)-OH | 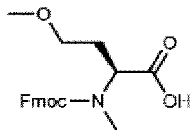 |
| Fmoc-MeMet(O2)-OH | 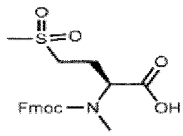 |
| Fmoc-Ile-OH | 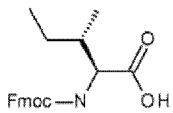 |
| Fmoc-Nle-OH | 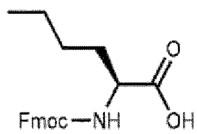 |
| Fmoc-Chg-OH | 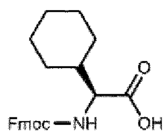 |
| Fmoc-Ala(cBu)-OH | 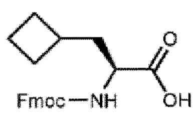 |

(continued)

| Abbreviation of Fmoc amino acid | Structure of FmocOH amino acid |
|---|---|
| Fmoc-Gly(cFent)-OH | 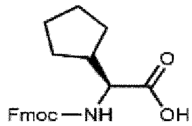 |
| Fmoc-Hle-OH | 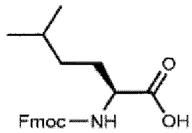 |
| Fmoc-Nva-OH | 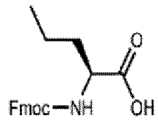 |
| Fmoc-Phe-OH | 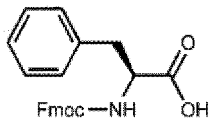 |
| Fmoc-Hph-OH | 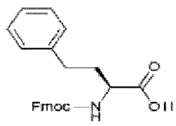 |
| Fmoc-Glu-mor | 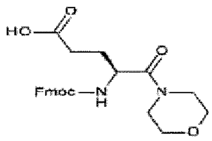 |
| Fmoc-Glu-pip | 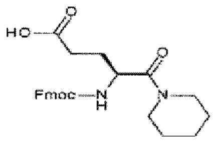 |
| Fmoc-MeGlu-pip | 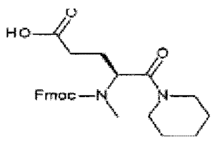 |
| Fmoc-Glu-NMe2 | 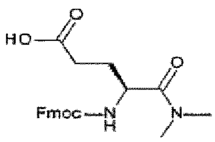 |
| Fmoc-MeGlu-NMe2 | 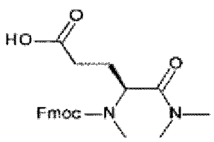 |

(continued)

| Abbreviation of Fmoc amino acid | Structure of FmocOH amino acid |
|---|---|
| Fmoc-MeCys(AcOH)-NMe2 | 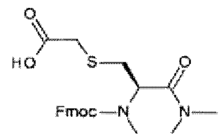 |
| Fmoc-bAla(2-Me2)-OH | 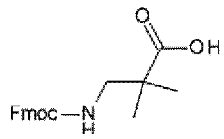 |
| Fmoc-Lys(Boc)-OH | 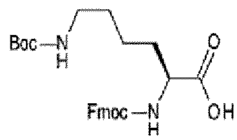 |
| Fmoc-Asn(Trt)-OH | 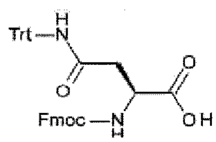 |
| Fmoc-Ser(tBu)-OH | 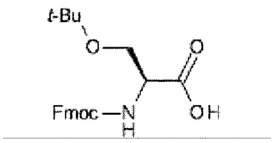 |

(Definition of functional group)

As the "halogen atom" herein, F, Cl, Br and I are exemplified.

As used herein, the "alkyl" is a monovalent group derived from an aliphatic hydrocarbon by removing any one hydrogen atom, and a subset of hydrocarbyl or hydrocarbon group structures which do not contain a hetero atom (which is an atom other than carbon and hydrogen atoms) or an unsaturated carbon-carbon bond and contain hydrogen and carbon atoms in the backbone. The alkyl includes not only a linear form but a branched form. The alkyl is specifically alkyl having 1 to 20 carbon atoms ($C_1$-$C_{20}$; hereinafter, "$C_p$-$C_q$" means that the number of carbon atoms is p to q), preferably $C_1$-$C_{10}$ alkyl, more preferably $C_1$-$C_6$ alkyl. Examples of the alkyl specifically include methyl, ethyl, n-propyl, i-propyl, n-butyl, s-butyl, t-butyl, isobutyl (2-methylpropyl), n-pentyl, s-pentyl (1-methylbutyl), t-pentyl (1,1-dimethylpropyl), neopentyl (2,2-dimethylpropyl), isopentyl (3-methylbutyl), 3-pentyl (1-ethylpropyl), 1,2-dimethylpropyl, 2-methylbutyl, n-hexyl, 1,1,2-trimethylpropyl, 1,2,2-trimethylpropyl, 1,1,2,2-tetramethylpropyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, and 2-ethylbutyl.

As used herein, the "alkenyl" is a monovalent group having at least one double bond (two adjacent $SP^2$ carbon atoms). Depending on the conformation of the double bond and a substituent (if present), the geometric morphology of the double bond can assume entgegen (E) or zusammen (Z) and cis or trans conformations. The alkenyl includes not only a linear form but a branched form. The alkenyl is preferably $C_2$-$C_{10}$ alkenyl, more preferably $C_2$-$C_6$ alkenyl. Examples thereof specifically include vinyl, allyl, 1-propenyl, 2-propenyl, 1-butenyl, 2-butenyl (which includes cis and trans), 3-butenyl, pentenyl, 3-methyl-2-butenyl, and hexenyl.

As used herein, the "alkynyl" is a monovalent group having at least one triple bond (two adjacent SP carbon atoms). The alkynyl includes not only a linear form but a branched form. The alkynyl is preferably $C_2$-$C_{10}$ alkynyl, more preferably $C_2$-$C_6$ alkynyl. Examples thereof specifically include ethynyl, 1-propynyl, propargyl, 3-butynyl, pentynyl, hexynyl, 3-phenyl-2-propynyl, 3-(2'-fluorophenyl)-2-propynyl, 2-hydroxy-2-propynyl, 3-(3-fluorophenyl)-2-propynyl, and 3-methyl-(5-phenyl)-4-pentynyl.

As used herein, the term "cycloalkyl" means a saturated or partially saturated cyclic monovalent aliphatic hydrocarbon group and includes a monocyclic ring, a bicyclo ring, and a spiro ring. The cycloalkyl is preferably $C_3$-$C_8$ cycloalkyl. Examples thereof specifically include cyclopropyl, cyclobutyl, cyclopentyl, cyclohexyl, cycloheptyl, cyclooctyl,

bicyclo[2.2.1]heptyl, and spiro[3.3]heptyl.

As used herein, the term "aryl" means a monovalent aromatic hydrocarbon ring and is preferably $C_6$-$C_{10}$ aryl. Examples of the aryl specifically include phenyl and naphthyl (e.g., 1-naphthyl and 2-naphthyl).

As used herein, the term "heterocyclyl" means a nonaromatic cyclic monovalent group containing a carbon atom as well as 1 to 5 heteroatoms. The heterocyclyl may have a double and/or triple bond in the ring. A carbon atom in the ring may form carbonyl through oxidation, and the ring may be a monocyclic ring or a condensed ring. The number of atoms constituting the ring is preferably 4 to 10 (4- to 10-membered heterocyclyl), more preferably 4 to 7 (4- to 7-membered heterocyclyl). Examples of the heterocyclyl specifically include azetidinyl, oxiranyl, oxetanyl, azetidinyl, dihydrofuryl, tetrahydrofuryl, dihydropyranyl, tetrahydropyranyl, tetrahydropyridyl, tetrahydropyrimidyl, morpholinyl, thiomorpholinyl, pyrrolidinyl, piperidinyl, piperazinyl, pyrazolidinyl, imidazolinyl, imidazolidinyl, oxazolidinyl, isoxazolidinyl, thiazolidinyl, isothiazolidinyl, 1,2-thiazinane, thiadiazolidinyl, oxazolidonyl, benzodioxanyl, benzoxazolyl, dioxolanyl, dioxanyl, tetrahydropyrrolo[1,2-c]imidazole, thietanyl, 3,6-diazabicyclo[3.1.1]heptanyl, 2,5-diazabicyclo[2.2.1]heptanyl, 3-oxa-8-azabicyclo[3.2.1]octanyl, sultam, and 2-oxaspiro[3.3]heptyl.

As used herein, the term "protected heterocyclyl" means a group in which one or more functional groups, for example, an amino group, present in the "heterocyclyl" defined above, is protected with an arbitrary protective group, and is preferably protected 4- to 7-membered heterocyclyl. Examples of the protective group specifically include Boc, Fmoc, Cbz, Troc, and Alloc. Examples of the protected heterocyclyl specifically include Boc-protected azetidine.

As used herein, the term "heterocycloalkylidene" means a divalent group that results from the removal of two hydrogen atoms from one carbon atom of the "heterocyclyl" defined above and has a free valence that constitutes a portion of a double bond. The heterocycloalkylidene is preferably 4- to 7-membered heterocycloalkylidene. Examples thereof specifically include tetrahydropyran-4-ylidene and azetidin-3-ylidene.

As used herein, the term "protected heterocycloalkylidene" means a group in which one or more functional groups, for example, an amino group, present in the "heterocycloalkylidene" defined above, is protected with an arbitrary protective group, and is preferably protected 4- to 7-membered heterocycloalkylidene. Examples of the protective group specifically include Boc, Fmoc, Cbz, Troc, and Alloc. Examples of the protected heterocyclyl specifically include Boc-protected azetidin-3-ylidene.

As used herein, the term "heteroaryl" means an aromatic cyclic monovalent group containing a carbon atom as well as 1 to 5 heteroatoms. The ring may be a monocyclic ring or a condensed ring with another ring and may be partially saturated. The number of atoms forming the ring is preferably 5 to 10 (5-to 10-membered heteroaryl), more preferably 5 to 7 (5- to 7-membered heteroaryl). Examples of the heteroaryl specifically include furyl, thienyl, pyrrolyl, imidazolyl, pyrazolyl, thiazolyl, isothiazolyl, oxazolyl, isoxazolyl, oxadiazolyl, thiadiazolyl, triazolyl, tetrazolyl, pyridyl, pyrimidyl, pyridazinyl, pyrazinyl, triazinyl, benzofuranyl, benzothienyl, benzothiadiazolyl, benzothiazolyl, benzoxazolyl, benzoxadiazolyl, benzimidazolyl, indolyl, isoindolyl, indazolyl, quinolyl, isoquinolyl, cinnolinyl, quinazolinyl, quinoxalinyl, benzodioxolyl, indolizinyl, and imidazopyridyl.

As used herein, the term "alkoxy" means an oxy group bonded to the "alkyl" defined above and is preferably $C_1$-$C_6$ alkoxy. Examples of the alkoxy specifically include methoxy, ethoxy, 1-propoxy, 2-propoxy, n-butoxy, i-butoxy, s-butoxy, t-butoxy, pentyloxy, and 3-methylbutoxy.

As used herein, the term "alkenyloxy" means an oxy group bonded to the "alkenyl" defined above and is preferably $C_2$-$C_6$ alkenyloxy. Examples of the alkenyloxy specifically include vinyloxy, allyloxy, 1-propenyloxy, 2-propenyloxy, 1-butenyloxy, 2-butenyloxy (which includes cis and trans), 3-butenyloxy, pentenyloxy, and hexenyloxy.

As used herein, the term "cycloalkoxy" means an oxy group bonded to the "cycloalkyl" defined above and is preferably $C_3$-$C_8$ cycloalkoxy. Examples of the cycloalkoxy specifically include cyclopropoxy, cyclobutoxy, and cyclopentyloxy.

As used herein, the term "aryloxy" means an oxy group bonded to the "aryl" defined above and is preferably $C_6$-$C_{10}$ aryloxy. Examples of the aryloxy specifically include phenoxy, 1-naphthyloxy, and 2-naphthyloxy.

As used herein, the term "amino" means $-NH_2$ in the narrow sense and means -NRR' in the broad sense. In this context, R and R' are each independently selected from hydrogen, alkyl, alkenyl, alkynyl, cycloalkyl, heterocyclyl, aryl, and heteroaryl, or R and R' form a ring together with the nitrogen atom bonded thereto. Examples of the amino preferably include $-NH_2$, mono-$C_1$-$C_6$ alkylamino, di-$C_1$-$C_6$ alkylamino, and 4- to 8-membered cyclic amino.

As used herein, the term "monoalkylamino" means a group of the "amino" defined above in which R is hydrogen, and R' is the "alkyl" defined above, and is preferably mono-$C_1$-$C_6$ alkylamino. Examples of the monoalkylamino specifically include methylamino, ethylamino, n-propylamino, i-propylamino, n-butylamino, s-butylamino, and t-butylamino.

As used herein, the term "dialkylamino" means a group of the "amino" defined above in which R and R' are each independently the "alkyl" defined above, and is preferably di-$C_1$-$C_6$ alkylamino. Examples of the dialkylamino specifically include dimethylamino and diethylamino.

As used herein, the term "cyclic amino" means a group of the "amino" defined above in which R and R' form a ring together with the nitrogen atom bonded thereto, and is preferably 4- to 8-membered cyclic amino. Examples of the cyclic amino specifically include 1-azetidyl, 1-pyrrolidyl, 1-piperidyl, 1-piperazyl, 4-morpholinyl, 3-oxazolidyl, 1,1-dioxidothiomorpholinyl-4-yl, and 3-oxa-8-azabicyclo[3.2.1]octan-8-yl.

As used herein, the term "protected amino" means an amino group protected with an arbitrary protective group. Examples of the protected amino specifically include amino protected with a protective group such as Boc, Fmoc, Cbz, Troc, Alloc, or Trt.

As used herein, the term "aminocarbonyl" means a carbonyl group bonded to the "amino" defined above and is preferably $-CONH_2$, mono-$C_1$-$C_6$ alkylaminocarbonyl, di-$C_1$-$C_6$ alkylaminocarbonyl, or 4- to 8-membered cyclic aminocarbonyl. Examples of the aminocarbonyl specifically include $-CONH_2$, dimethylaminocarbonyl, 1-azetidinylcarbonyl, 1-pyrrolidinylcarbonyl, 1-piperidinylcarbonyl, 1-piperazinylcarbonyl, 4-morpholinylcarbonyl, 3-oxazolidinylcarbonyl, 1,1-dioxidothiomorpholinyl-4-ylcarbonyl, and 3-oxa-8-azabicyclo[3.2.1]octane-8-ylcarbonyl.

As used herein, the term "alkenyloxycarbonyl" means a carbonyl group bonded to the "alkenyloxy" defined above and is preferably $C_2$-$C_6$ alkenyloxycarbonyl. Examples of the alkenyloxycarbonyl specifically include vinyloxycarbonyl, allyloxycarbonyl, 1-propenyloxycarbonyl, 2-propenyloxycarbonyl, 1-butenyloxycarbonyl, 2-butenyloxycarbonyl (which includes cis and trans), 3-butenyloxycarbonyl, pentenyloxycarbonyl, and hexenyloxycarbonyl.

As used herein, the term "alkylsulfonyl" means a sulfonyl group bonded to the "alkyl" defined above and is preferably $C_1$-$C_6$ alkylsulfonyl. Examples of the alkylsulfonyl specifically include methylsulfonyl.

In the present specification, the term "hydroxyalkyl" means a group in which one or more hydrogen atoms of the "alkyl" defined above are replaced with a hydroxy group, and is preferably hydroxy $C_1$-$C_6$ alkyl. Examples of the hydroxyalkyl specifically include hydroxymethyl, 1-hydroxyethyl, 2-hydroxyethyl, 2-hydroxy-2-methylpropyl, and 5-hydroxypentyl.

In the present specification, the term "haloalkyl" means a group in which one or more hydrogen atoms of the "alkyl" defined above are replaced with halogen, and is preferably halo $C_1$-$C_6$ alkyl, more preferably $C_1$-$C_6$ fluoroalkyl. Examples of the haloalkyl specifically include difluoromethyl, trifluoromethyl, 2,2-difluoroethyl, 2,2,2-trifluoroethyl, 3,3-difluoropropyl, 4,4-difluorobutyl, and 5,5-difluoropentyl.

In the present specification, the term "cyanoalkyl" means a group in which one or more hydrogen atoms of the "alkyl" defined above are replaced with cyano, and is preferably cyano-$C_1$-$C_6$ alkyl. Examples of the cyanoalkyl specifically include cyanomethyl and 2-cyanoethyl.

In the present specification, the term "aminoalkyl" means a group in which one or more hydrogen atoms of the "alkyl" defined above are replaced with the "amino" defined above, and is preferably amino $C_1$-$C_6$ alkyl. Examples of the aminoalkyl specifically include 1-pyridylmethyl, 2-(1-piperidyl)ethyl, 3-(1-piperidyl)propyl, and 4-aminobutyl.

In the present specification, the term "carboxyalkyl" means a group in which one or more hydrogen atoms of the "alkyl" defined above are replaced with carboxy, and is preferably carboxy $C_1$-$C_6$ alkyl. Examples of the carboxyalkyl specifically include carboxymethyl.

In the present specification, the term "alkenyloxycarbonylalkyl" means a group in which one or more hydrogen atoms of the "alkyl" defined above are replaced with the "alkenyloxycarbonyl" defined above, and is preferably $C_2$-$C_6$ alkenyloxycarbonyl-$C_1$-$C_6$ alkyl, more preferably $C_2$-$C_6$ alkenyloxycarbonyl-$C_1$-$C_2$ alkyl. Examples of the alkenyloxycarbonylalkyl specifically include allyloxycarbonylmethyl and 2-(allyloxycarbonyl)ethyl.

In the present specification, the term "alkoxyalkyl" means a group in which one or more hydrogen atoms of the "alkyl" defined above are replaced with the "alkoxy" defined above, and is preferably $C_1$-$C_6$ alkoxy-$C_1$-$C_6$ alkyl, more preferably $C_1$-$C_6$ alkoxy-$C_1$-$C_2$ alkyl. Examples of the alkoxyalkyl specifically include methoxymethyl, ethoxymethyl, 1-propoxymethyl, 2-propoxymethyl, n-butoxymethyl, i-butoxymethyl, s-butoxymethyl, t-butoxymethyl, pentyloxymethyl, 3-methylbutoxymethyl, 1-methoxyethyl, 2-methoxyethyl, and 2-ethoxyethyl.

In the present specification, the term "cycloalkylalkyl" means a group in which one or more hydrogen atoms of the "alkyl" defined above are replaced with the "cycloalkyl" defined above, and is preferably $C_3$-$C_8$ cycloalkyl-$C_1$-$C_6$ alkyl, more preferably $C_3$-$C_6$ cycloalkyl-$C_1$-$C_2$ alkyl. Examples of the cycloalkylalkyl specifically include cyclopropylmethyl, cyclobutylmethyl, cyclopentylmethyl, and cyclohexylmethyl.

In the present specification, the term "cycloalkoxyalkyl " means a group in which one or more hydrogen atoms of the "alkyl" defined above are replaced with the "cycloalkoxy" defined above, and is preferably $C_3$-$C_8$ cycloalkoxy-$C_1$-$C_6$ alkyl, more preferably $C_3$-$C_6$ cycloalkoxy-$C_1$-$C_2$ alkyl. Examples of the cycloalkoxyalkyl specifically include cyclopropoxymethyl and cyclobutoxymethyl.

In the present specification, the term "heterocyclylalkyl" means a group in which one or more hydrogen atoms of the "alkyl" defined above are replaced with the "heterocyclyl" defined above, and is preferably 4- to 7-membered heterocyclyl-$C_1$-$C_6$ alkyl, more preferably 4- to 7-membered heterocyclyl-$C_1$-$C_2$ alkyl. Examples of the heterocyclylalkyl specifically include 2-(tetrahydro-2H-pyran-4-yl)ethyl and 2-(azetidin-3-yl)ethyl.

In the present specification, the term "alkylsulfonylalkyl" means a group in which one or more hydrogen atoms of the "alkyl" defined above are replaced with the "alkylsulfonyl" defined above, and is preferably $C_1$-$C_6$ alkylsulfonyl-$C_1$-$C_6$ alkyl, more preferably $C_1$-$C_6$ alkylsulfonyl-$C_1$-$C_2$ alkyl. Examples of the alkylsulfonylalkyl specifically include methylsulfonylmethyl and 2-(methylsulfonyl)ethyl.

In the present specification, the term "aminocarbonylalkyl" means a group in which one or more hydrogen

atoms of the "alkyl" defined above are replaced with the "aminocarbonyl" defined above, and is preferably aminocarbonyl-$C_1$ to $C_6$ alkyl, more preferably aminocarbonyl-$C_1$-$C_4$ alkyl. Examples of the aminocarbonylalkyl specifically include methylaminocarbonylmethyl, dimethylaminocarbonylmethyl, t-butylaminocarbonylmethyl, 1-azetidinylcarbonylmethyl, 1-pyrrolidinylcarbonylmethyl, 1-piperidinylcarbonylmethyl, 4-morpholinylcarbonylmethyl, 2-(methylaminocarbonyl)ethyl, 2-(dimethylaminocarbonyl)ethyl, 2-(1-azetidinylcarbonyl)ethyl, 2-(1-pyrrolidinylcarbonyl)ethyl, 2-(4-morpholinylcarbonyl)ethyl, 3-(dimethylaminocarbonyl)propyl, and 4-(dimethylaminocarbonyl)butyl.

In the present specification, the term "aryloxyalkyl" means a group in which one or more hydrogen atoms of the "alkyl" defined above are replaced with the "aryloxy" defined above, and is preferably $C_6$-$C_{10}$ aryloxy-$C_1$-$C_6$ alkyl, more preferably $C_6$-$C_{10}$ aryloxy-$C_1$-$C_2$ alkyl. Examples of the aryloxyalkyl specifically include phenoxymethyl and 2-phenoxyethyl.

In the present specification, the term "aralkyl (arylalkyl)" means a group in which at least one hydrogen atom of the "alkyl" defined above is replaced with the "aryl" defined above, and is preferably $C_7$-$C_{14}$ aralkyl, more preferably $C_7$-$C_{10}$ aralkyl. Examples of the aralkyl specifically include benzyl, phenethyl, and 3-phenylpropyl.

In the present specification, the term "aralkoxy" means an oxy group bonded to the "aralkyl" defined above and is preferably $C_7$-$C_{14}$ aralkoxy, more preferably $C_7$-$C_{10}$ aralkoxy. Examples of the aralkoxy specifically include benzyloxy, phenethyloxy, and 3-phenylpropoxy.

In the present specification, the term "aralkoxyalkyl" means a group in which one or more hydrogen atoms of the "alkyl" defined above are replaced with the "aralkoxy" defined above, and is preferably $C_7$-$C_{14}$ alkoxy-$C_1$-$C_6$ alkyl, more preferably $C_7$-$C_{14}$ alkoxy-$C_1$-$C_2$ alkyl. Examples of the aralkoxyalkyl specifically include benzyloxymethyl and 1-(benzyloxy)ethyl.

In the present specification, the term "heteroarylalkyl" means a group in which at least one hydrogen atom of the "alkyl" defined above is replaced with the "heteroaryl" defined above, and is preferably 5- to 10-membered heteroaryl-$C_1$-$C_6$ alkyl, more preferably 5- to 10-membered heteroaryl-$C_1$-$C_2$ alkyl. Examples of the heteroarylalkyl specifically include 3-thienylmethyl, 4-thiazolylmethyl, 2-pyridylmethyl, 3-pyridylmethyl, 4-pyridylmethyl, 2-(2-pyridyl)ethyl, 2-(3-pyridyl)ethyl, 2-(4-pyridyl)ethyl, 2-(6-quinolyl)ethyl, 2-(7-quinolyl)ethyl, 2-(6-indolyl)ethyl, 2-(5-indolyl)ethyl, and 2-(5-benzofuranyl)ethyl.

In the present specification, the term "heteroarylalkoxy" means an oxy group bonded to the "heteroarylalkyl" defined above and is preferably 5- to 10-membered heteroaryl-$C_1$-$C_6$ alkoxy, more preferably 5-to 10-membered heteroaryl-$C_1$-$C_2$ alkoxy. Examples of the heteroarylalkoxy specifically include 3-thienylmethoxy and 3-pyridylmethoxy.

In the present specification, the term "heteroarylalkoxyalkyl" means a group in which one or more hydrogen atoms of the "alkyl" defined above are replaced with the "heteroarylalkoxy" defined above, and is preferably 5- to 10-membered heteroaryl-$C_1$-$C_6$ alkoxy-$C_1$-$C_6$ alkyl, more preferably 5- to 10-membered heteroaryl-$C_1$-$C_2$ alkoxy-$C_1$-$C_2$ alkyl. Examples of the heteroarylalkoxyalkyl specifically include 3-pyridylmethoxymethyl.

In the present specification, the term "heterocycloalkylidenealkyl" means a group in which one or more hydrogen atoms of the "alkyl" defined above are replaced with the "heterocycloalkylidene" defined above, and is preferably 4- to 7-membered heterocycloalkylidene-$C_1$-$C_6$ alkyl, more preferably 4- to 7-membered heterocycloalkylidene-$C_1$-$C_2$ alkyl. Examples of the heterarylalkoxyalkyl specifically include tetrahydro-4H-pyran-4-ylidenemethyl and azetidin-3-ylidenemethyl.

In the present specification, the term "alkoxyalkenyl" means a group in which one or more hydrogen atoms of the "alkenyl" defined above are replaced with the "alkoxy" defined above, and is preferably $C_1$-$C_6$ alkoxy $C_2$-$C_6$ alkenyl. Examples of the alkoxyalkenyl specifically include (E)-4-methoxybut-2-en-1-yl.

In the present specification, the term "aminocarbonylalkenyl" means a group in which one or more hydrogen atoms of the "alkenyl" defined above are replaced with the "aminocarbonyl" defined above, and is preferably aminocarbonyl-$C_2$-$C_6$ alkenyl. Examples of the aminocarbonylalkenyl specifically include (E)-3-(dimethyl aminocarbonylcarbonyl)-prop-2-en-1-yl.

In the present specification, the term "haloalkoxy" means a group in which one or more hydrogen atoms of the "alkoxy" defined above are replaced with halogen, and is preferably halo $C_1$-$C_6$ alkoxy. Examples of the haloalkoxy specifically include difluoromethoxy, trifluoromethoxy, 2,2-difluoroethoxy, and 2,2,2-trifluoroethoxy.

In the present specification, the term "alkylene" means a divalent group induced by the further removal of one arbitrary hydrogen atom from the "alkyl" described above, and is preferably $C_4$-$C_8$ alkylene. Examples of the alkylene specifically include $-CH_2-$, $-(CH_2)_2-$, $-(CH_2)_3-$, $-CH(CH_3)CH_2-$, $-C(CH_3)_2-$, $-(CH_2)_4-$, $-CH(CH_3)CH_2CH_2-$, $-C(CH_3)_2CH_2-$, $-CH_2CH(CH_3)CH_2-$, $-CH_2C(CH_3)_2-$, $-CH_2CH_2CH(CH_3)-$, $-(CH_2)_5-$, $-(CH_2)_6-$, $-(CH_2)_7-$, and $-(CH_2)_8-$.

In the present specification, the term "alicyclic ring" means a nonaromatic hydrocarbon ring. The alicyclic ring may have an unsaturated bond in the ring and may be a polycyclic ring having two or more rings. A carbon atom constituting the ring may form carbonyl through oxidation. The alicyclic ring is preferably a 3-to 8-membered alicyclic ring. Examples thereof specifically include a cyclopropane ring, a cyclobutane ring, a cyclopentane ring, a cyclohexane ring, a cycloheptane ring, a cyclooctane ring, and a bicyclo[2.2.1]heptane ring.

In the present specification, the term "saturated heterocyclic ring" means a nonaromatic heterocyclic ring containing a carbon atom as well as 1 to 5 heteroatoms without containing a double bond and/or a triple bond in the ring. The saturated heterocyclic ring may be a monocyclic ring or may form a condensed ring with another ring, for example, an aromatic ring such as a benzene ring. The saturated heterocyclic ring is preferably a 4- to 7-membered saturated heterocyclic ring. Examples thereof specifically include an azetidine ring, an oxetane ring, a tetrahydrofuran ring, a tetrahydropyran ring, a morpholine ring, a thiomorpholine ring, a pyrrolidine ring, a 4-oxopyrrolidine ring, a piperidine ring, a 4-oxopiperidine ring, a piperazine ring, a pyrazolidine ring, an imidazolidine ring, an oxazolidine ring, an isoxazolidine ring, a thiazolidine ring, an isothiazolidine ring, a thiadiazolidine ring, an oxazolidone ring, a dioxolane ring, a dioxane ring, a thietane ring, an octahydroindole ring, and an indoline ring.

The "peptide chain" in the present specification refers to a peptide chain of 1, 2, 3, 4 or more natural amino acids and/or non-natural amino acids linked through an amide bond and/or an ester bond. The peptide chain is preferably a peptide chain comprising 1 to 4 amino acid residues, more preferably a peptide chain consisting of 1 to 4 amino acid residues.

Examples of the "protective group for an amino group" in the present specification include carbamate-type protective groups, amide-type protective groups, arylsulfoneamide-type protective groups, alkylamine-type protective groups, and imide-type protective groups, and examples thereof specifically include a Fmoc group, a Boc group, an Alloc group, a Cbz group, a Teoc group, a trifluoroacetyl group, a pentafluoropropionyl group, a phthaloyl group, a benzenesulfonyl group, a tosyl group, a nosyl group, a dinitronosyl group, a t-Bu group, a trityl group, a cumyl group, a benzylidene group, a 4-methoxybenzylidene group, and a diphenylmethylidene group.

Examples of the "protective group for a carboxyl group" as used herein include alkyl ester-type protective groups, benzyl ester-type protective groups, and substituted alkyl ester-type protective groups. Examples of the protective group for a carboxyl group specifically include a methyl group, an ethyl group, a t-Bu group, a benzyl group, a trityl group, a cumyl group, a methoxytrityl group, a 2-(trimethylsilyl)ethyl group, a 2,2,2-trichloroethyl group, and an allyl group.

Examples of the "protective group for hydroxy" as used herein include alkyl ether-type protective groups, aralkyl ether-type protective groups, and silyl ether-type and carbonic acid ester-type protective groups. Examples of the protective group for hydroxy specifically include a methoxymethyl group, a benzyloxymethyl group, a tetrahydropyranyl group, a tert-butyl group, an allyl group, a 2,2,2-trichloroethyl group, a benzyl group, a 4-methoxybenzyl group, a trimethylsilyl group, a triethylsilyl group, a triisopropylsilyl group, a t-butyldimethylsilyl group, a t-butyldiphenylsilyl group, a methoxycarbonyl group, a 9-fluorenylmethoxycarbonyl group, and a 2,2,2-trichloroethoxycarbonyl group.

As used herein, the term "optionally substituted" means that a group may be substituted with an arbitrary substituent.

As used herein, the term "optionally protected" means that a group may be protected with an arbitrary protective group.

As used herein, the term "one or more" means a number of 1 or 2 or larger. When the term "one or more" is used in a context related to a substituent for a certain group, this term means a number from 1 to the maximum number of substituents accepted by the group. Examples of the term "one or more" specifically include 1, 2, 3, 4, 5, 6, 7, 8, 9, 10, and/or larger numbers.

The compound according to the present invention may be a salt thereof, preferably a chemically or pharmaceutically acceptable salt. The compound according to the present invention, or a salt thereof may be a solvate thereof, preferably a chemically or pharmaceutically acceptable solvate. Examples of the salt of the compound according to the present invention include: hydrochloride; hydrobromide; hydroiodide; phosphate; phosphonate; sulfate; sulfonate such as methanesulfonate and p-toluenesulfonate; carboxylate such as acetate, citrate, malate, tartrate, succinate, and salicylate; alkali metal salts such as sodium salt, and potassium salt; alkaline earth metal salts such as magnesium salt and calcium salt; and ammonium salts such as ammonium salt, alkylammonium salt, dialkylammonium salt, trialkylammonium salt, and tetraalkylammonium salt. These salts are produced by, for example, bringing the compound into contact with an acid or a base which can be used for production of a medicament. In the present invention, the solvate of the compound refers to a molecular group formed by the compound with a solvent, and is not particularly limited as long as it is a solvate formed by a solvent which is permitted to be taken concomitantly with administration of a drug. The solvate is a hydrate when the solvent is water. Examples of the solvate of the compound according to the present invention is preferably a hydrate, and examples of the hydrate specifically include mono- to decahydrate, preferably mono- to pentahydrate, more preferably mono- to trihydrate. The solvate of the compound according to the present invention includes not only solvates with a single solvent such as water, alcohol (e.g. methanol, ethanol, 1-propanol or 2-propanol), or dimethylformamide, but also solvates with a plurality of solvents.

In the present specification, the "amino acid" includes a natural amino acid and a non-natural amino acid. In the present specification, the "natural amino acid" refers to Gly, Ala, Ser, Thr, Val, Leu, Ile, Phe, Tyr, Trp, His, Glu, Asp, Gln, Asn, Cys, Met, Lys, Arg, or Pro. Examples of the non-natural amino acid include, but are not particularly limited to, β-amino acids, γ-amino acids, D-amino acids, N-substituted amino acids, α,α-disubstituted amino acids, amino acids having a side chain different from the natural one, and hydroxycarboxylic acid. In the present specification, the amino acid accepts an arbitrary conformation. The side chain of the amino acid can be selected without particular limitations

and is freely selected from a hydrogen atom as well as, for example, an alkyl group, an alkenyl group, an alkynyl group, an aryl group, a heteroaryl group, an aralkyl group, and a cycloalkyl group. One or two non-adjacent methylene groups in any of these groups may be substituted by an oxygen atom, a carbonyl group (-CO-), or a sulfonyl group ($-SO_2-$). A substituent may be added to each of the groups. Such a substituent is not limited and can be one or two or more substituents each independently freely selected from arbitrary substituents including a halogen atom, an O atom, a S atom, a N atom, a B atom, a Si atom, and a P atom. Specifically, examples thereof include an optionally substituted alkyl group, alkenyl group, alkynyl group, aryl group, heteroaryl group, aralkyl group, and cycloalkyl group. In one non-limiting aspect, the amino acid in the present specification may be a compound having a carboxy group and an amino group in the same molecule (even in this case, the amino acid also includes imino acids such as proline and hydroxyproline).

The term "side chain of an amino acid" in the present specification means an atomic group bonded to carbon ($\alpha$-carbon) bonded to an amino group and a carboxyl group in the case of an $\alpha$-amino acid. For example, the methyl group of Ala is a side chain of the amino acid. The atomic acid bonded to $\alpha$-carbon and/or $\beta$-carbon may form a side chain of the amino acid in the case of a $\beta$-amino acid, and the atomic group bonded to $\alpha$-carbon, $\beta$-carbon and/or $\gamma$-carbon may form a side chain of the amino acid in the case of a $\gamma$-amino acid.

The term "main chain of an amino acid" in the present specification means a branched portion formed by an amino group, $\alpha$-carbon and a carboxyl group in the case of an $\alpha$-amino acid, a branched portion formed by an amino group, $\beta$-carbon, $\alpha$-carbon and a carboxyl group in the case of a $\beta$-amino acid, and a branched portion formed by an amino group, $\gamma$-carbon, $\beta$-carbon, $\alpha$-carbon and a carboxyl group in the case of a $\gamma$-amino acid.

The terms "main chain of a peptide", the "main chain of a peptide compound" and the "main chain of a cyclic peptide compound" in the present specification each mean a structure formed by linkage of a plurality of the "amino acid main chains" through an amide bond.

The backbone amino group of the amino acid may be unsubstituted ($NH_2$ group) or may be substituted (i.e., a -NHR group wherein R represents alkyl, alkenyl, alkynyl, aryl, heteroaryl, aralkyl, or cycloalkyl optionally having a substituent, and one or two non-adjacent methylene groups in any of these groups may be substituted by an oxygen atom, a carbonyl group (-CO-), or a sulfonyl group ($-SO_2-$); and a carbon chain bonded to a N atom and a carbon atom at position $\alpha$ may form a ring, as in proline). The substituent of R is selected in the same manner as in the substituent for the amino acid side chain mentioned above. In the case of a substituted backbone amino group, the R is present in the "side chain of the amino acid" in the present specification. Such an amino acid having the substituted backbone amino group is referred to herein as the "N-substituted amino acid". In the present specification, examples of the "N-substituted amino acid" preferably include, but are not limited to, N-alkyl amino acids, N-$C_1$-$C_6$ alkyl amino acids, N-$C_1$-$C_4$ alkyl amino acids, and N-methyl amino acids. Proline is a natural amino acid, and is therefore excluded from non-natural N-substituted amino acid residues.

In the present specification, the "amino acid" forming a peptide compound includes all isotopes corresponding to each amino acid. The isotope of the "amino acid" is a form in which at least one atom is replaced at a ratio different from that in a natural amino acid with an atom identical thereto in atomic number (proton number) and different therefrom in mass number (total number of protons and neutrons). Examples of the isotope included in the "amino acid" forming a peptide compound herein include a hydrogen atom, a carbon atom, a nitrogen atom, an oxygen atom, a phosphorus atom, a sulfur atom, a fluorine atom, a chlorine atom, and the like, and they include $^{2}H$, $^{3}H$; $^{13}C$, $^{14}C$; $^{15}N$; $^{17}O$, $^{18}O$; $^{31}P$, $^{32}P$; $^{35}S$; $^{18}F$; $^{36}Cl$; and the like, respectively.

In the present specification, examples of the substituent containing a halogen atom include an alkyl group, a cycloalkyl group, an alkenyl group, an alkynyl group, an aryl group, a heteroaryl group, and an aralkyl group having halogen as a substituent and more specifically include fluoroalkyl, difluoroalkyl, and trifluoroalkyl.

Examples of the substituent containing an O atom include hydroxy (-OH), oxy (-OR), carbonyl (-C=O-R), carboxy ($-CO_2H$), oxycarbonyl(-C=O-OR), carbonyloxy (-O-C=O-R), thiocarbonyl (-C=O-SR), a carbonylthio group (-S-C=O-R), aminocarbonyl (-C=O-NHR), carbonylamino (-NH-C=O-R), oxycarbonylamino (-NH-C=O-OR), sulfonylamino ($-NH-SO_2-R$), aminosulfonyl ($-SO_2-NHR$), sulfamoylamino ($-NH-SO_2-NHR$), thiocarboxyl (-C=O-SH) and carboxylcarbonyl ($-C=O-CO_2H$) groups.

Examples of the oxy (-OR) include alkoxy, cycloalkoxy, alkenyloxy, alkynyloxy, aryloxy, heteroaryloxy, and aralkyloxy. The alkoxy is preferably $C_1$-$C_4$ alkoxy or $C_1$-$C_2$ alkoxy, particularly preferably methoxy or ethoxy.

Examples of the carbonyl (-C=O-R) include formyl (-C=O-H), alkylcarbonyl, cycloalkylcarbonyl, alkenylcarbonyl, alkynylcarbonyl, arylcarbonyl, heteroarylcarbonyl, and aralkylcarbonyl.

Examples of the oxycarbonyl (-C=O-OR) include alkyloxycarbonyl, cycloalkyloxycarbonyl, alkenyloxycarbonyl, alkynyloxycarbonyl, aryloxycarbonyl, heteroaryloxycarbonyl, and aralkyloxycarbonyl.

Examples of the carbonyloxy (-O-C=O-R) include alkylcarbonyloxy, cycloalkylcarbonyloxy, alkenylcarbonyloxy, alkynylcarbonyloxy, arylcarbonyloxy, heteroarylcarbonyloxy, and aralkylcarbonyloxy.

Examples of the thiocarbonyl (-C=O-SR) include alkylthiocarbonyl, cycloalkylthiocarbonyl, alkenylthiocarbonyl, alkynylthiocarbonyl, arylthiocarbonyl, heteroarylthiocarbonyl, and aralkylthiocarbonyl.

Examples of the carbonylthio (-S-C=O-R) include alkylcarbonylthio, cycloalkylcarbonylthio, alkenylcarbonylthio, alkynylcarbonylthio, arylcarbonylthio, heteroarylcarbonylthio, and aralkylcarbonylthio.

Examples of the aminocarbonyl (-C=O-NHR) include alkylaminocarbonyl (e.g. $C_1$-$C_6$ or $C_1$-$C_4$ alkylaminocarbonyl, particularly, ethylaminocarbonyl and methylaminocarbonyl), cycloalkylaminocarbonyl, alkenylaminocarbonyl, alkynylaminocarbonyl, arylaminocarbonyl, heteroarylaminocarbonyl, and aralkylaminocarbonyl. Examples thereof additionally include groups in which the H atom bonded to the N atom in -C=O-NHR is further replaced with alkyl, cycloalkyl, alkenyl, alkynyl, aryl, heteroaryl, or aralkyl.

Examples of the carbonylamino (-NH-C=O-R) include alkylcarbonylamino, cycloalkylcarbonylamino, alkenylcarbonylamino, alkynylcarbonylamino, arylcarbonylamino, heteroarylcarbonylamino, and aralkylcarbonylamino. Examples thereof additionally include groups in which the H atom bonded to the N atom in -NH-C=O-R is further replaced with alkyl, cycloalkyl, alkenyl, alkynyl, aryl, heteroaryl, or aralkyl.

Examples of the oxycarbonylamino (-NH-C=O-OR) include alkoxycarbonylamino, cycloalkoxycarbonylamino, alkenyloxycarbonylamino, alkynyloxycarbonylamino, aryloxycarbonylamino, heteroaryloxycarbonylamino, and aralkyloxycarbonylamino. Examples thereof additionally include groups in which the H atom bonded to the N atom in -NH-C=O-OR is further replaced with alkyl, cycloalkyl, alkenyl, alkynyl, aryl, heteroaryl, or aralkyl.

Examples of the sulfonylamino (-NH-$SO_2$-R) include alkylsulfonylamino, cycloalkylsulfonylamino, alkenylsulfonylamino, alkynylsulfonylamino, arylsulfonylamino, heteroarylsulfonylamino, and aralkylsulfonylamino. Examples thereof additionally include groups in which the H atom bonded to the N atom in -NH-$SO_2$-R is further replaced with alkyl, cycloalkyl, alkenyl, alkynyl, aryl, heteroaryl, or aralkyl.

Examples of the aminosulfonyl (-$SO_2$-NHR) include alkylaminosulfonyl, cycloalkylaminosulfonyl, alkenylaminosulfonyl, alkynylaminosulfonyl, arylaminosulfonyl, heteroarylaminosulfonyl, and aralkylaminosulfonyl. Examples thereof additionally include groups in which the H atom bonded to the N atom in -$SO_2$-NHR is further replaced with alkyl, cycloalkyl, alkenyl, alkynyl, aryl, heteroaryl, or aralkyl.

Examples of the sulfamoylamino (-NH-$SO_2$-NHR) include alkylsulfamoylamino, cycloalkylsulfamoylamino, alkenyl sulfamoyl amino, alkynyl sulfamoyl amino, arylsulfamoylamino, heteroaryl sulfamoyl amino, and aralkylsulfamoylamino. The two H atoms bonded to the N atoms in -NH-$SO_2$-NHR may be substituted by substituents each independently selected from the group consisting of alkyl, cycloalkyl, alkenyl, alkynyl, aryl, heteroaryl, and aralkyl, and these two substituents may form a ring.

Examples of the substituent containing a S atom include groups such as thiol (-SH), thio (-S-R), sulfinyl (-S=O-R), sulfonyl (-$SO_2$-R), and sulfo(-$SO_3$H).

Examples of the thio (-S-R) that can be selected include alkylthio, cycloalkylthio, alkenylthio, alkynylthio, arylthio, heteroarylthio, and aralkylthio.

Examples of the sulfonyl (-$SO_2$-R) include alkylsulfonyl, cycloalkylsulfonyl, alkenylsulfonyl, alkynylsulfonyl, arylsulfonyl, heteroarylsulfonyl, and aralkylsulfonyl.

Examples of the substituent containing a N atom include groups such as azide (-$N_3$; also referred to as an "azide group"), cyano (-CN), primary amino (-$NH_2$), secondary amino (-NH-R; also referred to as mono-substituted amino), tertiary amino (-NR(R'); also referred to as di-substituted amino), amidino (-C(=NH)-$NH_2$), substituted amidino (-C(=NR)-NR'R"), guanidino (-NH-C(=NH)-$NH_2$), substituted guanidino (-NR-C(=NR‴)-NR'R"), aminocarbonylamino (-NR-CO-NR'R"), pyridyl, piperidino, morpholino, and azetidinyl.

Examples of the secondary amino (-NH-R: mono-substituted amino) include alkylamino, cycloalkylamino, alkenylamino, alkynylamino, arylamino, heteroaryl amino, and aralkylamino.

Examples of the tertiary amino (-NR(R'): di-substituted amino) include an amino group having arbitrary two substituents each independently selected from alkyl, cycloalkyl, alkenyl, alkynyl, aryl, heteroaryl, and aralkyl, for example, alkyl(aralkyl)amino. These arbitrary two substituents may form a ring. Examples thereof specifically include dialkylamino, particularly, $C_1$-$C_6$ dialkylamino, $C_1$-$C_4$ dialkylamino, dimethylamino, and diethylamino. As used herein, the "$C_p$-$C_q$ dialkylamino group" refers to a group in which an amino group is substituted by two $C_p$-$C_q$ alkyl groups. The $C_p$-$C_q$ alkyl groups may be the same or different.

Examples of the substituted amidino (-C(=NR)-NR'R") include groups in which three substituents R, R', and R" on the N atoms are each independently selected from alkyl, cycloalkyl, alkenyl, alkynyl, aryl, heteroaryl, and aralkyl, for example, alkyl(aralkyl)(aryl)amidino.

Examples of the substituted guanidino (-NR-C(=NR‴)-NR'R") include groups in which R,R', R", and R‴ are each independently selected from alkyl, cycloalkyl, alkenyl, alkynyl, aryl, heteroaryl, and aralkyl, and groups in which these substituents form a ring.

Examples of the aminocarbonylamino (-NR-CO-NR'R") include groups in which R, R', and R" are each independently selected from a hydrogen atom, alkyl, cycloalkyl, alkenyl, alkynyl, aryl, heteroaryl, and aralkyl, and groups in which these substituents form a ring.

As used herein, the "amino acid residue" forming a peptide compound is sometimes referred to simply as an "amino acid".

As used herein, the "linear peptide compound" is formed by linkage of natural amino acids and/or non-natural amino acids through an amide bond or an ester bond, and is not particularly limited as long as the compound has no cyclic portion. The total number of natural amino acids and non-natural amino acids forming a linear peptide compound may be 1, 2, 3, 4, 5, 6, 7, 8, 9, 10, 11, 12, 13, 14, 15, 20, 25 or 30, and is preferably in the range of 6 to 20, 7 to 19, 7 to 18, 7 to 17, 7 to 16, 7 to 15, 8 to 14, or 9 to 13.

As used herein, the "cyclic peptide compound" is formed by linkage of natural amino acids and/or non-natural amino acids through an amide bond or an ester bond, and is not particularly limited as long as the compound has a cyclic portion. The total number of natural amino acids and non-natural amino acids forming a cyclic peptide compound may be 1, 2, 3, 4, 5, 6, 7, 8, 9, 10, 11, 12, 13, 14, 15, 20, 25 or 30, and is preferably in the range of 6 to 20, 7 to 19, 7 to 18, 7 to 17, 7 to 16, 7 to 15, 8 to 14, or 9 to 13.

As used herein, the term "cyclic portion" of a peptide compound means a ring-like portion formed by linkage of two or more amino acid residues. As used herein, the term "linear portion" which is used when a partial structure of a cyclic peptide compound is mentioned refers to a portion that is not present in the main chain structure of the cyclic portion and that has at least one amide bond and/or ester bond on the chain of the portion.

The number of amino acids forming the cyclic portion of the cyclic peptide compound in the present specification is not limited, and is, for example, 2 or more, 3 or more, 4 or more, 5 or more, 6 or more, 7 or more, 8 or more, 9 or more, 10 or more, 11 or more, 12 or more, and 30 or less, 20 or less, 18 or less, 16 or less, 15 or less, 14 or less, 13 or less, 12 or less, 11 or less, or 7, 8, 9, 10, 11, 12, 13, 14, 15 or 16. From the viewpoint of securing both membrane permeability and metabolic stability, the number of amino acids forming the cyclic portion is preferably 2 to 30, 2 to 15, or 5 to 15, more preferably 5 to 14, 7 to 14, or 8 to 14, further preferably 8 to 13, 9 to 13, 8 to 12, 8 to 11, or 9 to 12, particularly preferably 9 to 11.

In an aspect, the number of amino acids (number of units) of the linear portion is preferably 0 to 8, more preferably 0 to 5, more preferably 0 to 3. In one non-limiting aspect, the "linear portion" in the present specification may contain natural amino acids and non-natural amino acids (including chemically modified or skeleton-transformed amino acids).

In an aspect, the molecular weight of the cyclic peptide compound in the present specification may be 500 to 2000.

The "peptide compound" in the present specification may contain a pharmaceutically acceptable salt, or a solvate thereof.

As used herein, the term "side chain" is used in the context of a side chain of an amino acid, a side chain of a cyclic portion of a cyclic peptide compound, or the like, and means a portion that is not present in the main chain structure thereof.

As used herein, the term "number of amino acids" refers to the number of amino acid residues (amino acid units) forming a peptide compound, and means the number of amino acid units generated upon cleavage of amide bonds, ester bonds and cyclized portions which link the amino acids.

As used herein, the term "elongation reaction" means a reaction in which an amino acid or a peptide is elongated with amino acids or peptides. The elongation reaction can be carried out by a solid phase synthesis method applied to an amino acid or a peptide supported on a solid phase synthesis resin, or a liquid phase synthesis method in which a solid phase synthesis resin is not used.

As used herein, the term "supported on a solid phase synthesis resin" means that an amino acid or a peptide is bound to a solid phase synthesis resin to which an amino acid or a peptide is not bound.

The meaning of the term "and/or" as used herein includes any combination in which "and" and "or" are appropriately combined. Specifically, for example, the term "A, B and/or C" includes the following seven variations:
(i) A, (ii) B, (iii) C, (iv) A and B, (v) A and C, (vi) B and C, and (vii) A, B and C.

(Production method)

In an aspect, the present invention relates to a method for producing a peptide compound containing at least one N-substituted amino acid residue, a salt thereof or a solvate thereof by a solid phase process, wherein a peptide is supported on a solid phase synthesis resin before a first elongation reaction in the solid phase process.

In an aspect, the present invention relates to a method for producing a peptide compound containing at least one N-substituted amino acid residue, a salt thereof or a solvate thereof by a solid phase process, the method comprising a step of supporting a peptide on a solid phase synthesis resin.

That is, in the present invention, a peptide such as an oligopeptide, which has been prepared by a liquid phase process or the like in advance, is supported on a solid phase synthesis resin, and the peptide is subjected to elongation of a peptide chain in a solid phase process to synthesize a peptide compound having a desired amino acid sequence. In the present specification, the peptide supported on the solid phase synthesis resin before the elongation reaction in the solid phase process is sometimes referred to as a "starting peptide".

The first elongation reaction in the present specification is preferably one in which the starting peptide is elongated with an amino acid.

Heretofore, production of a peptide compound using a solid phase process has been performed by supporting an amino acid on a solid phase synthesis resin, and elongating the amino acid in a sequential manner. In this method, however, a first residue amino acid supported on a solid phase synthesis resin may be detached from the solid phase synthesis resin (premature cleavage) in a condensation step for elongating the amino acid supported on the solid phase synthesis resin (first residue amino acid residue) with a subsequent amino acid (second residue amino acid residue). Such detachment is noticeable in the case where a solid phase synthesis resin enabling a peptide compound to be isolated under mild conditions is used. In addition, there is a possibility that prior to elongation with a second residue amino acid residue, a first residue amino acid residue is elongated with the first residue amino acid residue detached due to premature cleavage, followed by elongation with the second residue amino acid residue, resulting in generation of an excessively elongated form. Further, for some non-natural amino acid residues, an elongation reaction in a solid phase process, for example, a reaction of elongation of a first residue amino acid residue with a second residue amino acid residue does not sufficiently proceed, and there is a possibility that while the second residue amino acid residue is not linked and remains absent, a subsequent amino acid is linked. These problems are noticeable in the case where the second residue amino acid residue is a N-substituted amino acid residue having a bulky side chain. By using the method of the present invention, these defects can be avoided to produce a desired peptide compound with a high yield and a high purity.

In the present invention, a peptide containing any number and any type of amino acid residues can be used as a starting peptide. Examples of such peptides specifically include oligopeptides containing two or more amino residues, with dipeptides or tripeptides being preferable. It is preferable that the amino group of the amino acid residue at the N-terminal of the starting peptide be protected with a protective group. Such a starting peptide can be produced using a method known in the art, for example, a liquid phase method. The method for preparing a starting peptide protected at the N-terminal is not limited. Specifically, for example, the starting peptide can be produced as follows. Using a condensation agent and an amino acid residue in which an amino group is protected, an amino acid residue in which a carboxyl group is protected is subjected to a reaction of elongation with the amino acid residue, the generated peptide is then subjected to a reaction of elimination of a protective group at the N-terminal and a reaction of elongation with an amino acid in which an amino group is protected, this procedure is repeated until a desired number of residues is obtained, and a reaction of elimination of a protective group at the C-terminal is carried out in the final step. Amino acid residues to be used as raw materials for production of the starting peptide can be acquired from a commercial supplier, or produced by a known method, for example, a method described in WO 2018/225864.

In an aspect, examples of the starting peptide include those in which the amino acid residue at the C-terminal (first residue amino acid residue) thereof is a non-natural amino acid residue, and the non-natural amino acid residue is preferably a N-substituted amino acid residue such as a N-alkylamino acid. The N-alkylamino acid is preferably an N-$C_1$-$C_6$ alkylamino acid, more preferably a N-methylamino acid. Without being bound by a particular theory, premature cleavage tends to more easily occur when the first residue amino acid residue is a N-substituted amino acid residue than when the amino acid residue is a N-non-substituted amino acid residue, and this tendency is more noticeable in the case where the amino acid residue has a bulky group on a side chain thereof. Therefore, the present invention is particularly useful for production of a peptide compound having such an amino acid residue as an amino acid residue at the C-terminal.

In an aspect, the amino acid residue at the C-terminal (first residue amino acid residue) of the starting peptide is supported on the solid phase synthesis resin by a carboxyl group bonded to a carbon atom at the $\alpha$-position, a carbon atom at the $\beta$-position or a carbon atom at the $\gamma$-position on the amino group. Examples of the amino acid residue supported on the solid phase synthesis resin by a carboxyl group bonded to a carbon atom at the $\beta$-position on the amino group include aspartic acid and derivatives thereof. Specifically, support of the amino acid residue on the solid phase synthesis resin by a carboxyl group present on the side chain of aspartic acid corresponds to support of the amino acid residue by a carboxyl group bonded to a carbon atom at the $\beta$-position on the amino group. Examples of the amino acid residue supported on the solid phase synthesis resin by a carboxyl group bonded to a carbon atom at the $\gamma$-position on the amino group include glutamic acid and derivatives thereof. Specifically, support of the amino acid residue on the solid phase synthesis resin by a carboxyl group present on the side chain of glutamic acid corresponds to support of the amino acid residue by a carboxyl group bonded to a carbon atom at the $\gamma$-position on the amino group. Normally, other natural amino acid residues and N-substituted amino acid residues thereof are each supported on the solid phase synthesis resin by a carboxyl group bonded to a carbon atom at the $\alpha$-position thereon.

In an aspect, the amino acid residue at the C-terminal (first residue amino acid residue) of the starting peptide may be aspartic acid, 2-aminobutanoic acid, glycine, alanine, valine, proline, tyrosine or 2-aminoisobutyric acid, and/or a N-substituted form or a derivative thereof. When the first residue amino acid residue is the above-mentioned amino acid residue, premature cleavage easily occurs. The N-substituted form of such an amino acid residue is preferably a N-alkyl form, more preferably a N-methyl form. Examples of the derivative of such an amino acid residue include those

in which any functional group (e.g. amino group, carboxyl group or hydroxyl group) that is not involved in binding to the solid phase synthesis resin and the second residue amino acid residue is protected with any substituent (e.g. protective group). In particular, examples of the derivative of aspartic acid specifically include those a free carboxyl group that is not involved in binding to the solid phase synthesis resin and the second residue amino acid residue is aminocarbonylated. Examples of the aminocarbonylated aspartic acid specifically include dialkyl-aminocarbonylated aspartic acids such as dimethyl-aminocarbonylated aspartic acid, and aspartic acids aminocarbonylated between the aspartic acid and a N atom of a saturated heterocyclic ring containing the N atom (e.g. azetidine ring, morpholine ring, pyrrolidine ring, piperidine ring or azepane ring), and these aspartic acids may be N-substituted forms such as N-alkyl forms. When the first residue amino acid residue is aspartic acid, or a N-substituted form and/or a derivative thereof, it is preferable that the amino acid residue be supported on the solid phase synthesis resin by a carboxyl group at the β-position on the amino group. Examples of the amino acid residue in which any functional group (e.g. amino group, carboxyl group or hydroxyl group) is protected with any protective group include those an amino group on the side chain of the amino acid residue is protected with a carbamate-type protective group such as a Boc group or a Cbz group, and examples thereof specifically include those in which an amino group on the side chain of Lys is protected with a Boc group. In addition, examples the above-mentioned amino acid residue include those in which an amide group on the side chain of the amino acid residue is protected with an alkylamine-type protective group such as a t-Bu group or a Trt group, examples thereof specifically include those in which an amide group on the side chain of Asn or Gln is protected with a Trt group, and those in which a hydroxyl group on the side chain of the amino acid residue is protected with an alkyl ether-type protective group such as a t-Bu group, and examples thereof specifically include those in which a hydroxyl group on the side chain of Ser, a hydroxyl group on the side chain of Thr or a hydroxyl group on the side chain of Tyr is protected with a t-Bu group.

In an aspect, examples of the starting peptide include those in which the amino acid residue adjacent to an amino acid residue at the C-terminal thereof (second residue amino acid) is a non-natural amino acid residue. The non-natural amino acid residue is preferably a N-substituted amino acid residue such as a N-alkyl amino acid and/or an amino acid derivative, and the N-substituted amino acid residue is preferably a N-methyl form. Examples of the derivative of such an amino acid residue include those in which any functional group (e.g. amino group, carboxyl group or hydroxyl group) that is not involved in binding to the adjacent amino acid residue is protected with any substituent (e.g. protective group). Examples specifically include those an amino group on the side chain of the amino acid residue is protected with a carbamate-type protective group such as a Boc group or a Cbz group, and examples thereof more specifically include those in which an amino group on the side chain of Lys is protected with a Boc group. In addition, examples the above-mentioned amino acid residue include those in which an amide group on the side chain of the amino acid residue is protected with an alkylamine-type protective group such as a t-Bu group or a Trt group, examples thereof specifically include those in which an amide group on the side chain of Asn or Gln is protected with a Trt group, and those in which a hydroxyl group on the side chain of the amino acid residue is protected with an alkyl ether-type protective group such as a t-Bu group, and examples thereof specifically include those in which a hydroxyl group on the side chain of Ser, a hydroxyl group on the side chain of Thr or a hydroxyl group on the side chain of Tyr is protected with a t-Bu group. Without being bound by a particular theory, premature cleavage tends to more easily occur when the second residue amino acid is a N-substituted amino acid residue than when the amino acid residue is a N-non-substituted amino acid residue, and the tendency is particularly noticeable in the case where the amino acid residue has a bulky group on a side chain thereof. Therefore, the present invention is particularly useful for production of a peptide compound having such a second residue amino acid residue.

In an aspect, both the amino acid residue at the C-terminal (first residue amino acid residue) of the starting peptide and the amino acid residue adjacent to the amino acid residue at the C-terminal (second residue amino acid residue) may be natural amino acid residues, and it is preferable that one or both thereof be non-natural amino acid residue(s).

In an aspect, the amino acid residue at the C-terminal (first residue amino acid residue) of the starting peptide and/or the amino acid residue adjacent to the amino acid residue at the C-terminal (second residue amino acid residue) have a bulky side chain. Examples of the natural amino acid having a bulky side chain include amino acids having a side chain having 2 or more carbon atoms, and examples thereof include Met, Phe, Tyr, Val, Leu, Ile, Trp, Arg, His, Glu, Lys, Gln, Asp, Asn, Cys, and Thr. The amino acid residue having a bulky side chain may include an amino acid residue having a bulky protective group on the side chain. For example, Ser itself does not have a bulky side chain, and Ser(tBu) in which the side chain of Ser is protected with tBu corresponds to an amino acid residue having a bulky side chain. The amino acid residue having a optionally substituted branched-chain alkyl group on the side chain of the amino acid residue may be an amino acid residue having a bulky side chain, regardless of whether the amino acid residue is natural or non-natural. It is preferable that such a branched-chain alkyl group be bonded to a carbon atom at the α-position on the carboxyl group of the amino acid residue, and the branching position of the branched-chain alkyl group is preferably a carbon atom at the β-position or the γ-position on the carboxyl group. For example, Val is one example of the amino acid residue having a branched-chain alkyl group on the carbon atom at the α-position of the carboxyl group of the amino acid residue and having a branch on the carbon atom at the β-position of the carboxyl group, and the Val-like amino

acid residue may be an amino acid residue having a bulky side chain. Leu is one example of the amino acid residue having a branched-chain alkyl group on the carbon atom at the α-position of the carboxyl group of the amino acid and having a branch on the carbon atom at the γ-position of the carboxyl group, and the Leu-like amino acid may be an amino acid residue having a bulky side chain. The number and type of substituents that may be present in the branched-chain alkyl group are not particularly limited, and the branched-chain alkyl may have 1 to 5 substituents independently selected from the group consisting of alkenyl, alkynyl, cycloalkyl, aryl, heterocyclyl, heteroaryl, alkoxy, alkenyloxy, cycloalkoxy, aryloxy, amino, aminocarbonyl, alkenyloxycarbonyl, alkylsulfonyl, hydroxy, halogen, cyano, carboxy, alkenyloxycarbonyl, aralkoxy, heteroarylalkoxy, alkoxyalkenyl, aminocarbonylaklenyl, and haloalkoxy. Without being bound by a particular theory, premature cleavage easily occurs when the first residue amino acid residue and/or the second residue amino acid residue have a bulky side chain, and therefore the present invention is particularly useful for production of a peptide compound having such an amino acid.

In an aspect, the amino acid residue at the C-terminal (first residue amino acid residue) of the starting peptide may be represented by the following formula (A).

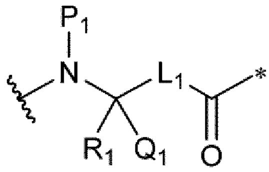
(A)

In formula (A), $L_1$ is a single bond, or $-CHM_1-$, $-CH_2CHM_1-$, $-CHM_1CH_2-$, $-(CH_2)_nS(CH_2)_m-$, $-(CH_2)_nSO(CH_2)_m-$, or $-(CH_2)_nSO_2(CH_2)_m-$, where n and m are each independently 1 or 2.

When $L_1$ is $-(CH_2)_nS(CH_2)_m-$, examples of the $-(CH_2)_nS(CH_2)_m-$ specifically include $-CH_2SCH_2-$, $-CH_2CH_2SCH_2-$, $-CH_2SCH_2CH_2-$, and $-CH_2CH_2SCH_2CH_2-$.

When $L_1$ is $-(CH_2)_nS(O)(CH_2)_m-$, examples of the $-(CH_2)_nS(O)(CH_2)_m-$ specifically include $-CH_2S(O)CH_2-$, $-CH_2CH_2S(O)CH_2-$, $-CH_2S(O)CH_2CH_2-$, and $-CH_2CH_2S(O)CH_2CH_2-$.

When $L_1$ is $-(CH_2)_nS(O)_2(CH_2)_m-$, examples of the $-(CH_2)_nS(O)_2(CH_2)_m-$ specifically include $-CH_2S(O)_2CH_2-$, $-CH_2CH_2S(O)_2CH_2-$, $-CH_2S(O)_2CH_2CH_2-$, and $-CH_2CH_2S(O)_2CH_2CH_2-$.

In formula (A), $R_1$ is hydrogen, $C_1$-$C_6$ alkyl, $C_2$-$C_6$ alkenyl, $C_2$-$C_6$ alkynyl, $C_1$-$C_6$ alkoxy $C_1$-$C_6$ alkyl, $C_7$-$C_{14}$ aralkyl, or aminocarbonyl (the amino is $-NH_2$, mono-$C_1$-$C_6$ alkylamino, di-$C_1$-$C_6$ alkylamino, or 4- to 8-membered cyclic amino), which is optionally substituted with one or more groups independently selected from the group consisting of halogen, oxo, hydroxy, $C_1$-$C_6$ alkyl, 4- to 7-membered heterocyclyl, aminocarbonyl (the amino is $-NH_2$, mono-$C_1$-$C_6$ alkylamino, di-$C_1$-$C_6$ alkylamino, or 4- to 8-membered cyclic amino), $C_1$-$C_6$ alkylsulfonyl, and $C_1$-$C_6$ alkoxy $C_1$-$C_6$ alkyl, or $R_1$ is a peptide chain containing one to four amino acid residues. When $R_1$ is a peptide chain containing 1 to 4 amino acid residues, the 1 to 4 amino acid residues forming the peptide chain may be natural amino acid residues, or non-natural amino acid residues, and may be the same, or different.

When $L_1$ is a single bond, $R_1$ is preferably hydrogen, $C_1$-$C_6$ alkyl, $C_1$-$C_6$ alkoxy $C_1$-$C_6$ alkyl optionally substituted with hydroxy, or $C_7$-$C_{14}$ aralkyl optionally substituted with one or more halogens or $C_1$-$C_6$ alkoxy $C_1$-$C_6$ alkyl.

When $L_1$ is a single bond, $R_1$ is more preferably hydrogen, methyl, isopropyl, $C_1$-$C_6$ alkoxy $C_1$-$C_2$ alkyl optionally substituted with hydroxy, fluorine, or benzyl optionally substituted with t-butoxy, and examples thereof specifically include hydrogen, (2-hydroxy-2-methyl-propyloxy)methyl, benzyl, 3-fluorobenzyl, and 4-fluorobenzyl.

When $L_1$ is $-CHM_1-$, $-CH_2CHM_1-$ or $-CHM_1CH_2-$, $R_1$ is preferably hydrogen, $C_1$-$C_6$ alkyl, halo $C_1$-$C_6$ alkyl, $C_2$-$C_6$ alkenyl, $C_2$-$C_6$ alkynyl, $C_1$-$C_6$ alkoxy $C_1$-$C_6$ alkyl (the $C_1$-$C_6$ alkoxy $C_1$-$C_6$ alkyl is optionally substituted with hydroxy, or aminocarbonyl (the amino is $-NH_2$, mono-$C_1$-$C_6$ alkylamino, di-$C_1$-$C_6$ alkylamino, or 4- to 8-membered cyclic amino)), $C_7$-$C_{14}$ aralkyl optionally substituted with one or more halogens, or aminocarbonyl (the amino is $-NH_2$, mono-$C_1$-$C_6$ alkylamino, di-$C_1$-$C_6$ alkylamino, or 4- to 8-membered cyclic amino, and the cyclic amino is optionally substituted with one or more halogens, one or more oxos, one or more $C_1$-$C_6$ alkyls, or 4- to 7-membered heterocyclyl).

When $L_1$ is $-CHM_1-$, $-CH_2CHM_1-$ or $-CHM_1CH_2-$, $R_1$ is more preferably hydrogen, $C_1$-$C_6$ alkyl, $C_1$-$C_6$ fluoroalkyl, $C_2$-$C_3$ alkenyl, $C_2$-$C_3$ alkynyl, $C_1$-$C_6$ alkoxy $C_1$-$C_2$ alkyl optionally substituted with mono-$C_1$-$C_4$ alkylaminocarbonyl, dimethylaminocarbonyl; 4- to 8-membered aminocarbonyl optionally substituted with one or more fluorine atoms, $C_1$-$C_4$ alkyls, or 4- to 7-membered heterocyclyls; or benzyl, or phenethyl.

When $L_1$ is $-CHM_1-$, $-CH_2CHM_1-$ or $-CHM_1CH_2-$, examples of $R_1$ specifically include hydrogen, methyl, isobutyl, trifluoromethyl, allyl, prop-2-yn-1-yl, (isopentyloxy)methyl, {2-(t-butylamino)-2-oxoethoxy}methyl, dimethylaminocarbonyl, azetidinylcarbonyl, pyrrolidinylcarbonyl, 3,3-dimethylpyrrolidinylcarbonyl, 3,3,4,4-tetrafluoropyrrolidinylcarbonyl, 4-methylpyrrolidinylcarbonyl, 4-(t-butyl)-piperidinylcarbonyl, 3,3,4,4,5,5-hexafluoropiperidinylcarbonyl, 3,3-difluoropiperidinylcarbonyl, 4,4-difluoropiperidinylcarbonyl, piperidinylcarbonyl, morpholinocarbonyl, oxazolidin-3-ylcarbonyl, 3-oxa-8-azabicyclo[3.2.1]octan-8-ylcarbonyl, 1,1-dioxidemorpholinylcarbonyl, 1-(oxetan-3-yl)-piperazin-4-ylcarbonyl, and

phenethyl.

When $L_1$ is $-(CH_2)_nS(CH_2)_m-$, $-(CH_2)_nS(O)(CH_2)_m$ or $-(CH_2)_nS(O)_2(CH_2)_m-$, $R_1$ is preferably aminocarbonyl (the amino is $-NH_2$, mono-$C_1$-$C_6$ alkylamino, di-$C_1$-$C_6$ alkylamino, or 4- to 8-membered cyclic amino).

In formula (A), $R_1$ and $P_1$ may form a 4- to 7-membered saturated heterocyclic ring together with a carbon atom bonded to $R_1$ and a nitrogen atom bonded to $P_1$.

When $R_1$ and $P_1$ form a 4- to 7-membered saturated heterocyclic ring, the 4- to 7-membered saturated heterocyclic ring is preferably an azetidine ring, a pyrrolidine ring, a piperidine ring, a piperazine ring or a morpholine ring.

In formula (A), $R_1$ and $Q_1$ may form a 3- to 8-membered alicyclic ring or a 4- to 7-membered saturated heterocyclic ring together with a carbon atom bonded thereto.

When $R_1$ and $Q_1$ form a 3- to 8-membered alicyclic ring or a 4- to 7-membered saturated heterocyclic ring, the 3- to 8-membered alicyclic ring is preferably a cyclopropane ring, a cyclobutane ring, a cyclopentane ring, or a cyclohexane ring, and the 4- to 7-membered saturated heterocyclic ring is a tetrahydrofuran ring, or a tetrahydropyran ring.

When $L_1$ is $-CHM_1-$, $-CH_2CHM_1-$ or $-CHM_1CH_2-$ in formula (A), $R_1$ and $M_1$ may form a 3- to 8-membered alicyclic ring together with a carbon atom bonded to $R_1$ and a carbon atom bonded to $M_1$.

When $R_1$ and $M_1$ form a 3- to 8-membered alicyclic ring, the 3- to 8-membered alicyclic ring is preferably a cyclopropane ring, or a cyclohexane ring.

When $L_1$ is $-CHM_1-$, $-CH_2CHM_1-$ or $-CHM_1CH_2-$ in formula (A), $M_1$ is hydrogen except when $R_1$ and $M_1$ form a 3- to 8-membered alicyclic ring.

In formula (A), $P_1$ is hydrogen, or $C_1$-$C_6$ alkyl, where the $C_1$-$C_6$ alkyl is optionally substituted with one or more groups independently selected from the group consisting of halogen, hydroxy, $C_1$-$C_6$ alkoxy, amino (the amino is $-NH_2$, mono-$C_1$-$C_6$ alkylamino, di-$C_1$-$C_6$ alkylamino, or 4- to 8-membered cyclic amino, which is optionally substituted with halogen), and aminocarbonyl (the amino is $-NH_2$, mono-$C_1$-$C_6$ alkylamino, di-$C_1$-$C_6$ alkylamino, or 4- to 8-membered cyclic amino), except when $R_1$ and $P_1$ form a 4- to 7-membered saturated heterocyclic ring.

$P_1$ is preferably hydrogen, or $C_1$-$C_6$ alkyl. Examples of such $P_1$ specifically include hydrogen, methyl, ethyl, and n-propyl.

$Q_1$ is hydrogen or $C_1$-$C_6$ alkyl except when $R_1$ and $Q_1$ form a 3- to 8-membered alicyclic ring or 4- to 7-membered saturated heterocyclic ring, preferably hydrogen, or methyl.

$R_1$ is preferably $-CONR_{1A}R_{1B}$, where $R_{1A}$ and $R_{1B}$ are each independently hydrogen or $C_1$-$C_6$ alkyl (preferably methyl), or $R_{1A}$ and $R_{1B}$ form a 4- to 8-membered saturated heterocyclic ring together with a nitrogen atom bonded thereto. The 4- to 8-membered saturated heterocyclic ring is optionally substituted with one or more groups independently selected from the group consisting of one or more halogens (preferably fluorine), one or more oxos, one or more $C_1$-$C_6$ alkyls (preferably $C_1$-$C_4$ alkyls), and 4- to 7-membered heterocyclyl (preferably oxetan-3-yl).

In formula (A), * represents a site of binding to the solid phase synthesis resin, and the wavy line represents a site of binding to the adjacent amino acid residue.

When $L_1$ is a single bond, examples of the amino acid residue represented by formula (A) specifically include MeSer(tBuOH), MeGly, MePhe, MePhe(3-F), MePhe(4-F), D-MePhe, MeVal, Pro, Aib, Ala, Gly, Tyr(tBu), and Val.

When $L_1$ is $-CHM_1-$, examples of the amino acid residue represented by formula (A) specifically include bAla, bMeAla, 2-ACHxC, 2-ACPnC, 3-CF3-bAla, Asp-mor, Asp-mor(26-bicyc), Asp-mor(SO2), Asp-NMe2, Asp-oxz, Asp-pip, Asp-pip(345-F6), Asp-pip(4-Me), Asp-pip-tBu, Asp-piz(oxe), Asp-pyrro, Asp-pyrro(34-F4), Asp-pyrro(3-Me2), D-(Propargyl)Gly-(C#CH2), D-3-Abu, D-3-MeAbu, D-Gly(Allyl)-(C#CH2), D-Hph-(C#CH2), D-Leu-(C#CH2), D-MeAsp-pyrro, D-MeLeu-(C#CH2), D-Pic(2)-(C#CH2), D-Pro-(C#CH2), D-Ser(iPen)-(C#CH2), D-Ser(NtBu-Aca)-(C#CH2), EtAsp-pip, MeAsp-aze, MeAsp-mor, MeAsp-mor(26-bicyc), MeAsp-mor(SO2), MeAsp-NMe2, MeAsp-oxz, MeAsp-pip, MeAsp-pip(345-F6), MeAsp-pip(3-F2), MeAsp-pip(4-F2), MeAsp-pip(4-Me), MeAsp-piz(oxe), MeAsp-pyrro, MeAsp-pyrro(34-F4), MeAsp-pyrro(3-Me2), nPrAsp-pip, and D-MeAsp-NMe2.

When $L_1$ is $-CH_2CHM_1-$ or $-CHM_1CH_2-$, examples of the amino acid residue represented by formula (A) specifically include Glu-mor, Glu-pip, MeGlu-pip, Glu-NMe2, and MeGlu-NMe2.

When $L_1$ is $-(CH_2)_nS(CH_2)_m-$, $-(CH_2)_nS(O)(CH_2)_m$ or $-(CH_2)_nS(O)_2(CH_2)_m-$, examples of the amino acid residue represented by formula (A) specifically include MeCys(AcOH)-NMe2.

In an aspect, the amino acid residue adjacent to the amino acid residue at the C-terminal (second residue amino acid residue) of the starting peptide may be represented by the following formula (B).

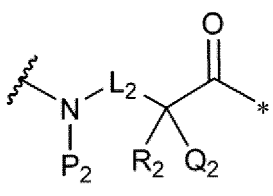

(B)

In formula (B), $L_2$ is a single bond, or $-CH_2-$.

In formula (B), $R_2$ is hydrogen, $C_1$-$C_6$ alkyl, $C_2$-$C_6$ alkynyl, $C_1$-$C_6$ alkoxy $C_1$-$C_6$ alkyl, $C_3$-$C_8$ cycloalkyl, $C_3$-$C_8$ cycloalkyl $C_1$-$C_6$ alkyl, $C_3$-$C_8$ cycloalkoxy $C_1$-$C_6$ alkyl, or $C_7$-$C_{14}$ aralkyl, which is optionally substituted with one or more groups independently selected from the group consisting of halogen, hydroxy, amino (the amino is $-NH_2$, protected amino, mono-$C_1$-$C_6$ alkylamino, di-$C_1$-$C_6$ alkylamino, or 4- to 8-membered cyclic amino, which is optionally substituted with halogen), aminocarbonyl (the amino is $-NH_2$, protected amino, mono-$C_1$-$C_6$ alkylamino, di-$C_1$-$C_6$ alkylamino, or 4- to 8-membered cyclic amino), and $C_1$-$C_6$ alkylsulfonyl.

When $L_2$ is a single bond, $R_2$ is preferably hydrogen, $C_1$-$C_6$ alkyl, halo $C_1$-$C_6$ alkyl, hydroxy $C_1$-$C_6$ alkyl, amino $C_1$-$C_6$ alkyl (the amino is $-NH_2$, protected amino, mono-$C_1$-$C_6$ alkylamino, di-$C_1$-$C_6$ alkylamino, or 4- to 8-membered cyclic amino, which is optionally substituted with one or more halogens), aminocarbonyl $C_1$-$C_6$ alkyl (the amino is $-NH_2$, protected amino, mono-$C_1$-$C_6$ alkylamino, di-$C_1$-$C_6$ alkylamino, or 4- to 8-membered cyclic amino, which is optionally substituted with one or more halogens), $C_1$-$C_6$ alkylsulfonyl $C_1$-$C_6$ alkyl, $C_2$-$C_6$ alkynyl, $C_1$-$C_6$ alkoxy $C_1$-$C_6$ alkyl optionally substituted with one or more halogens, $C_3$-$C_8$ cycloalkyl, $C_3$-$C_8$ cycloalkyl $C_1$-$C_6$ alkyl, $C_3$-$C_8$ cycloalkoxy $C_1$-$C_6$ alkyl, or $C_7$-$C_{14}$ aralkyl.

When $L_2$ is a single bond, $R_2$ is more preferably $C_1$-$C_6$ alkyl, fluoro-$C_1$-$C_6$ alkyl, hydroxy $C_1$-$C_4$ alkyl, protected amino $C_1$-$C_4$ alkyl, protected aminocarbonyl $C_1$-$C_4$ alkyl, methylsulfonyl $C_1$-$C_2$ alkyl, $C_2$-$C_3$ alkynyl, $C_1$-$C_4$ alkoxy $C_1$-$C_2$ alkyl optionally substituted with one or more fluorine, $C_3$-$C_6$ cycloalkyl, $C_3$-$C_6$ cycloalkyl $C_1$-$C_2$ alkyl, $C_3$-$C_6$ cycloalkoxy $C_1$-$C_2$ alkyl, benzyl, or phenethyl.

When $L_2$ is a single bond, examples of $R_2$ more specifically include methyl, ethyl, n-propyl, i-propyl, 2-methylpropyl, 1-methylpropyl, n-butyl, 2-methylbutyl, 3-methylbutyl, n-pentyl, propargyl, 3,3-difluorobutyl, 5,5-difluoropentyl, methoxymethyl, 1-methoxyethyl, 2-methoxyethyl, n-propoxymethyl, 1-hydroxyethyl, cyclopropoxymethyl, cyclobutoxymethyl, (2,2,2-trifluoroethoxy)methyl, 2-methylsulfonylethyl, cyclopropyl, cyclobutyl, cyclopentyl, cyclohexyl, cyclopropylmethyl, cyclobutylmethyl, cyclopentylmethyl, cyclohexylmethyl, benzyl, phenethyl, 4-(t-butoxycarbonylamino)butyl, tritylaminocarbonylmethyl, and t-butoxymethyl.

When $L_2$ is $-CH_2-$, $R_2$ is preferably $C_1$-$C_6$ alkyl, more preferably methyl.

In formula (B), $R_2$ and $P_2$ may form a 4- to 7-membered saturated heterocyclic ring together with a carbon atom bonded to $R_2$ and a nitrogen atom bonded to $P_2$.

When $R_2$ and $P_2$ form a 4- to 7-membered saturated heterocyclic ring, the 4- to 7-membered saturated heterocyclic ring is preferably an azetidine ring, a pyrrolidine ring, a piperidine ring, a piperazine ring or a morpholine ring.

In formula (B), $R_2$ and $Q_2$ may form a 3- to 8-membered alicyclic ring or a 4- to 7-membered saturated heterocyclic ring together with a carbon atom bonded thereto.

When $R_2$ and $Q_2$ form a 3- to 8-membered alicyclic ring or a 4- to 7-membered saturated heterocyclic ring, the 3- to 8-membered alicyclic ring is preferably a cyclopropane ring, a cyclobutane ring, a cyclopentane ring, or a cyclohexane ring, and the 4- to 7-membered saturated heterocyclic ring is preferably a tetrahydrofuran ring, or a tetrahydropyran ring.

In formula (B), $P_2$ is hydrogen, or $C_1$-$C_6$ alkyl, where the $C_1$-$C_6$ alkyl is optionally substituted with one or more groups independently selected from the group consisting of halogen, hydroxy, $C_1$-$C_6$ alkoxy, amino (the amino is $-NH_2$, mono-$C_1$-$C_6$ alkylamino, di-$C_1$-$C_6$ alkylamino, or 4- to 8-membered cyclic amino, which is optionally substituted with halogen), and aminocarbonyl (the amino is $-NH_2$, mono-$C_1$-$C_6$ alkylamino, di-$C_1$-$C_6$ alkylamino, or 4- to 8-membered cyclic amino), except when $R_2$ and $P_2$ form a 4- to 7-membered saturated heterocyclic ring. $P_2$ is preferably hydrogen or $C_1$-$C_2$ alkyl, and examples thereof specifically include hydrogen, and methyl.

$Q_2$ is hydrogen or $C_1$-$C_6$ alkyl except when $R_2$ and $Q_2$ form a 3- to 8-membered alicyclic ring or 4- to 7-membered saturated heterocyclic ring, preferably hydrogen or methyl.

* represents a site of binding to the amino acid residue at the C-terminal, and
the wavy line represents a site of binding to the adjacent amino acid residue or a protective group for the amino group.

Examples of the amino acid residue represented by formula (B) specifically include MeAla, MeLeu, MeCha, MeVal, MeAla(cPent), MeAla(cBu), MeAla(cPr), MeChg, MeGly(cPent), MeGly(cBu), MeGly(cPr), MeAbu, MeNva, MeNle, Val, Leu, MeNva(5-F2), MeHle, MeIle, MeSer(nPr), MeSer(cPr), MeHnl, MeHnl(7-F2), MePRA, MeSer(Me), MeThr, MeSer(cBu), MeSer(Tfe), MeThr(Me), MeHse(Me), MeMet(O2), Ile, Nle, Chg, Ala(cBu), Gly(cPent), Hle, Nva, Phe, Hph, Gly, Aib, Lys(Boc), Ala, D-MeVal, Asn(Trt), Ser(tBu), and bAla(2-Me2).

In an aspect, the peptide supported on the solid phase synthesis resin before the first elongation reaction in the solid phase process may be a dipeptide represented by the following formula (1):

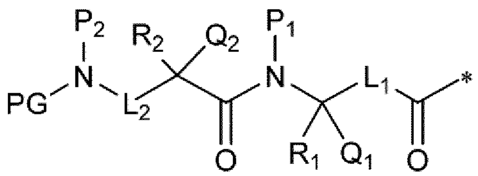

(1)

wherein

$L_1$ is a single bond, or $-CHM_1-$, $-CH_2CHM_1-$, $-CHM_1CH_2-$, $-(CH_2)_nS(CH_2)_m-$, $-(CH_2)_nSO(CH_2)_m-$, or $-(CH_2)_nSO_2(CH_2)_m-$, where n and m are each independently 1 or 2,

$R_1$ is hydrogen, $C_1$-$C_6$ alkyl, $C_2$-$C_6$ alkenyl, $C_2$-$C_6$ alkynyl, $C_1$-$C_6$ alkoxy $C_1$-$C_6$ alkyl, $C_7$-$C_{14}$ aralkyl, or aminocarbonyl (the amino is $-NH_2$, mono-$C_1$-$C_6$ alkylamino, di-$C_1$-$C_6$ alkylamino, or 4- to 8-membered cyclic amino), which is optionally substituted with one or more groups independently selected from the group consisting of halogen, oxo, hydroxy, $C_1$-$C_6$ alkyl, 4- to 7-membered heterocyclyl, aminocarbonyl (the amino is $-NH_2$, mono-$C_1$-$C_6$ alkylamino, di-$C_1$-$C_6$ alkylamino, or 4- to 8-membered cyclic amino), and $C_1$-$C_6$ alkylsulfonyl, or $R_1$ is a peptide chain containing one to four amino acid residues, or

$R_1$ and $P_1$ form a 4- to 7-membered saturated heterocyclic ring together with a carbon atom bonded to $R_1$ and a nitrogen atom bonded to $P_1$, or

$R_1$ and $Q_1$ form a 3- to 8-membered alicyclic ring or a 4- to 7-membered saturated heterocyclic ring together with a carbon atom bonded thereto, or

$R_1$ and $M_1$ form a 3- to 8-membered alicyclic ring together with a carbon atom bonded to $R_1$ and a carbon atom bonded to $M_1$,

$P_1$ is hydrogen, or $C_1$-$C_6$ alkyl, where the $C_1$-$C_6$ alkyl is optionally substituted with one or more groups independently selected from the group consisting of halogen, hydroxy, $C_1$-$C_6$ alkoxy, amino (the amino is $-NH_2$, mono-$C_1$-$C_6$ alkylamino, di-$C_1$-$C_6$ alkylamino, or 4- to 8-membered cyclic amino, which is optionally substituted with halogen), and aminocarbonyl (the amino is $-NH_2$, mono-$C_1$-$C_6$ alkylamino, di-$C_1$-$C_6$ alkylamino, or 4- to 8-membered cyclic amino), except when $R_1$ and $P_1$ form a 4- to 7-membered saturated heterocyclic ring,

$Q_1$ is hydrogen or $C_1$-$C_6$ alkyl except when $R_1$ and $Q_1$ form a 3- to 8-membered alicyclic ring or 4- to 7-membered saturated heterocyclic ring,

$M_1$ is hydrogen except when $R_1$ and $M_1$ form a 3- to 8-membered alicyclic ring,

$L_2$ is a single bond, or $-CH_2-$,

$R_2$ is hydrogen, $C_1$-$C_6$ alkyl, $C_2$-$C_6$ alkynyl, $C_1$-$C_6$ alkoxy $C_1$-$C_6$ alkyl, $C_3$-$C_8$ cycloalkyl, $C_3$-$C_8$ cycloalkyl $C_1$-$C_6$ alkyl, $C_3$-$C_8$ cycloalkoxy $C_1$-$C_6$ alkyl, or $C_7$-$C_{14}$ aralkyl, which is optionally substituted with one or more groups independently selected from the group consisting of halogen, hydroxy, amino (the amino is $-NH_2$, protected amino, mono-$C_1$-$C_6$ alkylamino, di-$C_1$-$C_6$ alkylamino, or 4- to 8-membered cyclic amino, which is optionally substituted with halogen), aminocarbonyl (the amino is $-NH_2$, protected amino, mono-$C_1$-$C_6$ alkylamino, di-$C_1$-$C_6$ alkylamino, or 4- to 8-membered cyclic amino), and $C_1$-$C_6$ alkylsulfonyl, or

$R_2$ and $P_2$ form a 4- to 7-membered saturated heterocyclic ring together with a carbon atom bonded to $R_2$ and a nitrogen atom bonded to $P_2$, or

$R_2$ and $Q_2$ form a 3- to 8-membered alicyclic ring or a 4- to 7-membered saturated heterocyclic ring together with a carbon atom bonded thereto, or

$P_2$ is hydrogen, or $C_1$-$C_6$ alkyl, where the $C_1$-$C_6$ alkyl is optionally substituted with one or more groups independently selected from the group consisting of halogen, hydroxy, $C_1$-$C_6$ alkoxy, amino (the amino is $-NH_2$, mono-$C_1$-$C_6$ alkylamino, di-$C_1$-$C_6$ alkylamino, or 4- to 8-membered cyclic amino, which is optionally substituted with halogen), and aminocarbonyl (the amino is $-NH_2$, mono-$C_1$-$C_6$ alkylamino, di-$C_1$-$C_6$ alkylamino, or 4- to 8-membered cyclic amino), except when $R_2$ and $P_2$ form a 4- to 7-membered saturated heterocyclic ring,

$Q_2$ is hydrogen or $C_1$-$C_6$ alkyl except when $R_2$ and $Q_2$ form a 3- to 8-membered alicyclic ring or 4- to 7-membered saturated heterocyclic ring,

* represents a site of binding to the solid phase synthesis resin, and

PG is a protective group for the amino group,

provided that both $P_1$ and $P_2$ are not hydrogen.

The dipeptide represented by the following formula (1) is preferably a dipeptide represented by the following formula (2):

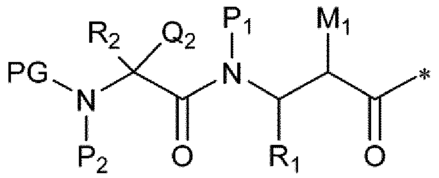

(2)

wherein

$R_1$ is hydrogen, $C_1$-$C_6$ alkyl, halo $C_1$-$C_6$ alkyl, $C_2$-$C_6$ alkenyl, $C_2$-$C_6$ alkynyl, $C_1$-$C_6$ alkoxy $C_1$-$C_6$ alkyl (the $C_1$-$C_6$ alkoxy $C_1$-$C_6$ alkyl is optionally substituted with hydroxy, or aminocarbonyl (the amino is - $NH_2$, mono-$C_1$-$C_6$ alkylamino, di-$C_1$-$C_6$ alkylamino, or 4- to 8-membered cyclic amino)), $C_7$-$C_{14}$ aralkyl optionally substituted with one or more halogens, or aminocarbonyl (the amino is -$NH_2$, mono-$C_1$-$C_6$ alkylamino, di-$C_1$-$C_6$ alkylamino, or 4- to 8-membered cyclic amino, and the cyclic amino is optionally substituted with one or more halogens, one or more oxos, one or more $C_1$-$C_6$ alkyls, or 4- to 7-membered heterocyclyl), or

$R_1$ and $M_1$ form a 3- to 8-membered alicyclic ring together with a carbon atom bonded to $R_1$ and a carbon atom bonded to $M_1$, or

$R_1$ and $P_1$ form a 4- to 7-membered saturated heterocyclic ring together with a nitrogen atom bonded to $P_1$ and a carbon atom bonded to $R_1$,

$M_1$ is hydrogen except when $R_1$ and $M_1$ form a 3- to 8-membered alicyclic ring,

$P_1$ is hydrogen or $C_1$-$C_6$ alkyl except when $R_1$ and $P_1$ form a 4- to 7-membered saturated heterocyclic ring,

$R_2$ is $C_1$-$C_6$ alkyl, halo $C_1$-$C_6$ alkyl, hydroxy $C_1$-$C_6$ alkyl, $C_1$-$C_6$ alkylsulfonyl $C_1$-$C_6$ alkyl, $C_2$-$C_6$ alkynyl, $C_1$-$C_6$ alkoxy $C_1$-$C_6$ alkyl optionally substituted with one or more halogens, $C_3$-$C_8$ cycloalkyl, $C_3$-$C_8$ cycloalkyl $C_1$-$C_6$ alkyl, $C_3$-$C_8$ cycloalkoxy $C_1$-$C_6$ alkyl, or $C_7$-$C_{14}$ aralkyl, or

$R_2$ and $P_2$ form a 4- to 7-membered saturated heterocyclic ring together with a nitrogen atom bonded to $P_2$ and a carbon atom bonded to $R_2$,

$P_2$ is hydrogen or $C_1$-$C_6$ alkyl except when $R_2$ and $P_2$ form a 4- to 7-membered saturated heterocyclic ring,

$Q_2$ is hydrogen,

* represents a site of binding to the solid phase synthesis resin, and

PG is a protective group for the amino group,

provided that both $P_1$ and $P_2$ are not hydrogen.

In an aspect, $L_1$, $P_1$, $Q_1$ and $R_1$ in formula (1) or formula (2) may be the same groups as $L_1$, $P_1$, $Q_1$ and $R_1$, respectively, in the formula (A), and $L_2$, $P_2$, $Q_2$ and $R_2$ in formula (1) or formula (2) may be the same groups as $L_2$, $P_2$, $Q_2$ and $R_2$, respectively, in the formula (B). However, it is preferable that neither $P_1$ nor $P_2$ be hydrogen in formula (1) or formula (2).

PG in formula (1) or formula (2) is a protective group for an amino group.

In an aspect, when the starting peptide is a dipeptide, non-limiting specific examples of the dipeptide include Fmoc-MeVal-MeAsp-pip, Fmoc-MeIle-MeAsp-pip, Fmoc-MeGly(cPent)-MeAsp-pip, Fmoc-MeChg-MeAsp-pip, Fmoc-MeLeu-MeAsp-pip, Fmoc-MeGly(cPent)-MeAsp-NMe2, Fmoc-MeLeu-MeAsp-NMe2, Fmoc-MeVal-D-3-MeAbu-OH, Fmoc-MeChg-D-3-MeAbu-OH, Fmoc-MeVal-MeGly-OH, Fmoc-MeVal-Asp-NMe2, Fmoc-Gly-MeAsp-NMe2, Fmoc-Aib-MeAsp-NMe2, Fmoc-D-MeVal-MeAsp-NMe2, Fmoc-MeVal-D-MeAsp-NMe2, Fmoc-bAla(2-Me2)-MeAsp-NMe2, Fmoc-bAla(2-Me2)-D-3-MeAbu-OH, Fmoc-MeLeu-MeVal-OH, Fmoc-MeVal-Pro-OH, Fmoc-Phe-Pro-OH, Fmoc-Lys(Boc)-Pro-OH, Fmoc-MeLeu-Aib-OH, Fmoc-MeVal-Gly-OH, Fmoc-Ala-Ala-OH, Fmoc-Gly-Tyr(tBu)-OH, Fmoc-Phe-Gly-OH, Fmoc-Asn(Trt)-Gly-OH, Fmoc-MeGly(cPent)-MeAsp-mor, Fmoc-Gly-Val-OH, and Fmoc-Ser(tBu)-Gly-OH.

In an aspect, when the starting peptide is a tripeptide, non-limiting specific examples of the tripeptide include Ala-Ala-Pro, Gly-Gly-Gly, Ala-Gly-Asp, N-substituted forms thereof, and derivatives thereof. The amino acid residues forming the tripeptide may be N-substituted, or derivatized. By using a solid phase synthesis resin on which a tripeptide is supported as described above, premature cleavage can be suppressed. When a sequence of amino acids of the same type is elongated, the reaction process can be shortened by performing one elongation with a peptide instead of carrying out an amino acid elongation reaction two or more times.

The "peptide compound" produced by the above-described method based on a solid phase process in the present invention is a linear peptide compound, and contains at least one, preferably at least two N-substituted amino acid residues (the number of the N-substituted amino acid residues is specifically, 2, 3, 4, 5, 6, 7, 8, 9, 10, 11, 12, 13, 14, 15, 20, 25, 30, preferably 5, 6 or 7, and preferably in the range of 2 to 30, 3 to 30, 6 to 20, 7 to 19, 7 to 18, 7 to 17, 7 to 16, 7 to 15, 8 to 14 or 9 to 13) and at least one N-non-substituted amino acid residue, in addition to natural amino acid residues and non-natural amino acid residues whose total number meets the above-described conditions. The ratio of the number of N-substituted amino acid residues present in such a peptide compound is, for example, 30% or more,

40% or more, 50% or more, 60% or more, 70% or more, or 80% or more with respect to the total number of amino acid residues forming the peptide compound. The N-substituted amino acid residues in the present invention may be non-natural N-substituted amino acid residues other than proline. The peptide compound according to the present invention may include a repeated sequence, for example, a repeated sequence of 2 amino acid residues, 3 amino acid residues, 4 amino acid residues or 5 amino acid residues, and the number of repetitions thereof is preferably 2 or less, or 3 or less. The peptide compound according to the present invention is more preferably free of such a repeated sequence.

In an aspect, the "solid phase synthesis resin" for use in the present invention is not particularly limited as long as it can be used for synthesis of a peptide compound by a solid phase process. Examples of such the solid phase synthesis resin specifically include those removable under acidic conditions, such as CTC resin, Sieber resin, Wang resin, SASRIN resin, tritylchloride resin (Trt resin), 4-methyltritylchloride resin (Mtt resin) and 4-methoxytritylchloride resin (Mmt). The resin can be appropriately selected in line with a functional group on the side of an amino acid to be used. For example, when carboxylic acid (main chain carboxylic acid, or side chain carboxylic acid typified by Asp or Glu), or a hydroxy group on the aromatic ring (phenol group typified by Tyr) is used as the functional group on the amino acid side, the resin that is used is preferably tritylchloride resin (Trt resin) or 2-chlorotritylchloride resin (CTC resin). When an aliphatic hydroxy group (aliphatic alcohol group typified by Ser or Thr) is used as the functional group on the amino acid side, the resin that is used is preferably tritylchloride resin (Trt resin), 2-chlorotritylchloride resin (CTC resin) or 4-methyltritylchloride resin (Mtt resin). In the present specification, the resin is sometimes referred to as resin. The solid phase synthesis resin can be linked to an amino acid at any position, which is not limited to an amino acid at the C-terminal in the peptide. It is preferable that the carboxyl group of the amino acid at the C-terminal be linked to the solid phase synthesis resin, and the carboxyl group may be a carboxyl group on the main chain or a carboxyl group on the side chain. When a resin having a trityl skeleton at a linker site, specifically CTC resin, Trt resin, Mtt resin or Mmt resin, is used as the solid phase synthesis resin, premature cleavage easily occurs, and therefore the method of the present invention is particularly useful.

The type of polymer forming the resin is not particularly limited. In the case of resin formed of polystyrene, either resins of 100-200 mesh or resins of 200-400 mesh may be used. The crosslinking ratio is not particularly limited, and resins crosslinked with DVB (divinylbenzene) at 1% are preferable. Examples of the type of polymer forming the resin include Tentagel and Chemmatrix.

In an aspect, the present invention may comprise a step of supporting a peptide on a solid phase synthesis resin. As the reaction condition in this step, any reaction condition known in the art can be used, and there is no particular limitation. It is preferable to apply, for example, the reaction condition described in WO 2013/100132 or WO 2018/225864, or the reaction condition described in Solid Phase Synthesis Handbook issued by Merck on May 1, 2002.

Examples of the step of supporting the peptide on the solid phase synthesis resin specifically include, but are not limited to, the following.

By swelling a resin with an appropriate solvent, and applying a peptide (protected peptide) solution, in which an amino group is protected with a protective group, to a solid phase synthesis resin in the presence of a base, the protected peptide can be supported on the resin. Examples of the solvent for use in the swelling and the solvent for use in the preparation of the peptide solution include halogen-based solvents such as DCM (dichloromethane), chloroform and DCE (1,2-dichloroethane); ether-based solvents such as THF (tetrahydrofuran), 2-methyltetrahydrofuran, 4-methyltetrahydrofuran, dioxane, DME (1,2-dimethoxyethane), TBME (t-butyl methyl ether), CPME (cyclopentyl methyl ether) and isosorbide dimethyl ether; ketone-based solvents such as acetone, MEK (methyl ethyl ketone), 4-methyl-2-pentanone and cyclopentanone, urea-based solvents such as TMU (N,N,N',N'-tetramethylurea), DMI (1,3-dimethyl-2-imidazolidinone) and DMPU (N,N'-dimethylpropylene urea); amide-based solvents such as DMF (N,N-dimethylformamide), DMA (N,N-dimethylacetamide), NFM (N-formylmorpholine), NMP (N-methyl-2-pyrrolidone) and NBP (N-butyl-2-pyrrolidone); sulfoxide-based solvents such as DMSO (dimethylsolfoxide); sulfone-based solvents such as sulfolane; ester-based solvents such as ethyl acetate, propyl acetate, isopropyl acetate, butyl acetate, isobutyl acetate and GVL ($\gamma$-valerolactone); aromatic solvents such as anisole, toluene, $\alpha,\alpha,\alpha$-torifluorotoluene, 1,2-dichlorobenzene and benzene; and carbonate-based solvents such as dimethyl carbonate, diethyl carbonate, ethylene carbonate and propylene carbonate. In addition thereto, solvents such as acetonitrile, methanol, ethylene glycol, water, silene and limonene can be used. Solvents obtained by mixing two or more of the above-mentioned solvents at any ratio can also be used. Examples of the protective group for an amino group which can be used include carbamate-type protective groups such as a Fmoc group, a Boc group, an Alloc group, a Cbz group and a Teoc group; amide-type protective groups such as a trifluoroacetyl group; arylsulfoneamide-type protective groups such as a benzenesulfonyl group, a tosyl group, a nosyl group and a dinitronosyl group; alkylamine-type protective groups such as a t-Bu group, a trityl group and a cumyl group; and imide-type protective groups such as a benzylidene group, a 4-methoxybenzylidene group and a diphenylmethylidene group. Examples of the base that can be used include tertiary amine bases such as triethylamine, DIPEA (N,N-diisopropylethylamine), NMM (N-methylmorpholine) and DABCO (1,4-diazabicyclo[2.2.2]-octane); and pyridine-based bases such as pyridine, lutidine, collidine and DMAP (4-dimethylaminopyridine).

In an aspect, the present invention further comprises a step of elongating a peptide supported on a solid phase

synthesis resin with one or more amino acids. By this step, a peptide compound having a desired amino acid sequence can be obtained. For this step, a method known in the art can be used, and for example, the method described in WO 2013/100132, WO 2018/225851 or WO 2018/225864, or the method described in Solid Phase Synthesis Handbook issued by Merck on May 1, 2002 can be applied.

Examples of the step of elongating a peptide supported on a solid phase synthesis resin with one or more amino acid residues specifically include, but are not limited, to the following.

A step of eliminating a protective group at the N-terminal of the peptide supported on the solid phase synthesis resin, and a step of applying an amino acid residue in which an amino group is protected with a protective group (protected amino acid residue) and a condensation reagent to the peptide in a solvent in the presence or absence of a base are carried out. By repeating the two steps, the peptide can be elongated with a plurality of amino acid residues. Examples of the protective group at the N-terminal which can be used include carbamate-type protective groups such as a Fmoc group, a Boc group, an Alloc group, a Cbz group and a Teoc group; amide-type protective groups such as a trifluoroacetyl group; arylsulfoneamide-type protective groups such as a benzenesulfonyl group, a tosyl group, a nosyl group and a dinitronosyl group; alkylamine-type protective groups such as a t-Bu group, a trityl group and a cumyl group; and imide-type protective groups such as a benzylidene group, a 4-methoxybenzylidene group and a diphenylmethylidene group, and a Fmoc group is preferably used. When a Fmoc group is used as a protective group, piperidine, DBU (1,8-diazabicyclo[5.4.0]-7-undecene), DBN (1,5-diazabicyclo[4.3.0]-5-nonene), or the like can be used for elimination thereof. Examples of the condensation reagent that can be used include combinations of carbodiimide-based condensation agents such as DCC (N,N'-dicyclohexylcarbodiimide), DIC (N,N'-diisopropylcarbodiimide) and EDCI (1-ethyl-3-(3-dimethylaminopropyl)carbodiimide hydroxide) with activating agents such as HOAt (1-hydroxy-7-azabenzotriazole), HOBt (1-hydroxybenzotriazole), HOOBt (3,4-dihydro-3-hydroxy-4-oxo-1,2,3-benzotriazine) and oxyma (cyano(hydroxyimino)ethyl acetate); uronium salt-based condensation agents such as HATU (O-(7-aza-1H-benzotriazol-1-yl)-N,N,N',N'-tetramethyluronium hexafluorophosphate), HBTU (O-(1H-benzotriazol-1-yl)-N,N,N',N'-tetramethyluronium hexafluorophosphate), HCTU (O-(6-chloro-1H-benzotriazol-1-yl)-N,N,N',N'-tetramethyluronium hexafluorophosphate) and COMU ((1-cyano-2-ethoxy-2-oxoethylideneaminoxy)dimethylaminomorpholinocarbenium hexafluorophosphate); phosphonium salt-based condensation agents such as PyAOP ((7-azabenzotriazol-1-yloxy)trispyrolidinophosphonium hexafluorophosphate), PyBOP (1H-benzotriazol-1-yloxy-tri(pyrrolidino)phosphonium hexafluorophosphate) and PyOxim ([ethylcyano(hydroxyimino)acetat-$O^2$]tri-1-pyrrolidinylphosphonium hexafluorophosphate); and formamidinium salt-based condensation agents such as 1-chloro-N,N-2-trimethyl-1-propenylamine (Ghosez agent), TCFH (chloro-N,N,N',N'-tetramethylformamidinium hexafluorophosphate), PyCIU (N,N,N',N'-bis(tetramethylene)chloroformamidinium hexafluorophosphate), BTFFH (fluoro-N,N,N',N'-bis(tetramethylene)formamidinium hexafluorophosphate) and TFFH (fluoro-N,N,N',N'-tetramethylamidinium hexafluorophosphate. Examples of the base that can be used include tertiary amine bases such as triethylamine, DIPEA (N,N-diisopropylethylamine), NMM (N-methylmorpholine) and DABCO (1,4-diazabicyclo[2.2.2]-octane); and pyridine-based bases such as pyridine, lutidine, collidine and DMAP (4-dimethylaminopyridine). Examples of the solvent include halogen-based solvents such as DCM (dichloromethane), chloroform and DCE (1,2-dichloroethane); ether-based solvents such as THF (tetrahydrofuran), 4-methyltetrahydrofuran, 4-methyltetrahydrofuran, dioxane, DME (1,2-dimethoxyethane), TBME (t-butyl methyl ether), CPME (cyclopentyl methyl ether) and isosorbide dimethyl ether; ketone-based solvents such as acetone, MEK (methyl ethyl ketone), 4-methyl-2-pentanone and cyclopentanone, urea-based solvents such as TMU (N,N,N',N'-tetramethylurea), DMI (1,3-dimethyl-2-imidazolidinone) and DMPU (N,N'-dimethylpropylene urea); amide-based solvents such as DMF (N,N-dimethylformamide), DMA (N,N-dimethylacetamide), NFM (N-formylmorpholine), NMP (N-methyl-2-pyrrolidone) and NBP (N-butyl-2-pyrrolidone); sulfoxide-based solvents such as DMSO (dimethylsolfoxide); sulfone-based solvents such as sulfolane; ester-based solvents such as ethyl acetate, propyl acetate, isopropyl acetate, butyl acetate, isobutyl acetate and GVL ($\gamma$-valerolactone); aromatic solvents such as anisole, toluene, $\alpha,\alpha,\alpha$-torifluorotoluene, 1,2-dichlorobenzene and benzene; and carbonate-based solvents such as dimethyl carbonate, diethyl carbonate, ethylene carbonate and propylene carbonate. In addition thereto, solvents such as acetonitrile, methanol, ethylene glycol, water, silene and limonene can be used. Solvents obtained by mixing two or more of the above-mentioned solvents at any ratio can also be used.

In an aspect, the present invention relates to a method for producing a cyclic peptide compound, the method further comprising the steps of: obtaining a linear peptide compound produced by the above-described method of the present invention, a salt thereof, or a solvate thereof; removing the solid phase synthesis resin; and cyclizing a C-terminal-side group and a N-terminal-side group to form a cyclic portion.

For both the step of removing the peptide compound from the solid phase synthesis resin and the step of cyclizing a C-terminal-side group and a N-terminal-side group of the peptide compound to form a cyclic portion, a method known in the art can be used, and for example, the method described in WO 2013/100132, WO 2018/225851 or WO 2018/225864, or the method described in Solid Phase Synthesis Handbook issued by Merck on May 1, 2002 can be applied.

Examples of the step of removing the peptide compound from the solid phase synthesis resin specifically include, but are not limited to, the following.

By elongating the peptide chain to a desired amino acid sequence, then swelling the solid phase synthesis resin, and then applying an acidic solution, the peptide compound can be removed from the resin. Examples of the solvent for use in the swelling include halogen-based solvents such as DCM (dichloromethane), chloroform and DCE (1,2-dichloroethane); ether-based solvents such as THF (tetrahydrofuran), 4-methyltetrahydrofuran, 4-methyltetrahydrofuran, dioxane, DME (1,2-dimethoxyethane), TBME (t-butyl methyl ether), CPME (cyclopentyl methyl ether) and isosorbide dimethyl ether; ketone-based solvents such as acetone, MEK (methyl ethyl ketone), 4-methyl-2-pentanone and cyclopentanone, urea-based solvents such as TMU (N,N,N',N'-tetramethyl urea), DMI (1,3-dimethyl-2-imidazolidinone) and DMPU (N,N'-dimethylpropylene urea); amide-based solvents such as DMF (N,N-dimethylformamide), DMA (N,N-dimethylacetamide), NFM (N-formylmorpholine), NMP (N-methyl-2-pyrrolidone) and NBP (N-butyl-2-pyrrolidone); sulfoxide-based solvents such as DMSO (dimethylsolfoxide); sulfone-based solvents such as sulfolane; ester-based solvents such as ethyl acetate, propyl acetate, isopropyl acetate, butyl acetate, isobutyl acetate and GVL ($\gamma$-valerolactone); aromatic solvents such as anisole, toluene, $\alpha,\alpha,\alpha$-torifluorotoluene, 1,2-dichlorobenzene and benzene; and carbonate-based solvents such as dimethyl carbonate, diethyl carbonate, ethylene carbonate and propylene carbonate. In addition thereto, solvents such as acetonitrile, methanol, ethylene glycol, water, silene and limonene can be used. Solvents obtained by mixing two or more of the above-mentioned solvents at any ratio can also be used. For the acidic solution, fluoroalcohols such as TFE (2,2,2-trifluoroethanol) and HFIP (1,1,1,3,3,3-hexafluoroisopropyl alcohol), carboxylic acids such as TFA (trifluoroacetic acid), hydrochloric acid and the like can be used.

In an aspect, the "solid phase synthesis resin" for use in the present invention is preferably one that enables isolation of a peptide compound synthesized by a solid phase process and having an intended amino acid sequence, for example, under mild acidic conditions that do not cause removal of a protective group on the side chain of an amino acid forming the peptide compound. Examples of the protective group on the side chain of the amino acid specifically include Boc, Trt, THP, and tBu. Examples of the amino acid having a protective group on the side chain is Tyr(tBu), Ser(tBu), Thr(tBu), Asp(tBu), Glu(tBu), Trp(Boc), Lys(Boc), His(Boc), Ser(Trt), Thr(Trt), Trp(Trt), Lys(Trt), His(Trt), Asn(Trt), Gln(Trt), Ser(THP), Thr(THP), and N-alkyl forms, e.g. N-methyl forms, thereof.

In an aspect, the "solid phase synthesis resin" for use in the present invention is preferably one that enables isolation of the supported peptide compound under mild acidic conditions. For examples, a resin having a trityl skeleton at a linker site, specifically CTC resin, Trt resin, Mtt resin or Mmt resin, or a resin having a diphenylmethyl skeleton at a linker site, specifically Sieber resin is preferable. Such a solid phase synthesis resin is more preferably CTC resin or Sieber resin, most preferably CTC resin.

In an aspect, the mild acidic conditions may include temperature conditions. The "solid phase synthesis resin" for use in the present invention is preferably one that can be removed under mild acidic conditions at a temperature around room temperature, e.g. room temperature $\pm$ 10°C.

In an aspect, the mild acidic conditions are acidic conditions at a pH of 2 or more. In an aspect, the mild acidic conditions may include conditions of using a dilute acid solution. As used herein, the dilute acid solution means an acid diluted with a non-acidic solvent, and may be prepared by mixing an acid with a non-acidic solvent. Examples of the acid that is used for the dilute solution include acids having a pKa in water of -1 or more, 1 or more, 2 or more, 3 or more, 4 or more, 5 or more, 6 or more, 7 or more, 8 or more, 9 or more, 10 or more, 11 or more, or 12 or more, and examples thereof specifically include TFA, 2,2,2-trifluoroethanol, 1,1,1,3,3,3-hexafluoroisopropyl alcohol, trichloroacetic acid, acetic acid, formic acid, oxalic acid, and mixtures thereof. Such an acid is preferably TFA, 2,2,2-trifluoroethanol, 1,1,1,3,3,3-hexafluoroisopropyl alcohol, acetic acid, or a mixture thereof, more preferably TFA. The volume percentage of the acid in the dilute solution may be 60% or less, 50% or less, 40% or less, 30% or less, 20% or less, 10% or less, 5% or less, 4% or less, 3% or less, 2% or less, or 1% or less, and is preferably 20% or less, 10% or less, 5% or less, 4% or less, 3% or less, 2% or less, or 1% or less, more preferably 10% or less, 5% or less, 4% or less, 3% or less, 2% or less, or 1% or less. The non-acidic solvent that is used for the dilute acid solution is preferably DCM, dichloroethane, water, or 2-MeTHF, or a mixed solvent thereof, more preferably DCM. Examples of the dilute acid solution specifically include DCM solutions containing TFA at 20 vol% or less, DCM solutions containing TFA at 10 vol% or less, DCM solutions containing TFA at 5 vol% or less, DCM solutions containing TFA at 1 vol% or less, DCM solutions containing acetic acid at 20% or less and 2,2,2-trifluoroethanol at 20% or less, and DCM solutions containing 1,1,1,3,3,3-hexafluoroisopropyl alcohol at 20% or less.

Examples of the step of cyclizing a C-terminal-side group and a N-terminal-side group of the peptide compound to form a cyclic portion specifically include, but are not limited to, the following.

By applying a condensation reagent in the presence or absence of a base to a linear peptide compound dissolved in an appropriate solvent, a C-terminal-side group and a N-terminal-side group of the peptide compound can be cyclized to form a cyclic portion. Examples of the solvent include halogen-based solvents such as DCM (dichloromethane), chloroform and DCE (1,2-dichloroethane); ether-based solvents such as THF (tetrahydrofuran), 4-methyltetrahydrofuran, 4-methyltetrahydropyran, dioxane, DME (1,2-dimethoxyethane), TBME (t-butyl methyl ether), CPME (cyclopentyl methyl ether) and isosorbide dimethyl ether; ketone-based solvents such as acetone, MEK (methyl ethyl ketone), 4-methyl-2-pentanone and cyclopentanone, urea-based solvents such as TMU (N,N,N',N'-tetramethylurea), DMI (1,3-dimethyl-2-imidazolidinone) and DMPU (N,N'-dimethylpropylene urea); amide-based solvents such as DMF (N,N-dimethylformamide), DMA (N,N-dimethylacetamide), NFM (N-formylmorpholine), NMP (N-methyl-2-pyrrolidone) and NBP (N-butyl-2-pyrrolidone); sulfoxide-based solvents such as DMSO (dimethylsolfoxide); sulfone-based solvents such as sulfolane; ester-based solvents such as ethyl acetate, propyl acetate, isopropyl acetate, butyl acetate, isobutyl acetate and GVL (γ-valerolactone); aromatic solvents such as anisole, toluene, α,α,α-torifluorotoluene, 1,2-dichlorobenzene and benzene; and carbonate-based solvents such as dimethyl carbonate, diethyl carbonate, ethylene carbonate and propylene carbonate. In addition thereto, solvents such as acetonitrile, silene and limonene can be used. Solvents obtained by mixing two or more of the above-mentioned solvents at any ratio can also be used. Examples of the condensation reagent that can be used include combinations of carbodiimide-based condensation agents such as DCC (N,N'-dicyclohexylcarbodiimide), DIC (N,N'-diisopropylcarbodiimide) and EDCI (1-ethyl-3-(3-dimethylaminopropyl)carbodiimide hydroxide) with activating agents such as HOAt (1-hydroxy-7-azabenzotriazole), HOBt (1-hydroxybenzotriazole), HOOBt (3,4-dihydro-3-hydroxy-4-oxo-1,2,3-benzotriazine) and oxyma (cyano(hydroxyimino)ethyl acetate); uronium salt-based condensation agents such as HATU (O-(7-aza-1H-benzotriazol-1-yl)-N,N,N',N'-tetramethyluronium hexafluorophosphate), HBTU (O-(1H-benzotriazol-1-yl)-N,N,N',N'-tetramethyluronium hexafluorophosphate), HCTU (O-(6-chloro-1H-benzotriazol-1-yl)-N,N,N',N'-tetramethyluronium hexafluorophosphate) and COMU ((1-cyano-2-ethoxy-2-oxoethylideneaminoxy)dimethylaminomorpholinocarbenium hexafluorophosphate); phosphonium salt-based condensation agents such as PyAOP ((7-azabenzotriazol-1-yloxy)trispyrolidinophosphonium hexafluorophosphate), PyBOP (1H-benzotriazol-1-yloxy-tri(pyrrolidino)phosphonium hexafluorophosphate) and PyOxim ([ethylcyano(hydroxyimino)acetat-$O^2$]tri-1-pyrrolidinylphosphonium hexafluorophosphate); and formamidinium salt-based condensation agents such as 1-chloro-N,N-2-trimethyl-1-propenylamine (Ghosez agent), TCFH (chloro-N,N,N',N'-tetramethylformamidinium hexafluorophosphate), PyClU (N,N,N',N'-bis(tetramethylene)chloroformamidinium hexafluorophosphate), BTFFH (fluoro-N,N,N',N'-bis(tetramethylene)formamidinium hexafluorophosphate) and TFFH (fluoro-N,N,N',N'-tetramethylamidinium hexafluorophosphate. Examples of the base that can be used include tertiary amine bases such as triethylamine, DIPEA (N,N-diisopropylethylamine), NMM (N-methylmorpholine) and DABCO (1,4-diazabicyclo[2.2.2]-octane); and pyridine-based bases such as pyridine, lutidine, collidine and DMAP (4-dimethylaminopyridine).

In an aspect, the number of N-substituted amino acids present at the peptide site in the cyclic peptide compound is preferably 2 or more, 3 or more, 4 or more, or 5 or more, more preferably 6 or more, further preferably 7 or more, particularly preferably 8 or more, and preferably 20 or less, 15 or less, 14 or less, 13 or less, 12 or less, 10 or less, or 9 or less as an example. In the present specification, the number of N-substituted amino acids present in the cyclic peptide compound is, for example, 30% or more, 40% or more, 50% or more, 60% or more, 70% or more, or 80% or more with respect to the number of amino acids forming the cyclic portion.

In an aspect, the present invention relates to a method for improving a recovery ratio of a peptide compound as compared to elongation with amino acid residues one by one, wherein a peptide is supported on a solid phase synthesis resin before a first elongation reaction in production of a peptide compound containing at least one N-substituted amino acid residue, a salt thereof or a solvate thereof by a solid phase process. That is, in the present invention, a peptide compound having an intended amino acid sequence is obtained by elongating a peptide chain by a solid phase process using a peptide (preferably a dipeptide or a tripeptide) as a starting material, and as compared to a case where a peptide compound having the same amino acid sequence is produced by a solid phase process using an amino acid as a starting material, the recovery ratio of an intended peptide compound can be improved by, for example, 5% or more, 10% or more, 20% or more, 30% or more, 40% or more, or 50% or more.

In an aspect, the present invention relates to a method for suppressing generation of impurities as compared to elongation with amino acid residues one by one, wherein a peptide is supported on a solid phase synthesis resin before a first elongation reaction in production of a peptide compound containing at least one N-substituted amino acid residue, a salt thereof or a solvate thereof by a solid phase process. That is, in the present invention, a peptide compound having an intended amino acid sequence is obtained by elongating a peptide chain by a solid phase process using a peptide (preferably a dipeptide or a tripeptide) as a starting material, and as compared to a case where a peptide compound having the same amino acid sequence is produced by a solid phase process using an amino acid as a starting material, generation of impurities (e.g. an epimeric form, an excessively elongated form and an amino acid-deficient form) can be suppressed by, for example, 0.5% or more, 1% or more, 5% or more, 10% or more, 20% or more, 30% or more, or 40%% or more.

In an aspect, the present invention relates to a method for suppressing premature cleavage as compared to elongation with amino acid residues one by one, wherein a peptide is supported on a solid phase synthesis resin before a first elongation reaction in production of a peptide compound containing at least one N-substituted amino acid residue, a salt thereof or a solvate thereof by a solid phase process. That is, in the present invention, a peptide compound having an intended amino acid sequence is obtained by elongating a peptide chain by a solid phase process using a peptide (preferably a dipeptide or a tripeptide) as a starting material, and as compared to a case where a peptide compound having the same amino acid sequence is produced by a solid phase process using an amino acid as a starting material, premature cleavage can be significantly suppressed, for example, by 5% or more, 10% or more, 20% or more, 30% or

more, 40% or more, or 50% or more.

The compound according to the present invention, a salt thereof, or a solvate thereof includes all stereoisomers (e.g. enantiomers and diastereomers (including cis- and trans-geometric isomers)), racemates of the isomers, and other mixtures. For example, the compound of the present invention may have one or more asymmetric points, and the present invention includes racemic mixtures, diastereomer mixtures, and enantiomers of such compounds.

When the compound according to the present invention is obtained as a free form, the compound can be conventionally transformed into a state of a salt, a hydrate thereof or a solvate thereof which may be formed by the compound.

When the compound according to the present invention is obtained as a salt, a hydrate or a solvate of the compound, the compound can be conventionally transformed into a free form thereof.

All references cited herein are incorporated herein by reference.

Examples

The present invention will be described in more detail on the basis of Example, which should not be construed as limiting the present invention. All starting materials and reagents were obtained from commercial suppliers, or synthesized in accordance with known methods.

The table below shows LCMS analysis conditions.

[Table 1]

| Analysis condition | Apparatus | Column (I.D × length) (mm) | Mobile phase | Gradient (A/B) | Flow rate (mL/min) | Column temperature (°C) | Wavelength |
|---|---|---|---|---|---|---|---|
| SQDFA05 | Acquity UPLC/SQD2 | Ascentis Express C18 (2.1x50) | A) 0.1% FA.water B) 0.1% FA.acetonitrile | 95/5 (initial) => 0/100 (1.0 minute) => 0/100 (0.4 minutes) | 1.0 | 35 | 210-400nm PDA total |
| SQDFA05_55deg | Acquity UPLC/SQD2 | Ascentis Express C18 (2.1x50) | A) 0.1% FA.water B) 0.1% FA.acetonitrile | 95/5 (initial) => 0/100 (1.0 minute) => 0/100 (0.4 minutes) | 1.0 | 55 | 210-400nm PDA total |
| SQDFA05long | Acquity UPLC/SQD2 | Ascentis Express C18 (2.1x50) | A) 0.1% FA.water B) 0.1% FA.acetonitrile | 95/5 (initial) => 0/100 (4.5 minutes) => 0/100 (0.5 minutes) | 1.0 | 35 | 210-400nm PDA total |
| SQDAA50 | Acquity UPLC/SQD2 | Ascentis Express C18 (2.1x50) | A) 10mM AA.water B) methanol | 50/50 (initial) => 0/100 (0.7 minutes) => 0/100 (0.7 minutes) | 1.0 | 35 | 210-400nm PDA total |
| SQDAA50long | Acquity UPLC/SQD2 | Ascentis Express C18 (2.1x50) | A) 10mM AA.water B) methanol | 50/50 (initial) => 0/100 (4.5 minutes) => 0/100 (0.5 minutes) | 1.0 | 35 | 210-400nm PDA total |
| SMDmethod_1 | Shimadzu /2020 | Ascentis Express C18 (2.1x50) | A) 0.1% FA. water B) 0.1% FA. acetonitrile | 95/5 (initial) => 5/95 (2.5 minutes) => 5/95 (1.2 minutes) => 95/5 (0.1 minutes) | 1.0 | 40 | 190-400nm PDA total |
| SMDmethod_2 | Shimadzu /2020 | Ascentis Express C18 (2.1x50) | A) 0.1% FA. water B) 0.1% FA. acetonitrile | 95/5 (initial) => 5/95 (2.0 minutes) => 5/95 (0.7 minutes) => 95/5 (0.1 minutes) | 1.0 | 40 | 190-400nm PDA total |
| SMDmethod_3 | Shimadzu /2020 | Ascentis Express C18 (3.0x50) | A) 0.05% TFA. water B) 0.05% TFA. acetonitrile | 70/30 (initial) => 30/70 (3.2 minutes) => 5/95 (0.5 minutes) => 5/95 (1.0 minute) => 95/5 (0.1 minutes) | 1.0 | 40 | 190-400nm PDA total |
| SMDmethod_4 | Shimadzu /2020 | HALO C18 (3.0x30) | A) 0.05% TFA. water B) 0.05% TFA. acetonitrile | 95/5 (initial) => 0/100 (0.7 minutes) => 0/100 (0.4 minutes) => 95/5 (0.02 minutes | 1.5 | 40 | 190-400nm PDA total |
| SMDmethod_5 | Shimadzu /2020 | HALO C18 (3.0x30) | A) 0.05% TFA. water B) 0.05% TFA. acetonitrile | 95/5 (initial) => 0/100 (1.1 minute) => 0/100 (0.6 minutes) => 95/5 (0.05 minutes) | 1.5 | 40 | 190-400nm PDA total |
| SMDmethod_6 | Shimadzu /2020 | HALO C18 (3.0x30) | A) 0.05% TFA. water B) 0.05% TFA. acetonitrile | 95/5 (initial) => 0/100 (1.1 minutes) => 0/100 (0.6 minutes) => 95/5 (0.05 minutes) | 1.3 | 40 | 190-400nm PDA total |
| SMDmethod_7 | Shimadzu /2020 | Shim-pack XR-ODS (3.0x50) | A) 0.05% TFA. water B) 0.05% TFA. acetonitrile | 60/40 (initial) => 5/95 (3.0 minutes) => 5/95 (0.7 minutes) => 95/5 (0.05 minutes) | 1.2 | 40 | 190-400nm PDA total |

Example 1: Synthesis of compound such as amino acid for use in peptide synthesis using peptide synthesizing machine

For peptide synthesis described in the present specification, amino acids or peptides shown in Tables 2, 3, 4 and 5, and resins for support thereof were used.

Example 1-1. Fmoc-amino acid and peptide purchased and used as such

Fmoc-amino acids and peptides shown in Table 2 were purchased from commercial suppliers.

[Table 2]

| **Abbreviation** | Structure |
|---|---|
| **Fmoc-Ile-OH** | 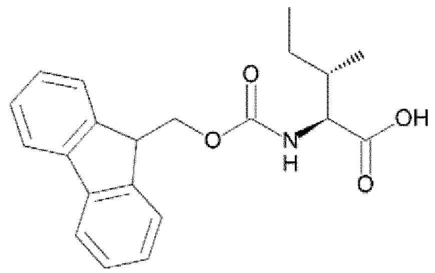 |
| **Fmoc-Ala-OH** | 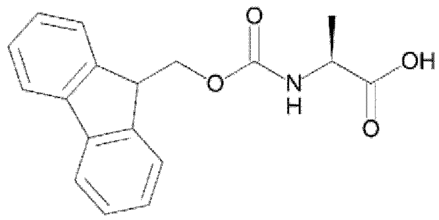 |
| **Fmoc-Lys (Boc) - OH** | 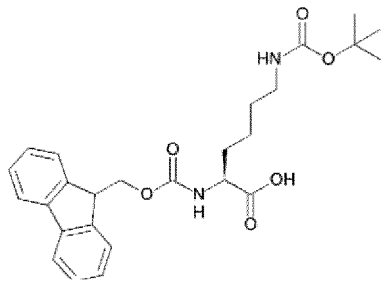 |
| **Fmoc-Asn (Trt)-OH** | 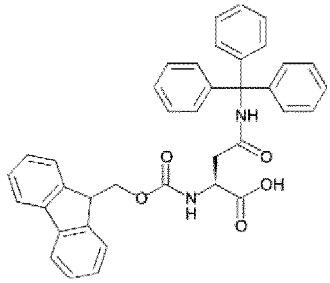 |
| **Fmoc-Ser (tBu) - OH** | 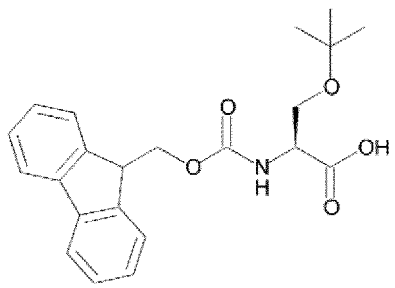 |

(continued)

| **Abbreviation** | Structure |
|---|---|
| **Fmoc-Gly-OH** | 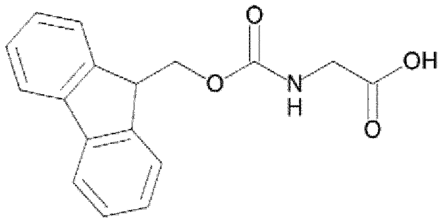 |
| **Fmoc-Phe-OH** | 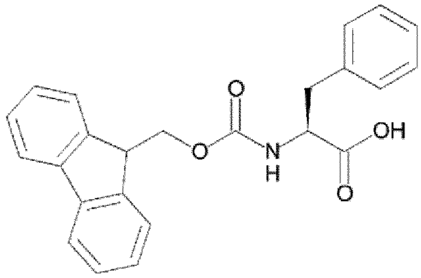 |
| **Fmoc**-Tyr **(tBu)-OH** | 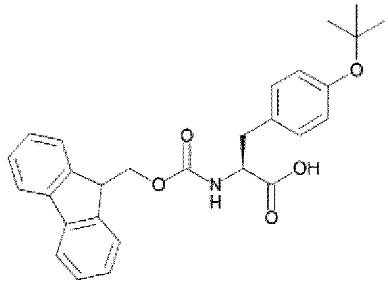 |
| **Fmoc-Val-OH** | 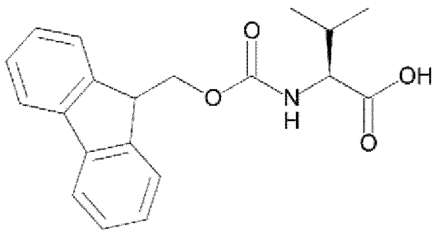 |
| **Fmoc-Leu-OH** | 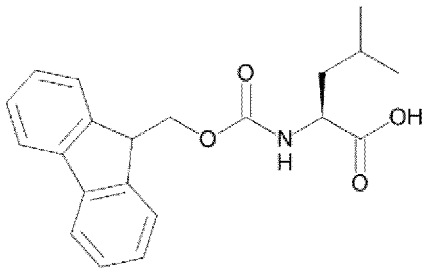 |

(continued)

| Abbreviation | Structure |
|---|---|
| **Fmoc-Aib-OH** | 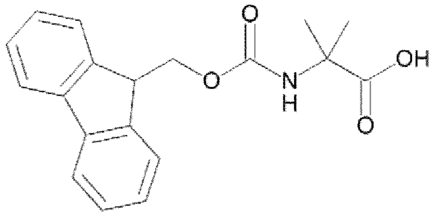 |
| **Fmoc-Pro-OH** | 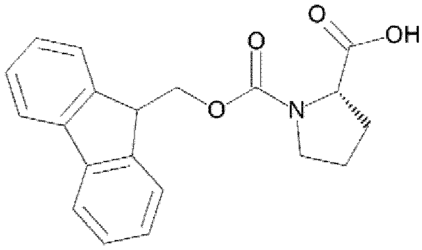 |
| **Fmoc-MeIle-OH** | 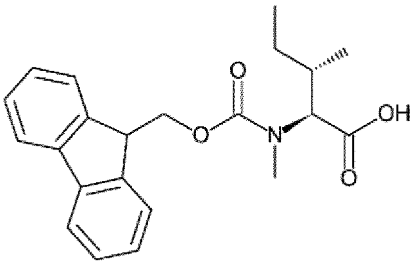 |
| **Fmoc-MeChg-OH** | 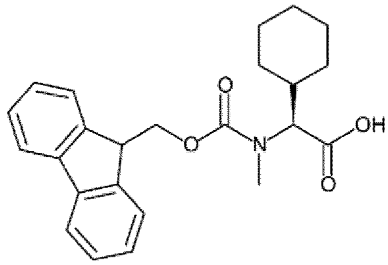 |
| **Fmoc-HeGly-OH** | 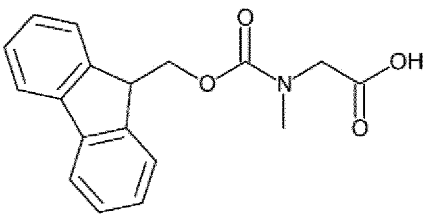 |
| **Fmoc-bAla (2-Me2)-OH** | 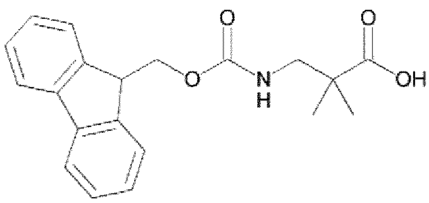 |

(continued)

| Abbreviation | Structure |
|---|---|
| **Fmoc-MeVal-OH** | 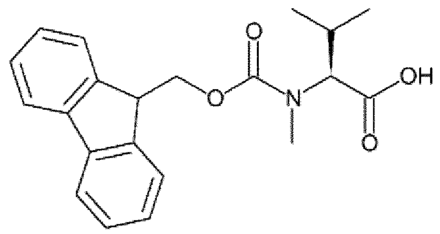 |
| **Fmoc-MeGly (cPent) -OH** | 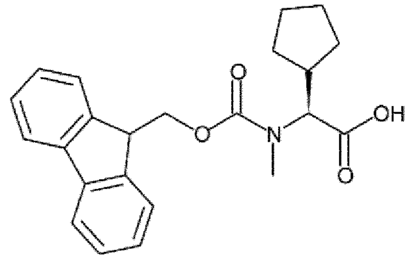 |
| **Fmoc-MeLeu-OH** | 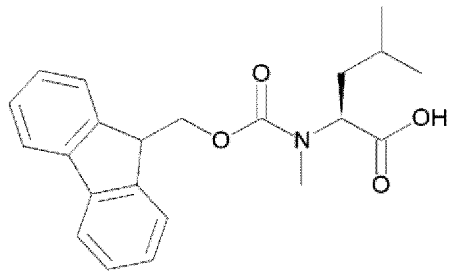 |
| **Fmoc-MeAla-OH** | 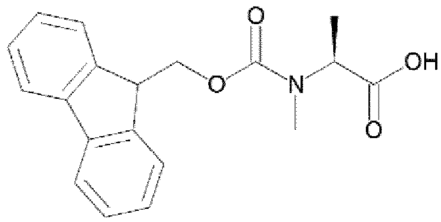 |
| **Fmoc-MePhe-OH** | 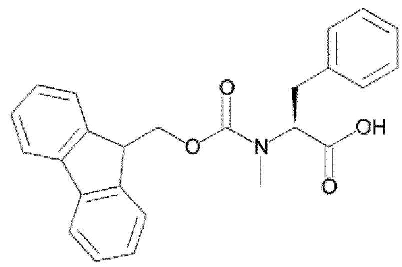 |
| **Fmoc-D-3-MeAbu-OH** | 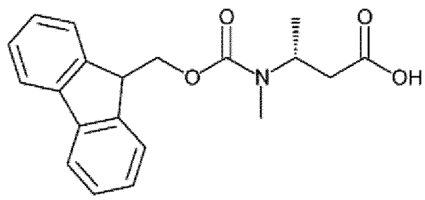 |

(continued)

| Abbreviation | Structure |
|---|---|
| **Fmoc-Lys (Boc)- Pro-OH** | 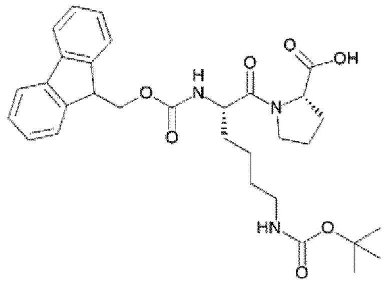 |
| **Fmoc-Ala-Ala-OH** | 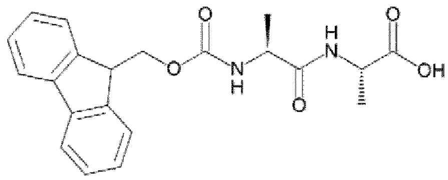 |
| **Fmoc-Asn (Trt)-Gly-OH** | 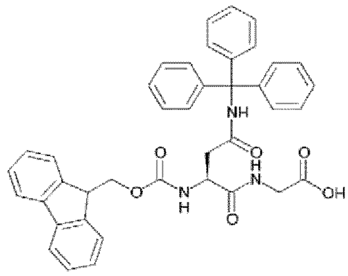 |
| | |
| **Abbreviation** | **Structure** |
| **Fmoc-Ser (tBu)- Gly-OH** | 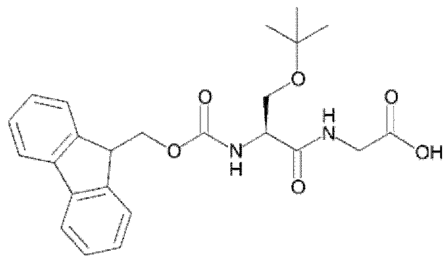 |
| **Fmoc-D-MeVal-OH** | 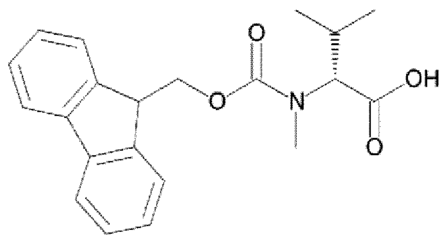 |

(continued)

| Abbreviation | Structure |
|---|---|
| **Fmoc-Phe-Pro-OH** | 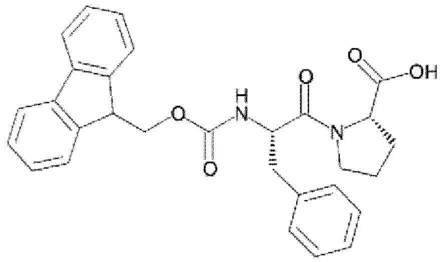 |
| **Fmoc-Gly-Tyr (tBu) - OH** | 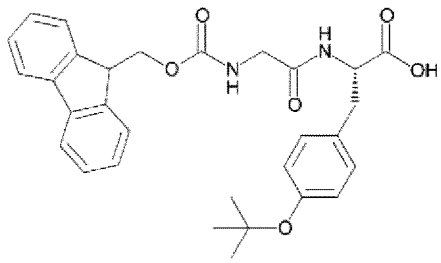 |
| **Fmoc-Phe-Gly-OH** | 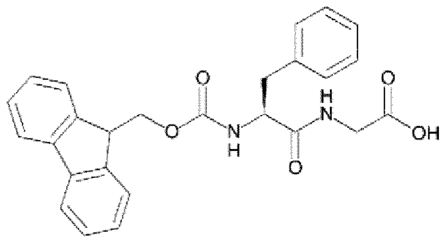 |
| **Fmoc-Gly-Val-OH** | 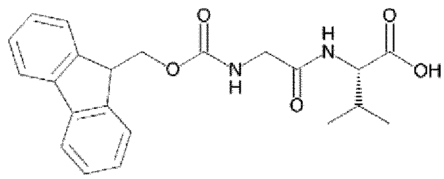 |
| | |
| **Abbreviation** | Structure |
| **Fmoc-Ala-Ala-Pro-OH** | 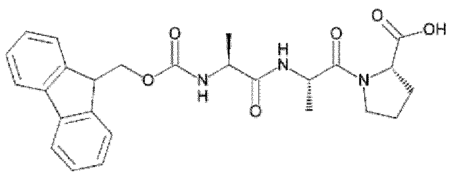 |

Example 1-2. Synthesis of Fmoc-dipeptide

The Fmoc-peptides shown in Table 3 were synthesized in accordance with the scheme shown below.

[Table 3]

| Compound No. | Abbreviation |
|---|---|
| aa2-001 | Fmoc-MeVal-MeAsp-pip |

(continued)

| Compound No. | Abbreviation |
|---|---|
| aa2-002 | Fmoc-MeIle-MeAsp-pip |
| aa2-003 | Fmoc-MeGly(cPent)-MeAsp-piρ |
| aa2-004 | Fmoc-MeChg-MeAsp-pip |
| aa2-005 | Fmoc-MeLeu-MeAsp-pip |
| aa2-006 | Fmoc-MeGly(cPent)-MeAsp-NMe2 |
| aa2-007 | Fmoc-MeLeu-MeAsp-NMe2 |
| aa2-008 | Fmoc-MeYal-D-3-MeAbu-OH |
| aa2-009 | Fmoc-MeChg-D-3-MeAbu-OH |
| aa2-010 | Fmoc-MeVal-MeGly-OH |
| aa2-011 | Fmoc-MeGly(cPent)-MeAsp-mor |
| aa2-012 | Fmoc-MeVal-Asp-NMe2 |
| aa2-013 | Fmoc-Gly-MeAsp-NMe2 |
| aa2-014 | Fmoc-Aib-MeAsp-NMe2 |
| aa2-015 | Fmoc-bAla(2-Me2)-D-3-MeAbu-OH |
| aa2-016 | Fmoc-MeLeu-MeVal-OH |
| aa2-017 | Fmoc-MeVal-Pro-OH |
| aa2-018 | Fmoc-MeLeu-Aib-OH |
| aa2-019 | Fmoo-MeVal-Gly-OH |
| aa2-020 | Fmoc-D-MeVal-MeAsp-NMe2 |
| aa2-021 | Fmoc-bAla(2-Me2)-MeAsp-NMe2 |
| aa2 022 | Fmoc-MeVal-D-MeAsp-NMe2 |
| aa2-023 | Fmoc-Ala-MeGly(cPent)-MeAsp-NMe2 |

Synthesis of compound aa2-001, Fmoc-MeVal-MeAsp-pip

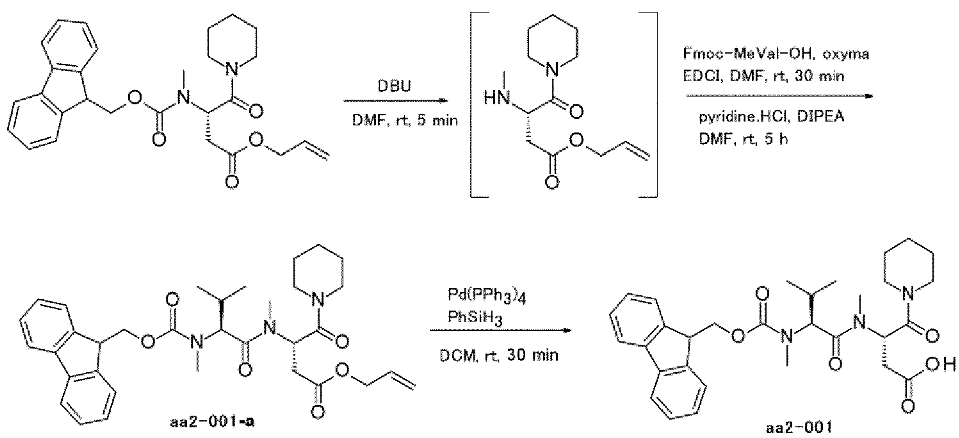


aa2-001-a

aa2-001

Synthesis of compound aa2-001-a, prop-2-enyl (3S)-3-[[(2S)-2-[9H-fluoren-9-ylmethoxycarbonyl(methyl)amino]-3-methylbutanoyl]-methylamino]-4-oxo-4-piperidin-1-ylbutanoate (Fmoc-MeVal-MeAsp(OAl)-pip)

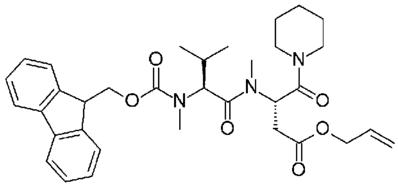

Fmoc-MeAsp(OAl)-pip as a starting raw material was synthesized in accordance with the method described in WO 2018/225864.

Under nitrogen gas flow, Fmoc-MeVal-OH (4.25 g, 12.0 mmol), EDCI (3.23 g, 16.8 mmol), oxyma (2.05 g, 14.4 mmol) and DMF (40 mL) were added into a reaction vessel. The reaction liquid was stirred for 30 minutes to obtain an active ester solution of Fmoc-MeVal-OH.

Under nitrogen gas flow, Fmoc-MeAsp(OAl)-pip (5.00 g, 10.5 mmol) and DMF (40 mL) were added into a reaction vessel, and DBU (1.60 g, 10.5 mmol) was then added at room temperature. The reaction liquid was stirred for 5 minutes, and pyridine hydrochloride (1.33 g, 11.5 mmol) was then added at room temperature under nitrogen gas flow. The reaction liquid was stirred for 10 minutes, the active ester solution of Fmoc-MeVal-OH prepared as described above and DIPEA (1.49 g, 11.5 mmol) were then added at room temperature under nitrogen gas flow, and the reaction liquid was stirred for 5 hours. The reaction liquid was concentrated under reduced pressure, and the obtained residue was purified by reverse-phase silica gel column chromatography (water/acetonitrile = 95/5 $\rightarrow$ 20/80) to obtain 3.03 g of compound aa2-001-a as a pink solid (yield: 48%).

LCMS (ESI) m/z = 590.6 $(M+H)^+$
Retention time: 1.06 min (analysis condition SQDFA05)

Synthesis of compound aa2-001, (3S)-3-[[(2S)-2-[9H-fluoren-9-ylmethoxycarbonyl(methyl)aminol-3-methylbutanoyl]-methylamino]-4-oxo-4-piperidin-1-ylbutanoic acid (Fmoc-MeVal-MeAsp-pip)

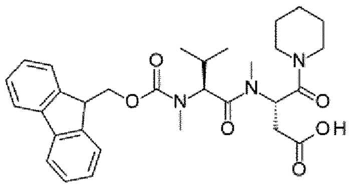

Under nitrogen gas flow, compound aa2-001-a (1.18 g, 2.00 mmol) and tetrakistriphenylphosphinepaladium (23.1 mg, 0.020 mmol) were added into a reaction vessel, and DCM (4.0 mL) was then added. To the reaction liquid phenylsilane (0.152 g, 1.40 mmol) was added dropwise, and the reaction liquid was then stirred at room temperature for 30 minutes. The reaction liquid was diluted with TBME (11.8 mL), and then extracted with a saturated aqueous sodium bicarbonate solution/water (1/1, 11.8 mL), and further extracted with water (5.9 mL). The combined aqueous layer was made acidic to a pH of about 2 by adding a 85% aqueous phosphoric acid solution (0.700 mL, 10.2 mmol) thereto, and the aqueous layer was extracted twice with DCM (11.8 mL). The combined organic layer was washed with a saturated aqueous sodium chloride solution/water (1/1, 11.8 mL), and the organic layer was then dried over sodium sulfate. The drying material was removed by filtration, and the filtrate was then concentrated under reduced pressure to obtain 1.06 g of compound aa2-001 as a light brown amorphous crystal (yield: 97%).

LCMS (ESI) m/z = 550.5 $(M+H)^+$
Retention time: 0.93 min (analysis condition SQDFA05)

Synthesis of compound aa2-002, Fmoc-MeIle-MeAsp-pip

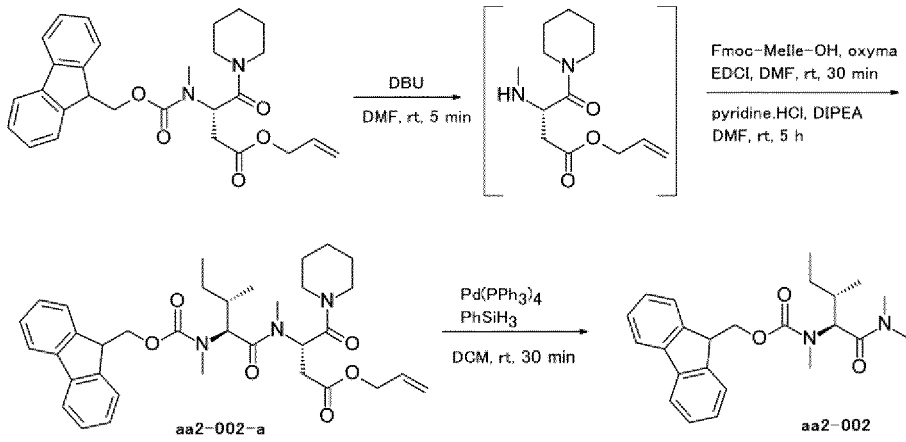


Synthesis of compound aa2-002-a, prop-2-enyl (3S)-3-[[(2S,3S)-2-[9H-fluoren-9-ylmethoxycarbonyl(methyl)amino]-3-methylpentanoyl]-methylamino]-4-oxo-4-piperidin-1-ylbutanoate (Fmoc-MeIle-MeAsp(OAl)-pip)

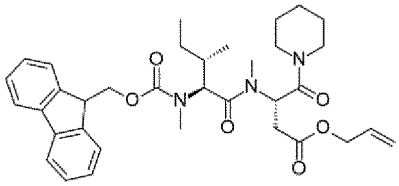

Compound Fmoc-MeAsp(OAl)-pip (10.0 g, 21.0 mmol) and Fmoc-MeIle-OH (6.80 g, 18.5 mmol) were provided as starting raw materials, and the same procedure as in the synthesis of compound aa2-001-a was carried out to obtain 3.64 g of compound aa2-002 as an orange solid (yield: 29%).

LCMS (ESI) m/z = 604.6 $(M+H)^+$
Retention time: 1.10 min (analysis condition SQDFA05)

Synthesis of compound aa2-002, (3S)-3-[[(2S,3S)-2-[9H-fluoren-9-ylmethoxycarbonyl(methyl)amino]-3-methylpentanoyl]-methylamino]-4-oxo-4-piperidin-1-ylbutanoic acid (Fmoc-MeIle-MeAsp-pip)

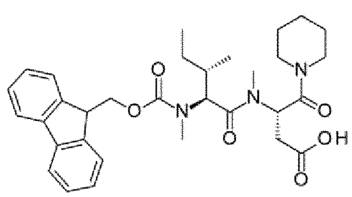

Compound aa2-002-a (1.21 g, 2.00 mmol) was provided as a starting raw material, and the same procedure as in the synthesis of compound aa2-001 was carried out to obtain 1.05 g of compound aa2-002 as a light brown amorphous crystal (yield: 93%).

LCMS (ESI) m/z = 564.7 $(M+H)^+$
Retention time: 0.99 min (analysis condition SQDFA05)

Synthesis of compound aa2-003, Fmoc-MeGly(cPent)-MeAsp-pip

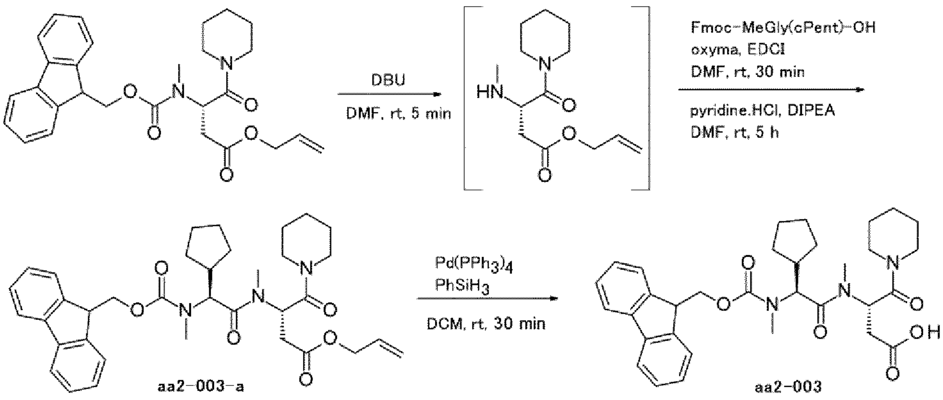


Synthesis of compound aa2-003-a, prop-2-enyl (3S)-3-[[(2S)-2-cyclopentyl-2-[9H-fluoren-9-ylmethoxycarbonyl(methyl)aminolacetyl]-methylamino]-4-oxo-4-piperidin-1-ylbutanoate(Fmoc-MeGly(cPent)-MeAsp(OAl)-pip)

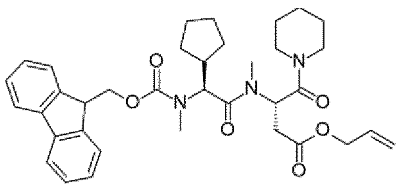

Compound Fmoc-MeAsp(OAl)-pip (10.0 g, 21.0 mmol) and Fmoc-MeGly(cPent)-OH (8.75 g, 23.1 mmol) were provided as starting raw materials, and the same procedure as in the synthesis of compound aa2-001-a was carried out to obtain 3.49 g of compound aa2-003-a as an orange solid (yield: 27%).

LCMS (ESI) m/z = 616.6 $(M+H)^+$
Retention time: 1.11 min (analysis condition SQDFA05)

Synthesis of compound aa2-003, (3S)-3-[[(2S)-2-cyclopentyl-2-[9H-fluoren-9-ylmethoxycarbonyl(methyl)aminolacetyl]-methylamino]-4-oxo-4-piperidin-1-ylbutanoate(Fmoc-MeGly(cPent)-MeAsp-pip)

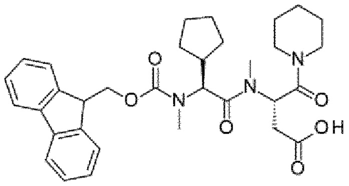

Compound aa2-003-a (1.23 g, 2.00 mmol) was provided as a starting raw material, and the same procedure as in the synthesis of compound aa2-001 was carried out to obtain 1.12 g of compound aa2-003 as a light brown amorphous crystal (yield: 97%).

LCMS (ESI) m/z = 576.8 $(M+H)^+$
Retention time: 0.95 min (analysis condition SQDFA05)

Synthesis of compound aa2-004, Fmoc-MeChg-MeAsp-pip

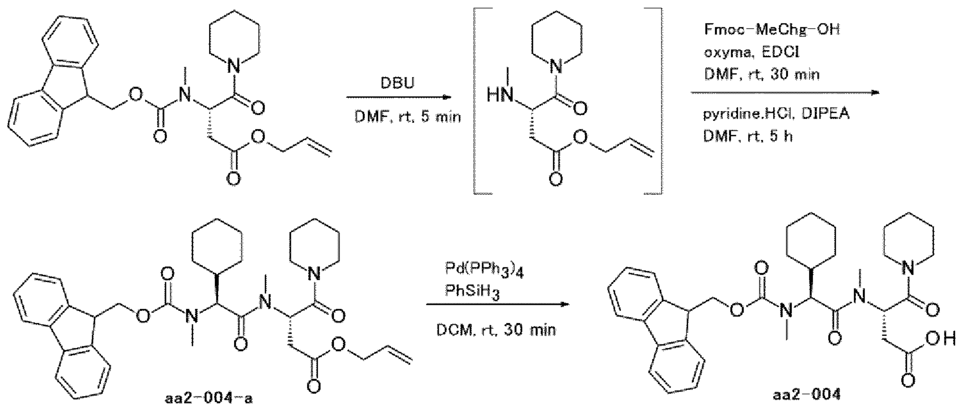


Synthesis of compound aa2-004-a, prop-2-enyl (3S)-3-[[(2S)-2-cyclohexyl-2-[9H-fluoren-9-ylmethoxycarbonyl(methyl)aminolacetyl]-methylamino]-4-oxo-4-piperidin-1-ylbutanoate(Fmoc-MeChg-MeAsp(OAl)-pip)

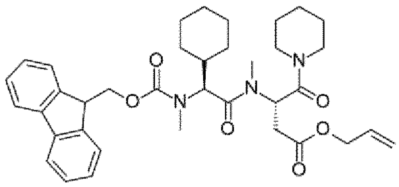

Compound Fmoc-MeAsp(OAl)-pip (10.0 g, 21.0 mmol) and Fmoc-MeChg-OH (9.10 g, 23.1 mmol) were provided as starting raw materials, and the same procedure as in the synthesis of compound aa2-001-a was carried out to obtain 5.52 g of compound aa2-004-a as a yellow solid (yield: 42%).

LCMS (ESI) m/z = 630.6 $(M+H)^+$
Retention time: 1.13 min (analysis condition SQDFA05)

Synthesis of compound aa2-004, (3S)-3-[[(2S)-2-cyclohexyl-2-[9H-fluoren-9-ylmethoxycarbonyl(methyl)aminolacetyl]-methylamino]-4-oxo-4-piperidin-1-ylbutanoate(Fmoc-MeChg-MeAsp-pip)

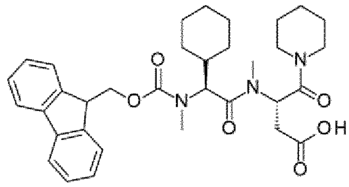

Compound aa2-004-a (1.26 g, 2.00 mmol) was provided as a starting raw material, and the same procedure as in the synthesis of compound aa2-001 was carried out to obtain 0.953 g of compound aa2-004 as a light brown amorphous crystal (yield: 81%).

LCMS (ESI) m/z = 590.6 $(M+H)^+$
Retention time: 0.97 min (analysis condition SQDFA05)

Synthesis of compound aa2-005, Fmoc-MeLeu-MeAsp-pip

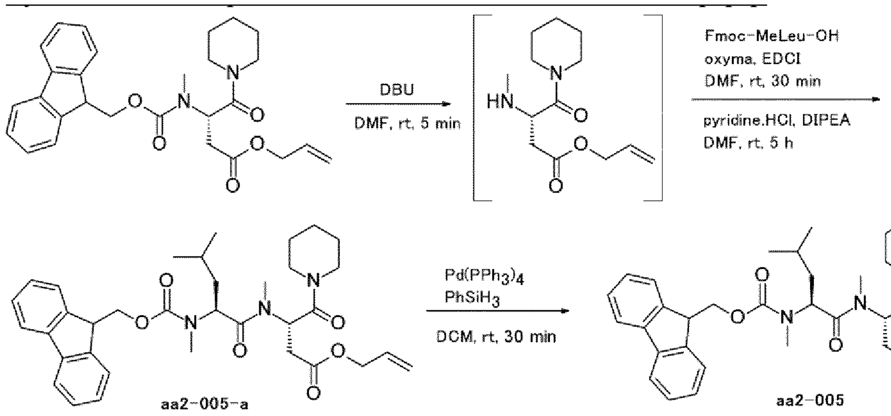


Synthesis of compound aa2-005-a, prop-2-enyl (3S)-3-[[(2S)-2-[9H-fluoren-9-ylmethoxycarbonyl(methyl)amino]-4-methylpentanoyl]-methylamino]-4-oxo-4-piperidin-1-ylbutanoate

(Fmoc-MeLeu-MeAsp(OAl)-pip)

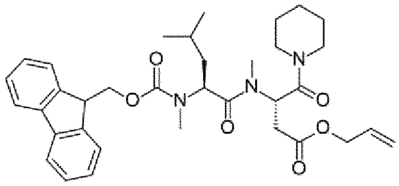

Compound Fmoc-MeAsp(OAl)-pip (8.00 g, 16.79 mmol) and Fmoc-MeLeu-OH (6.48 g, 17.63 mmol) were provided as starting raw materials, and the same procedure as in the synthesis of compound aa2-001-a was carried out to obtain 5.00 g of compound aa2-005-a as a yellow amorphous crystal (yield: 49%).

LCMS (ESI) m/z = 604.6 $(M+H)^+$
Retention time: 1.10 min (analysis condition SQDFA05)

Synthesis of compound aa2-005, (3S)-3-[[(2S)-2-[9H-fluoren-9-ylmethoxycarbonyl(methyl)amino]-4-methylpentanoyl]-methylamino]-4-oxo-4-piperidin-1-ylbutanoic acid (Fmoc-MeLeu-MeAsp-pip)

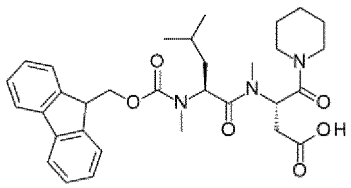

Compound aa2-005-a (1.21 g, 2.00 mmol) was provided as a starting raw material, and the same procedure as in the synthesis of compound aa2-001 was carried out to obtain 1.09 g of compound aa2-005 as a light brown amorphous crystal (yield: 96%).

LCMS (ESI) m/z = 564.5 $(M+H)^+$
Retention time: 0.94 min (analysis condition SQDFA05)

Synthesis of compound aa2-006, Fmoc-MeGly(cPent)-MeAsp-NMe2

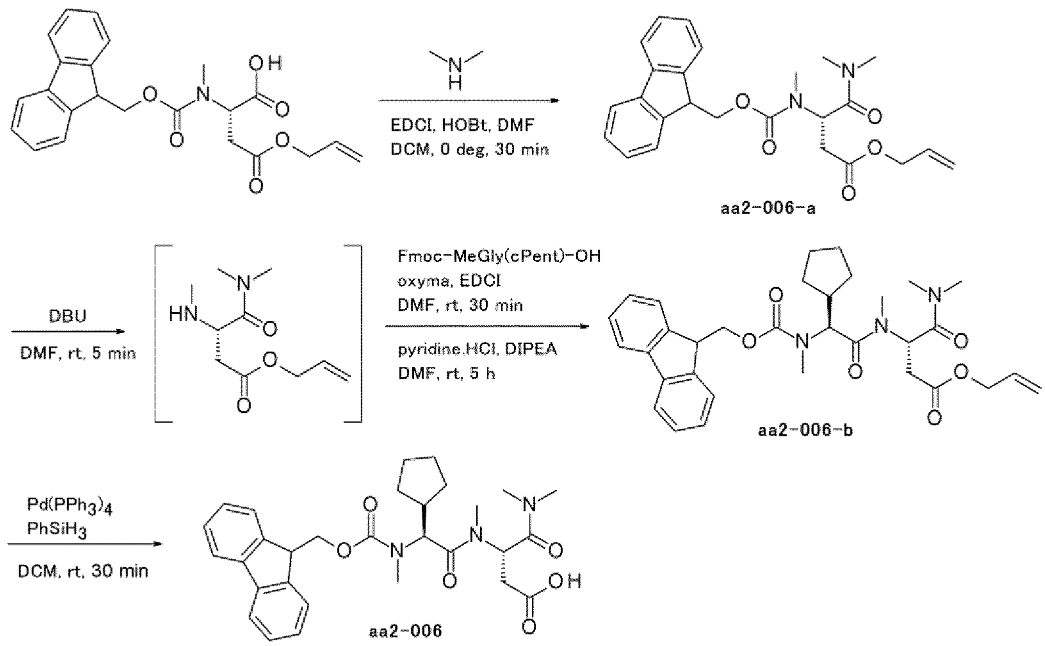


Synthesis of compound aa2-006-a, prop-2-enyl (3S)-4-(dimethylamino)-3-r9H-fluoren-9-ylmethoxycarbonyl(methyl)amino]-4-oxobutanoate (Fmoc-MeAsp(OAl)-NMe2)

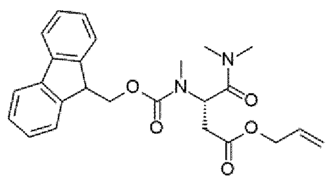

Fmoc-MeAsp(OAl)-OH as a starting raw material was synthesized in accordance with the method described in WO 2018/225864. Under nitrogen gas flow, EDCI (16.9 g, 88.0 mmol) and DMF (144 mL) were added into a reaction vessel, and cooled to 0°C. Solutions of HOBt (10.9 g, 80.6 mmol) and Fmoc-MeAsp(OAl)-OH (30.0 g, 73.3 mmol) in DCM/DMF (60 mL/60 mL) were sequentially added at 0°C, and the mixture was stirred at 0°C for 30 minutes. Dimethylamine (2 mol/L THF solution, 40.5 mL, 80.6 mmol) was added dropwise at 0°C, and the mixture was then stirred at 0°C for 30 minutes. To the reaction liquid was added ethyl acetate (300 mL), the organic layer was sequentially washed twice with a 1 mol/L aqueous hydrochloric acid solution (240 mL), twice with water (300 mL), twice with a saturated aqueous sodium bicarbonate solution/water (1/1, 300 mL), and with a saturated aqueous sodium chloride solution/water (1/1, 300 mL), and the organic layer was then dried over sodium sulfate. The drying material was removed by filtration, and the filtrate was then concentrated under reduced pressure to obtain 32.7 g of compound aa2-006-a as a light brown amorphous crystal (yield: 102%).

LCMS (ESI) m/z = 437.2 $(M+H)^+$
Retention time: 0.86 min (analysis condition SQDFA05)

Synthesis of compound aa2-006-b, prop-2-enyl (3S)-3-[[(2S)-2-cyclolpentyl-2-[9H-fluoren-9-ylmethoxycarbonyl(methyl)aminolacelyll -methyl amino] -4-(dimethylamino)-4-oxobutanoate (Fmoc-MeGly(cPent)-MeAsp(OAl)-NMe2)

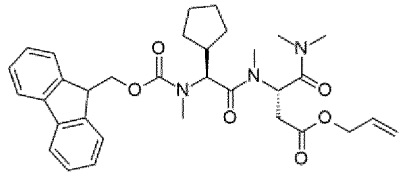

Compound aa2-006-a (8.50 g, 19.5 mmol) and Fmoc-MeGly(cPent)-OH (7.76 g, 20.5 mmol) were provided as starting raw materials, and the same procedure as in the synthesis of compound aa2-001-a was carried out to obtain 5.02 g of compound aa2-006-b as a yellow amorphous crystal (yield: 43%).

LCMS (ESI) m/z = 576.5 $(M+H)^+$
Retention time: 1.02 min (analysis condition SQDFA05)

Synthesis of compound aa2-006, (3S)-3-[[(2S)-2-cyclolpenlyl-2-[9H-fluoren-9-ylmethoxycarbonyl(methyl)amino]acelyl]-methylamino]-4-(dimethylamino)-4-oxobutanoic acid (Fmoc-MeGly(cPent)-MeAsp-NMe2)

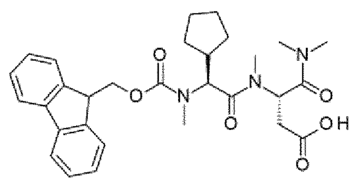

Compound aa2-006-b (0.921 g, 1.60 mmol) was provided as a starting raw material, and the same procedure as in the synthesis of compound aa2-001 was carried out to obtain 0.786 g of compound aa2-006 as a light brown amorphous crystal (yield: 92%).

LCMS (ESI) m/z = 536.5 $(M+H)^+$
Retention time: 0.85 min (analysis condition SQDFA05)

Synthesis of compound aa2-007, Fmoc-MeLeu-MeAsp-NMe2

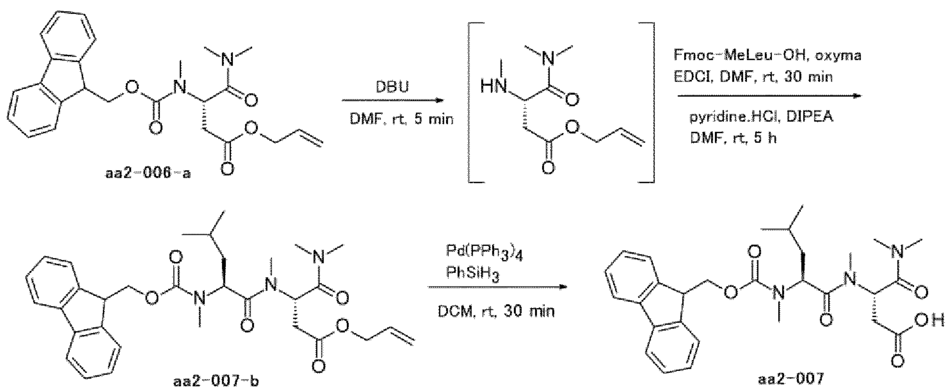

aa2-006-a
aa2-007-b
aa2-007

Synthesis of compound aa2-007-b, prop-2-enyl (3S)-4-(dimethylamino)-3-[[(2S)-2-[9H-fluoren-9-ylmethoxycarbonyl(methyl)amino]-4-methylpentanoyl]-methylamino]-4-oxobutanoate(Fmoc-MeLeu-MeAsp(OAl)-NMe2)

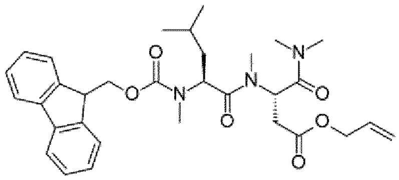

Compound aa2-006-a (8.50 g, 19.5 mmol) and Fmoc-MeLeu-OH (7.76 g, 20.5 mmol) were provided as starting raw materials, and the same procedure as in the synthesis of compound aa2-001-a was carried out to obtain 5.00 g of compound aa2-007-b as a yellow amorphous crystal (yield: 48%).

LCMS (ESI) m/z = 564.5 $(M+H)^+$
Retention time: 1.02 min (analysis condition SQDFA05)

Synthesis of compound aa2-007, (3S)-4-(dimethylamino)-3-[[(2S)-2-[9H-fluoren-9-ylmethoxycarbonyl(methyl)amino]-4-methylpentanoyl]-methylamino]-4-oxobutanoic acid (Fmoc-MeLeu-MeAsp-NMe2)

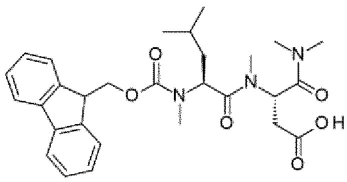

Compound aa2-007-b (1.13 g, 2.00 mmol) was provided as a starting raw material, and the same procedure as in the synthesis of compound aa2-001 was carried out to obtain 0.995 g of compound aa2-007 as a light brown amorphous crystal (yield: 95%).

LCMS (ESI) m/z = 524.5 $(M+H)^+$
Retention time: 0.86 min (analysis condition SQDFA05)

Synthesis of compound aa2-008, Fmoc-MeVal-D-3-MeAbu-OH

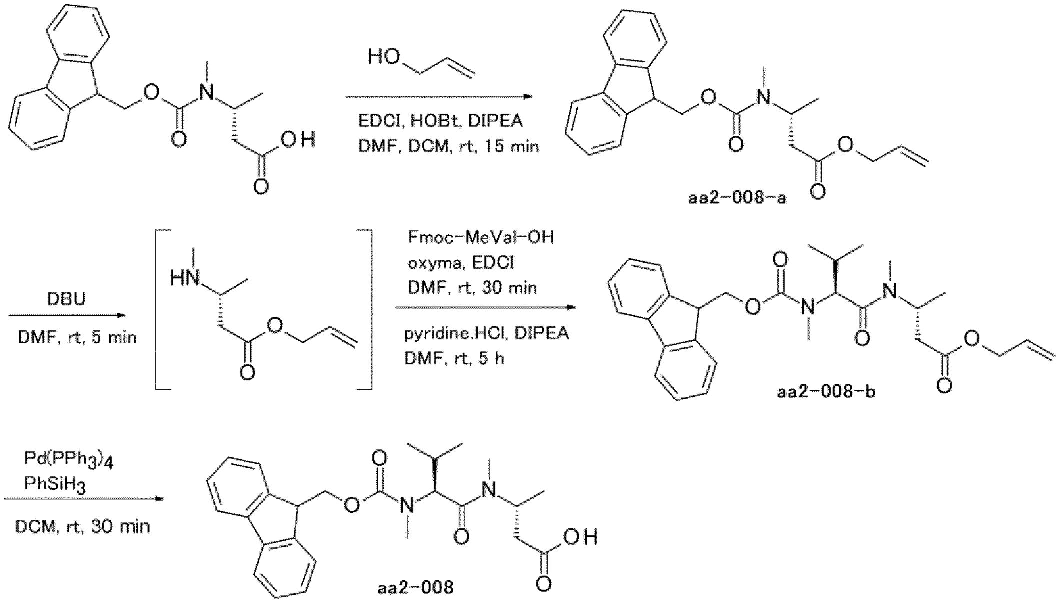


Synthesis of compound aa2-008-a, prop-2-enyl (3R)-3-[9H-fluoren-9-ylmethoxycarbonyl(methyl)aminolbutanoate (Fmoc-D-3-MeAbu(OAl)

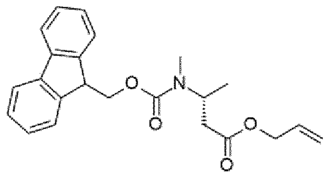

Under nitrogen gas flow, Fmoc-D-3-MeAbu-OH (20.0 g, 59.0 mmol), EDCI (17.0 g, 88.5 mmol), HOBt (13.6 g, 88.5 mmol), allyl alcohol (8.1 mL, 118 mmol), DMF (140 mL) and DCM (40 mL) were sequentially added into a reaction vessel at 0°C, and stirred at 0°C for 30 minutes. Subsequently, DIPEA (15.4 mL, 88.5 mmol) was added, and the mixture was stirred at 0°C for 30 minutes, and then stirred at room temperature for 15 minutes. To the reaction liquid was added ethyl acetate (200 mL), the organic layer was washed with a saturated aqueous sodium bicarbonate solution (200 mL), and the organic layer was then dried over sodium sulfate. The drying material was removed by filtration, and the filtrate was then concentrated under reduced pressure to obtain 22.1 g of a crude product of compound aa2-008-a as a yellow oily material (yield: 99%).

LCMS (ESI) m/z = 380.1 $(M+H)^+$
Retention time: 2.12 min (analysis condition SMDmethod_1)

Synthesis of compound aa2-008-b, prop-2-enyl (3R)-3-[[(2S)-2-[9H-fluoren-9-ylmethoxycarbonyl(methyl)amino]-3-methylbutanoyl]-methylamino]butanoate (Fmoc-MeVal-D-3-MeAbu-OA1)

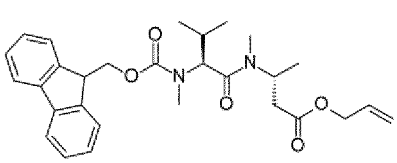

Compound aa2-008-a (6.00 g, 15.8 mmol) and Fmoc-MeVal-OH (5.82 g, 16.62 mmol) were provided as starting raw materials, and the same procedure as in the synthesis of compound aa2-001-a was carried out to obtain 3.79 g of

compound aa2-008-b as a brown amorphous crystal (yield: 49%).

LCMS (ESI) m/z = 493.5 $(M+H)^+$
Retention time: 1.03 min (analysis condition SQDFA05)

Synthesis of compound aa2-008, (3R)-3-[[(2S)-2-[9H-fluoren-9-ylmethoxycarbonyl(methyl)amino]-3-methylbutanoyl]-methylamino]butanoic acid (Fmoc-MeVal-D-3-MeAbu-OH)

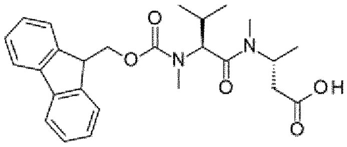

Compound aa2-008-b (0.788 g, 1.60 mmol) was provided as a starting raw material, and the same procedure as in the synthesis of compound aa2-001 was carried out to obtain 0.607 g of compound aa2-008 as a light brown amorphous crystal (yield: 84%).

LCMS (ESI) m/z = 453.4 $(M+H)^+$
Retention time: 0.85 min (analysis condition SQDFA05)

Synthesis of compound aa2-009, Fmoc-MeChg-D-3-MeAbu-OH

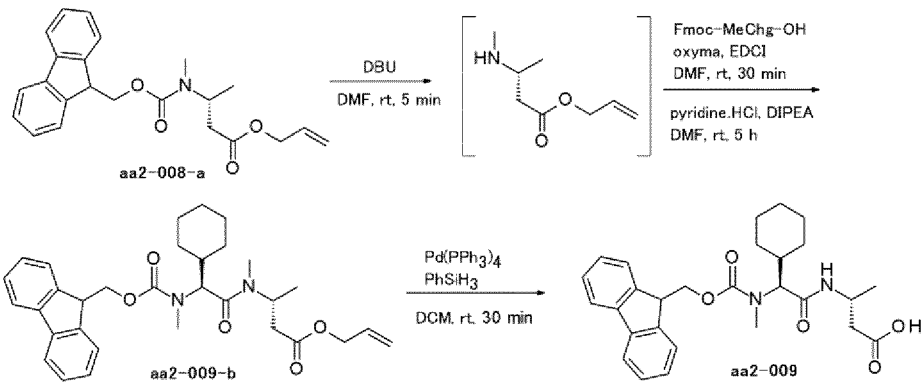


Synthesis of compound aa2-009-b, prop-2-enyl (3R)-3-[[(2S)-2-cyclohexyl-2-[9H-fluoren-9-ylmethoxycarbonyl(methyl)amino]acetyl]-methylamino]butanoate (Fmoc-MeChg-D-3-MeAbu-OAl)

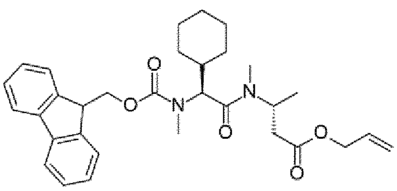

Compound aa2-008-a (4.70 g, 12.4 mmol) and Fmoc-MeChg-OH (5.10 g, 13.0 mmol) were provided as starting raw materials, and the same procedure as in the synthesis of compound aa2-001-a was carried out to obtain 3.70 g of compound aa2-009-b as a yellow oily material (yield: 56%).

LCMS (ESI) m/z = 533.6 $(M+H)^+$
Retention time: 1.11 min (analysis condition SQDFA05)

Synthesis of compound aa2-009, (3R)-3-[[(2S)-2-cyclohexyl-2-[9H-fluoren-9-ylmethoxycarbonyl(methyl)amino]acetyl]-methylamino]butanoic acid (Fmoc-MeChg-D-3-MeAbu-OH)

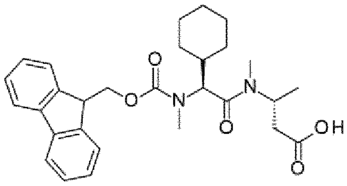

Compound aa2-009-b (0.799 g, 1.50 mmol) was provided as a starting raw material, and the same procedure as in the synthesis of compound aa2-001 was carried out to obtain 0.501 g of compound aa2-009 as a light brown amorphous crystal (yield: 68%).

LCMS (ESI) m/z = 493.5 $(M+H)^+$
Retention time: 0.95 min (analysis condition SQDFA05)

Synthesis of compound aa2-010, Fmoc-MeVal-MeGly-OH

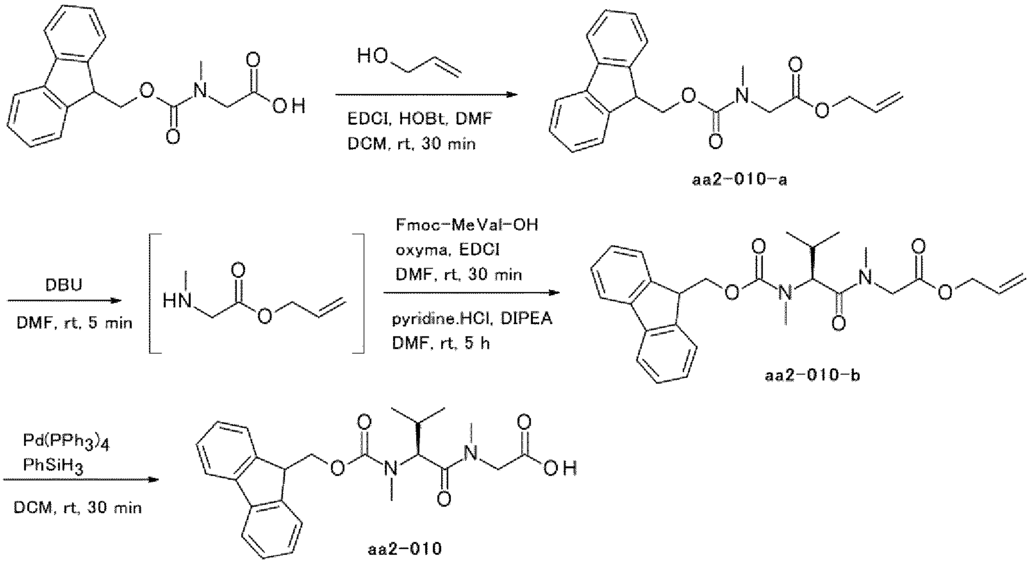


Synthesis of compound aa2-010-a, prop-2-enyl 2-[9H-fluoren-9-ylmethoxycarbonyl(methyl)amino]butanoate (Fmoc-MeGly-OAl)

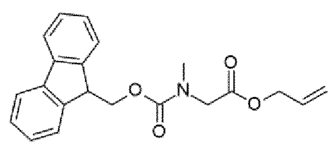

Under nitrogen gas flow, Fmoc-MeGly-OH (6.00 g, 19.3 mmol), EDCI (4.43 g, 23.1 mmol), HOBt (3.12 g, 23.1

mmol), allyl alcohol (1.23 mL, 21.2 mmol), DMF (40 mL) and DCM (12 mL) were added sequentially into a reaction vessel, and stirred at room temperature for 30 minutes. To the reaction liquid was added ethyl acetate (60 mL), the organic layer was sequentially washed with a 1 mol/L aqueous hydrochloric acid solution (60 mL), a saturated aqueous sodium bicarbonate solution (60 mL) and a saturated aqueous sodium chloride solution (60 mL), and the organic layer was concentrated under reduced pressure to obtain 6.00 g of a crude product of compound aa2-010-a as a yellow oily material (yield: 89%).

LCMS (ESI) m/z = 352.1 $(M+H)^+$
Retention time: 1.75 min (analysis condition SMDmethod_2)

Synthesis of compound aa2-010-b, prop-2-enyl 2-[[(2S)-2-[9H-fluoren-9-ylmethoxycarbonyl(methyl)amino]-3-methylbutanoyl]-methylamino]acetate (Fmoc-MeVal-MeGly-OAl)

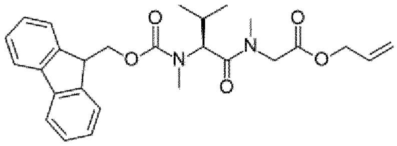

Compound aa2-010-a (6.00 g, 17.07 mmol) and Fmoc-MeVal-OH (6.63 g, 18.76 mmol) were provided as starting raw materials, and the same procedure as in the synthesis of compound aa2-001-a was carried out to obtain 3.37 g of compound aa2-010-b as a pink amorphous crystal (yield: 43%).

LCMS (ESI) m/z = 465.5 $(M+H)^+$
Retention time: 1.00 min (analysis condition SQDFA05)

Synthesis of compound aa2-010, 2-[[(2S)-2-[9H-fluoren-9-ylmethoxycarbonyl(methyl)amino]-3-methylbutanoyl]-methylamino]acetic acid (Fmoc-MeVal-MeGly-OH)

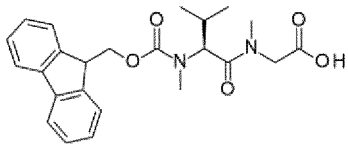

Compound aa2-010-b (0.929 g, 2.00 mmol) was provided as a starting raw material, and the same procedure as in the synthesis of compound aa2-001 was carried out to obtain 0.841 g of compound aa2-010 as a light brown amorphous crystal (yield: 99%).

LCMS (ESI) m/z = 425.4 $(M+H)^+$
Retention time: 0.83 min (analysis condition SQDFA05)

Synthesis of compound aa2-011, Fmoc-MeGly(cPent)-MeAsp-mor

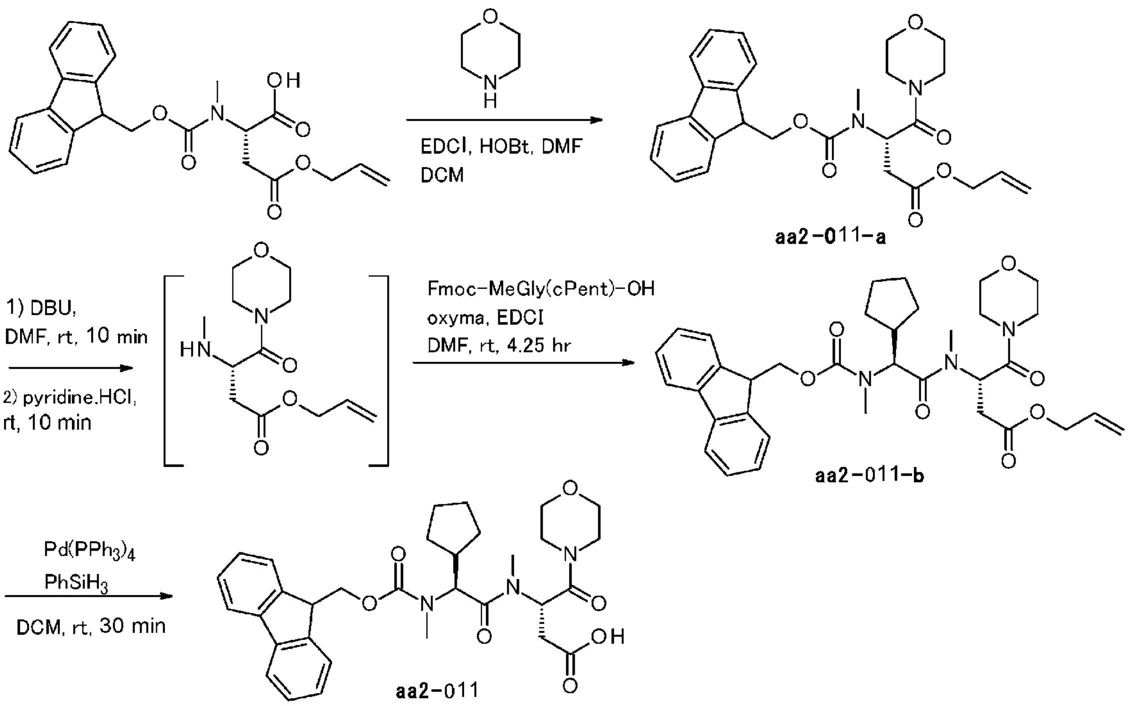


Synthesis of compound aa2-011-a, allyl (3S)-3-[9H-fluoren-9-ylmethoxycarbonyl(methyl)amino]-4-morpholino-4-oxo-butanoate (Fmoc-MeAsp(OAl)-mor)

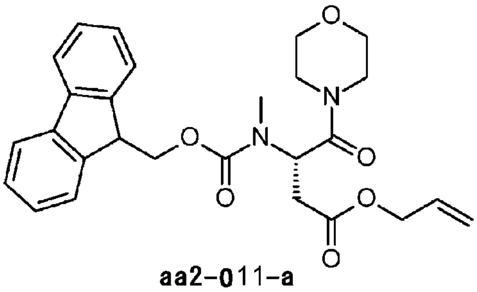

aa2-011-a

Fmoc-MeAsp(OAl)-OH as a starting raw material was synthesized in accordance with the method described in WO 2018/225864. The condensation reaction of the carboxyl group with morpholine was carried out by using morpholine instead of dimethylamine used for synthesis of compound aa2-006-a.

Synthesis of compound aa2-011-b, allyl (3S)-3-[[(2S)-2-cyclopentyl-2-[9H-fluoren-9-ylmethoxycarbonyl(methyl)aminolacetyl]-methylamino]-4-morpholino-4-oxobutanoate (Fmoc-MeGly(cPent)-MeAsp)(OAl)-mor)

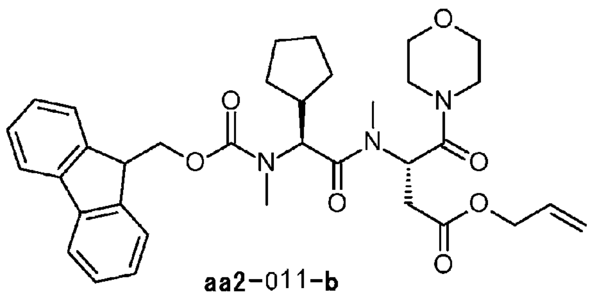

aa2-011-b

Under nitrogen atmosphere, DBU (3.15 mL, 20.90 mmol) was added dropwise to a solution of compound aa2-011-a (10 g, 20.90 mmol) in dehydrated DMF (50 mL) at room temperature, and the mixture was stirred for 10 minutes. To the reaction mixture was added pyridine hydrochloride (2.66 g, 22.99 mmol) at room temperature, and the mixture was stirred for 10 minutes. To this reaction mixture was added a separately prepared active ester solution (described later) at room temperature, followed by dropwise addition of DIPEA (4.01 mL, 22.99 mmol). The obtained reaction mixture was stirred at room temperature for 4 hours and 15 minutes, and ethyl acetate (100 mL), hexane (20 mL) and a 1 mol/L aqueous hydrochloric acid solution (100 mL) were then added. The obtained mixture was extracted with ethyl acetate-hexane (5 : 1) (total amount of organic phase: about 300 mL), the organic layer was washed with a 1 mol/L aqueous hydrochloric acid solution (100 mL), water (100 mL), an aqueous sodium bicarbonate solution (100 mL x 2), and a saturated aqueous sodium chloride solution (100 mL), and dried over sodium sulfate, and the solvent was then removed by distillation under reduced pressure. The obtained crude product was purified by normal-phase silica gel chromatography (hexane-ethyl acetate) to obtain 9.98 g of compound aa2-011-b (yield: 81%).

The active ester solution was prepared in the following manner. Under nitrogen atmosphere, dehydrated DMF (55 mL) was added to a mixture of (2S)-2-cyclopentyl-2-[9H-fluoren-9-ylmethoxycarbonyl(methyl)amino]acetic acid (7.53 g, 19.85 mmol), WSCI·HCl (5.21 g, 27.2 mmol) and Oxyma (3.56 g, 25.08 mmol) at room temperature, and the mixture was stirred for 40 minutes. The reaction mixture thus obtained was used as an active ester solution.

LCMS (ESI) m/z = 618 $(M+H)^+$
Retention time: 0.96 min (analysis condition SQDFA05)

Synthesis of compound aa2-011, (3S)-3-[[(2S)-2-cyclopentyl-2-[9H-fluoren-9-ylmethoxycarbonyl(methyl)aminolacetyl]-methylamino]-4-morpholino-4-oxobutanoic acid (Fmoc-MeGly(cPent)-MeAsp-mor)

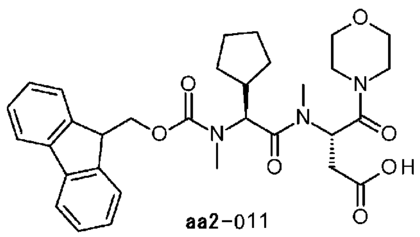

aa2-011

Under nitrogen atomosphere, dehydrated DCM (15 ml) was added to a mixture of compound aa2-011-b (9.90 g, 16.03 mmol) and tetrakis(triphenylphosphine)palladium (0) (0.185 g, 0.16 mmol), and the mixture was stirred at room temperature. To the obtained solution, phenylsilane (1.38 mL, 11.22mmol) was added dropwise. The obtained reaction mixture was stirred for 30 minutes, MTBE (100 mL) was then added, and an aqueous sodium bicarbonate solution (100 mL) (saturated aqueous solution diluted by 2 times) was then slowly added dropwise to stop the reaction. The obtained mixture was extracted twice with water (total amount of aqueous phase: about 150 mL), and to the aqueous phase was added DCM (100 mL) and phosphoric acid (5.6 mL). The obtained mixture was extracted twice with DCM (total amount of organic phase: about 200 ml), the organic phase was washed with a saturated aqueous sodium chloride solution (80 mL x 2), and dried over sodium sulfate, and the solvent was then removed by distillation under reduced pressure to obtain 9.57 g of compound aa2-011 (yield: 100%). The compound was used for the subsequent reaction.

LCMS (ESI) m/z = 578 $(M+H)^+$
Retention time: 0.79 min (analysis condition SQDFA05)

Synthesis of compound aa2-012, Fmoc-MeVal-Asp-NMe2

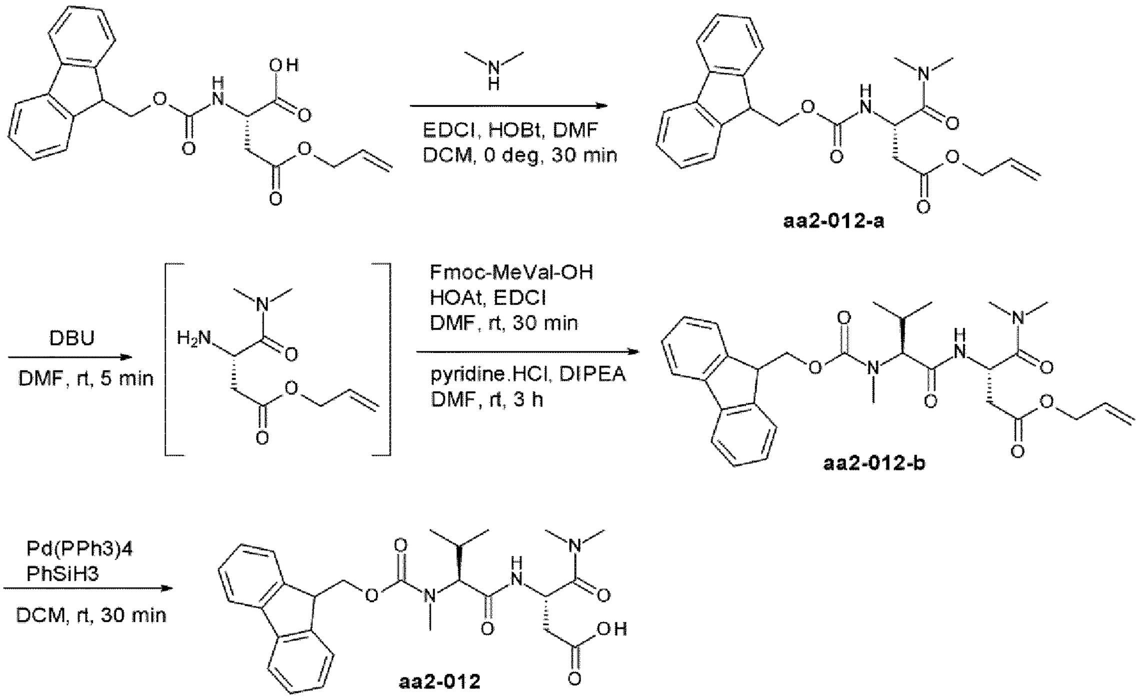


Synthesis of compound aa2-012-a, prop-2-enyl (3S)-4-(dimethylamino)-3-(9H-fluoren-9-ylmethoxycarbonylamino)-4-oxobutanoate (Fmoc-Asp(OAl)-NMe2)

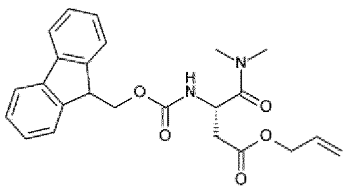

Fmoc-Asp(OAl)-OH (100.0 g, 252.9 mmol) purchased from a commercial supplier was provided as a starting raw material, and the same procedure as in the synthesis of compound aa2-006-a was carried out to obtain 89 g of compound aa2-012-a as a yellow oily material (yield: 82%).

LCMS (ESI) m/z = 423.2 $(M+H)^+$
Retention time: 2.24 min (analysis condition SMDmethod_3)

Synthesis of compound aa2-012-b, prop-2-enyl (3S)-4-(dimethylamino)-3-[[(2S)-2-[9H-fluoren-9-ylmethoxycarbonyl(methyl)amino]-3-methylbutanoyl]amino]-4-oxobutanoate (Fmoc-MeVal-Asp(OAl)-NMe2

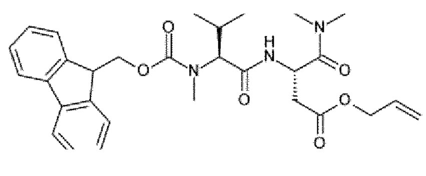

Compound aa2-012-a (5.0 g, 11.8 mmol) and Fmoc-MeVal-OH (4.39 g, 12.4 mmol) were provided as starting raw materials, and the same procedure as in the synthesis of compound aa2-001-a was carried out to obtain 3.4 g of compound aa2-012-b as a gray-white solid (yield: 53%).

LCMS (ESI) m/z = 536.5 $(M+H)^+$
Retention time: 0.94 min (analysis condition SQDFA05)

Synthesis of compound aa2-012, (3S)-4-(dimethylamino)-3-[[(2S)-2-[9H-fluoren-9-ylmethoxycarbonyl(methyl)amino1-3-methylbutanoyllamino1-4-oxobutanoic acid (Fmoc-MeVal-Asp-NMe2)

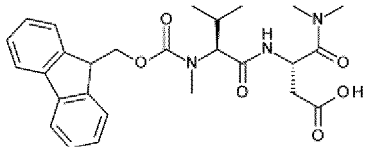

Compound aa2-012-b (1.16 g, 2.17 mmol) was provided as a starting raw material, and the same procedure as in the synthesis of compound aa2-001 was carried out to obtain 1.02 g of compound aa2-012 as a white amorphous crystal (yield: 95%).

LCMS (ESI) m/z = 496.5 $(M+H)^+$
Retention time: 0.78 min (analysis condition SQDFA05)

Synthesis of compound aa2-013, Fmoc-Gly-MeAsp-NMe2

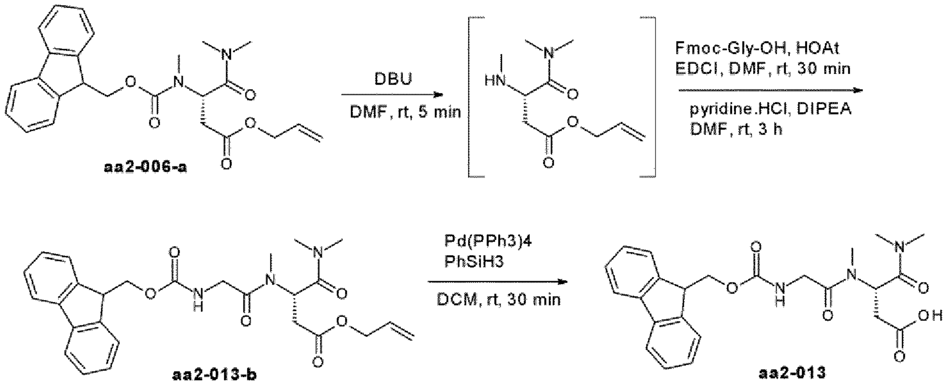


Synthesis of compound aa2-013-b, prop-2-enyl (3S)-4-(dimethylamino)-3-[[2-(9H-fluoren-9-ylmethoxycarbonylamino)acelyl]methylamino]-4-oxobutanoate (Fmoc-Gly-MeAsp(OAl)-NMe2)

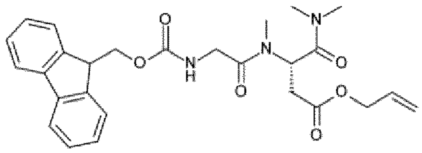

Compound aa2-006-a (5.0 g, 11.5 mmol) and Fmoc-Gly-OH (7.15 g, 24.1 mmol) were provided as starting raw materials, and the same procedure as in the synthesis of compound aa2-001-a was carried out to obtain 3.9 g of compound aa2-013-b as a yellow amorphous crystal (yield: 68%).

LCMS (ESI) m/z = 494.5 $(M+H)^+$
Retention time: 0.81 min (analysis condition SQDFA05)

Synthesis of compound aa2-013, (3S)-4-(dimethylamino)-3-[[2-(9H-fluoren-9-ylmethoxycarbonylamino)acetyl]methylamino]-4-oxobutanoic acid (Fmoc-Gly-MeAsp-NMe2)

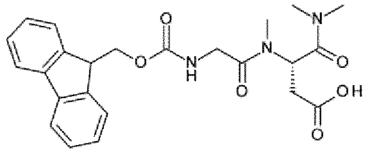

Compound aa2-013-b (1.07 g, 2.17 mmol) was provided as a starting raw material, and the same procedure as in the synthesis of compound aa2-001 was carried out to obtain 1.01 g of compound aa2-013 as a light brown amorphous crystal (yield: 103%).

LCMS (ESI) m/z = 454.4 $(M+H)^+$
Retention time: 0.67 min (analysis condition SQDFA05)

Synthesis of compound aa2-014, Fmoc-Aib-MeAsp-NMe2

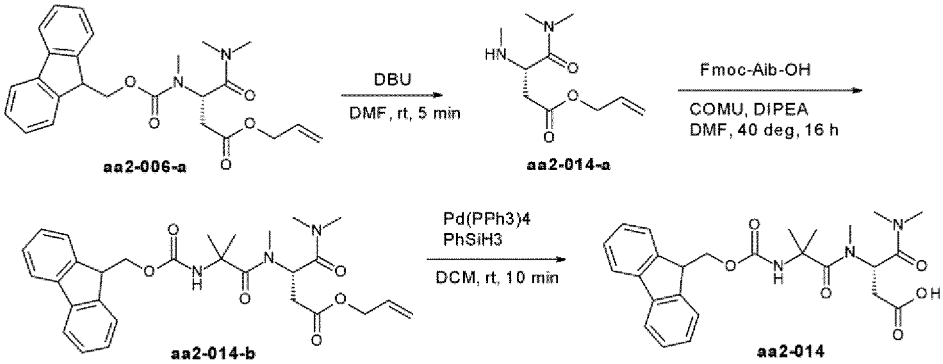

aa2-006-a aa2-014-a aa2-014-b aa2-014

Synthesis of compound aa2-014-a, prop-2-enyl (3S)-4-(dimethylamino)-3-(methylamino)-4-oxobutanoate (H-MeAsp(OAl)-NMe2)

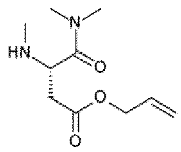

Compound aa2-006-a (18.0 g, 41.2 mmol) and DCM (180 mL) were added into a reaction vessel, and DBU (6.28 g, 41.2 mmol) was then added at room temperature. The reaction liquid was stirred for 5 minutes, the reaction liquid was then concentrated under reduced pressure, and the obtained residue was purified by normal-phase silica gel column chromatography (petroleum ether/ethyl acetate = 100/0 -> 0/100, and subsequently DCM/methanol = 100/0 → 85/15) to obtain 8.1 g of compound aa2-014-a as a yellow oily material (yield: 87%).

LCMS (ESI) m/z = 215.2 $(M+H)^+$
Retention time: 0.41 min (analysis condition SMDmethod_4)

Synthesis of compound aa2-014-b, prop-2-enyl (3S)-4-(dimethylamino)-3-[[2-[9H-fluoren-9-ylmethoxycarbonylamino)-2-methylpropanoyl]-methylamino]-4-oxobutanoate (Fmoc-Aib-MeAsp)(OAl)-NMe2)

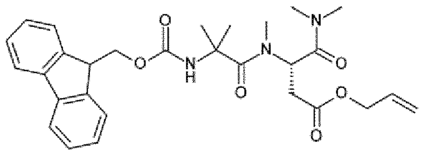

Under nitrogen gas flow, compound aa2-014-a (4.35 g, 20.3 mmol), Fmoc-Aib-OH (6.0 g, 18.4 mmol), COMU (11.8 g, 27.7 mmol) and DMF (60 mL) were added into a reaction vessel, and DIPEA (2.38 g, 18.4 mmol) was then added dropwise at 0°C. The reaction liquid was stirred at 40°C for 16 hours. Thereafter, to the reaction liquid was added ethyl acetate (120 mL), the organic layer was sequentially washed twice with a 1 mol/L aqueous hydrochloric acid solution (60 mL), once with water (60 mL), twice with a saturated aqueous sodium bicarbonate solution (60 mL), and with a saturated aqueous sodium chloride solution (60 mL), and the organic layer was then dried over sodium sulfate. The drying material was removed by filtration, the filtrate was then concentrated under reduced pressure, and the obtained residue was purified by normal-phase silica gel column chromatography (petroleum ether/ethyl acetate = 100/0 → 50/50, and subsequently reverse-phase silica gel column chromatography (water/acetonitrile = 95/5 → 50/50) to obtain 3.31 g of compound aa2-014-b as a yellow solid (yield: 37%).

LCMS (ESI) m/z = 522.5 $(M+H)^+$
Retention time: 0.85 min (analysis condition SQDFA05)

Synthesis of compound aa2-014, (3S)-4-(dimethylamino)-3-[[2-(9H-fluoren-9-ylmethoxycarbonylamino)-2-methylpropanoyl]-methylamino]-4-oxobutanoic acid (Fmoc-Aib-MeAsp-NMe2)

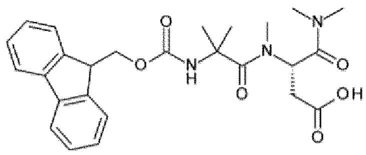

Compound aa2-014-b (1.14 g, 2.19 mmol) was provided as a starting raw material, and the same procedure as in the synthesis of compound aa2-001 was carried out to obtain 1.04 g of compound aa2-014 as a light brown amorphous crystal (yield: 99%).

LCMS (ESI) m/z = 482.4 $(M+H)^+$
Retention time: 0.70 min (analysis condition SQDFA05)

Synthesis of compound aa2-015, Fmoc-bAla(2-Me2)-D-3-MeAbu-OH

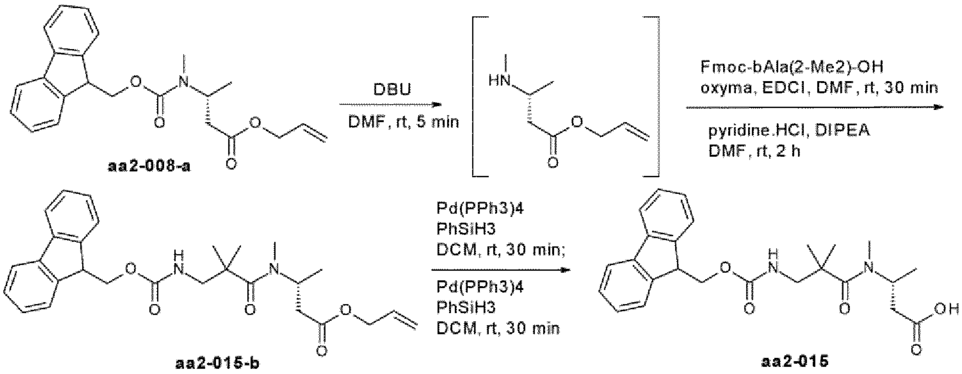


aa2-008-a

aa2-015-b

aa2-015

Synthesis of compound aa2-015-b, prop-2-enyl (3R)-3-[[3-[9H-fluoren-9-ylmethoxycarbonylamino)-2,2-dimethylpropanoyl-methylamino]butanoate (Fmoc-bAla(2-Me2)-D-3-MeAbu-OAl)

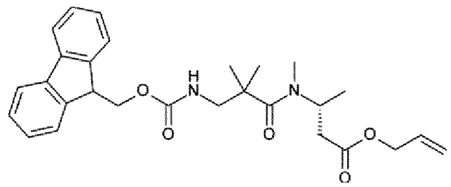

Compound aa2-008-a (8.0 g, 21.1 mmol) and Fmoc-bAla(2-Me2)-OH (7.5 g, 22.2 mmol) were provided as starting raw materials, and the same procedure as in the synthesis of compound aa2-001-a was carried out to obtain 1.28 g of compound aa2-015-b as a light yellow oily material (yield: 13%).

LCMS (ESI) m/z = 479.5 $(M+H)^+$
Retention time: 1.00 min (analysis condition SQDFA05)

Synthesis of compound aa2-015, (3R)-3-[[3-[9H-fluoren-9-ylmethoxycarbonylamino)-2,2-dimethylpropanoyl-methylamino]butanoic acid (Fmoc-bAla(2-Me2)-D-3-MeAbu-OH)

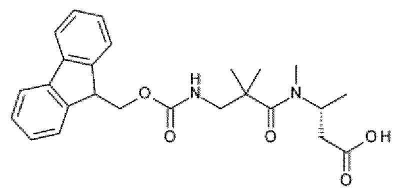

Under nitrogen gas flow, compound aa2-015-b (602 mg, 1.26 mmol) and tetrakistriphenylphosphinepaladium (14.5 mg, 0.013 mmol) were added into a reaction vessel, and DCM (2.5 mL) was then added. To the reaction liquid, phenylsilane (95 mg, 0.88 mmol) was added dropwise, and the reaction liquid was then stirred at room temperature for 30 minutes. Subsequently, tetrakistriphenylphosphinepaladium (14.5 mg, 0.013 mmol) and phenylsilane (95 mg, 0.88 mmol) were added, and the mixture was stirred at room temperature for 30 minutes. The reaction liquid was diluted with TBME (6.0 mL), and then extracted with a saturated aqueous sodium bicarbonate solution/water (1/1, 6.0 mL), and further extracted with water (3.0 mL). The combined aqueous layer was made acidic to a pH of about 2 by adding a 85% aqueous phosphoric acid solution (0.516 mL, 7.55 mmol) thereto, and the aqueous layer was extracted twice with DCM (6.0 mL). The combined organic layer was washed with a saturated aqueous sodium chloride solution/water (1/1, 6.0 mL), and the organic layer was then dried over sodium sulfate. The drying material was removed by filtration, the filtrate was then concentrated under reduced pressure, and the obtained residue was purified by reverse-phase silica gel column chromatography (water/acetonitrile = 90/10 -> 30/70) to obtain 285 mg of compound aa2-015 as a white solid (yield: 52%).

LCMS (ESI) m/z = 439.5 $(M+H)^+$
Retention time: 0.80 min (analysis condition SQDFA05)

Synthesis of compound aa2-016, Fmoc-MeLeu-MeVal-OH

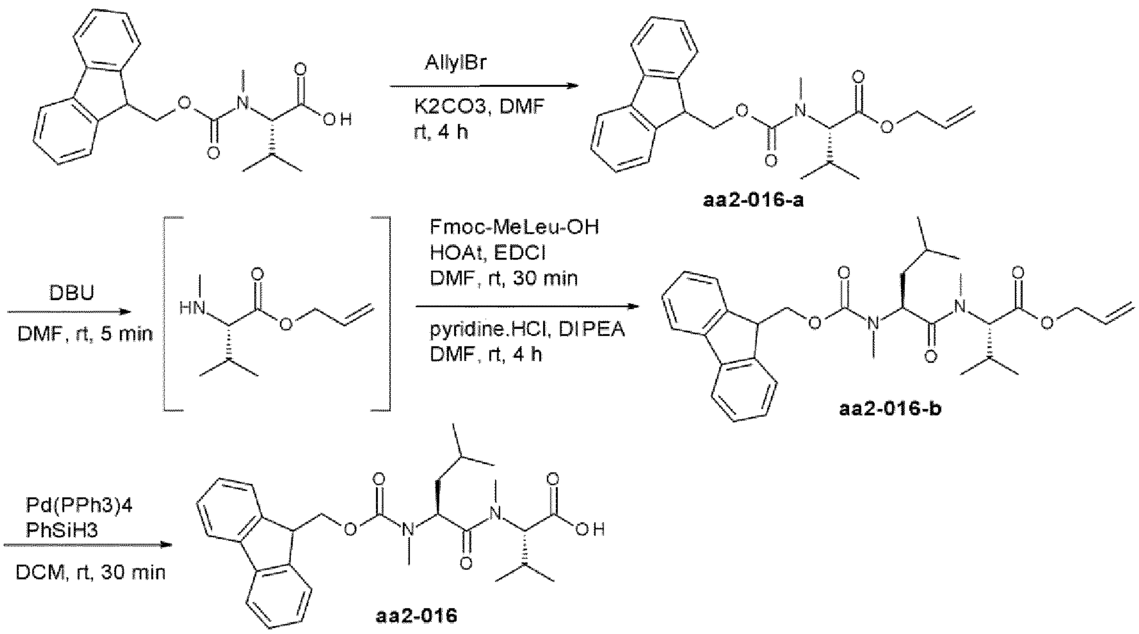


Synthesis of compound aa2-016-a, prop-2-enyl (2S)-2-[9H-fluoren-9-ylmethoxycarbonyl(methyl)amino]-3-methylbutanoate (Fmoc-MeVal-OAl)

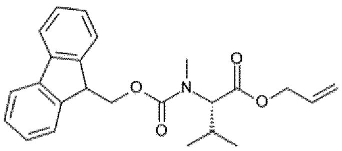

Under nitrogen gas flow, Fmoc-MeVal-OH (2.00 g, 5.66 mmol), allyl bromide (0.75 g, 6.20 mmol), potassium carbonate (1.17 g, 8.47 mmol) and DMF (20 mL) were added into a reaction vessel, and the reaction liquid was stirred at room temperature for 4 hours. Filtration was performed, and the filtrate was then neutralized with a 1 mol/L aqueous hydrochloric acid solution at 0°C, and extracted three times with ethyl acetate. The combined organic layer was washed once with water, and twice with a saturated aqueous sodium chloride solution, and the organic layer was then dried over sodium sulfate. The drying material was removed by filtration, the filtrate was then concentrated under reduced pressure, and the obtained residue was purified by normal-phase silica gel column chromatography (petroleum ether/ethyl acetate = 100/0 $\rightarrow$ 90/10) to obtain 1.9 g of compound aa2-016-a as a colorless oily material (yield: 85%).

LCMS (ESI) m/z = 394.2 $(M+H)^+$
Retention time: 1.21 min (analysis condition SMDmethod_5)

Synthesis of compound aa2-016-b, prop-2-enyl (2S)-2-[[(2S)-2-[9H-fluoren-9-ylmethoxycarbonyl(methyl)amino]-4-methylpentanoyl]-methylamino]-3-methylbutanoate(Fmoc-MeLeu-MeVal-OAl).

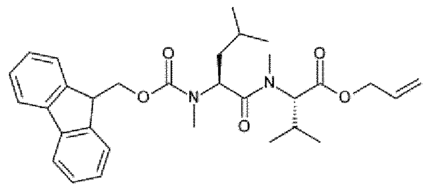

Compound aa2-016-a (1.0 g, 2.64 mmol) and Fmoc-MeLeu-OH (1.02 g, 2.77 mmol) were provided as starting raw materials, and the same procedure as in the synthesis of compound aa2-001-a was carried out to obtain 0.90 g of compound aa2-016-b as a colorless oily material (yield: 64%).

LCMS (ESI) m/z = 521.6 $(M+H)^+$
Retention time: 1.16 min (analysis condition SQDFA05)

Synthesis of compound aa2-016, (2S)-2-[[(2S)-2-[9H-fluoren-9-ylmethoxycarbonyl(methyl)amino]-4-methylpentanoyl]-methylamino]-3-methylbutanoic acid (Fmoc-MeLeu-MeVal-OH)

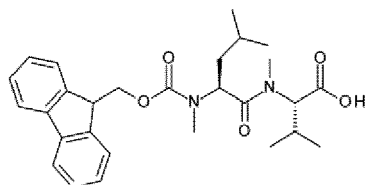

Compound aa2-016-b (417 mg, 0.80 mmol) was provided as a starting raw material, and the same procedure as in the synthesis of compound aa2-001 was carried out to obtain 185 mg of compound aa2-016 as a light brown amorphous crystal (yield: 48%).

LCMS (ESI) m/z = 481.5 $(M+H)^+$
Retention time: 0.97 min (analysis condition SQDFA05)

Synthesis of compound aa2-017, Fmoc-MeVal-Pro-OH

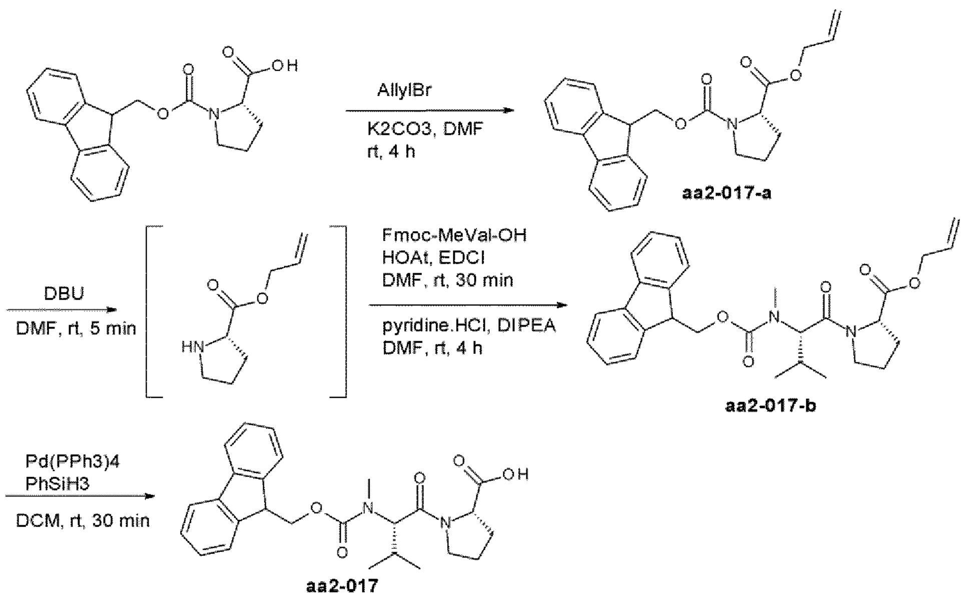

Synthesis of compound aa2-017-a, 2-O-prop-2-enyl 1-O-(9H-fluoren-9-ylmethyl) (2S)-pyrrolidine-1,2-dicarboxylate (Fmoc-Pro-OAl)

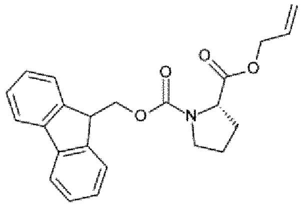

Fmoc-Pro-OH (5.00 g, 14.8 mmol) was provided as a starting raw material, and the same procedure as in the synthesis of compound aa2-016-a was carried out to obtain 5.3 g of compound aa2-017-a as a colorless oily material (yield: 94%).

LCMS (ESI) m/z = 378.2 $(M+H)^+$
Retention time: 1.10 min (analysis condition SMDmethod_5)

Synthesis of compound aa2-017-b, prop-2-enyl (2S)-1-[(2S)-2-[9H-fluoren-9-ylmethoxycarbonyl(methyl)amino]-3-methylbutanoyl]pyrrolidine-2-carboxylate (Fmoc-MeVal-Pro-OAl)

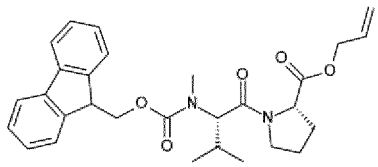

Compound aa2-017-a (5.3 g, 14.0 mmol) and Fmoc-MeVal-OH (5.21 g, 14.7 mmol) were provided as starting raw materials, and the same procedure as in the synthesis of compound aa2-001-a was carried out to obtain 5.2 g of compound aa2-017-b as a colorless oily material (yield: 74%).

LCMS (ESI) m/z = 491.5 $(M+H)^+$
Retention time: 1.00 min (analysis condition SQDFA05)

Synthesis of compound aa2-017, (2S)-1-[(2S)-2-[9H-fluoren-9-ylmethoxycarbonyl(methyl)aminol-3-methylbutanoyl]pyrrolidine-2-carboxylic acid (Fmoc-MeVal-Pro-OH)

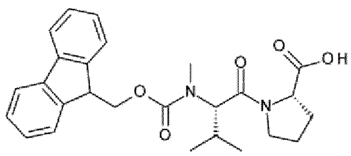

Compound aa2-017-b (1.57 g, 3.20 mmol) was provided as a starting raw material, and the same procedure as in the synthesis of compound aa2-001 was carried out to obtain 1.35 g of compound aa2-017 as a light brown amorphous crystal (yield: 94%).

LCMS (ESI) m/z = 451.5 $(M+H)^+$
Retention time: 0.81 min (analysis condition SQDFA05)

Synthesis of compound aa2-018, Fmoc-MeLeu-Aib-OH

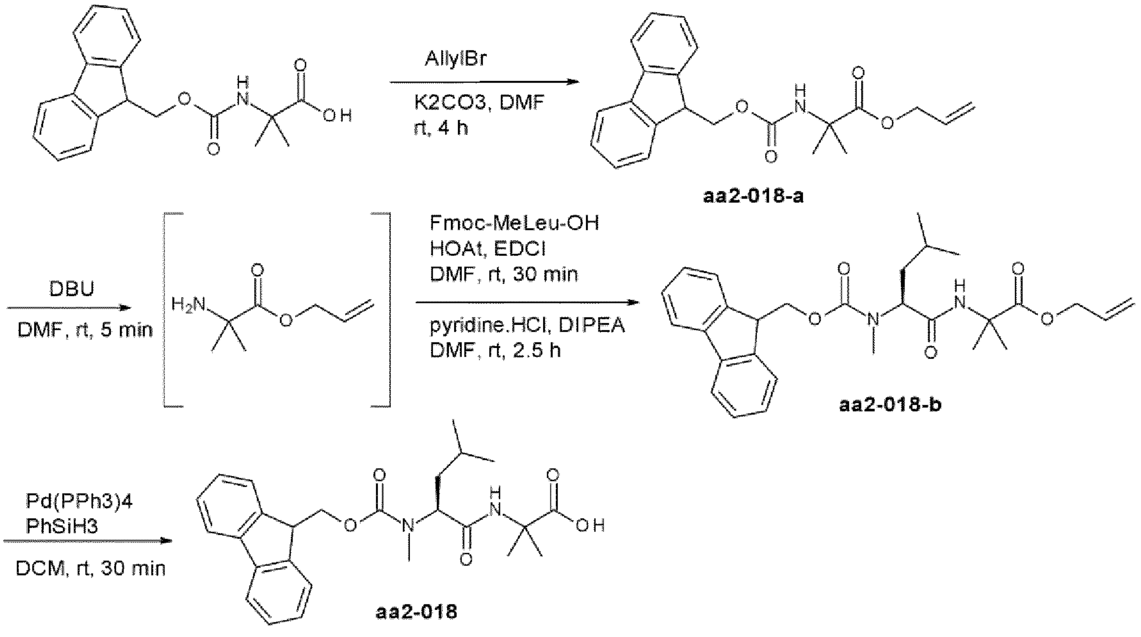


Synthesis of compound aa2-018-a, prop-2-enyl 2-[9H-fluoren-9-ylmethoxycarbonylamino)-2-methylpropanoate (Fmoc-Aib-OAl)

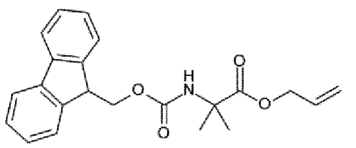

Fmoc-Aib-OH (7.00 g, 21.5 mmol) was provided as a starting raw material, and the same procedure as in the synthesis of compound aa2-016-a was carried out to obtain 7.5 g of compound aa2-018-a as a colorless oily material (yield: 94%).

LCMS (ESI) m/z = 366.1 $(M+H)^+$
Retention time: 1.08 min (analysis condition SMDmethod_5)

Synthesis of compound aa2-018-b, prop-2-enyl 2-[[(2S)-2-[9H-fluoren-9-ylmethoxycarbonyl(methyl)amino1-4-methylpentanoyl]amino1-2-methylpropanoate (Fmoc-MeLeu-Aib-OAl)

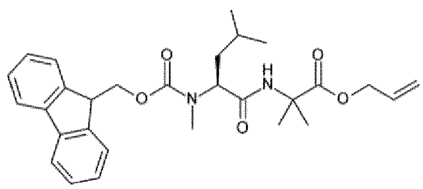

Compound aa2-018-a (7.00 g, 19.2 mmol) and Fmoc-MeLeu-OH (7.39 g, 20.1 mmol) were provided as starting raw materials, and the same procedure as in the synthesis of compound aa2-001-a was carried out to obtain 6.2 g of

compound aa2-018-b as a yellow oily material (yield: 65%).

LCMS (ESI) m/z = 493.5 $(M+H)^+$
Retention time: 1.07 min (analysis condition SQDFA05)

Synthesis of compound aa2-018, 2-[[(2S)-2-[9H-fluoren-9-ylmethoxycarbonyl(methyl)aminol-4-methylpentanoyl]amino]-2-methylpropanoic acid (Fmoc-MeLeu-Aib-OH)

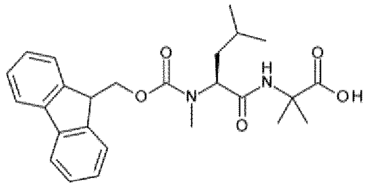

Compound aa2-018-b (985 mg, 2.00 mmol) was provided as a starting raw material, and the same procedure as in the synthesis of compound aa2-001 was carried out to obtain 608 mg of compound aa2-018 as a light brown amorphous crystal (yield: 67%).

LCMS (ESI) m/z = 453.5 $(M+H)^+$
Retention time: 0.91 min (analysis condition SQDFA05)

Synthesis of compound aa2-019, Fmoc-MeVal-Gly-OH

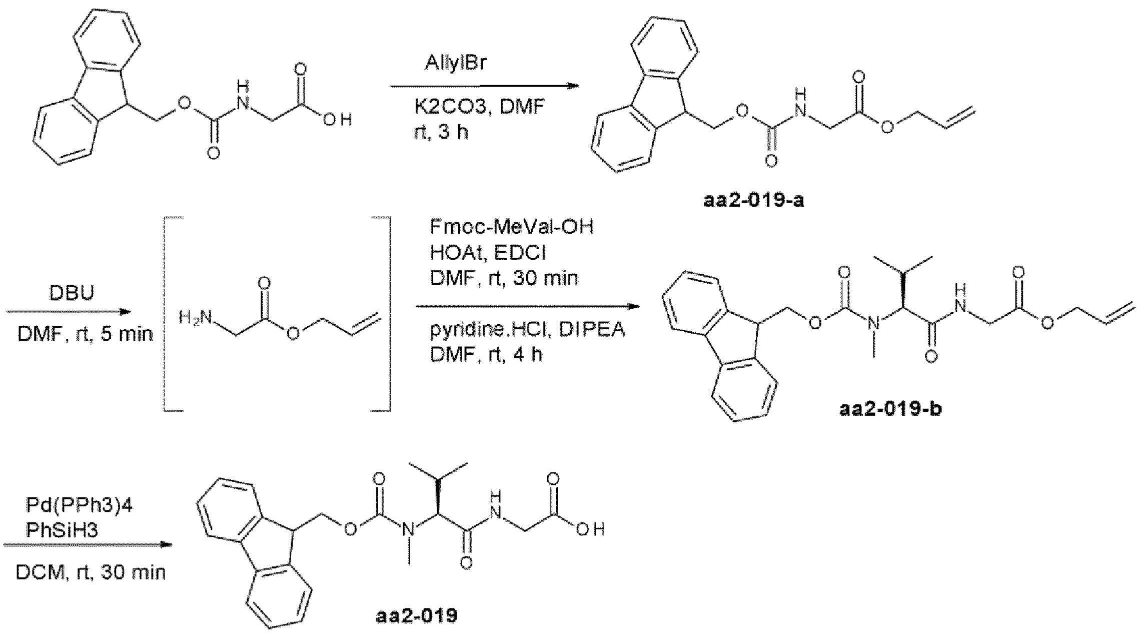


Synthesis of compound aa2-019-a, prop-2-enyl 2-(9H-fluoren-9-ylmethoxycarbonylamino)acetate (Fmoc-Gly-OAl)

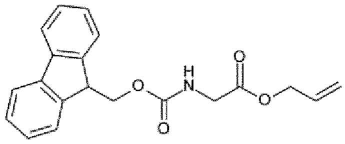

Fmoc-Gly-OH (7.00 g, 23.5 mmol) was provided as a starting raw material, and the same procedure as in the synthesis of compound aa2-016-a was carried out to obtain 7.5 g of compound aa2-019-a as a colorless oily material (yield: 91%).

LCMS (ESI) m/z = 338.2 $(M+H)^+$
Retention time: 1.16 min (analysis condition SMDmethod_6)

Synthesis of compound aa2-019-b, prop-2-enyl 2-[[(2S)-2-[9H-fluoren-9-ylmethoxycarbonyl(methyl)aminol-3-methylbutanoyl]amino] acetate (Fmoc-MeVal-Gly-OAl)

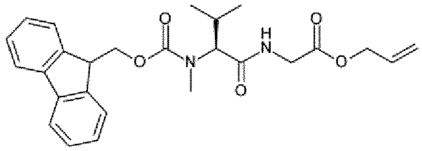

Compound aa2-019-a (5.0 g, 14.8 mmol) and Fmoc-MeVal-OH (5.5 g, 15.6 mmol) were provided as starting raw materials, and the same procedure as in the synthesis of compound aa2-001-a was carried out to obtain 4.3 g of compound aa2-019-b as a colorless oily material (yield: 64%).

LCMS (ESI) m/z = 451.5 $(M+H)^+$
Retention time: 0.96 min (analysis condition SQDFA05)

Synthesis of compound aa2-019, 2-[[(2S)-2-[9H-fluoren-9-ylmethoxycarbonyl(methyl)amino]-3-methylbutanoyl]amino]acetic acid (Fmoc-MeVal-Gly-OH)

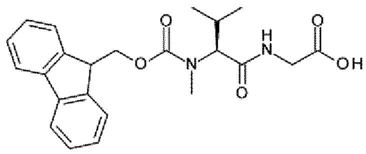

Compound aa2-019-b (1.47 g, 3.27 mmol) was provided as a starting raw material, and the same procedure as in the synthesis of compound aa2-001 was carried out to obtain 1.31 g of compound aa2-019 as a light brown amorphous crystal (yield: 98%).

LCMS (ESI) m/z = 411.4 $(M+H)^+$
Retention time: 0.79 min (analysis condition SQDFA05)

Synthesis of compound aa2-020, Fmoc-D-MeVal-MeAsp-NMe2

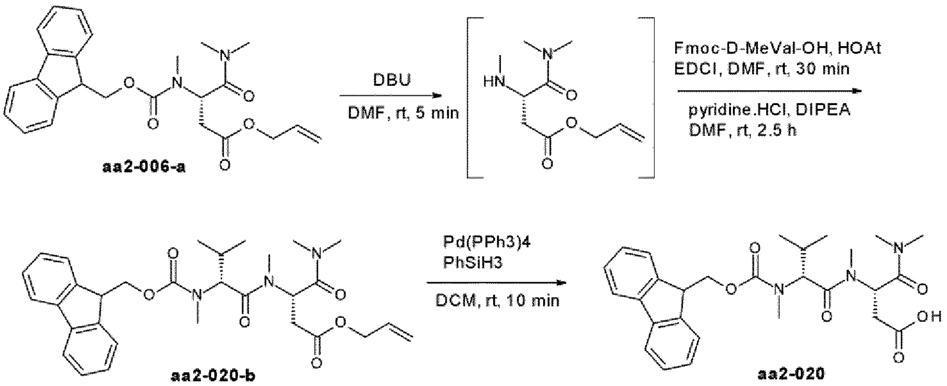


aa2-006-a

aa2-020-b

aa2-020

Synthesis of compound aa2-020-b, prop-2-enyl (3S)-4-(dimethylamino)-3-[[(2R)-2-[9H-fluoren-9-ylmethoxycarbonyl(methyl)amino]-3-methylbutanoyl]-methylamino]-4-oxobutanoate (Fmoc-D-MeVal-MeAsp(OAl)-NMe2)

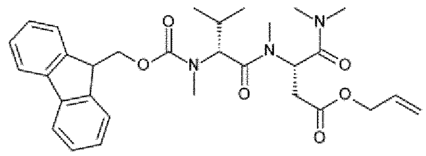

Compound aa2-006-a (5.00 g, 11.5 mmol) and Fmoc-D-MeVal-OH (4.08 g, 11.5 mmol) were provided as starting raw materials, and the same procedure as in the synthesis of compound aa2-001-a was carried out to obtain 3.1 g of compound aa2-020-b as a white solid (yield: 49%).

LCMS (ESI) m/z = 550.5 $(M+H)^+$
Retention time: 0.98 min (analysis condition SQDFA05)

Synthesis of compound aa2-020, (3S)-4-(dimethylamino)-3-[[(2R)-2-[9H-fluoren-9-ylmethoxycarbonyl(methyl)amino]-3-methylbutanoyl]-methylamino]-4-oxobutanoic acid (Fmoc-D-MeVal-MeAsp-NMe2)

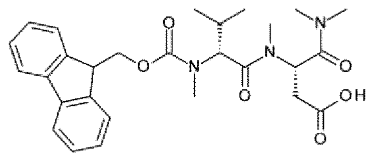

Compound aa2-020-b (1.37 g, 2.50 mmol) was provided as a starting raw material, and the same procedure as in the synthesis of compound aa2-001 was carried out to obtain 1.20 g of compound aa2-020 as a white amorphous crystal (yield: 94%).

LCMS (ESI) m/z = 510.5 $(M+H)^+$
Retention time: 0.79 min (analysis condition SQDFA05)

Synthesis of compound aa2-021, Fmoc-bAla(2-Me2)-MeAsp-NMe2

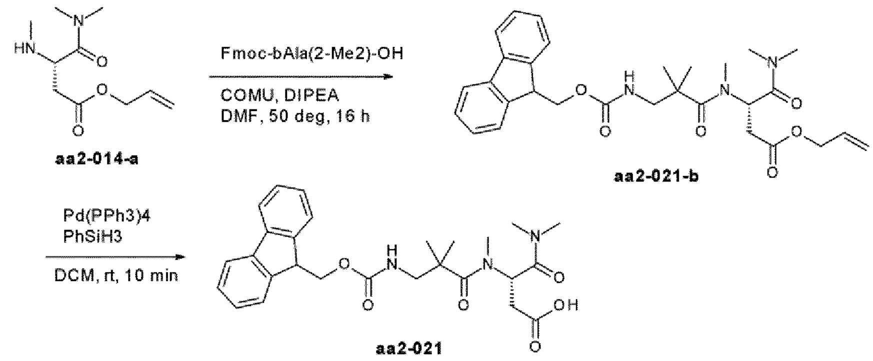

aa2-014-a
aa2-021-b
aa2-021

Synthesis of compound aa2-021-b, prop-2-enyl (3S)-4-(dimethylamino)-3-[[3-[9H-fluoren-9-ylmethoxycarbonylamino)-22-dimethylpropanoyl]-methylamino]-4-oxobutanoate(Fmoc-bAla(2-Me2)-MeAsp(OAl)-NMe2)

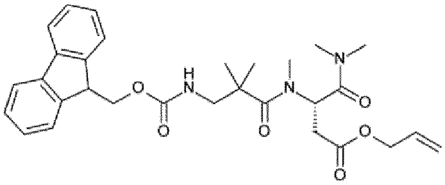

Compound aa2-014-a (5.92 g, 27.6 mmol) and Fmoc-bAla(2-Me2)-OH (8.6 g, 25.3 mmol) were provided as starting raw materials, and the same procedure as in the synthesis of compound aa2-014-b was carried out to obtain 3.63 g of compound aa2-021-b as a yellow solid (yield: 26%).

LCMS (ESI) m/z = 536.6 $(M+H)^+$
Retention time: 0.90 min (analysis condition SQDFA05)

Synthesis of compound aa2-021, (3S)-4-(dimethylamino)-3-[[3-(9H-fluoren-9-ylmethoxycarbonylamino)-2,2-dimethylpropanoyl]-methylamino]-4-oxobutanoic acid (Fmoc-bAla(2-Me2)-MeAsp-NMe2)

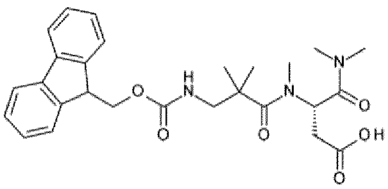

Compound aa2-021-b (1.07 g, 2.00 mmol) was provided as a starting raw material, and the same procedure as in the synthesis of compound aa2-001 was carried out to obtain 874 mg of compound aa2-021 as a light brown amorphous crystal (yield: 88%).

LCMS (ESI) m/z = 496.5 $(M+H)^+$
Retention time: 0.74 min (analysis condition SQDFA05)

Synthesis of compound aa2-022, Fmoc-MeVal-D-MeAsp-NMe2

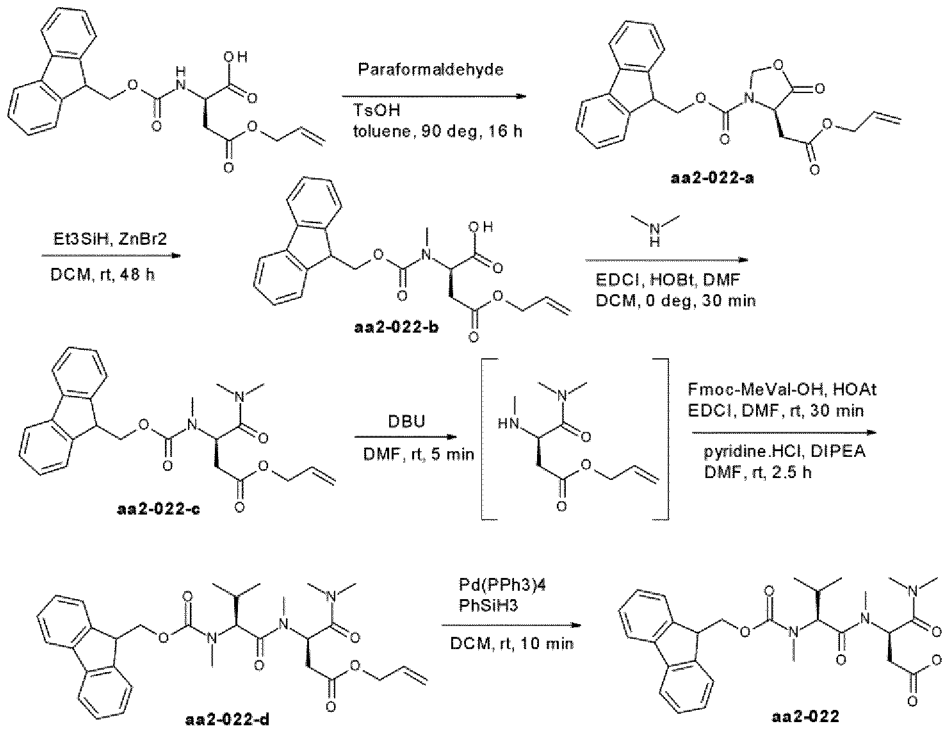


Synthesis of compound aa2-022-a, 9H-fluoren-9-ylmethyl (4R)-5-oxo-4-(2-oxo-2-prop-2-enoxyethyl)-1,3-oxazolidine-3-carboxylate

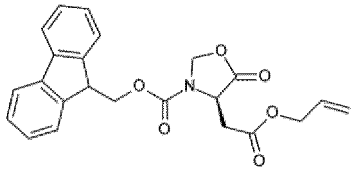

Under nitrogen gas flow, Fmoc-D-Asp(OAl)-OH (20.0 g, 50.6 mmol), paraformaldehyde (4.56 g, 152 mmol), p-toluenesulfonic acid (0.09 g, 0.506 mmol) and toluene (500 mL) were added into a reaction vessel, and the reaction liquid was stirred at 90°C for 16 hours. The reaction mixture was cooled to room temperature, and the solvent was then removed by distillation under reduced pressure. The obtained residue was dissolved in TBME, the solution was washed with an aqueous sodium carbonate solution, and the organic layer was then dried over sodium sulfate. The drying material was removed by filtration, and the filtrate was then concentrated under reduced pressure to obtain 20 g of compound aa2-022-a as a yellow oily material (yield: 90%).

LCMS (ESI) m/z = 408.2 $(M+H)^+$
Retention time: 1.05 min (analysis condition SMDmethod_5)

Synthesis of compound aa2-022-b, (2R)-2-[9H-fluoren-9-ylmethoxycarbonyl(methyl)amino]-4-oxo-4-prop-2-enoxybutanoic acid (Fmoc-D-MeAsp(OAl)-OH)

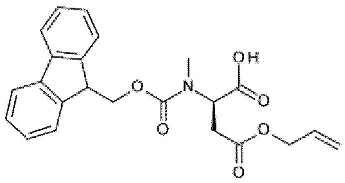

Under nitrogen gas flow, compound aa2-022-a (20 g, 49.1 mmol), triethylsilane (11.4 g, 98.2 mmol), DCM (200 mL) and zinc bromide (11.1 g, 49.1 mmol) were added into a reaction vessel, the reaction liquid was stirred at room temperature for 48 hours, and the solvent was then removed by distillation under reduced pressure. The obtained residue was dissolved in an aqueous potassium carbonate solution, and the solution was washed twice with hexane. The aqueous phase was made to have a pH of 2 with hydrochloric acid, and then extracted three times with ethyl acetate, and the organic phase was dried over sodium sulfate. The drying material was removed by filtration, and the filtrate was then concentrated under reduced pressure to obtain 15 g of compound aa2-022-b as a white solid (yield: 93%).

LCMS (ESI) m/z = 410.2 $(M+H)^+$
Retention time: 0.98 min (analysis condition SMDmethod_5)

Synthesis of compound aa2-022-c, prop-2-enyl (3R)-4-(dimethylamino)-3-[9H-fluoren-9-ylmethoxycarbonyl(methyl)amino]-4-oxobutanoate (Fmoc-D-MeAsp(OAl)-NMe2)

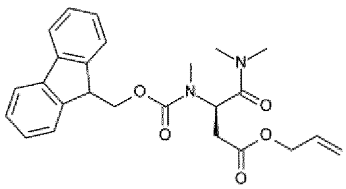

Compound aa2-022-b (15.7 g, 38.3 mmol) was provided as a starting raw material, and the same procedure as in the synthesis of compound aa2-006-a was carried out to obtain 13 g of compound aa2-022-c as a yellow oily material (yield: 78%).

LCMS (ESI) m/z = 437.2 $(M+H)^+$
Retention time: 1.86 min (analysis condition SMDmethod_7)

Synthesis of compound aa2-022-d, prop-2-enyl (3R)-4-(dimethylamino)-3-[[(2S)-2-[9H-fluoren-9-ylmethoxycarbonyl(methyl)amino]-3-methylbutanoyl]-methylamino]-4-oxobutanoate (Fmoc-MeVal-D-MeAsp(OAl)- NMe2)

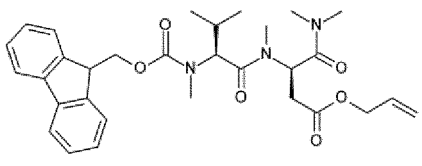

Compound aa2-022-c (7.00 g, 16.0 mmol) and Fmoc-MeVal-OH (6.23 g, 17.6 mmol) were provided as starting raw materials, and the same procedure as in the synthesis of compound aa2-001-a was carried out to obtain 3.34 g of compound aa2-022-d as a white solid (yield: 35%).

LCMS (ESI) m/z = 550.6 $(M+H)^+$
Retention time: 0.97 min (analysis condition SQDFA05)

Synthesis of compound aa2-022, (3R)-4-(dimethylamino)-3-[[(2S)-2-[9H-fluoren-9-ylmethoxycarbonyl(methyl)amino]-3-methylbutanoyl]-methylamino]-4-oxobutanoic acid (Fmoc-MeVal-D-MeAsp-NMe2)

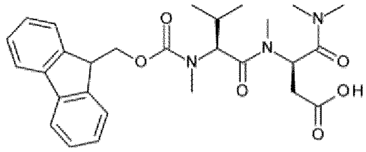

Compound aa2-022-d (1.37 g, 2.50 mmol) was provided as a starting raw material, and the same procedure as in the synthesis of compound aa2-001 was carried out to obtain 1.26 g of compound aa2-022 as a white amorphous crystal (yield: 99%).

LCMS (ESI) m/z = 510.5 $(M+H)^+$
Retention time: 0.79 min (analysis condition SQDFA05)

Synthesis of compound aa2-023, Fmoc-Ala-MeGly(cPent)-MeAsp-NMe2

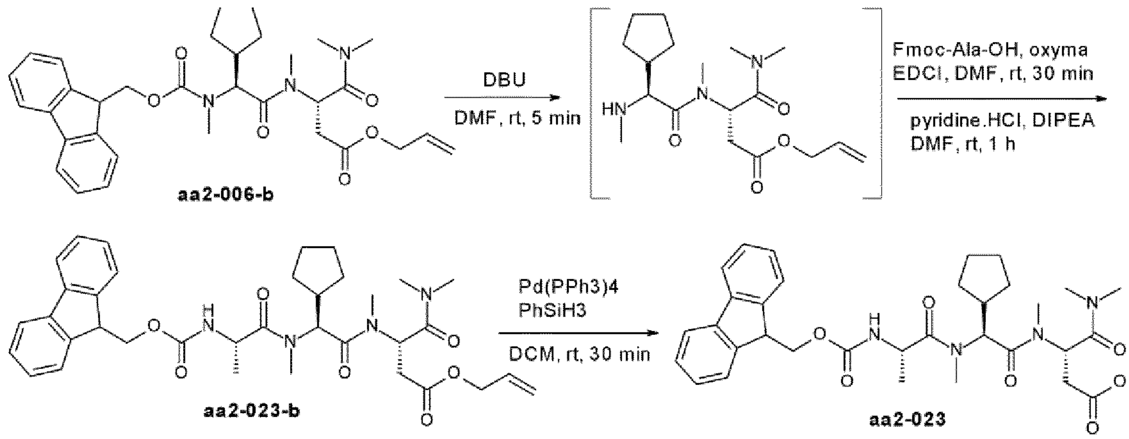


Synthesis of compound aa2-023-b, prop-2-enyl (3S)-3-[[(2S)-2-cyclopentyl-2-[[(2S)-2-(9H-fluoren-9-ylmethoxycarbonylamino)propanoyl]-methylamino]acetyl]-methylamino]-4-(dimethylamino)-4-oxobutanoate (Fmoc-Ala-MeGly(cPent)-MeAsp(OAl)-NMe2)

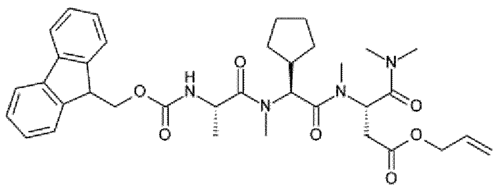

Compound aa2-006-b (1.17 g, 2.03 mmol) and Fmoc-Ala-OH (601 mg, 1.93 mmol) were provided as starting raw materials, and the same procedure as in the synthesis of compound aa2-001-a was carried out to obtain 575 mg of compound aa2-023-b as a yellow amorphous crystal (yield: 44%).

LCMS (ESI) m/z = 647.7 $(M+H)^+$
Retention time: 0.98 min (analysis condition SQDFA05)

Synthesis of compound aa2-023, (3S)-3-[[(2S)-2-cyclopentyl-2-[[(2S)-2-(9H-fluoren-9-ylmethoxycarbonylamino)propanoyl]-methylamino]acetyl]-methylamino]-4-(dimethylamino)-4-oxobutanoic acid (Fmoc-Ala-MeGly(cPent)-MeAsp-NMe2)

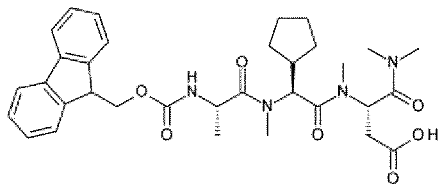

Compound aa2-023-b (575 mg, 0.889 mmol) was provided as a starting raw material, and the same procedure as in the synthesis of compound aa2-001 was carried out to obtain compound aa2-023 as a crude product. The obtained crude product was dissolved in DMSO, and the solution was purified by reverse-phase silica gel column chromatography (water/acetonitrile = 85/15 → 20/80) to obtain 476 mg of compound aa2-023 as a white solid (yield: 88%).

LCMS (ESI) m/z = 607.6 $(M+H)^+$
Retention time: 0.83 min (analysis condition SQDFA05)

Example 1-3. Synthesis of Fmoc-amino acid

The Fmoc-amino acids shown in Table 4 were synthesized in accordance with the scheme shown below.

[Table 4]

| Compound No. | Abbreviation |
|---|---|
| aa3-001 | Fmoc-MeAsp-pip |
| aa3-002 | Fmoc-MeAsp-NMe2 |
| aa3-003 | Fmoc-Asp-NMe2 |

Synthesis of compound aa3-001, (3S)-3-[9H-fluoren-9-ylmethoxycarbonyl(methyl)amino]-4-oxo-4-piperidin-1-ylbutanoic acid (Fmoc-MeAsp-pip)

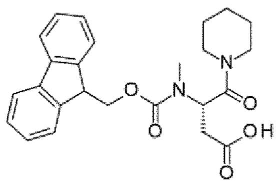

Compound aa3-001 was synthesized in accordance with the method described in WO 2018/225864.

Synthesis of compound aa3-002, (3S)-4-(dimethylamino)-3-[9H-fluoren-9-ylmethoxycarbonyl(methyl)amino]-4-oxobutanoic acid (Fmoc-MeAsp-NMe2)

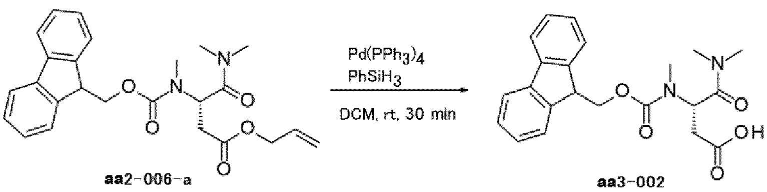


aa2-006-a aa3-002

Compound aa2-006-a (32.0 g, 73.3 mmol) was provided as a starting raw material, and the same procedure as in the synthesis of compound aa2-001 was carried out to obtain 25.1 g of compound aa3-002 as a light brown amorphous crystal (yield: 86%).

LCMS (ESI) m/z = 397.2 $(M+H)^+$
Retention time: 0.68 min (analysis condition SQDFA05)

<u>Synthesis of compound aa3-003, Fmoc-Asp-NMe2</u>

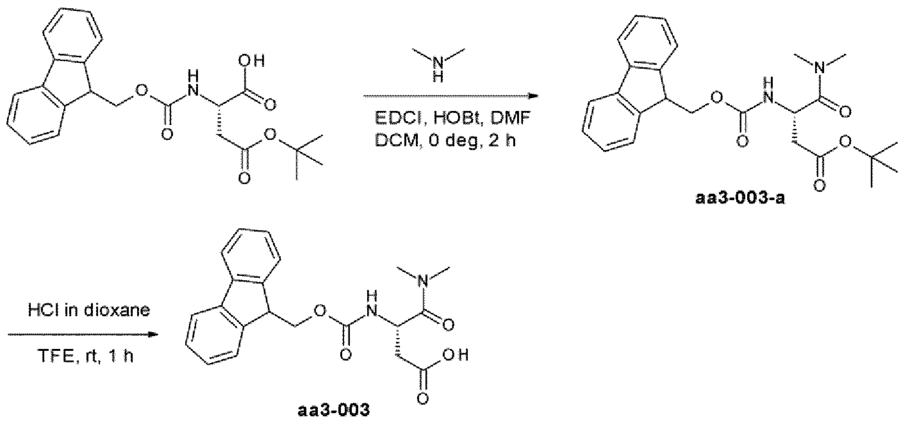


aa3-003-a

aa3-003

<u>Synthesis of compound aa3-003-a, (3S)-4-(dimethylamino)-3-[9H-fluoren-9-ylmethoxycarbonylamino)-4-oxobutanoic acid-2-methylpropan-2-yl (Fmoc-Asp(OtBu)-NMe2)</u>

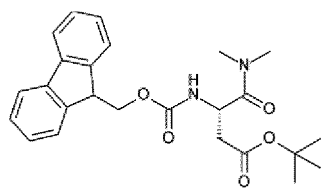

Fmoc-Asp(OtBu)-OH (25.0 g, 60.8 mmol) purchased from a commercial supplier was provided as a starting raw material, and the same procedure as in the synthesis of compound aa2-006-a was carried out to obtain 29.8 g of compound aa3-003-a as a crude product.

LCMS (ESI) m/z = 461.3 $(M+Na)^+$
Retention time: 0.88 min (analysis condition SQDFA05)

<u>Synthesis of compound aa3-003, (3S)-4-(dimethylamino)-3-(9H-fluoren-9-ylmethoxycarbonylamino)-4-oxobutanoic acid (Fmoc-MeAsp-NMe2)</u>

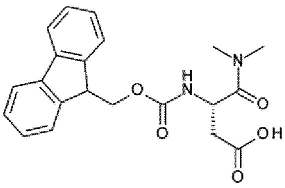

A crude product of compound aa3-003-a (29.8 g) and TFE (270 mL) were added into a reaction vessel, a 4 mol/L hydrochloric acid in dioxane solution (15.2 mL, 60.8 mmol) was added portion wise, and the reaction liquid was then stirred at room temperature for 1 hour. The reaction liquid was diluted with TBME (500 mL), and then extracted with a 5% saturated aqueous sodium carbonate solution (600 mL). The obtained aqueous layer was made acidic to a pH of about 2 to 3 by adding a 85% aqueous phosphoric acid solution (40 to 50 mL) thereto, and the aqueous layer was extracted with TBME (400 mL). The obtained organic layer was washed with a 10% aqueous sodium chloride solution (400 mL) and water (400 mL), and the organic layer was then dried over sodium sulfate. The drying material was removed by filtration, and the filtrate was then concentrated under reduced pressure to obtain 21.4 g of compound aa3-003 (yield: 92%).

LCMS (ESI) m/z = 383.2 $(M+H)^+$
Retention time: 0.66 min (analysis condition SQDFA05)

<u>Example 1-4. Synthesis of resin supporting amino acid and peptide</u>

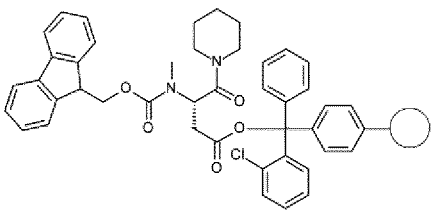

Fmoc-MeAsp(O-Trt(2-Cl)-resin)-pip

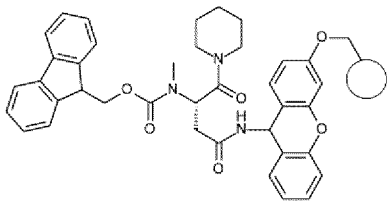

Fmoc-MeAsp(NH-Sieber resin)-pip

In the present specification, a polymer or resin site may be expressed by O when the compound is bound to a polymer or resin. For the purpose of clarifying the reaction point of the resin site, the chemical structure of the reaction site may be expressed with the compound connected to O. For example, in the above-described Fmoc-MeAsp(O-Trt(2-Cl)resin)-pip structure, the 2-chlorotrityl group of the resin is bonded to the side-chain carboxylic acid of MeAsp through an ester bond, and in the Fmoc-MeAsp(NH-Sieber resin)-pip, the 9H-xanthen-9-amino group of the resin is bonded to the side-chain carboxylic acid of MeAsp through an amide bond. The term "pip" means piperidine, and in the above-described structure, the carboxylic acid group at the C-terminal forms an amide bond with piperidine. 2-Chlorotrityl chloride resin (1.25 to 1.69 mmol/g, 100-200 mesh, 1% DVD) was purchased from WATANABE CHEMICAL INDUSTRIES, LTD. and SUNRESIN, and Fmoc-NH-Sieber resin (0.69 mmol/g, 100-200 mesh, 1% DVB) was purchased from Novabiochem.

The Fmoc-amino acids or peptide-supporting resins shown in Table 5 were synthesized in accordance with the scheme shown below.

[Table 5]

| Compound No. | Abbreviation |
|---|---|
| aa2-001-resin | Fmoc-MeVal-MeAsp(O-Trt(2-Cl)resin)-pip |
| aa2-002-resin | Fmoc-MeIle-MeAsp(O-Trt(2-Cl)resin)-pip |
| aa2-003-resin | Fmoc-MeGly(cPent)-MeAsp(O-Trt(2-Cl)resin)-pip |
| aa2-004-resin | Fmoc-MeChg-MeAsp(O-Trt(2-Cl)resin)-pip |
| aa2-005-resin | Fmoc-MeLeu-MeAsp(O-Trt(2-Cl)resin)-pip |
| aa2-006-resin | Fmoc-MeGly(cPent)-MeAsp(O-Trt(2-Cl)resin)-NMe2 |
| aa2-007-resin | Fmoc-MeLeu-MeAsp(O-Trt(2-Cl)resin)-NMe2 |
| aa2-008-resin | Fmoc-MeVal-D-3-MeAbu-O-Trt(2-Cl)resin |
| aa2-009-resin | Fmoc-MeChg-D-3-MeAbu-O-Trt(2-Cl)resin |
| aa2-010-resin | Fmoc-MeVal-MeGly-O-Trt(2-Cl)resin |
| aa2-011-resin | Fmoc-MeGly(cPent)-MeAsp(O-Trt(2-Cl)resin)-mor |
| aa2-012-resin | Fmoc-MeVal-Asp(O-Trt(2-Cl)resin)-NMe2 |
| aa2-013-resin | Fmoc-Gly-MeAsp(O-Trt(2-Cl)resin)-NMe2 |
| aa2-014-resin | Fmoc-Aib-MeAsp(O-Trt(2-Cl)resin)-NMe2 |
| aa2-015-resin | Fmoc-bAla(2-Me2)-D-3-MeAbu-O-Trt(2-Cl)resin |
| aa2-016-resin | Fmoc-MeLeu-MeVal-O-Trt(2-Cl)resin |
| aa2-017-resin | Fmoc-MeVal-Pro-O-Trt(2-Cl)resin |
| aa2-018-resin | Fmoc-MeLeu-Aib-O-Trt(2-Cl)resin |
| aa2-019-resin | Fmoc-MeVal-Gly-O-Trt(2-Cl)resin |
| aa2-020-resin | Fmoc-D-MeVal-MeAsp(O-Trt(2-Cl)resin)-NMe2 |
| aa2-021-resin | Fmoc-bAla(2-Me2)-MeAsp(O-Trt(2-Cl)resin)-NMe2 |
| aa2-022-resin | Fmoc-MeVal-D-MeAsp(O-Trt(2-Cl)resin)-NMe2 |
| aa2-023-resin | Fmoc-Ala-MeGly(cPent)-MeAsp(O-Trt(2-Cl)resin)-NMe2 |
| aa2-024-resin | Fmoc-Phe-Pro-O-Trt(2-Cl)resin |
| aa2-025-resin | Fmoc-Lys(Boc)-Pro-O-Trt(2-Cl)resin |
| aa2-026-resin | Fmoc-Gly-Tyr(tBu)-O-Trt(2-Cl)resin |
| aa2-027-resin | Fmoc-Ala-Ala-O-Trt(2-Cl)resin |
| aa2-028-resin | Fmoc-Phe-Gly-O-Trt(2-Cl)resin |
| aa2-029-resin | Fmoc-Asn(Trt)-Gly-O-Trt(2-Cl)resin |
| aa2-030-resin | Fmoc-Gly-Val-O-Trt(2-Cl)resin |
| aa2-031-resin | Fmoc-Ser(tBu)-Gly-O-Trt(2-Cl)resin |

| | |
|---|---|
| aa2-032-resin | Fmoc-Ala-Ala-Pro-O-Trt(2-Cl)resin |
| aa3-001-resin | Fmoc-MeAsp(O-Trt(2-Cl)resin)-pip |
| aa3-002-resin | Fmoc-MeAsp(O-Trt(2-Cl)resin)-NMe2 |
| aa3-003-resin | Fmoc-Asp(O-Trt(2-Cl)resin)-NMe2 |
| aa4-001-resin | Fmoc-D-3-MeAbu-O-Trt(2-Cl)resin |
| aa4-002-resin | Fmoc-MeGly-O-Trt(2-Cl)resin |
| aa4-003-resin | Fmoc-MeVal-O-Trt(2-Cl)resin |
| aa4-004-resin | Fmoc-Pro-O-Trt(2-Cl)resin |
| aa4-005-resin | Fmoc-Aib-O-Trt(2-Cl)resin |
| aa4-006-resin | Fmoc-Gly-O-Trt(2-Cl)resin |
| aa5-001-resin | Fmoc-MeVal-MeAsp(NH-Sieber resin)-pip |

Synthesis of compound aa2-001-resin, (3S)-3-[[(2S)-2-[9H-fluoren-9-ylmethoxycarbonyl(methyl)amino]-3-methylbutanoyl]-methylamino]-4-oxo-4-piperidin-1-ylbutanoic acid-2-chlorotrityl resin (Fmoc-MeVal-MeAsp(O-Trt(2-Cl)resin)-pip)

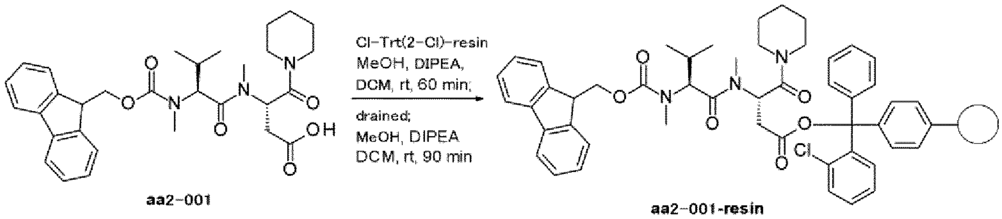


aa2-001 aa2-001-resin

A reaction vessel with a filter was charged with 2-chlorotrityl chloride resin (1.25 mmol/g, 3.10 g, 3.87 mmol) and DCM (21.7 mL), and shaken at room temperature for 45 minutes. Nitrogen pressure was applied to remove DCM, and to compound aa2-001 (1.06 g, 1.94 mmol), methanol (0.627 mL, 15.5 mmol) and DIPEA (1.62 mL, 9.29 mmol), DCM was then added to a total amount of 21.7 mL. The mixed liquid thus obtained was added into the reaction vessel, and the reaction vessel was shaken at room temperature for 60 minutes. Nitrogen pressure was applied to remove the reaction liquid, and to methanol (4.39 mL, 108 mmol) and DIPEA (1.62 mL, 9.29 mmol), DCM was then added to a total amount of 21.7 mL. The mixed liquid thus obtained was added into the reaction vessel, and the reaction vessel was shaken at room temperature for 90 minutes. Nitrogen pressure was applied to remove the reaction liquid, DCM (21.7 mL) was then added, the reaction vessel was shaken for 5 minutes, and nitrogen pressure was then applied to remove the reaction liquid. The resin washing operation using DCM was repeated five times, and the obtained resin was dried overnight under reduced pressure to obtain 3.58 g of aa2-001-resin.

Fmoc quantitation method

For determining the loading rate, a reaction vessel was charged with the obtained aa2-001-resin (11.94 mg), DMF (4.0 mL) was added, and the reaction vessel was shaken at room temperature for 30 minutes. Thereafter, DBU (40 μL) was added, and the reaction vessel was shaken at 30°C for 15 minutes. Thereafter, DMF was added so that the amount of the reaction mixed liquid was 10.0 mL, and 80 μL of the resulting solution was diluted with DMF (920 μL). The diluted solution obtained was analyzed by LC/MS (injection volume: 5 μL), and from the UVarea value of dibenzofulvene (294 nm: 4211.62, 304 nm: 3791.08), the loading rate on aa2-001-resin was calculated to be 0.363 mmol/g. (A calibration curve was prepared on the basis of the UVarea values of dibenzofulvene at wavelengths of 294 nm and 304 nm every measurement day using a mixed solution of Fmoc-Gly-OH having a known concentration (purchased from a commercial supplier) and DBU as a reference material, and an average value of the loading rate calculated at the wavelengths using the calibration curve was defined as the loading rate on the resin.)

Synthesis of compound aa2-002-resin, (3S)-3-[[(2S,3S)-2-[9H-fluoren-9-ylmethoxycarbonyl(methyl)amino]-3-methylpentanoyl]-methylamino]-4-oxo-4-piperidin-1-ylbutanoic acid-2-chlorotrityl resin (Fmoc-MeIle-MeAsp(O-Trt(2-Cl)res-in)-pip)

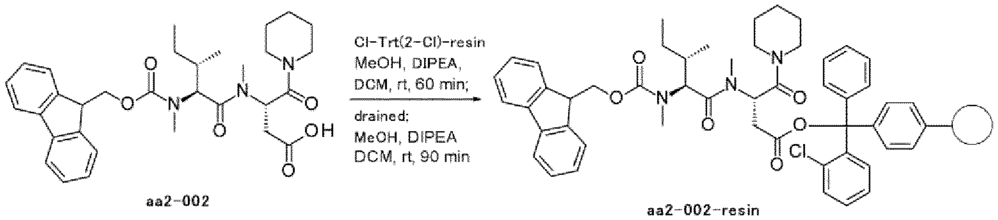


aa2-002 aa2-002-resin

2-Chlorotrityl chloride resin (1.25 mmol/g, 2.98 g, 3.72 mmol) and compound aa2-002 (1.05 g, 1.86 mmol) were provided as starting raw materials, and the same procedure as in the synthesis of compound aa2-001-resin was carried out to obtain 3.54 g of compound aa2-002-resin. The loading rate calculated by the Fmoc quantitation method using a dry resin (10.47 mg) was 0.326 mmol/g. (UVarea value at 294 nm: 3648.96 and UVarea value at 304 nm: 3280.91)

Synthesis of compound aa2-003-resin, (3S)-3-[[(2S)-2-cyclopentyl-2-[9H-fluoren-9-ylmethoxycarbonyl(methyl)amino]acetyl]-methylamino]-4-oxo-4-piperidin-1-ylbutanoic acid-2-chlorotrityl resin (Fmoc-MeGly(cPent)-MeAsp(O-Trt(2-Cl)resin)-pip)

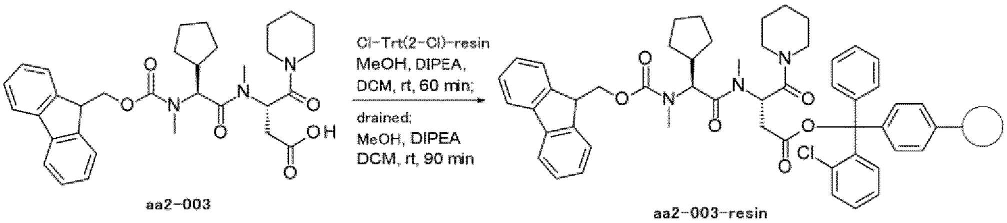


aa2-003 aa2-003-resin

2-Chlorotrityl chloride resin (1.25 mmol/g, 3.11 g, 3.88 mmol) and compound aa2-003 (1.12 g, 1.94 mmol) were provided as starting raw materials, and the same procedure as in the synthesis of compound aa2-001-resin was carried out to obtain 3.73 g of compound aa2-003-resin. The loading rate calculated by the Fmoc quantitation method using a dry resin (11.34 mg) was 0.362 mmol/g. (UVarea value at 294 nm: 3979.93 and UVarea value at 304 nm: 3588.46)

Synthesis of compound aa2-004-resin, (3S)-3-[[(2S)-2-cyclohexyl-2-[9H-fluoren-9-ylmethoxycarbonyl(methyl)aminolacetyl]-methylamino]-4-oxo-4-piperidin-1-ylbutanoic acid-2-chlorotrityl resin (Fmoc-MeGly-MeAsp(O-Trt(2-Cl)res-in)-pip)

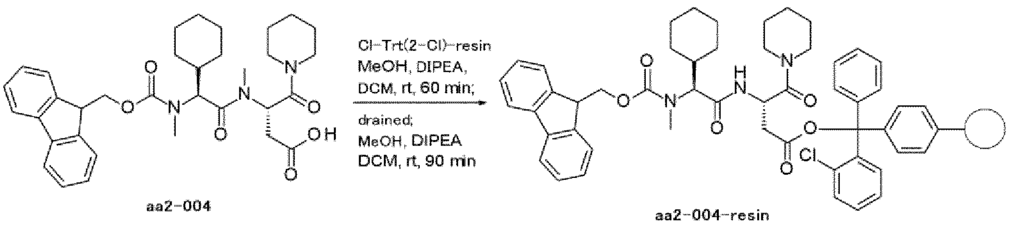


aa2-004 aa2-004-resin

2-Chlorotrityl chloride resin (1.25 mmol/g, 2.57 g, 3.22 mmol) and compound aa2-004 (0.949 g, 1.61 mmol) were provided as starting raw materials, and the same procedure as in the synthesis of compound aa2-001-resin was carried out to obtain 3.07 g of compound aa2-004-resin. The loading rate calculated by the Fmoc quantitation method

using a dry resin (10.09 mg) was 0.347 mmol/g. (UVarea value at 294 nm: 3412.72 and UVarea value at 304 nm: 3069.21)

Synthesis of compound aa2-005-resin, (3S)-3-[[(2S)-2-[9H-fluoren-9-ylmethoxycarbonyl(methyl)amino]-4-methylpentanoyl]-methylamino]-4-oxo-4-piperidin-1-ylbutanoic acid-2-chlorotrityl resin (Fmoc-MeLeu-MeAsp(O-Trt(2-Cl)resin)-pip)

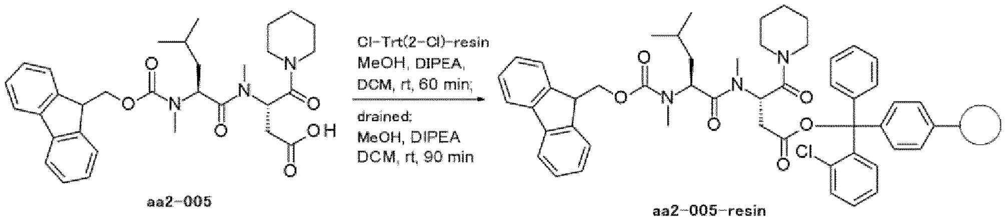


2-Chlorotrityl chloride resin (1.25 mmol/g, 3.09 g, 3.86 mmol) and compound aa2-005 (1.09 g, 1.93 mmol) were provided as starting raw materials, and the same procedure as in the synthesis of compound aa2-001-resin was carried out to obtain 3.57 g of compound aa2-005-resin. The loading rate calculated by the Fmoc quantitation method using a dry resin (10.42 mg) was 0.355 mmol/g. (UVarea value at 294 nm: 3592.54 and UVarea value at 304 nm: 3232.59)

Synthesis of compound aa2-006-resin, (3S)-3-[[(2S)-2-cyclopentyl-2-[9H-fluoren-9-ylmethoxycarbonyl(methyl)aminolacelyl]-methylamino]-4-(dimethylamino)-4-oxobutanoic acid-2-chlorotrityl resin (Fmoc-MeGly(cPent)-MeAsp(O-Trt(2-Cl)resin)-NMe2)

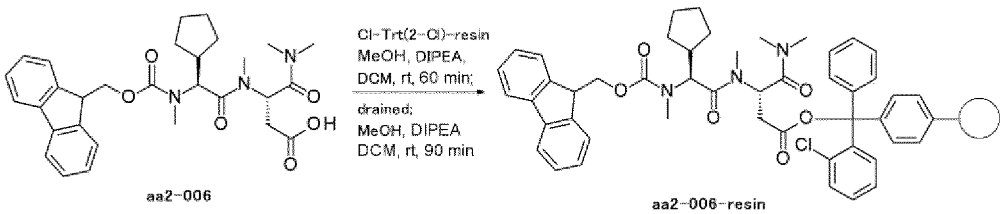


2-Chlorotrityl chloride resin (1.25 mmol/g, 2.35 g, 2.94 mmol) and compound aa2-006 (0.786 g, 1.47 mmol) were provided as starting raw materials, and the same procedure as in the synthesis of compound aa2-001-resin was carried out to obtain 2.72 g of compound aa2-006-resin. The loading rate calculated by the Fmoc quantitation method using a dry resin (11.77 mg) was 0.345 mmol/g. (UVarea value at 294 nm: 3965.86 and UVarea value at 304 nm: 3566.11)

Synthesis of compound aa2-007-resin, (3S)-4-(dimethylamino)-3-[[(2S)-2-[9H-fluoren-9-ylmethoxycarbonyl(methyl)amino]-4-methylpentanoyl]-methylamino]-4-oxobutanoic acid-2-chlorotrityl resin (Fmoc-MeLeu-MeAsp)(O-Trt(2-Cl)resin)-NMe2)

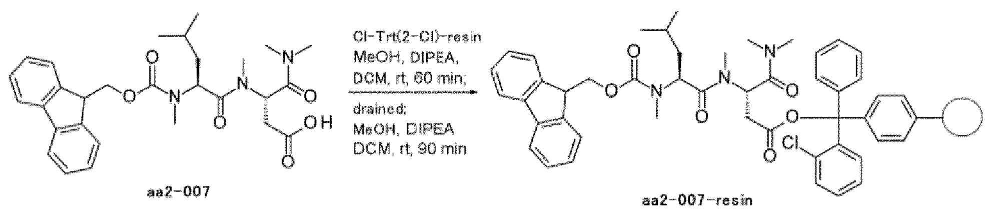


2-Chlorotrityl chloride resin (1.25 mmol/g, 3.04 g, 3.80 mmol) and compound aa2-007 (0.995 g, 1.90 mmol) were provided as starting raw materials, and the same procedure as in the synthesis of compound aa2-001-resin was carried out to obtain 3.51 g of compound aa2-007-resin. The loading rate calculated by the Fmoc quantitation method

using a dry resin (10.88 mg) was 0.384 mmol/g. (UVarea value at 294 nm: 4057.77 and UVarea value at 304 nm: 3645.68)

Synthesis of compound aa2-008-resin, (3R)-3-[[(2S)-2-[9H-fluoren-9-ylmethoxycarbonyl(methyl)amino]-3-methylbutanoyl]-methylamino]butanoic acid-2-chlorotrityl resin (Fmoc-MeVal-D-3-MeAbu-O-Trt(2-Cl)resin)

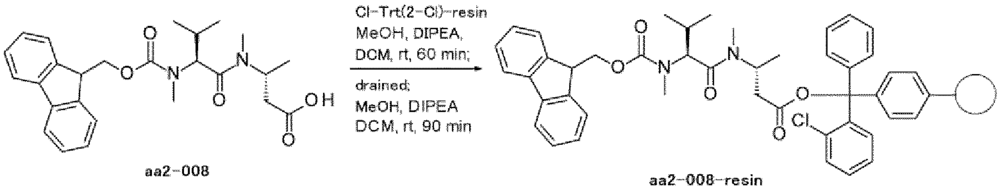


aa2-008 aa2-008-resin

2-Chlorotrityl chloride resin (1.25 mmol/g, 2.15 g, 2.68 mmol) and compound aa2-008 (0.607 g, 1.34 mmol) were provided as starting raw materials, and the same procedure as in the synthesis of compound aa2-001-resin was carried out to obtain 2.47 g of compound aa2-008-resin. The loading rate calculated by the Fmoc quantitation method using a dry resin (11.13 mg) was 0.415 mmol/g. (UVarea value at 294 nm: 4513.80 and UVarea value at 304 nm: 4054.24)

Synthesis of compound aa2-009-resin, (3R)-3-[[(2S)-2-cyclohexyl-2-[9H-fluoren-9-ylmethoxycarbonyl(methyl)amino]acetyl]-methylamino]butanoic acid-2-chlorotrityl resin (Fmoc-MeChg-D-3-MeAbu-O-Trt(2-Cl)resin)

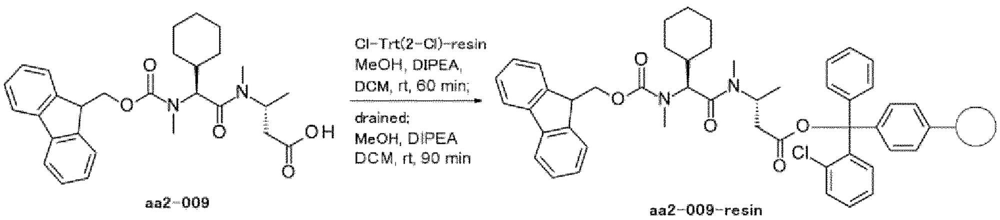


aa2-009 aa2-009-resin

2-Chlorotrityl chloride resin (1.25 mmol/g, 1.62 g, 2.03 mmol) and compound aa2-009 (0.500 g, 1.02 mmol) were provided as starting raw materials, and the same procedure as in the synthesis of compound aa2-001-resin was carried out to obtain 1.91 g of compound aa2-009-resin. The loading rate calculated by the Fmoc quantitation method using a dry resin (12.74 mg) was 0.397 mmol/g. (UVarea value at 294 nm: 4946.37 and UVarea value at 304 nm: 4444.88)

Synthesis of compound aa2-010-resin, 2-[[(2S)-2-[9H-fluoren-9-ylmethoxycarbonyl(methyl)amino]-3-methylbutanoyl]-methylamino]acetic acid-2-chlorotrityl resin (Fmoc-MeVal-MeGly-O-Trt(2-Cl)resin)

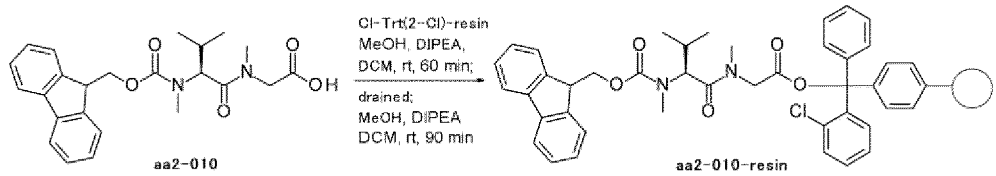


aa2-010 aa2-010-resin

2-Chlorotrityl chloride resin (1.25 mmol/g, 3.17 g, 3.96 mmol) and compound aa2-010 (0.841 g, 1.98 mmol) were provided as starting raw materials, and the same procedure as in the synthesis of compound aa2-001-resin was carried out to obtain 3.49 g of compound aa2-010-resin. The loading rate calculated by the Fmoc quantitation method using a dry resin (11.25 mg) was 0.374 mmol/g. (UVarea value at 294 nm: 4080.46 and UVarea value at 304 nm: 3686.94)

Synthesis of compound aa2-011-resin, (3S)-3-[[(2S)-2-cyclolentyl-2-[9H-fluoren-9-ylmethoxycarbonyl(methyl)amino]acetyl]-methylamino]-4-morpholino-4-oxobutanoic acid-2-chlorotrityl resin (Fmoc-MeGly(cPent)-MeAsp(O-Trt(2-Cl)resin)-mor)

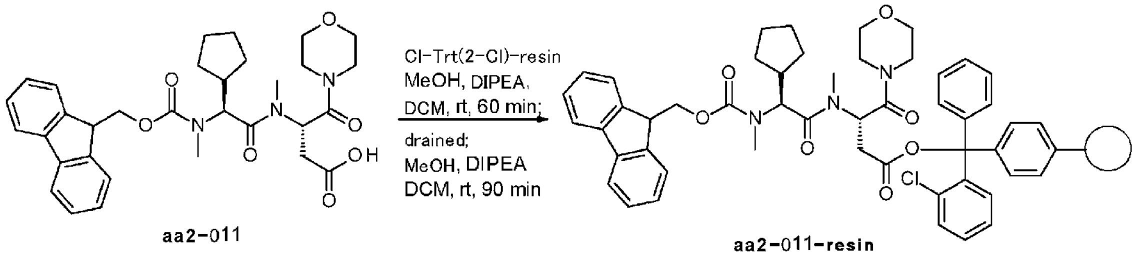


aa2-011 aa2-011-resin

A reaction vessel (400 mL) with a filter was charged with 2-chlorotrityl chloride resin (1.36 mmol/g, 20.5 g, 15.07 mmol) and DCM (140 mL), and left standing at room temperature for 1 hour. Nitrogen pressure was applied to remove DCM, and a solution of compound aa2-011 (9.50 g, 16.45 mmol), methanol (5.32 mL, 132 mmol) and DIPEA (13.8 mL, 79 mmol) in DCM (140 mL) was then added into the reaction vessel. The reaction vessel was shaken at 25°C at 60 rpm for 60 minutes. Nitrogen pressure was applied to remove the reaction liquid, and a solution of methanol (20 mL, 493 mmol) and DIPEA (13.8 mL, 79 mmol) in DCM (140 mL) was then added into the reaction vessel. The reaction vessel was shaken at 25°C at 60 rpm for 60 minutes. Nitrogen pressure was applied to remove the reaction liquid, DCM (140 mL) was then added, and the mixture was mixed, and then discharged by application of nitrogen pressure. The resin washing operation using DCM was repeated five times in total, and the obtained resin was dried for an entire day under reduced pressure to obtain 26.89 g of aa2-011-resin. The loading rate of amino acids on the resin was calculated as follows. A reaction vessel was charged with the obtained compound aa2-011-resin (10 mg), DMF (2 mL) was added, and the mixture was left standing at room temperature for 1 hour. Thereafter, DBU (40 μL) was added, and the reaction vessel was shaken at 25°C for 30 minutes. Thereafter, DMF (8 mL) was added to the reaction mixed liquid, and 1 ml of the resulting solution was diluted with DMF (11.5 mL). The absorbance (294 nm) of the diluted solution obtained was measured (using UV-1600 PC (cell length: 1.0 cm) from Shimadzu Corporation), and the loading rate on compound aa2-011-resin was calculated to be 0.415 mmol/g.

Synthesis of compound aa2-012-resin, (3S)-4-(dimethylamino)-3-[[(2S)-2-[9H-fluoren-9-ylmethoxycarbonyl(methyl)amino]-3-methylbutanoyl]amino]-4-oxobutanoic acid-2-chlorotrityl resin (Fmoc-MeVal-Asp)(O-Trt(2-Cl)resin)-NMe2)

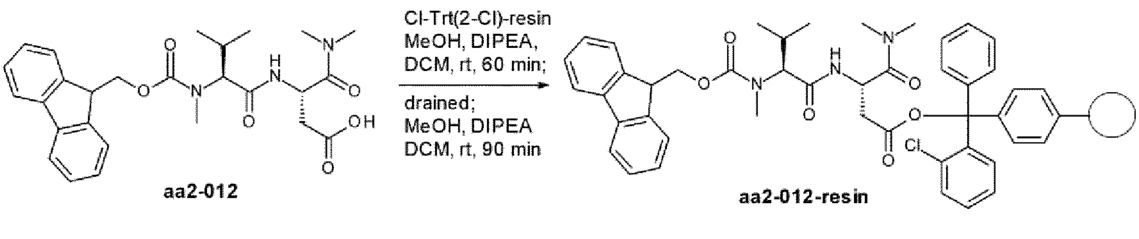


aa2-012 aa2-012-resin

2-Chlorotrityl chloride resin (1.25 mmol/g, 3.28 g, 4.10 mmol) and compound aa2-012 (1.02 g, 2.05 mmol) were provided as starting raw materials, and the same procedure as in the synthesis of compound aa2-001-resin was carried out to obtain 3.73 g of compound aa2-012-resin. The loading rate calculated by the Fmoc quantitation method using a dry resin (12.55 mg) was 0.373 mmol/g. (UVarea value at 294 nm: 5042.25 and UVarea value at 304 nm: 4531.21)

Synthesis of compound aa2-013-resin, (3S)-4-(dimethylamino)-3-[[2-(9H-fluoren-9-ylmethoxycarbonylamino)acetyl]-methylamino]-4-oxobutanoic acid-2-chlorotrityl resin (Fmoc-Gly-MeAsp(O-Trt(2-Cl)resin)-NMe2)

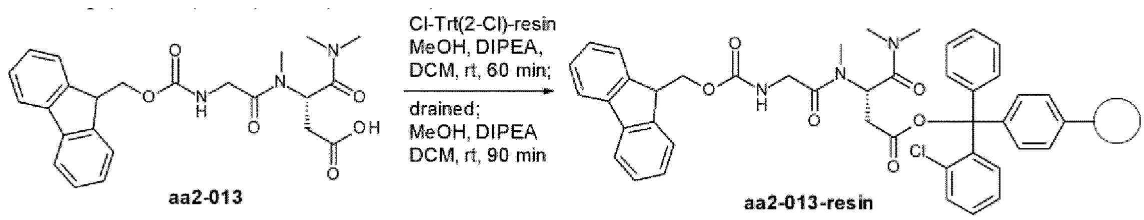


aa2-013 aa2-013-resin

2-Chlorotrityl chloride resin (1.25 mmol/g, 3.57 g, 4.46 mmol) and compound aa2-013 (1.01 g, 2.23 mmol) were provided as starting raw materials, and the same procedure as in the synthesis of compound aa2-001-resin was carried out to obtain 3.98 g of compound aa2-013-resin. The loading rate calculated by the Fmoc quantitation method using a dry resin (12.33 mg) was 0.386 mmol/g. (UVarea value at 294 nm: 5134.21 and UVarea value at 304 nm: 4593.14)

Synthesis of compound aa2-014-resin, (3S)-4-(dimethylamino)-3-[[2-(9H-fluoren-9-ylmethoxycarbonylamino)-2-methylpropanoyl]-methylamino]-4-oxobutanoic acid-2-chlorotrityl resin (Fmoc-Aib-MeAso(O-Trt(2-Cl)resin)-NMe2)

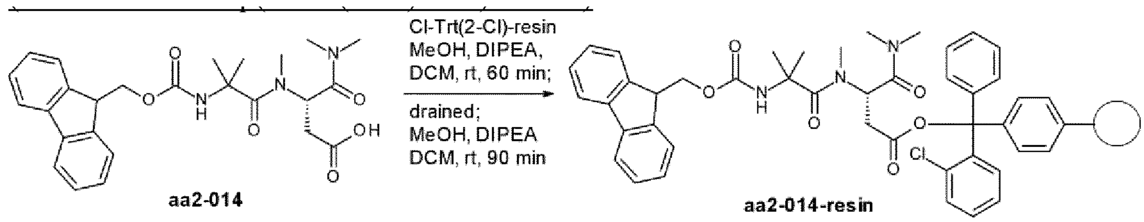


aa2-014 aa2-014-resin

2-Chlorotrityl chloride resin (1.25 mmol/g, 3.46 g, 4.32 mmol) and compound aa2-014 (1.04 g, 2.16 mmol) were provided as starting raw materials, and the same procedure as in the synthesis of compound aa2-001-resin was carried out to obtain 3.96 g of compound aa2-014-resin. The loading rate calculated by the Fmoc quantitation method using a dry resin (10.22 mg) was 0.413 mmol/g. (UVarea value at 294 nm: 4205.24 and UVarea value at 304 nm: 3774.43)

Synthesis of compound aa2-015-resin, (3R)-3-[[3-(9H-fluoren-9-ylmethoxycarbonylamino)-2,2-dimethylpropanoyl]-methylamino]butanoic acid-2-chlorotrityl resin (Fmoc-bAla(2-Me2)-D-3-MeAbu-O-Trt(2-Cl)resin)

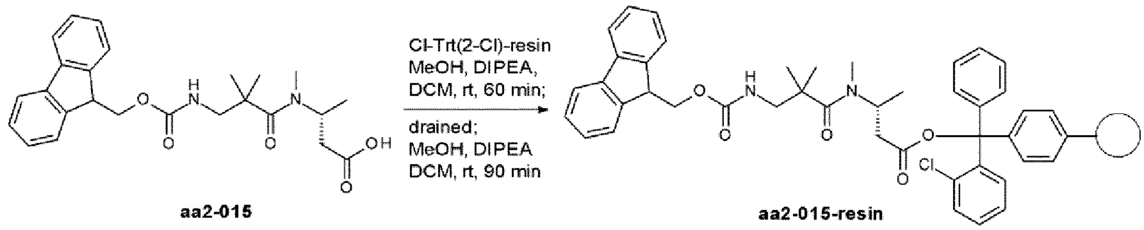


aa2-015 aa2-015-resin

2-Chlorotrityl chloride resin (1.25 mmol/g, 1.02 g, 1.28 mmol) and compound aa2-015 (281 mg, 0.640 mmol) were provided as starting raw materials, and the same procedure as in the synthesis of compound aa2-001-resin was carried out to obtain 1.14 g of compound aa2-015-resin. The loading rate calculated by the Fmoc quantitation method using a dry resin (10.46 mg) was 0.443 mmol/g. (UVarea value at 294 nm: 4615.90 and UVarea value at 304 nm: 4143.20)

Synthesis of compound aa2-016-resin, (2S)-2-[[(2S)-2-[9H-fluoren-9-ylmethoxycarbonyl(methyl)amino]-4-methylpentanoyl]-methylamino]-3-methylbutanoic acid-2-chlorotrityl resin (Fmoc-MeLeu-MeVal-O-Trt(2-Cl)resin)

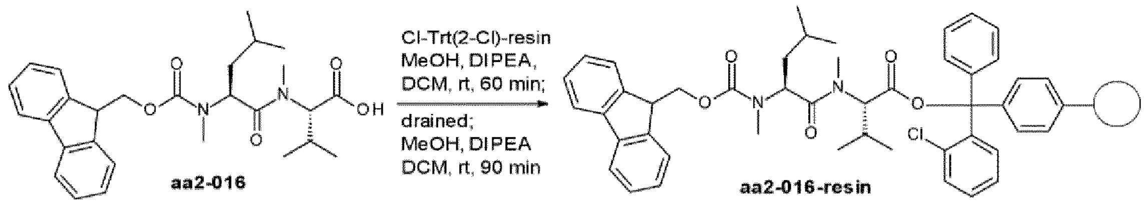


2-Chlorotrityl chloride resin (1.25 mmol/g, 617 mg, 0.771 mmol) and compound aa2-016 (185 mg, 0.386 mmol) were provided as starting raw materials, and the same procedure as in the synthesis of compound aa2-001-resin was carried out to obtain 663 mg of compound aa2-016-resin. The loading rate calculated by the Fmoc quantitation method using a dry resin (12.18 mg) was 0.348 mmol/g. (UVarea value at 294 nm: 4325.21 and UVarea value at 304 nm: 3876.60)

Synthesis of compound aa2-017-resin, (2S)-1-[(2S)-2-[9H-fluoren-9-ylmethoxycarbonyl(methyl)amino]-3-methylbutanoyl]pyrrolidine-2-carboxylic acid-2-chlorotrityl resin (Fmoc-MeVal-Pro-O-Trt(2-Cl)resin)

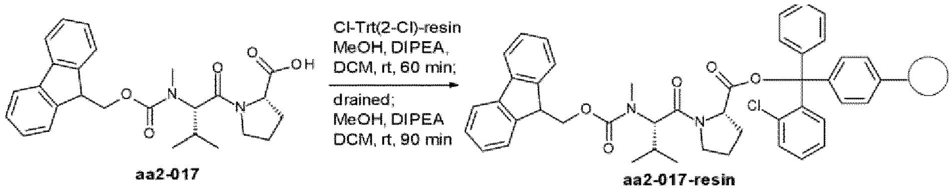


2-Chlorotrityl chloride resin (1.25 mmol/g, 4.79 g, 5.99 mmol) and compound aa2-017 (1.35 g, 3.00 mmol) were provided as starting raw materials, and the same procedure as in the synthesis of compound aa2-001-resin was carried out to obtain 5.40 g of compound aa2-017-resin. The loading rate calculated by the Fmoc quantitation method using a dry resin (10.38 mg) was 0.364 mmol/g. (UVarea value at 294 nm: 3850.10 and UVarea value at 304 nm: 3472.31)

Synthesis of compound aa2-018-resin, 2-[[(2S)-2-[9H-fluoren-9-ylmethoxycarbonyl(methyl)amino]-4-methylpentanoyl]amino]-2-methylpropanoic acid-2-chlorotrityl resin (Fmoc-MeLeu-Aib-O-Trt(2-Cl)resin)

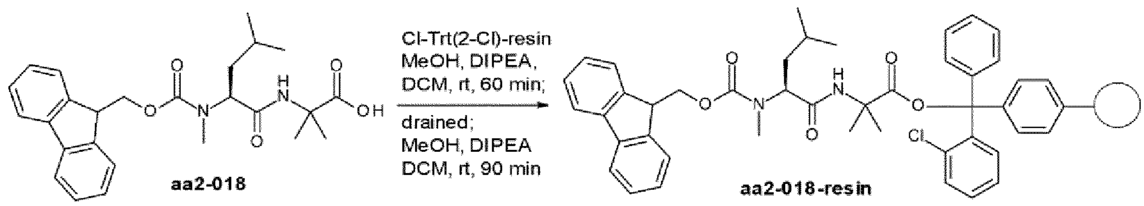


2-Chlorotrityl chloride resin (1.25 mmol/g, 2.15 g, 2.69 mmol) and compound aa2-018 (608 mg, 1.34 mmol) were provided as starting raw materials, and the same procedure as in the synthesis of compound aa2-001-resin was carried out to obtain 2.29 g of compound aa2-018-resin. The loading rate calculated by the Fmoc quantitation method using a dry resin (9.61 mg) was 0.300 mmol/g. (UVarea value at 294 nm: 2934.11 and UVarea value at 304 nm: 2651.64)

Synthesis of compound aa2-019-resin, 2-[[(2S)-2-[9H-fluoren-9-ylmethoxycarbonyl(methyl)amino]-3-methylbutanoyl]amino]acetic acid-2-chlorotrityl resin (Fmoc-MeVal-Gly-O-Trt(2-Cl)resin)

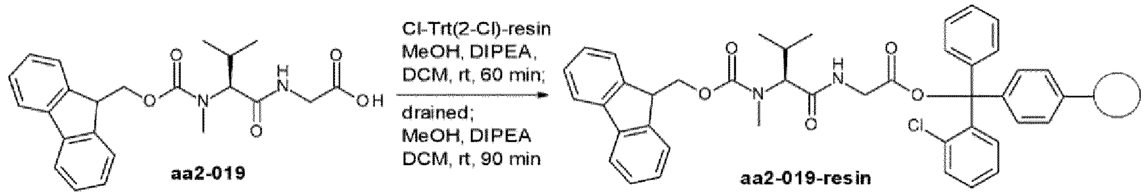


aa2-019 aa2-019-resin

2-Chlorotrityl chloride resin (1.25 mmol/g, 5.11 g, 6.38 mmol) and compound aa2-019 (1.31 g, 3.19 mmol) were provided as starting raw materials, and the same procedure as in the synthesis of compound aa2-001-resin was carried out to obtain 5.46 g of compound aa2-019-resin. The loading rate calculated by the Fmoc quantitation method using a dry resin (10.73 mg) was 0.303 mmol/g. (UVarea value at 294 nm: 3312.44 and UVarea value at 304 nm: 2987.09)

Synthesis of compound aa2-020-resin, (3S)-4-(dimethylamino)-3-[[(2R)-2-[9H-fluoren-9-ylmethoxycarbonyl(methyl)amino]-3-methylbutanoyl]-methylamino]-4-oxobutanoic acid-2-chlorotrityl resin (Fmoc-D-MeVal-MeAsp(O-Trt(2-Cl)resin)-NMe2)

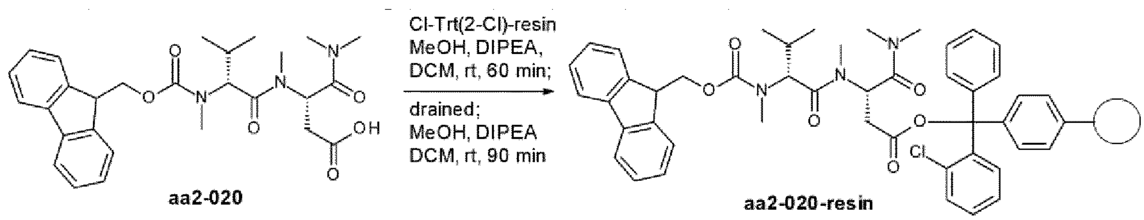


aa2-020 aa2-020-resin

2-Chlorotrityl chloride resin (1.25 mmol/g, 3.77 g, 4.71 mmol) and compound aa2-020 (1.20 g, 2.36 mmol) were provided as starting raw materials, and the same procedure as in the synthesis of compound aa2-001-resin was carried out to obtain 4.47 g of compound aa2-020-resin. The loading rate calculated by the Fmoc quantitation method using a dry resin (11.75 mg) was 0.396 mmol/g. (UVarea value at 294 nm: 4635.95 and UVarea value at 304 nm: 4167.33)

Synthesis of compound aa2-021-resin, (3S)-4-(dimethylamino)-3-[[3-(9H-fluoren-9-ylmethoxycarbonylamino)-22-dimethylpropanoyl]-methylamino]-4-oxobutanoic acid-2-chlorotrityl resin (Fmoc-bAla(2-Me2)-MeAsp(O-Trt(2-Cl)resin)-NMe2)

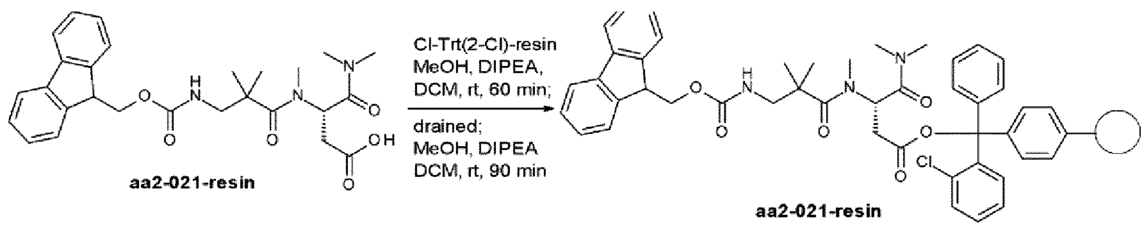


aa2-021-resin aa2-021-resin

2-Chlorotrityl chloride resin (1.25 mmol/g, 2.82 g, 3.53 mmol) and compound aa2-021 (874 mg, 1.76 mmol) were provided as starting raw materials, and the same procedure as in the synthesis of compound aa2-001-resin was carried out to obtain 3.24 g of compound aa2-021-resin. The loading rate calculated by the Fmoc quantitation method using a dry resin (9.82 mg) was 0.407 mmol/g. (UVarea value at 294 nm: 3979.96 and UVarea value at 304 nm: 3574.96)

Synthesis of compound aa2-022-resin, (3R)-4-(dimethylamino)-3-[[(2S)-2-[9H-fluoren-9-ylmethoxycarbonyl(methyl)amino]-3-methylbutanoyl]-methylamino]-4-oxobutanoic acid-2-chlorotrityl resin (Fmoc-MeVal-D-MeAsp(O-Trt(2-Cl)resin)-NMe2)

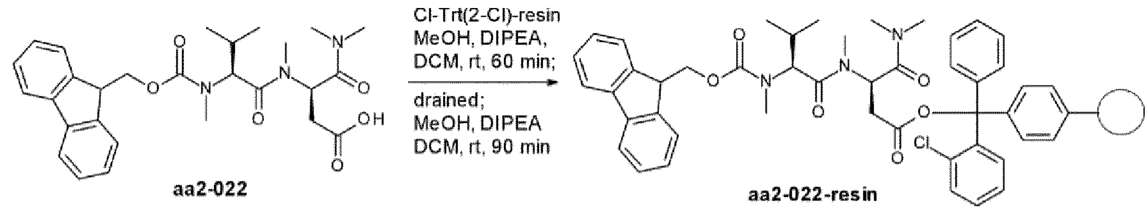


2-Chlorotrityl chloride resin (1.25 mmol/g, 3.97 g, 4.96 mmol) and compound aa2-022 (1.26 g, 2.48 mmol) were provided as starting raw materials, and the same procedure as in the synthesis of compound aa2-001-resin was carried out to obtain 4.75 g of compound aa2-022-resin. The loading rate calculated by the Fmoc quantitation method using a dry resin (10.79 mg) was 0.396 mmol/g. (UVarea value at 294 nm: 4264.54 and UVarea value at 304 nm: 3819.26)

Synthesis of compound aa2-023-resin, (3S)-3-[[(2S)-2-cyclolpentyl-2-[[(2S)-2-(9H-fluoren-9-ylmethoxycarbonylamino)propanoyl]-methylamino]acetyl]-methylamino]-4-(dimethylamino)-4-oxobutanoic acid-2-chlorotrityl resin (Fmoc-Ala-MeGly(cPent)-MeAsp)(O-Trt(2-Cl)resin)-NMe2)

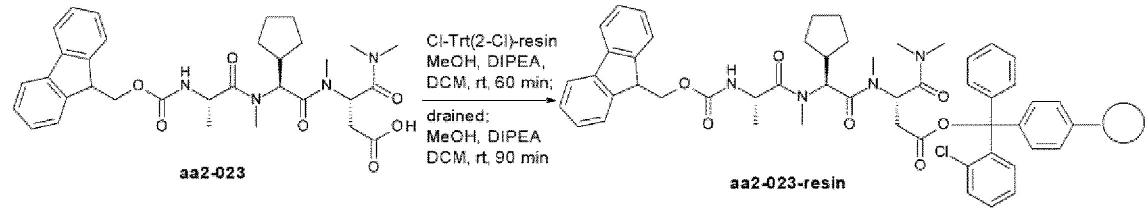


2-Chlorotrityl chloride resin (1.25 mmol/g, 700 mg, 0.875 mmol) and compound aa2-023 (265 mg, 0.437 mmol) were provided as starting raw materials, and the same procedure as in the synthesis of compound aa2-001-resin was carried out to obtain 801 mg of compound aa2-023-resin. The loading rate calculated by the Fmoc quantitation method using a dry resin (9.35 mg) was 0.378 mmol/g. (UVarea value at 294 nm: 3477.62 and UVarea value at 304 nm: 3130.63)

Synthesis of compound aa2-024-resin, (2S)-1-[(2S)-2-(9H-fluoren-9-ylmethoxycarbonylamino)-3-phenylpropanoyl]pyrrolidine-2-carboxylic acid-2-chlorotrityl resin (Fmoc-Phe-Pro-O-Trt(2-Cl)resin)

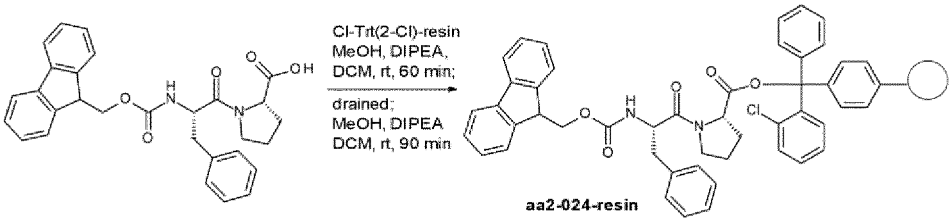


2-Chlorotrityl chloride resin (1.25 mmol/g, 1.12 g, 1.40 mmol) and Fmoc-Phe-Pro-OH (339 mg, 0.700 mmol) were provided as starting raw materials, and the same procedure as in the synthesis of compound aa2-001-resin was carried out to obtain 1.23 g of compound aa2-024-resin. The loading rate calculated by the Fmoc quantitation method using a dry resin (9.62 mg) was 0.383 mmol/g. (UVarea value at 294 nm: 3782.71 and UVarea value at 304 nm: 3403.88)

Synthesis of compound aa2-025-resin, (2S)-1-[(2S)-2-(9H-fluoren-9-ylmethoxycarbonylamino)-6-[(2-methylpropan-2-yl)oxycarbonylamino]hexanoyl]pyrrolidine-2-carboxylic acid-2-chlorotrityl resin (Fmoc-Lys(Boc)-Pro-O-Trt(2-Cl)resin)

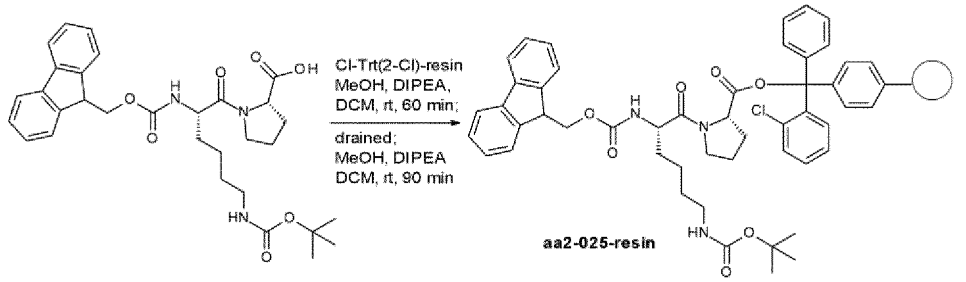


aa2-025-resin

2-Chlorotrityl chloride resin (1.25 mmol/g, 1.12 g, 1.40 mmol) and Fmoc-Lys(Boc)-Pro-OH (396 mg, 0.700 mmol) were provided as starting raw materials, and the same procedure as in the synthesis of compound aa2-001-resin was carried out to obtain 1.24 g of compound aa2-025-resin. The loading rate calculated by the Fmoc quantitation method using a dry resin (11.50 mg) was 0.339 mmol/g. (UVarea value at 294 nm: 4200.46 and UVarea value at 304 nm: 3772.96)

Synthesis of compound aa2-026-resin, (2S)-2-[[2-(9H-fluoren-9-ylmethoxycarbonylamino)acetyl]amino]-3-[4-[(2-methylpropan-2-yl)oxy]phenyl]propanoic acid-2-chlorotrityl resin (Fmoc-Gly-Tyr(tBu)-O-Trt(2-Cl)resin)

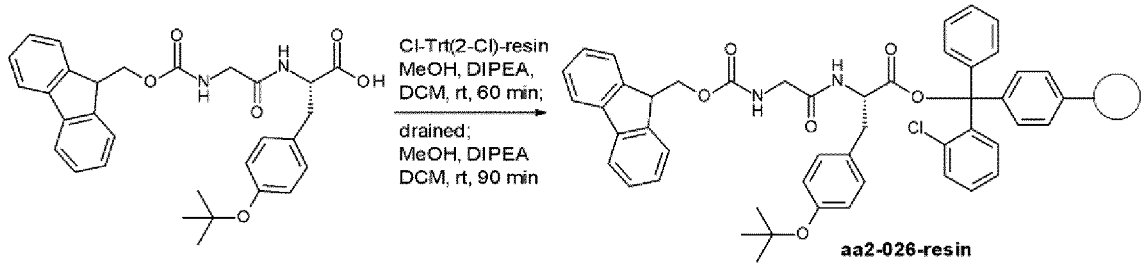


aa2-026-resin

2-Chlorotrityl chloride resin (1.25 mmol/g, 1.12 g, 1.40 mmol) and Fmoc-Gly-Tyr(tBu)-OH (362 mg, 0.700 mmol) were provided as starting raw materials, and the same procedure as in the synthesis of compound aa2-001-resin was carried out to obtain 1.15 g of compound aa2-026-resin. The loading rate calculated by the Fmoc quantitation method using a dry resin (9.88 mg) was 0.244 mmol/g. (UVarea value at 294 nm: 2478.91 and UVarea value at 304 nm: 2222.03)

Synthesis of compound aa2-027-resin, (2S)-2-[[(2S)-2-(9H-fluoren-9-ylmethoxycarbonylamino)propanoyl]amino]propanoic acid-2-chlorotrityl resin (Fmoc-Ala-Ala-O-Trt(2-Cl)resin)

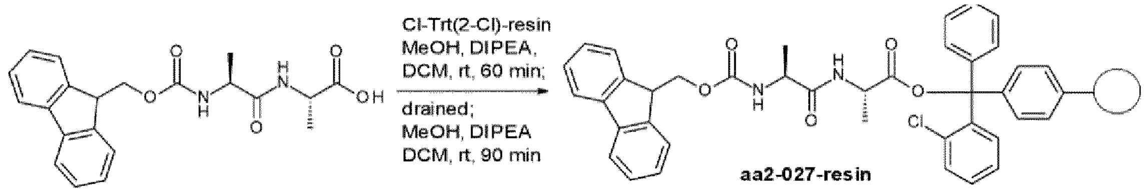


aa2-027-resin

2-Chlorotrityl chloride resin (1.25 mmol/g, 1.28 g, 1.60 mmol) and Fmoc-Ala-Ala-OH (306 mg, 0.800 mmol) were provided as starting raw materials, and the same procedure as in the synthesis of compound aa2-001-resin was carried out to obtain 1.39 g of compound aa2-027-resin. The loading rate calculated by the Fmoc quantitation method using a dry resin (9.69 mg) was 0.334 mmol/g. (UVarea value at 294 nm: 3496.53 and UVarea value at 304 nm: 3128.64)

Synthesis of compound aa2-028-resin, 2-[[(2S)-2-(9H-fluoren-9-ylmethoxycarbonylamino)-3-phenylpropanoyl]amino]acetic acid-2-chlorotrityl resin (Fmoc-Phe-Gly-O-Trt(2-Cl)resin)

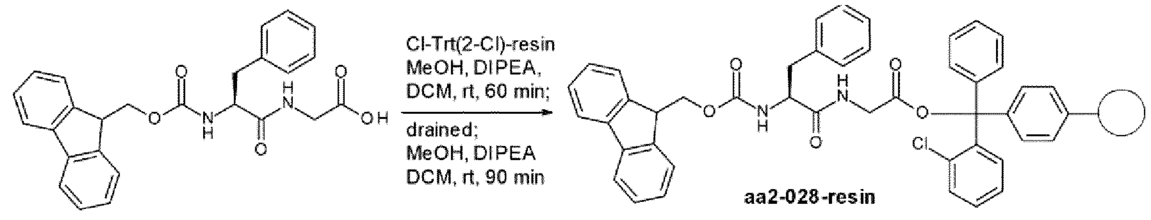


2-Chlorotrityl chloride resin (1.25 mmol/g, 1.28 g, 1.60 mmol) and Fmoc-Phe-Gly-OH (356 mg, 0.800 mmol) were provided as starting raw materials, and the same procedure as in the synthesis of compound aa2-001-resin was carried out to obtain 1.36 g of compound aa2-028-resin. The loading rate calculated by the Fmoc quantitation method using a dry resin (10.12 mg) was 0.248 mmol/g. (UVarea value at 294 nm: 2703.55 and UVarea value at 304 nm: 2442.89)

Synthesis of compound aa2-029-resin, 2-[[(2S)-2-(9H-fluoren-9-ylmethoxycarbonylamino-4-oxo-4-(tritylamino)butanoyl]amino]acetic acid-2-chlorotrityl resin (Fmoc-Asn(Trt)-Gly-O- Trt(2-Cl)resin)

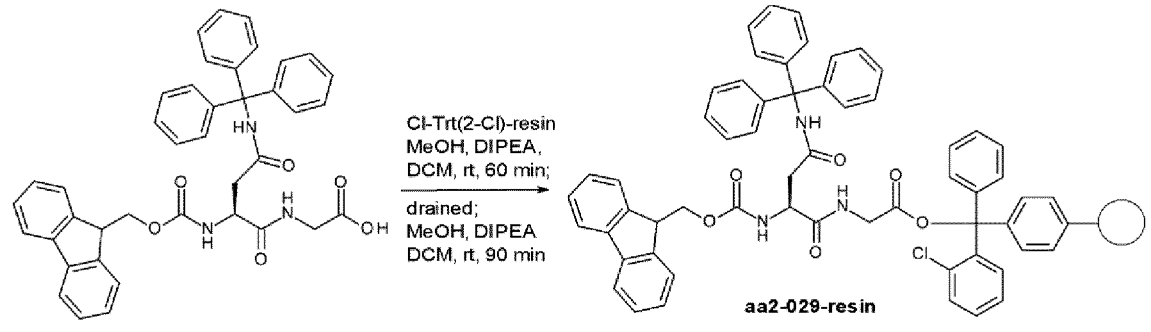


2-Chlorotrityl chloride resin (1.25 mmol/g, 1.12 g, 1.40 mmol) and Fmoc-Asn(Trt)-Gly-OH (458 mg, 0.700 mmol) were provided as starting raw materials, and the same procedure as in the synthesis of compound aa2-001-resin was carried out to obtain 1.15 g of compound aa2-029-resin. The loading rate calculated by the Fmoc quantitation method using a dry resin (9.67 mg) was 0.259 mmol/g. (UVarea value at 294 nm: 2698.79 and UVarea value at 304 nm: 2427.13)

Synthesis of compound aa2-030-resin, (2S)-2-[[2-(9H-fluoren-9-ylmethoxycarbonylamino)acelyl]amino]-3-methylbutanoic acid-2-chlorotrityl resin (Fmoc-Gly-Val-O-Trt(2-Cl)resin)

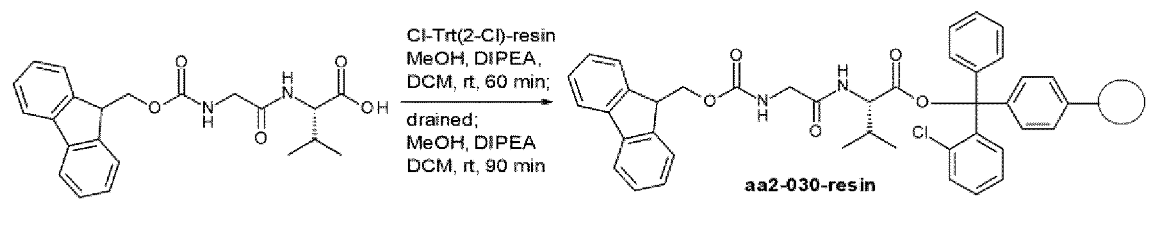


2-Chlorotrityl chloride resin (1.25 mmol/g, 1.28 g, 1.60 mmol) and Fmoc-Gly-Val-OH (317 mg, 0.800 mmol) were provided as starting raw materials, and the same procedure as in the synthesis of compound aa2-001-resin was carried out to obtain 1.38 g of compound aa2-030-resin. The loading rate calculated by the Fmoc quantitation method using a dry resin (10.06 mg) was 0.278 mmol/g. (UVarea value at 294 nm: 3013.51 and UVarea value at 304 nm: 2712.54)

Synthesis of compound aa2-031-resin, 2-[[(2S)-2-(9H-fluoren-9-ylmethoxycarbonylamino)-3-[(2-methylpropan-2-yl)oxy]propanoyl]amino]acetic acid-2-chlorotrityl resin (Fmoc-Ser(tBu)-Gly-O-Trt(2-Cl)resin)

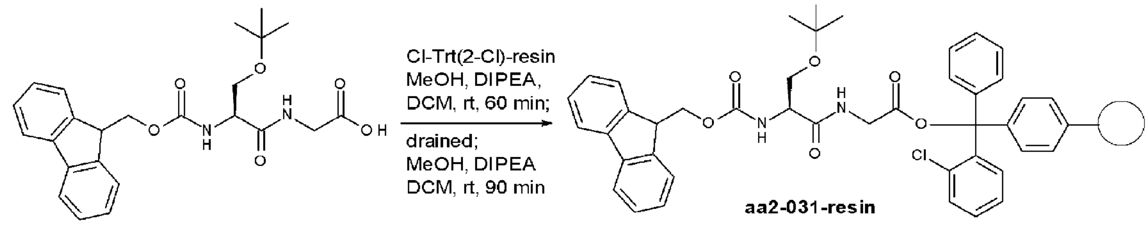


aa2-031-resin

2-Chlorotrityl chloride resin (1.25 mmol/g, 1.12 g, 1.40 mmol) and Fmoc-Ser(tBu)-Gly-OH (308 mg, 0.700 mmol) were provided as starting raw materials, and the same procedure as in the synthesis of compound aa2-001-resin was carried out to obtain 1.10 g of compound aa2-031-resin. The loading rate calculated by the Fmoc quantitation method using a dry resin (10.35 mg) was 0.258 mmol/g. (UVarea value at 294 nm: 2884.14 and UVarea value at 304 nm: 2584.43)

Synthesis of compound aa2-032-resin, (2S)-1-[(2S)-2-[[(2S)-2-(9H-fluoren-9-ylmethoxycarbonylamino)propanoyl]amino]propanoyl]pyrrolidine-2-carboxylic acid-2-chlorotrityl resin (Fmoc-Ala-Ala-Pro-O-Trt(2-Cl)resin)

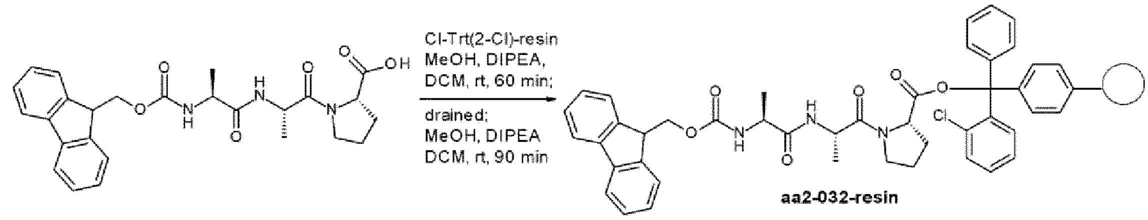


aa2-032-resin

2-Chlorotrityl chloride resin (1.25 mmol/g, 1.12 g, 1.40 mmol) and Fmoc-Ala-Ala-Pro-OH (336 mg, 0.700 mmol) were provided as starting raw materials, and the same procedure as in the synthesis of compound aa2-001-resin was carried out to obtain 1.24 g of compound aa2-032-resin. The loading rate calculated by the Fmoc quantitation method using a dry resin (11.15 mg) was 0.393 mmol/g. (UVarea value at 294 nm: 4495.05 and UVarea value at 304 nm: 4047.48)

Synthesis of compound aa3-001-resin, (3S)-3-[9H-fluoren-9-ylmethoxycarbonyl(methyl)amino]-4-oxo-4-piperidin-1-ylbutanoic acid-2-chlorotrityl resin (Fmoc-MeAsp(O-Trt(2-Cl)resin)-pip)

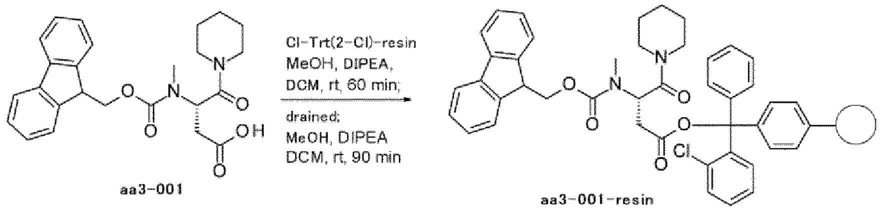


aa3-001 aa3-001-resin

2-Chlorotrityl chloride resin (1.44 mmol/g, 44.5 g, 64.1 mmol) and compound aa3-001 (14.0 g, 32.1 mmol) were provided as starting raw materials, and the same procedure as in the synthesis of compound aa2-001-resin was carried out to obtain 52.7 g of compound aa3-001-resin. The loading rate calculated by the Fmoc quantitation method using a dry resin (11.29 mg) was 0.455 mmol/g. (UVarea value at 294 nm: 5277.47 and UVarea value at 304 nm: 4746.13)

Synthesis of compound aa3-002-resin, (3S)-4-(dimethylamino)-3-[9H-fluoren-9-ylmethoxycarbonyl(methyl)amino]-4-oxobutanoic acid-2-chlorotrityl resin (Fmoc-MeAsp(O-Trt(2-Cl)resin)-NMe2)

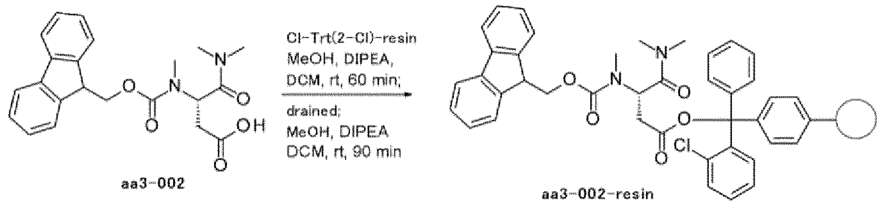


aa3-002 aa3-002-resin

2-Chlorotrityl chloride resin (1.60 mmol/g, 8.83 g, 14.1 mmol) and compound aa3-002 (2.80 g, 7.06 mmol) were provided as starting raw materials, and the same procedure as in the synthesis of compound aa2-001-resin was carried out to obtain 10.4 g of compound aa3-002-resin. The loading rate calculated by the Fmoc quantitation method using a dry resin (11.04 mg) was 0.442 mmol/g. (UVarea value at 294 nm: 4990.63 and UVarea value at 304 nm: 4516.89)

Synthesis of compound aa3-003-resin, (3S)-4-(dimethylamino)-3-[9H-fluoren-9-ylmethoxycarbonylamino)-4-oxobutanoic acid-2-chlorotrityl resin (Fmoc-Asp(O-Trt(2-Cl)resin)-NMe2)

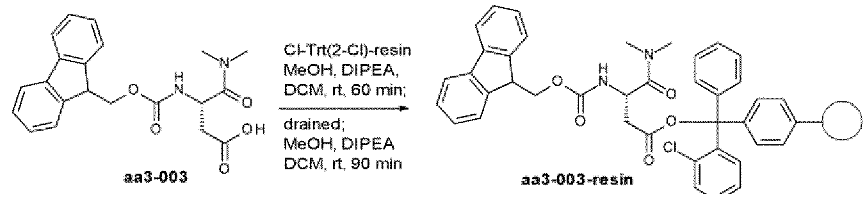


**aa3-003** **aa3-003-resin**

2-Chlorotrityl chloride resin (1.44 mmol/g, 39.0 g, 56.2 mmol) and compound aa3-003 (10.7 g, 28.0 mmol) were provided as starting raw materials, and the same procedure as in the synthesis of compound aa2-001-resin was carried out to obtain 45.0 g of compound aa3-003-resin. The loading rate calculated by the Fmoc quantitation method using a dry resin (10.48 mg) was 0.469 mmol/g. (UVarea value at 294 nm: 4929.82 and UVarea value at 304 nm: 4428.76)

Synthesis of compound aa4-001-resin, (3R)-3-[9H-fluoren-9-ylmethoxycarbonyl(methyl)amino]butanoic acid-2-chlorotrityl resin (Fmoc-D-3-MeAbu-O-Trt(2-Cl)resin)

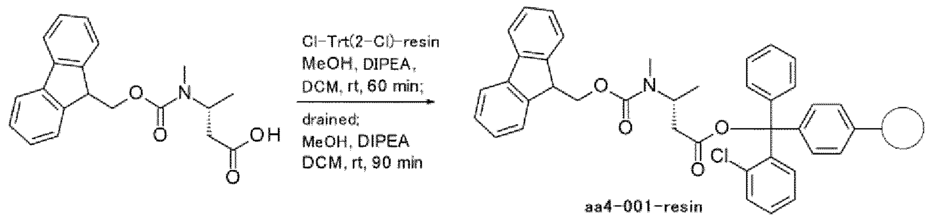


aa4-001-resin

A reaction vessel with a filter was charged with 2-chlorotrityl chloride resin (1.60 mmol/g, 25.0 g, 40.0 mmol) and DCM (125 mL), and shaken at room temperature for 20 minutes. Nitrogen pressure was applied to remove DCM, and to compound Fmoc-D-3-MeAbu-OH (3.60 g, 10.6 mmol), methanol (0.859 mL, 21.2 mmol) and DIPEA (12.3 mL, 70.7 mmol), DCM was then added to a total amount of 145 mL. The mixed liquid thus obtained was added into the reaction vessel, and the reaction vessel was shaken at room temperature for 30 minutes. Nitrogen pressure was applied to remove the reaction liquid, and to methanol (12.5 mL, 143 mmol) and DIPEA (12.5 mL, 71.8 mmol), DCM was then added to a total amount of 250 mL. The mixed liquid thus obtained was added into the reaction vessel, and the reaction vessel was shaken at room temperature for 90 minutes. Nitrogen pressure was applied to remove the reaction liquid, DCM (180 mL) was then added, the reaction vessel was shaken for 5 minutes, and nitrogen pressure was then applied

to remove the reaction liquid. The resin washing operation using DCM was repeated three times, and the obtained resin was dried overnight under reduced pressure to obtain 28.3 g of aa4-001-resin. The loading rate calculated by the Fmoc quantitation method using a dry resin (10.36 mg) was 0.369 mmol/g. (UVarea value at 294 nm: 3920.38 and UVarea value at 304 nm: 3530.84)

Synthesis of compound aa4-002-resin, 2-[9H-fluoren-9-ylmethoxycarbonyl(methyl)amino]acetic acid-2-chlorotrityl resin (Fmoc-MeGly-O-Trt(2-Cl)resin)

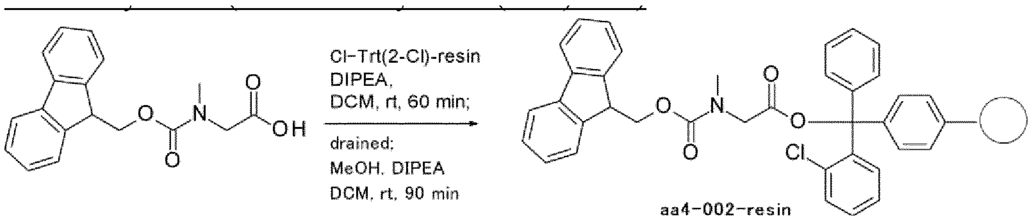


aa4-002-resin

A reaction vessel with a filter was charged with 2-chlorotrityl chloride resin (1.60 mmol/g, 12.3 g, 19.7 mmol) and DCM (125 mL), and shaken at room temperature for 20 minutes. Nitrogen pressure was applied to remove DCM, and a mixed liquid of Fmoc-MeGly-OH (3.07 g, 9.87 mmol), DIPEA (8.25 mL, 47.4 mmol) and DCM (110 mL) was added into the reaction vessel. The reaction vessel was shaken at room temperature for 60 minutes. Nitrogen pressure was applied to remove the reaction liquid, and a mixed liquid of methanol (12.8 mL, 316 mmol), DIPEA (8.25 mL, 47.4 mmol) and DCM (110 mL) was then added into the reaction vessel. The reaction vessel was shaken at room temperature for 90 minutes. Nitrogen pressure was applied to remove the reaction liquid, DCM (180 mL) was then added, the reaction vessel was shaken for 5 minutes, and nitrogen pressure was then applied to remove the reaction liquid. The resin washing operation using DCM was repeated twice, and the obtained resin was dried overnight under reduced pressure to obtain 22.2 g of compound aa4-002-resin. The loading rate calculated by the Fmoc quantitation method using a dry resin (10.00 mg) was 0.573 mmol/g. (UVarea value at 294 nm: 5879.66 and UVarea value at 304 nm: 5289.40)

Synthesis of compound aa4-003-resin, (2S)-2-[9H-fluoren-9-ylmethoxycarbonyl(methyl)amino]-3-methylbutanoic acid-2-chlorotrityl resin (Fmoc-MeVal-O-Trt(2-Cl)resin)

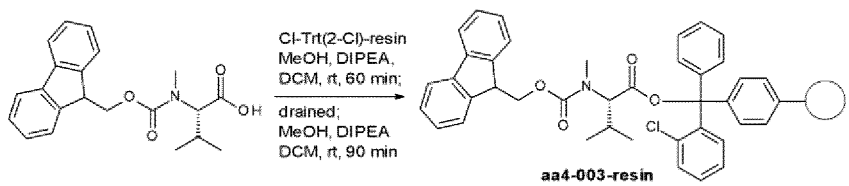


aa4-003-resin

2-Chlorotrityl chloride resin (1.25 mmol/g, 3.03 g, 3.79 mmol) and Fmoc-MeVal-OH (669 mg, 1.89 mmol) were provided as starting raw materials, and the same procedure as in the synthesis of compound aa2-001-resin was carried out to obtain 3.37 g of compound aa4-003-resin. The loading rate calculated by the Fmoc quantitation method using a dry resin (10.21 mg) was 0.436 mmol/g. (UVarea value at 294 nm: 4751.39 and UVarea value at 304 nm: 4274.97)

Synthesis of compound aa4-004-resin, 1-O-(9H-fluoren-9-ylmethyl) (2S)-pyrrolidine-1,2-dicarboxylate 2-O-2-chlorotrimethyl resin (Fmoc-Pro-O-Trt(2-Cl)resin)

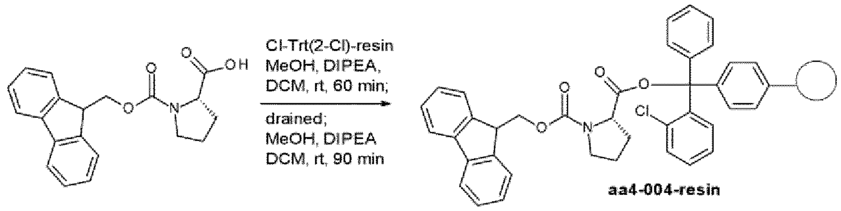


aa4-004-resin

2-Chlorotrityl chloride resin (1.69 mmol/g, 25.0 g, 42.3 mmol) and Fmoc-Pro-OH (7.13 mg, 21.1 mmol) were provided as starting raw materials, and the same procedure as in the synthesis of compound aa2-001-resin was carried out to obtain 28.8 g of compound aa4-004-resin. The loading rate calculated by the Fmoc quantitation method using a dry resin (10.87 mg) was 0.432 mmol/g. (UVarea value at 294 nm: 4714.30 and UVarea value at 304 nm: 4225.61)

Synthesis of compound aa4-005-resin, 2-[9H-fluoren-9-ylmethoxycarbonylamino]-2-methylpropanoic acid-2-chlorotrityl resin (Fmoc-Aib-O-Trt(2-Cl)resin)

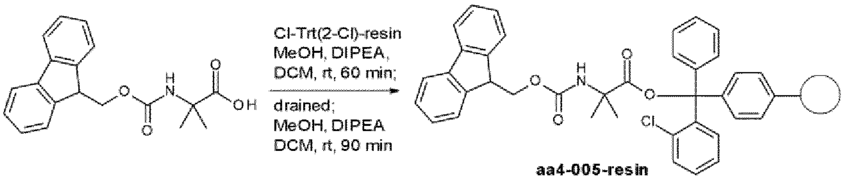


aa4-005-resin

2-Chlorotrityl chloride resin (1.25 mmol/g, 3.15 g, 3.93 mmol) and Fmoc-Aib-OH (640 mg, 1.97 mmol) were provided as starting raw materials, and the same procedure as in the synthesis of compound aa2-001-resin was carried out to obtain 3.41 g of compound aa4-005-resin. The loading rate calculated by the Fmoc quantitation method using a dry resin (10.43 mg) was 0.390 mmol/g. (UVarea value at 294 nm: 4336.95 and UVarea value at 304 nm: 3918.58)

Synthesis of compound aa4-006-resin, 2-[9H-fluoren-9-ylmethoxycarbonylamino]acetic acid-2-chlorotrityl resin (Fmoc-Gly-O-Trt(2-Cl)resin)

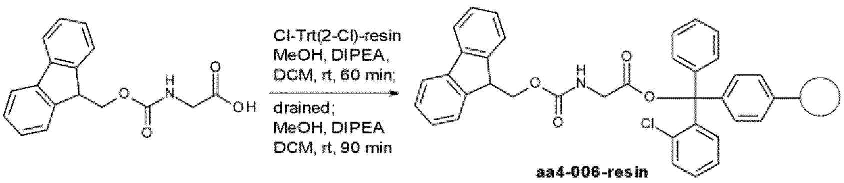


aa4-006-resin

2-Chlorotrityl chloride resin (1.25 mmol/g, 2.40 g, 3.00 mmol) and Fmoc-Gly-OH (446 mg, 1.50 mmol) were provided as starting raw materials, and the same procedure as in the synthesis of compound aa2-001-resin was carried out to obtain 2.39 g of compound aa4-006-resin. The loading rate calculated by the Fmoc quantitation method using a dry resin (10.06 mg) was 0.250 mmol/g. (UVarea value at 294 nm: 2563.25 and UVarea value at 304 nm: 2311.09)

Synthesis of compound aa5-001-resin, 9H-fluoren-9-ylmethyl N-[(2S)-1-[[(2S)-4-[(Sieber resin)amino]-1,4-dioxo-1-piperidin-1-ylbutan-2-yl]-methylamino]-3-methyl-1-oxobutan-2-yl]-N-methylcarbamate (Fmoc-MeVal-MeAsp(NH-Sieber resin)-pip)

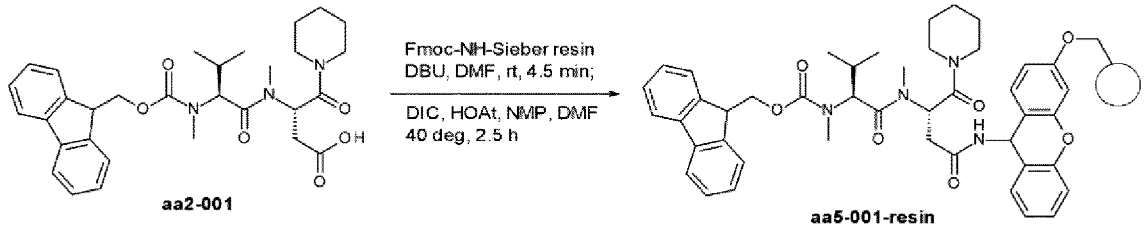


aa2-001

aa5-001-resin

A solid phase reaction vessel with a filter (frit) was charged with Fmoc-NH-Sieber resin (0.69 mmol/g, 600 mg, 0.414 mmol), DCM (7.2 mL) was added, the mixture was left standing for 30 minutes to swell the resin, and the solution was then discharged from the frit. A DMF solution of DBU (2%v/v, 4.2 mL) was added into the solid phase reaction vessel containing the resin, the mixture was reacted at room temperature for 4.5 minutes to carry out a Fmoc group removal reaction, and the solution was then discharged from the frit. DMF (4.2 mL) was added thereto, the mixture was left standing for 5 minutes, and the solution was then discharged from the frit. This resin washing step was repeated three more times. Subsequently, compound aa2-001 (594 mg, 1.08 mmol) was mixed with a solution (1.80 mL) of HOAt (92 mg, 0.676 mmol) in NMP and a solution (2.16 mL) of DIC (192 mg, 1.52 mmol) in DMF, the mixture was then added to the resin, a condensation reaction was carried out by reacting the mixture for 2.5 hours with the solid phase reaction vessel heated to 40°C, and the solution was then discharged from the frit. Subsequently, the resin was washed four times with DMF (4.2 mL), and then five times with DCM (4.2 mL), and the obtained resin was dried overnight under reduced pressure to obtain 688 mg of compound aa5-001-resin. The loading rate calculated by the Fmoc quantitation method using a dry resin (11.46 mg) was 0.538 mmol/g. (UVarea value at 294 nm: 6072.34 and UVarea value at 304 nm: 5457.70)

Example 2. Experiment for comparison of recovery ratio and purity of peptide in the case of supporting dipeptide with those in the case of supporting single amino acid in solid phase synthesis of peptide

In this Example, tripeptides each represented by Fmoc-AA3-AA2-AA1-resin were synthesized under the following three conditions by a solid phase reaction using a peptide synthesizing machine, and the recovery ratios and purities of the tripeptides were compared.

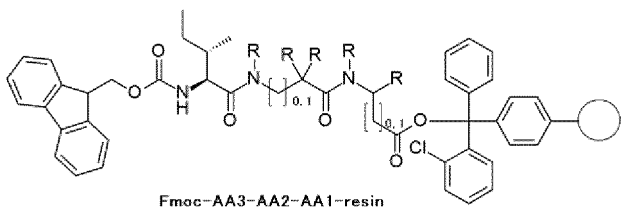

Fmoc-AA3-AA2-AA1-resin

[Table 6]

| | Raw material | AA2 elongation condition | AA3 elongation condition |
|---|---|---|---|
| Condition 1 | Fmoc-AA2-AA1-resin | - | HOAt, 40°C, 2.5 hr |
| Condition 2 | Fmoc-AA1-resin | HOAt, 40°C, 2.5 hr | HOAt, 40°C, 2.5 hr |
| Condition 3 | Fmoc-AA1-resin | oxyma, 50°C, 10 hr | HOAt, 40°C, 2.5 hr |

[Table 7]

| Compound No. | Abbreviation |
|---|---|
| pd2-001-resin | Fmoc-Ile-MeVal-MeAsp(O-Trt(2-Cl)resin)-pip |
| pd2-002-resin | Fmoc-Ile-MeIle-MeAsp(O-Trt(2-Cl)resin)-pip |
| pd2-003-resin | Fmoc-Ile-MeGly(cPent)-MeAsp(O-Trt(2-Cl)resin)-pip |
| pd2-004-resin | Fmoc-Ile-MeChg-MeAsp(O-Trt(2-Cl)resin)-pip |

(continued)

| Compound No. | Abbreviation |
|---|---|
| pd2-005-resin | Fmoc-Ile-MeLeu-MeAsp(O-Trt(2-Cl)resin)-pip |
| pd2-006-resin | Fmoc-Ile-MeGly(cPent)-MeAsp(O-Trt(2-Cl)resin)-NMe2 |
| pd2-007-resin | Fmoc-Ile-MeLeu-MeAsp(O-Trt(2-Cl)resin)-NMe2 |
| pd2-008-resin | Fmoc-Ile-MeVal-D-3-MeAbu-O-Trt(2-Cl)resin |
| pd2-009-resin | Fmoc-Ile-MeChg-D-3-MeAbu-O-Trt(2-Cl)resin |
| pd2-010-resin | Fmoc-Ile-MeVal-MeGly-O-Trt(2-Cl)resin |
| pd2-011-resin | Fmoc-Ile-MeVal-Asp(O-Trt(2-Cl)resin)-NMe2 |
| pd2-012-resin | Fmoc-Ile-Gly-MeAsp(O-Trt(2-Cl)resin)-NMe2 |
| pd2-013-resin | Fmoc-Ile-Aib-MeAsp(O-Trt(2-Cl)resin)-NMe2 |
| pd2-014-resin | Fmoc-Ile-bAla(2-Me2)-D-3-MeAbu-O-Trt(2-Cl)resin |
| pd2-015-resin | Fmoc-Ile-MeLeu-MeVal-O-Trt(2-Cl)resin |
| pd2-016-resin | Fmoc-Ile-MeVal-Pro-O-Trt(2-Cl)resin |
| pd2-017-resin | Fmoc-Ile-Phe-Pro-O-Trt(2-Cl)resin |
| pd2-018-resin | Fmoc-Ile-Lys(Boc)-Pro-O-Trt(2-Cl)resin |
| pd2-019-resin | Fmoc-Ile-MeLeu-Aib-O-Trt(2-Cl)resin |
| pd2-020-resin | Fmoc-Ile-MeVal-Gly-O-Trt(2-Cl)resin |

The synthesis of peptides by a solid phase reaction as described in this Example was performed by the Fmoc method using a peptide synthesizing machine (Multipep RS manufactured by Intavis Inc.). Detailed procedures of operations were as described in a manual attached to the synthesizing machine.

Detailed synthesis conditions in Example 2 are shown in the following synthesis method 1.

Synthesis method 1

As an activating agent for a Fmoc-protected amino acid (0.6 mol/L) and a carboxylic acid for forming an intended peptide, HOAt or oxyma (0.375 mol/L) was dissolved in NMP to prepare solution 1. When the Fmoc-protected amino acid was hardly soluble, DMSO was added at 20 to 30% (v/v) to prepare solution 1. In addition, the solution was mixed with DMF to a DIC concentration of 10% (v/v) to prepare solution 2. A solid phase reaction vessel with a filter (frit) was charged with a resin (100 mg) supporting the Fmoc amino acid or peptide prepared in Example 1-4, and was set in the peptide synthesizing machine. DCM (1.2 mL) was added, the mixture was left standing for 30 minutes to swell the resin, and the solution was then discharged from the frit. Solutions 1 and 2 were set in the peptide synthesizing machine, and automatic synthesis with the peptide synthesizing machine was started.

A DMF solution of DBU (2%v/v, 0.7 mL) was added into the solid phase reaction vessel containing the resin, and the mixture was reacted at room temperature to carry out a Fmoc group removal reaction. The reaction was carried out for 4.5 minutes in deprotection at the first residue, and for 10 minutes in deprotection at the second and subsequent residues, and the solution was then discharged from the frit. DMF (0.7 mL) was added thereto, the mixture was left standing for 5 minutes, and the solution was then discharged from the frit. This resin washing step was repeated three more times. Subsequently, solution 1 (0.3 mL) was mixed with solution 2 (0.36 mL) in a mixing vial of the synthesizing machine, the mixture was then added to the resin, a condensation reaction of the amino group on the resin with the Fmoc-protected amino acid by reacting the mixture for 2.5 hours or 10 hours with the solid phase reaction vessel heated to 40°C or 50°C, and the solution was then discharged from the frit. Subsequently, the resin was washed three times with DMF (0.7 mL). The Fmoc group removal reaction, followed by the condensation reaction of the Fmoc amino acid, was taken as a cycle, and the cycle was repeated to elongate a peptide on a resin surface.

After completion of the peptide elongation, the obtained resin was washed four times with DMF (0.7 mL), then washed four times with DCM (0.7 mL), and dried naturally at room temperature for 48 hours. A part of the obtained resin (about 10 mg) was put in a reaction vessel, and the loading rate of the peptide on the resin was calculated in accordance with Fmoc quantitation method described in Example 1-4. A part of the obtained resin (about 20 mg) was put in a reaction

vessel, a TFE/DCM solution (1/1, 1 mL) with or without 0.75% (v/v) DIPEA was added, and the reaction vessel was shaken at room temperature for 2 hours to carry out a peptide isolation reaction. After the reaction, the isolating solution was analyzed by LCMS to identify the product on the resin.

The definition of the recovery ratio and the method for calculation of the recovery ratio in Example 2 are described in the following recovery ratio calculation method.

Recovery ratio calculation method

In Example 2, the recovery ratio was defined as follows, and the ratio of detachment of the amino acid and the peptide from the resin during the solid phase reaction, in other words, the premature cleavage suppression ratio was evaluated.

Recovery ratio = loading rate on reaction product supporting resin (mmol/g) ÷ loading rate on resin (mmol/g) at intended product generation ratio of 100% (equation 1)

The loading rate on resin (mmol/g) at an intended product generation ratio of 100% is calculated as follows.

Loading rate on resin (mmol/g) at intended product generation ratio of 100% = loading rate on starting raw material resin (mmol/g) × weight of starting raw material resin (g) ÷ weight of resin (g) at intended product generation ratio of 100% (equation 2)

The weight of resin (g) at an intended product generation ratio of 100% is calculated as follows.

Weight of resin (g) at intended product generation ratio of 100% = weight of starting raw material resin (g) - weight of amino acid or peptide component on starting raw material resin (g) + weight of peptide component on resin (g) at intended product generation ratio of 100% (equation 3)

The weight of the amino acid or peptide component on starting raw material resin (g) is calculated as follows.

Weight of amino acid or peptide component on starting raw material resin (g) = weight of starting raw material resin (g) × loading rate on starting raw material resin (mmol/g) × molecular weight of amino acid or peptide component on starting raw material resin (g/mol) × 0.001 (mol/mmol) (equation 4)

The weight of the peptide component on resin (g) at an intended product generation ratio of 100% is calculated as follows.

Weight of peptide component on resin (g) at intended product generation ratio of 100% = weight of starting raw material resin (g) × loading rate on starting raw material resin (mmol/g) × molecular weight of peptide component of intended product (g/mol) × 0.001 (mol/mmol) (equation 5)

Substitution of equations 3, 4 and 5 into equation 2 gives the following.

Loading rate on resin (mmol/g) at intended product generation ratio of 100% = loading rate on starting raw material resin (mmol/g) ÷ (1 - loading rate on starting raw material resin (mmol/g) × molecular weight of amino acid or peptide component on starting raw material resin (g/mol) × 0.001 (mol/mmol) + loading rate on starting raw material resin (mmol/g) × molecular weight of peptide component of intended product (g/mol) × 0.001 (mol/mmol)) = 1 ÷ (1 ÷ support on starting raw material resin (mmol/g) - molecular weight of amino acid or peptide component on starting raw material resin (g/mol) × 0.001 (mol/mmol) + molecular weight of peptide component of intended product (g/mol) × 0.001 (mol/mmol)) (equation 6)

Substitution of equation 6 into equation 1 gives the following equation to calculate the recovery ratio.

Recovery ratio = loading rate on reaction product-supporting resin (mmol/g) × (1 ÷ loading rate on starting raw material resin (mmol/g) - molecular weight of amino acid or peptide component on starting raw material resin (g/mol) × 0.001 (mol/mmol) + molecular weight of peptide component of intended product (g/mol) × 0.001 (mol/mmol)

Synthesis of compound pd2-001-resin, (3S)-3-[[(2S)-2-[[(2S,3S)-2-(9H-fluoren-9-ylmethoxycarbonylamino)-3-methylpentanoyl]-methylamino]-3-methylbutanoyl]-methylamino]-4-oxo-4-piperidin-1-ylbutanoic acid-2-chlorotrityl resin (Fmoc-Ile-MeV al-MeAsp(O- Trt(2-Cl)resin)-pip) Synthesis of pd2-001-resin under condition 1

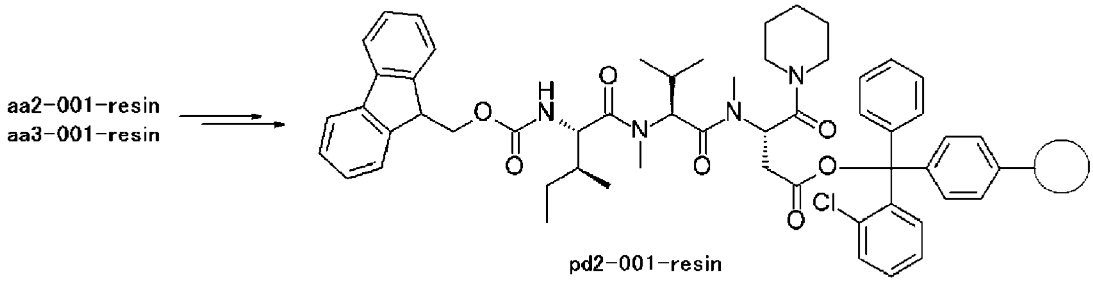


pd2-001-resin

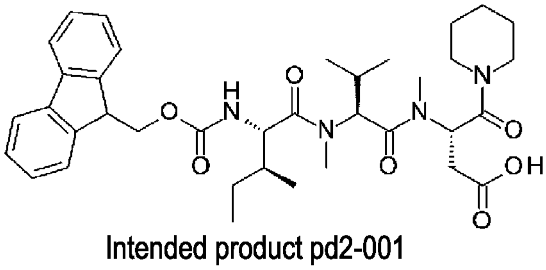

Intended product pd2-001

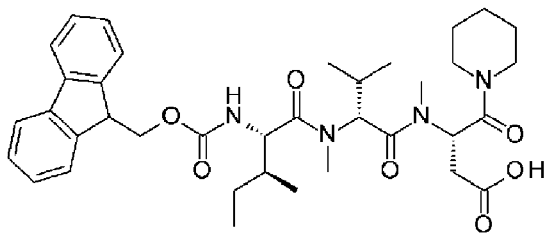

Epimeric form pd2-001-a

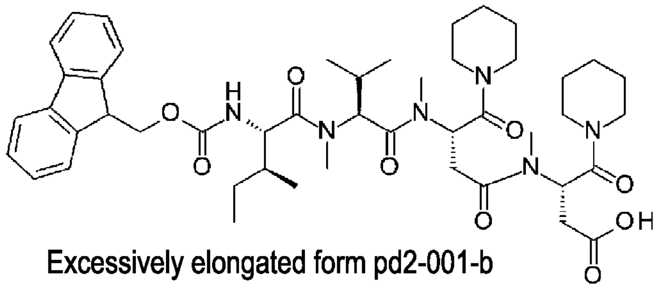

Excessively elongated form pd2-001-b

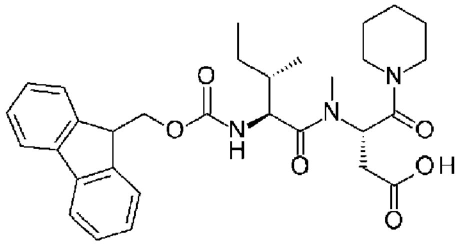

AA2-deficient form pd2-001-c

The dipeptide-supporting resin aa2-001-resin (Fmoc-MeVal-MeAsp(O-Trt(2-Cl)resin)-pip) (100 mg, 0.363

mmol/g) prepared in Example 1-4 was provided as a starting raw material, and elongation was performed with Fmoc-Ile-OH (HOAt, 40°C, 2.5 hours) in accordance with synthesis method 1 to synthesize pd2-001-resin. The loading rate calculated by the Fmoc quantitation method using a dry resin (11.36 mg) was 0.344 mmol/g. (UVarea value at 294 nm: 3907.18 and UVarea value at 304 nm: 3521.50)

The recovery ratio is calculated from the following equation in accordance with the recovery ratio calculation method.

$$\text{Recovery ratio} = 0.344 \times (1 \div 0.363 - 549.67 \times 0.001 + 662.83 \times 0.001) = 98.7\%$$

Analysis of the isolating reaction liquid by LCMS showed that the purity of intended product pd2-001 was 98.2 area%, and epimeric form pd2-001-a was observed at 1.8 area%. This epimeric form is an impurity already observed in the stage of preparation of compound aa2-001, and is not an impurity derived from this step of performing elongation with AA3 (Ile here).

Analysis condition: SQDAA50long

[Table 8]

| | | Molecular weight | LCMS (ESI) m/z | Retention time (min) | LC area% |
|---|---|---|---|---|---|
| pd2-001 | Intended product | 662.83 | 663.7 (M+H)+ | 1.78 | 98.2 |
| pd2-001-a | Epimeric form | 662.83 | 663.7 (M+H)+ | 2.01 | 1.8 |

Synthesis of pd2-001-resin under condition 2

The amino acid-supported resin aa3-001-resin (Fmoc-MeAsp(O-Trt(2-Cl)resin)-pip) (100 mg, 0.455 mmol/g) prepared in Example 1-4 was provided as a starting raw material, and elongation was performed with Fmoc-MeVal-OH (HOAt, 40°C, 2.5 hours) and then with Fmoc-Ile-OH (HOAt, 40°C, 2.5 hours) in accordance with synthesis method 1 to synthesize pd2-001-resin. The loading rate calculated by the Fmoc quantitation method using a dry resin (10.45 mg) was 0.292 mmol/g. (UVarea value at 294 nm: 3046.82 and UVarea value at 304 nm: 2746.87)

The recovery ratio is calculated from the following equation in accordance with the recovery ratio calculation method.

$$\text{Recovery ratio} = 0.292 \times (1 \div 0.455 - 436.51 \times 0.001 + 662.83 \times 0.001) = 70.8\%$$

Analysis of the isolating reaction liquid by LCMS showed that the purity of intended product pd2-001 was 61.6 area%, epimeric form pd2-001-a was observed at 6.5 area%, excessively elongated form pd2-001-b was observed at 2.4 area%, and AA2-deficient form pd2-001-c was observed at 29.5 area%. In this context, the term "excessively elongated form pd2-001-b" refers to a compound formed such that AA1 (MeAsp-pip here) is detached from the resin during elongation with AA2 (MeVal here), and AA1 supported on the resin is elongated with the detached AA1, i.e. a compound such that two AA1s are bound to each other, followed by elongation with AA2 and AA3. In subsequent Examples, the excessively elongated form also refers to a compound in which two AA1s are bound to each other unless otherwise specified.

Analysis condition: SQDAA50long

[Table 9]

| | | Molecular weight | LCMS (ESI) m/z | Retention time (min) | LC area% |
|---|---|---|---|---|---|
| pd2-001 | Intended product | 662.83 | 663.7 (M+H)+ | 1.78 | 61.6 |
| pd2-001-a | Epimeric form | 662.83 | 663.7 (M+H)+ | 2.00 | 6.5 |

(continued)

| | | Molecular weight | LCMS (ESI) m/z | Retention time (min) | LC area% |
|---|---|---|---|---|---|
| pd2-001-b | Excessively elongated form | 859.08 | 859.8 (M+H)+ | 2.18 | 2.4 |
| pd2-001-c | AA2-deficient form | 549.67 | 550.5 (M+H)+ | 1.63 | 29.5 |

Synthesis of pd2-001-resin under condition 3

The amino acid-supported resin aa3-001-resin (Fmoc-MeAsp(O-Trt(2-Cl)resin)-pip) (100 mg, 0.455 mmol/g) prepared in Example 1-4 was provided as a starting raw material, and elongation was performed with Fmoc-MeVal-OH (oxyma, 50°C, 10 hours), and then with Fmoc-Ile-OH (HOAt, 40°C, 2.5 hours) in accordance with synthesis method 1 to synthesize pd2-001-resin. The loading rate calculated by the Fmoc quantitation method using a dry resin (10.89 mg) was 0.332 mmol/g. (UVarea value at 294 nm: 3612.81 and UVarea value at 304 nm: 3267.35)

The recovery ratio is calculated from the following equation in accordance with the recovery ratio calculation method.

$$\text{Recovery ratio} = 0.332 \times (1 \div 0.455 - 436.51 \times 0.001 + 662.83 \times 0.001) = 80.5\%$$

Analysis of the isolating reaction liquid by LCMS showed that the purity of intended product pd2-001 was 93.7 area%, epimeric form pd2-001-a was observed at 2.6 area%, and excessively elongated form pd2-001-b was observed at 3.7 area%.

Analysis condition: SQDAA50long

[Table 10]

| | | Molecular weight | LCMS (ESI) m/z | Retention time (min) | LC area% |
|---|---|---|---|---|---|
| pd2-001 | Intended product | 662.83 | 663.6 (M+H)+ | 1.79 | 93.7 |
| pd2-001-a | Epimeric form | 662.83 | 663.7 (M+H)+ | 2.02 | 2.6 |
| pd2-001-b | Excessively elongated form | 859.08 | 859.8 (M+H)+ | 2.20 | 3.7 |

The table below collectively shows the above results.

[Table 11]

| | Recovery ratio (%) | Intended product pd2-001 (area%) | Epimeric form pd2-001-a (area%) | Excessively elongated form pd2-001-b (area%) | AA2-deficient form pd2-001-c (area%) |
|---|---|---|---|---|---|
| Condition 1 | 98.7 | 98.2 | 1.8 | - | - |
| Condition 2 | 70.8 | 61.6 | 6.5 | 2.4 | 29.5 |
| Condition 3 | 80.5 | 93.7 | 2.6 | 3.7 | - |

Under condition 1 where a dipeptide was supported, an intended product was obtained with a high recovery ratio and a high purity.

When a single amino acid was supported, generation of an excessively elongated form and generation of an AA2-deficient form due to poor elongation of AA2 that is a site difficult to elongate were observed in addition to a decrease in recovery ratio due to premature cleavage under normal condition 2. Even under condition 3 that is adaptable to a

sequence difficult to elongate, a decrease in recovery ratio due to premature cleavage was observed, and there was an increase in generation of an excessively elongated form although an AA2-deficient form was not generated.

In condition 1, an epimeric form (pd2-001-a) was observed as an impurity as in conditions 2 and 3. As described above, this is an impurity already observed in the stage of preparation of compound aa2-001. That is, the impurity can be avoided by precisely purifying compound aa2-001 prepared by a liquid phase process. On the other hand, under conditions 2 and 3 that correspond to a common peptide synthesis method, the purity decreases due to epimerization proceeding on the solid phase. When AA2 is an amino acid difficult to elongate, so that epimerization easily occurs during elongation, a peptide elongation method using the dipeptide-supporting resin is advantageous because the method may enable avoidance of a decrease in purity of the peptide due to epimerization.

Synthesis of compound pd2-002-resin, (3S)-3-[[(2S,3S)-2-[[(2S,3S)-2-(9H-fluoren-9-ylmethoxycarbonylamino)-3-methylpentanoyl]-methylamino]-3-methylpentanoyl]-methylamino]-4-oxo-4-piperidin-1-ylbutanoic acid-2-chlorotrityl resin (Fmoc-Ile-MeIle-MeAsp(O-Trt(2-Cl)resin)-pip)

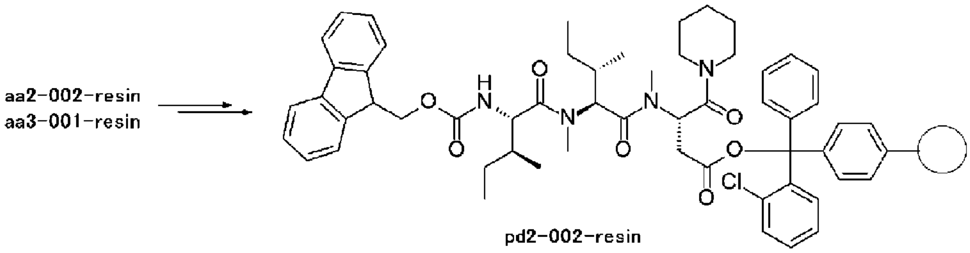


pd2-002-resin

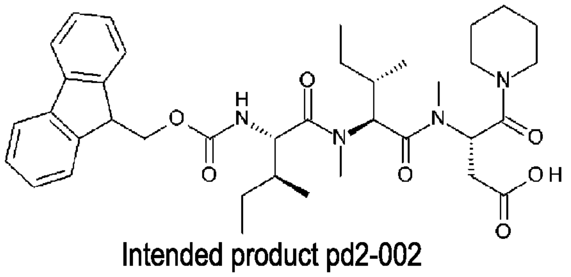

Intended product pd2-002

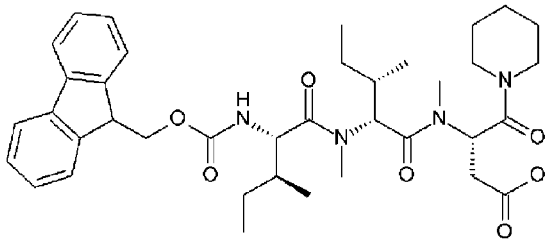

Epimeric form pd2-002-a

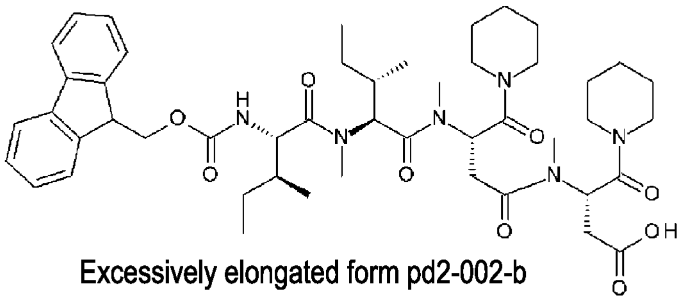

Excessively elongated form pd2-002-b

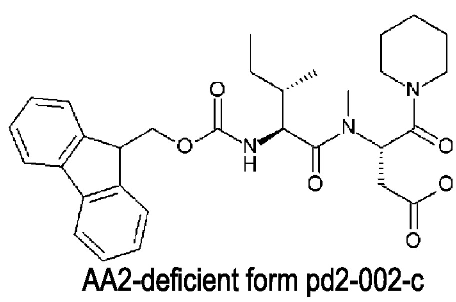

AA2-deficient form pd2-002-c

Synthesis of pd2-002-resin under condition 1

The dipeptide-supporting resin aa2-002-resin (Fmoc-MeIle-MeAsp(O-Trt(2-Cl)resin)-pip) (100 mg, 0.326 mmol/g) prepared in Example 1-4 was provided as a starting raw material, and elongation was performed with Fmoc-Ile-OH (HOAt, 40°C, 2.5 hours) in accordance with synthesis method 1 to synthesize pd2-002-resin. The loading rate calculated by the Fmoc quantitation method using a dry resin (10.24 mg) was 0.336 mmol/g. (UVarea value at 294 nm: 3446.83 and UVarea value at 304 nm: 3102.30)

The recovery ratio is calculated from the following equation in accordance with the recovery ratio calculation method.

$$\text{Recovery ratio} = 0.336 \times (1 \div 0.326 - 563.70 \times 0.001 + 676.86 \times 0.001) = 106.9\%$$

Analysis of the isolating reaction liquid by LCMS showed that the purity of intended product pd2-002 was 99.4 area%, and epimeric form pd2-002-a was observed at 0.6 area%.

Analysis condition: SQDAA50long

[Table 12]

| | | Molecular weight | LCMS (ESI) m/z | Retention time (min) | LC area% |
|---|---|---|---|---|---|
| pd2-002 | Intended product | 676.86 | 677.6 (M+H)+ | 2.00 | 99.4 |
| pd2-002-a | Epimeric form | 676.86 | 677.7 (M+H)+ | 2.20 | 0.6 |

Synthesis of pd2-002-resin under condition 2

The amino acid-supported resin prepared in Example 1-4 (aa3-001-resin (Fmoc-MeAsp(O-Trt(2-Cl)resin)-pip)) (100 mg, 0.455 mmol/g) was provided as a starting raw material, and elongation was performed with Fmoc-MeIle-OH (HOAt, 40°C, 2.5 hours), and then with Fmoc-Ile-OH (HOAt, 40°C, 2.5 hours) in accordance with synthesis method 1 to synthesize pd2-002-resin. The loading rate calculated by the Fmoc quantitation method using a dry resin (10.81 mg) was 0.326 mmol/g. (UVarea value at 294 nm: 3524.40 and UVarea value at 304 nm: 3171.55)
The recovery ratio is calculated from the following equation in accordance with the recovery ratio calculation method.

$$\text{Recovery ratio} = 0.326 \times (1 \div 0.455- 436.51 \times 0.001 + 676.86 \times 0.001) = 79.5\%$$

Analysis of the isolating reaction liquid by LCMS showed that the purity of intended product pd2-002 was 58.0 area%, epimeric form pd2-002-a was observed at 1.4 area%, excessively elongated form pd2-002-b was observed at 1.8 area%, and AA2-deficient form pd2-002-c was observed at 38.8 area%.

Analysis condition: SQDAA50long

[Table 13]

| | | Molecular weight | LCMS (ESI) m/z | Retention time (min) | LC area% |
|---|---|---|---|---|---|
| pd2-002 | Intended product | 676.86 | 677.6 (M+H)+ | 2.00 | 58.0 |
| pd2-002-a | Epimeric form | 676.86 | 677.7 (M+H)+ | 2.20 | 1.4 |
| pd2-002-b | Excessively elongated form | 873.11 | 873.8 (M+H)+ | 2.37 | 1.8 |
| pd2-002-c | AA2-deficient form | 549.67 | 550.5 (M+H)+ | 1.62 | 38.8 |

Synthesis of pd2-002-resin under condition 3

The amino acid-supported resin aa3-001-resin (Fmoc-MeAsp(O-Trt(2-Cl)resin)-pip) (100 mg, 0.455 mmol/g) prepared in Example 1-4 was provided as a starting raw material, and elongation was performed with Fmoc-MeIle-OH (oxyma, 50°C, 10 hours), and then with Fmoc-Ile-OH (HOAt, 40°C, 2.5 hours) in accordance with synthesis method 1 to synthesize pd2-002-resin. The loading rate calculated by the Fmoc quantitation method using a dry resin (10.41 mg) was 0.330 mmol/g. (UVarea value at 294 nm: 3437.60 and UVarea value at 304 nm: 3100.91)
The recovery ratio is calculated from the following equation in accordance with the recovery ratio calculation method.

$$\text{Recovery ratio} = 0.330 \times (1 \div 0.455- 436.51 \times 0.001 + 676.86 \times 0.001) = 80.5\%$$

Analysis of the isolating reaction liquid by LCMS showed that the purity of intended product pd2-002 was 95.2 area%, epimeric form pd2-002-a was observed at 1.4 area%, and excessively elongated form pd2-002-b was observed at 3.4 area%.

Analysis condition: SQDAA50long

[Table 14]

| | | Molecular weight | LCMS (ESI) m/z | Retention time (min) | LC area% |
|---|---|---|---|---|---|
| pd2-002 | Intended product | 676.86 | 677.7 (M+H)+ | 2.01 | 95.2 |
| pd2-002-a | Epimeric form | 676.86 | 677.6 (M+H)+ | 2.22 | 1.4 |
| pd2-002-b | Excessively elongated form | 873.11 | 873.9 (M+H)+ | 2.39 | 3.4 |

The table below collectively shows the above results.

[Table 15]

| | Recovery ratio (%) | Intended product pd2-002 (area%) | Epimeric form pd2-002-a (area%) | Excessively elongated form pd2-002-b (area%) | AA2-deficient form pd2-002-c (area%) |
|---|---|---|---|---|---|
| Condition 1 | 106.9 | 99.4 | 0.6 | - | - |
| Condition 2 | 79.5 | 58.0 | 1.4 | 1.8 | 38.8 |
| Condition 3 | 80.5 | 95.2 | 1.4 | 3.4 | - |

Under condition 1 where a dipeptide was supported, an intended product was obtained with a high recovery ratio and a high purity.

When a single amino acid was supported, generation of an excessively elongated form and generation of an AA2-deficient form due to poor elongation of AA2 that is a site difficult to elongate were observed in addition to a decrease in recovery ratio due to premature cleavage under normal condition 2. Even under condition 3 that is adaptable to a sequence difficult to elongate, a decrease in recovery ratio due to premature cleavage was observed, and there was an increase in generation of an excessively elongated form although an AA2-deficient form was not generated.

Synthesis of compound pd2-003-resin, (3S)-3-[[(2S)-2-cyclopentyl-2-[[(2S,3S)-2-(9H-fluoren-9-ylmethoxycarbonylamino)-3-methylpentanoyl]-methylamino]acetyl]-methylamino]-4-oxo-4-piperidin-1-ylbutanoic acid-2-chlorotrityl resin (Fmoc-Ile-MeGly(cPent)-MeAsp(O-Trt(2-Cl)resin)-pip)

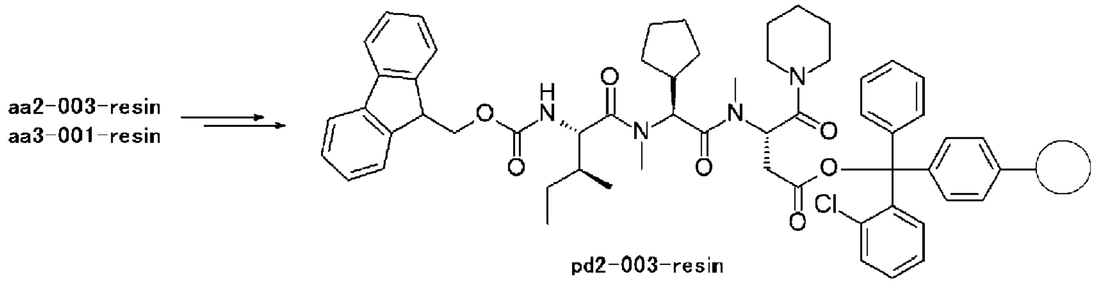


pd2-003-resin

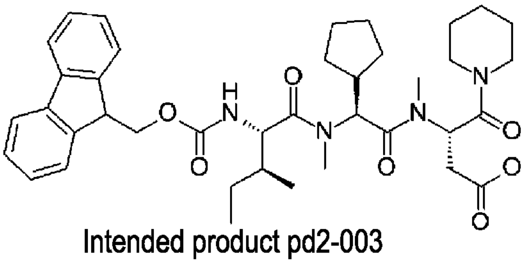

Intended product pd2-003

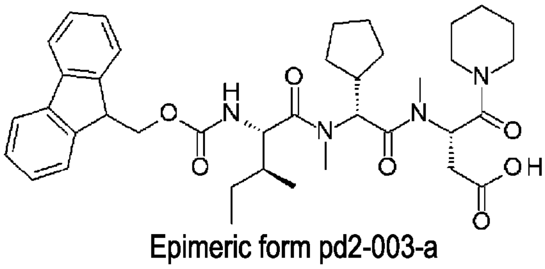

Epimeric form pd2-003-a

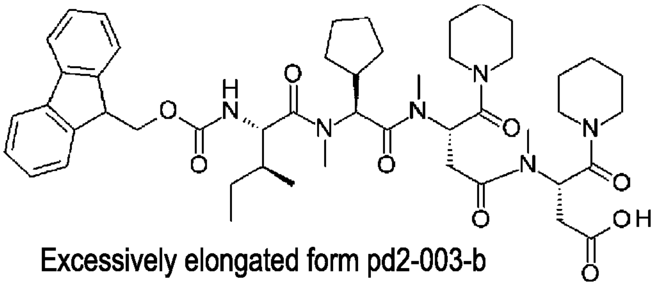

Excessively elongated form pd2-003-b

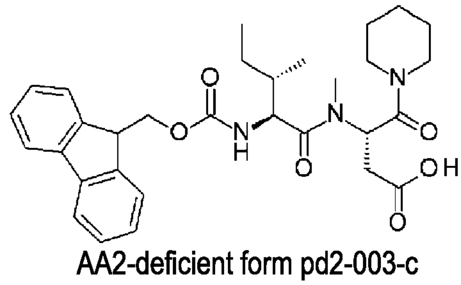

AA2-deficient form pd2-003-c

Synthesis of pd2-003-resin under condition 1

The dipeptide-supporting resin aa2-003-resin (Fmoc-MeGly(cPent)-MeAsp(O-Trt(2-Cl)resin)-pip) (100 mg, 0.362 mmol/g) prepared in Example 1-4 was provided as a starting raw material, and elongation was performed with Fmoc-Ile-OH (HOAt, 40°C, 2.5 hours) in accordance with synthesis method 1 to synthesize pd2-003-resin. The loading rate calculated by the Fmoc quantitation method using a dry resin (10.14 mg) was 0.348 mmol/g. (UVarea value at 294 nm: 3532.70 and UVarea value at 304 nm: 3179.43)
The recovery ratio is calculated from the following equation in accordance with the recovery ratio calculation method.

$$\text{Recovery ratio} = 0.348 \times (1 \div 0.362- 575.71 \times 0.001 + 688.87 \times 0.001) = 100.1\%$$

Analysis of the isolating reaction liquid by LCMS showed that the purity of intended product pd2-003 was 99.8 area%, and epimeric form pd2-003-a was observed at 0.2 area%.

Analysis condition: SQDAA50long

[Table 16]

| | | Molecular weight | LCMS (ESI) m/z | Retention time (min) | LC area% |
|---|---|---|---|---|---|
| pd2-003 | Intended product | 688.87 | 689.7 (M+H)+ | 2.06 | 99.8 |
| pd2-003-a | Epimeric form | 688.87 | 689.6 (M+H)+ | 2.27 | 0.2 |

Synthesis of pd2-003-resin under condition 2

The amino acid-supported resin aa3-001-resin (Fmoc-MeAsp(O-Trt(2-Cl)resin)-pip) (100 mg, 0.455 mmol/g) prepared in Example 1-4 was provided as a starting raw material, and elongation was performed with Fmoc-MeGly(cPent)-OH (HOAt, 40°C, 2.5 hours), and then with Fmoc-Ile-OH (HOAt, 40°C, 2.5 hours) in accordance with synthesis method 1 to synthesize pd2-003-resin. The loading rate calculated by the Fmoc quantitation method using a dry resin (10.05 mg) was 0.320 mmol/g. (UVarea value at 294 nm: 3216.77 and UVarea value at 304 nm: 2905.18)
The recovery ratio is calculated from the following equation in accordance with the recovery ratio calculation method.

$$\text{Recovery ratio} = 0.320 \times (1 \div 0.455- 436.51 \times 0.001 + 688.87 \times 0.001) = 78.4\%$$

Analysis of the isolating reaction liquid by LCMS showed that the purity of intended product pd2-003 was 83.9 area%, epimeric form pd2-003-a was observed at 1.2 area%, excessively elongated form pd2-003-b was observed at 3.9 area%, and AA2-deficient form pd2-003-c was observed at 11.0 area%.

Analysis condition: SQDAA50long

[Table 17]

| | | Molecular weight | LCMS (ESI) m/z | Retention time (min) | LC area% |
|---|---|---|---|---|---|
| pd2-003 | Intended product | 688.87 | 689.6 (M+H)+ | 2.06 | 83.9 |
| pd2-003-a | Epimeric form | 688.87 | 689.7 (M+H)+ | 2.27 | 1.2 |
| pd2-003-b | Excessively elongated form | 885.12 | 885.8 (M+H)+ | 2.43 | 3.9 |
| pd2-003-c | AA2-deficient form | 549.67 | 550.5 (M+H)+ | 1.63 | 11.0 |

Synthesis of pd2-003-resin under condition 3

The amino acid-supported resin aa3-001-resin (Fmoc-MeAsp(O-Trt(2-Cl)resin)-pip) (100 mg, 0.455 mmol/g) prepared in Example 1-4 was provided as a starting raw material, and elongation was performed with Fmoc-MeGly(cPent)-OH (oxyma, 50°C, 10 hours), and then with Fmoc-Ile-OH (HOAt, 40°C, 2.5 hours) in accordance with synthesis method 1 to synthesize pd2-003-resin. The loading rate calculated by the Fmoc quantitation method using a dry resin (9.45 mg) was 0.232 mmol/g. (UVarea value at 294 nm: 2194.45 and UVarea value at 304 nm: 1975.12)
The recovery ratio is calculated from the following equation in accordance with the recovery ratio calculation method.

$$\text{Recovery ratio} = 0.232 \times (1 \div 0.455 - 436.51 \times 0.001 + 688.87 \times 0.001) = 56.8\%$$

Analysis of the isolating reaction liquid by LCMS showed that the purity of intended product pd2-003 was 91.4 area%, epimeric form pd2-003-a was observed at 0.1 area%, and excessively elongated form pd2-003-b was observed at 8.5 area%.

Analysis condition: SQDAA50long

[Table 18]

| | | Molecular weight | LCMS (ESI) m/z | Retention time (min) | LC area% |
|---|---|---|---|---|---|
| pd2-003 | Intended product | 688.87 | 689.7 (M+H)+ | 2.09 | 91.4 |
| pd2-003-a | Epimeric form | 688.87 | 689.5 (M+H)+ | 2.29 | 0.1 |
| pd2-003-b | Excessively elongated form | 885.12 | 885.8 (M+H)+ | 2.45 | 8.5 |

The table below collectively shows the above results.

[Table 19]

| | Recovery ratio (%) | Intended product pd2-003 (area%) | Epimeric form pd2-003-a (area%) | Excessively elongated form pd2-003-b (area%) | AA2-deficient form pd2-003-c (area%) |
|---|---|---|---|---|---|
| Condition 1 | 100.1 | 99.8 | 0.2 | - | - |

(continued)

| | Recovery ratio (%) | Intended product pd2-003 (area%) | Epimeric form pd2-003-a (area%) | Excessively elongated form pd2-003-b (area%) | AA2-deficient form pd2-003-c (area%) |
|---|---|---|---|---|---|
| Condition 2 | 78.4 | 83.9 | 1.2 | 3.9 | 11.0 |
| Condition 3 | 56.8 | 91.4 | 0.1 | 8.5 | - |

Under condition 1 where a dipeptide was supported, an intended product was obtained with a high recovery ratio and a high purity.

When a single amino acid was supported, generation of an excessively elongated form and generation of an AA2-deficient form due to poor elongation of AA2 that is a site difficult to elongate were observed in addition to a decrease in recovery ratio due to premature cleavage under normal condition 2. Even under condition 3 that is adaptable to a sequence difficult to elongate, a decrease in recovery ratio due to premature cleavage was observed, and there was an increase in generation of an excessively elongated form although an AA2-deficient form was not generated.

<u>Synthesis of compound pd2-004-resin, (3S)-3-[[(2S)-2-cyclohexyl-2-[[(2S,3S)-2-(9H-fluoren-9-ylmethoxycarbonylamino)-3-methylpentanoyl]-methylamino]acety]-methylamino]-4-oxo-4-piperidin-1-ylbutanoic acid-2-chlorotrityl resin (Fmoc-Ile-MeChg-MeAsp(O-Trt(2-Cl)resin)-pip)</u>

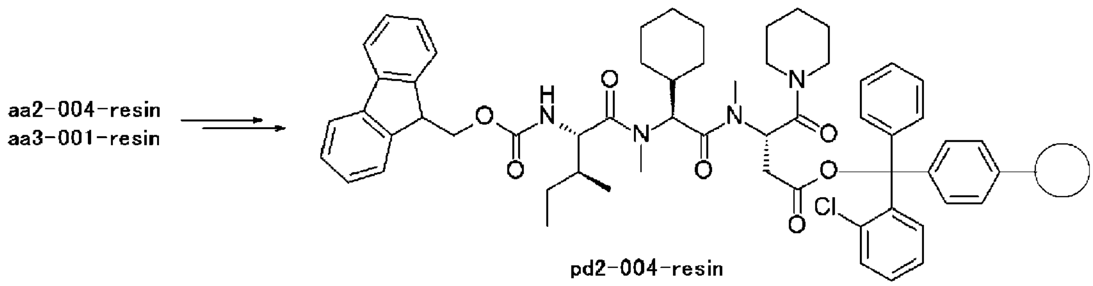


pd2-004-resin

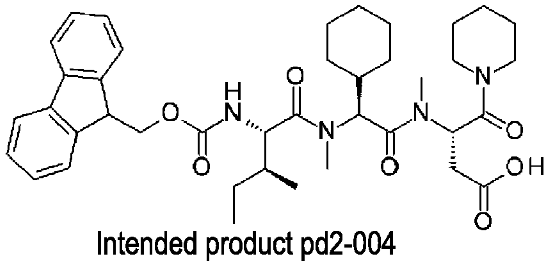

Intended product pd2-004

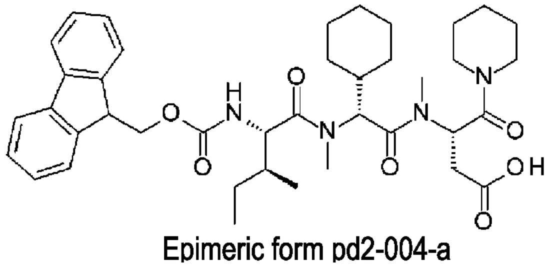

Epimeric form pd2-004-a

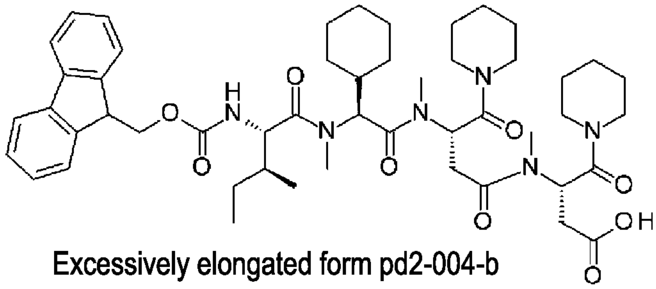

Excessively elongated form pd2-004-b

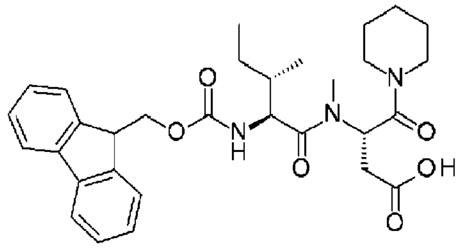

AA2-deficient form pd2-004-c

Synthesis of pd2-004-resin under condition 1

The dipeptide-supporting resin aa2-004-resin (Fmoc-MeChg-MeAsp(O-Trt(2-Cl)resin)-pip) (100 mg, 0.347 mmol/g) prepared in Example 1-4 was provided as a starting raw material, and elongation was performed with Fmoc-Ile-OH (HOAt, 40°C, 2.5 hours) in accordance with synthesis method 1 to synthesize pd2-004-resin. The loading rate calculated by the Fmoc quantitation method using a dry resin (10.66 mg) was 0.328 mmol/g. (UVarea value at 294 nm: 3502.36 and UVarea value at 304 nm: 3152.54)
The recovery ratio is calculated from the following equation in accordance with the recovery ratio calculation method.

$$\text{Recovery ratio} = 0.328 \times (1 \div 0.347 - 589.73 \times 0.001 + 702.89 \times 0.001) = 98.2\%$$

Analysis of the isolating reaction liquid by LCMS showed that the purity of intended product pd2-004 was 99.2 area%, and epimeric form pd2-004-a was observed at 0.8 area%.

Analysis condition: SQDAA50long

[Table 20]

| | | Molecular weight | LCMS (ESI) m/z | Retention time (min) | LC area% |
|---|---|---|---|---|---|
| pd2-004 | Intended product | 702.89 | 703.6 (M+H)+ | 2.23 | 99.2 |
| pd2-004-a | Epimeric form | 702.89 | 703.6 (M+H)+ | 2.43 | 0.8 |

Synthesis of pd2-004-resin under condition 2

The amino acid-supported resin aa3-001-resin (Fmoc-MeAsp(O-Trt(2-Cl)resin)-pip) (100 mg, 0.455 mmol/g) prepared in Example 1-4 was provided as a starting raw material, and elongation was performed with Fmoc-MeChg-OH (HOAt, 40°C, 2.5 hours), and then with Fmoc-Ile-OH (HOAt, 40°C, 2.5 hours) in accordance with synthesis method 1 to synthesize pd2-004-resin. The loading rate calculated by the Fmoc quantitation method using a dry resin (9.68 mg) was 0.314 mmol/g. (UVarea value at 294 nm: 3037.14 and UVarea value at 304 nm: 2743.09)
The recovery ratio is calculated from the following equation in accordance with the recovery ratio calculation method.

$$\text{Recovery ratio} = 0.314 \times (1 \div 0.455 - 436.51 \times 0.001 + 702.89 \times 0.001) = 77.4\%$$

Analysis of the isolating reaction liquid by LCMS showed that the purity of intended product pd2-004 was 74.1 area%, epimeric form pd2-004-a was observed at 2.3 area%, excessively elongated form pd2-004-b was observed at 4.0 area%, and AA2-deficient form pd2-004-c was observed at 19.5 area%.

Analysis condition: SQDAA50long

[Table 21]

| | | Molecular weight | LCMS (ESI) m/z | Retention time (min) | LC area% |
|---|---|---|---|---|---|
| pd2-004 | Intended product | 702.89 | 703.6 (M+H)+ | 2.24 | 74.1 |
| pd2-004-a | Epimeric form | 702.89 | 703.6 (M+H)+ | 2.43 | 2.3 |
| pd2-004-b | Excessively elongated form | 899.14 | 899.8 (M+H)+ | 2.58 | 4.0 |
| pd2-004-c | AA2-deficient form | 549.67 | 550.5 (M+H)+ | 1.63 | 19.5 |

Synthesis of pd2-004-resin under condition 3

The amino acid-supported resin aa3-001-resin (Fmoc-MeAsp(O-Trt(2-Cl)resin)-pip) (100 mg, 0.455 mmol/g) prepared in Example 1-4 was provided as a starting raw material, and elongation was performed with Fmoc-MeChg-OH (oxyma, 50°C, 10 hours), and then with Fmoc-Ile-OH (HOAt, 40°C, 2.5 hours) in accordance with synthesis method 1 to synthesize pd2-004-resin. The loading rate calculated by the Fmoc quantitation method using a dry resin (9.91 mg) was 0.226 mmol/g. (UVarea value at 294 nm: 2235.50 and UVarea value at 304 nm: 2016.81)
The recovery ratio is calculated from the following equation in accordance with the recovery ratio calculation method.

$$\text{Recovery ratio} = 0.226 \times (1 \div 0.455 - 436.51 \times 0.001 + 702.89 \times 0.001) = 55.7\%$$

Analysis of the isolating reaction liquid by LCMS showed that the purity of intended product pd2-004 was 91.7 area%, epimeric form pd2-004-a was observed at 0.6 area%, and excessively elongated form pd2-004-b was observed at 7.7 area%.

Analysis condition: SQDAA50long

[Table 22]

| | | Molecular weight | LCMS (ESI) m/z | Retention time (min) | LC area% |
|---|---|---|---|---|---|
| pd2-004 | Intended product | 702.89 | 703.6 (M+H)+ | 2.26 | 91.7 |
| pd2-004-a | Epimeric form | 702.89 | 703.7 (M+H)+ | 2.44 | 0.6 |
| pd2-004-b | Excessively elongated form | 899.14 | 899.8 (M+H)+ | 2.60 | 7.7 |

The table below collectively shows the above results.

[Table 23]

| | Recovery ratio (%) | Intended product pd2-004 (area%) | Epimeric form pd2-004-a (area%) | Excessively elongated form pd2-004-b (area%) | AA2-deficient form pd2-004-c (area%) |
|---|---|---|---|---|---|
| Condition 1 | 98.2 | 99.2 | 0.8 | - | - |
| Condition 2 | 77.4 | 74.1 | 2.3 | 4.0 | 19.5 |
| Condition 3 | 55.7 | 91.7 | 0.6 | 7.7 | - |

Under condition 1 where a dipeptide was supported, an intended product was obtained with a high recovery ratio and a high purity.

When a single amino acid was supported, generation of an excessively elongated form and generation of an AA2-deficient form due to poor elongation of AA2 that is a site difficult to elongate were observed in addition to a decrease in recovery ratio due to premature cleavage under normal condition 2. Even under condition 3 that is adaptable to a sequence difficult to elongate, a decrease in recovery ratio due to premature cleavage was observed, and there was an increase in generation of an excessively elongated form although an AA2-deficient form was not generated.

Synthesis of compound pd2-005-resin, (3S)-3-[[(2S)-2-[[(2S,3S)-2-(9H-fluoren-9-ylmethoxycarbonylamino)-3-methylpentanoyl]-methylamino]-4-methylpentanoyl]-methylamino]-4-oxo-4-piperidin-1-ylbutanoic acid-2-chlorotrityl resin (Fmoc-Ile-MeLeu-MeAsp(O-Trt(2-Cl)resin)-pip)

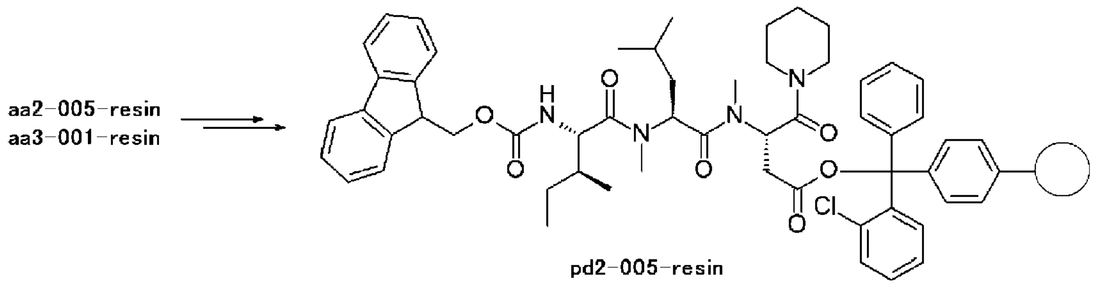


pd2-005-resin

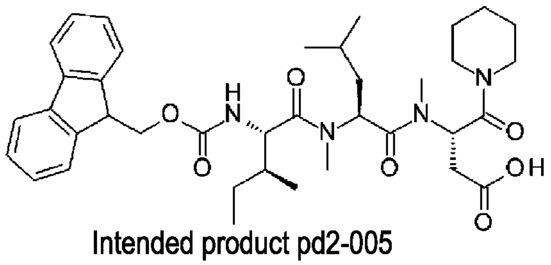

Intended product pd2-005

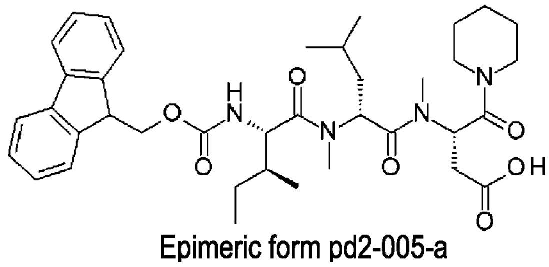

Epimeric form pd2-005-a

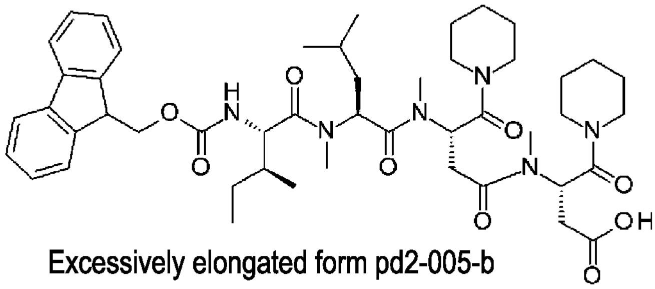

Excessively elongated form pd2-005-b

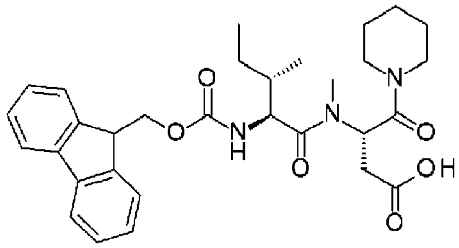

AA2-deficient form pd2-005-c

Synthesis of pd2-005-resin under condition 1

The dipeptide-supporting resin aa2-005-resin (Fmoc-MeLeu-MeAsp(O-Trt(2-Cl)resin)-pip) (100 mg, 0.355 mmol/g) prepared in Example 1-4 was provided as a starting raw material, and elongation was performed with Fmoc-Ile-OH (HOAt, 40°C, 2.5 hours) in accordance with synthesis method 1 to synthesize pd2-005-resin. The loading rate calculated by the Fmoc quantitation method using a dry resin (10.81 mg) was 0.342 mmol/g. (UVarea value at 294 nm: 3697.14 and UVarea value at 304 nm: 3330.28)
The recovery ratio is calculated from the following equation in accordance with the recovery ratio calculation method.

$$\text{Recovery ratio} = 0.342 \times (1 \div 0.355 - 563.70 \times 0.001 + 676.86 \times 0.001) = 100.2\%$$

Analysis of the isolating reaction liquid by LCMS showed that the purity of intended product pd2-005 was 99.9 area%, and epimeric form pd2-005-a was observed at 0.1 area%.

Analysis condition: SQDAA50long

[Table 24]

| | | Molecular weight | LCMS (ESI) m/z | Retention time (min) | LC area% |
|---|---|---|---|---|---|
| pd2-005 | Intended product | 676.86 | 677.7 (M+H)+ | 2.03 | 99.9 |
| pd2-005-a | Epimeric form | 676.86 | 677.6 (M+H)+ | 2.23 | 0.1 |

Synthesis of pd2-005-resin under condition 2

The amino acid-supported resin prepared in Example 1-4 (aa3-001-resin (Fmoc-MeAsp(O-Trt(2-Cl)resin)-pip) (100 mg, 0.455 mmol/g) was provided as a starting raw material, and elongation was performed with Fmoc-MeLeu-OH (HOAt, 40°C, 2.5 hours), and then with Fmoc-Ile-OH (HOAt, 40°C, 2.5 hours) in accordance with synthesis method 1 to synthesize pd2-005-resin. The loading rate calculated by the Fmoc quantitation method using a dry resin (10.74 mg) was 0.369 mmol/g. (UVarea value at 294 nm: 3953.19 and UVarea value at 304 nm: 3570.96)
The recovery ratio is calculated from the following equation in accordance with the recovery ratio calculation method.

$$\text{Recovery ratio} = 0.369 \times (1 \div 0.455 - 436.51 \times 0.001 + 676.86 \times 0.001) = 90.0\%$$

Analysis of the isolating reaction liquid by LCMS showed that the purity of intended product pd2-005 was 98.3 area%, epimeric form pd2-005-a was observed at 0.1 area%, and excessively elongated form pd2-005-b was observed at 1.6 area%. In this substrate, AA2-deficient form pd2-005-c was not observed even under condition 2.

Analysis condition: SQDAA50long

[Table 25]

| | | Molecular weight | LCMS (ESI) m/z | Retention time (min) | LC area% |
|---|---|---|---|---|---|
| pd2-005 | Intended product | 676.86 | 677.6 (M+H)+ | 2.03 | 98.3 |
| pd2-005-a | Epimeric form | 676.86 | 677.6 (M+H)+ | 2.23 | 0.1 |
| pd2-005-b | Excessively elongated form | 873.11 | 873.8 (M+H)+ | 2.41 | 1.6 |
| pd2-005-c | AA2-deficient form | 549.67 | not detected | | |

Synthesis of pd2-005-resin under condition 3

The amino acid-supported resin aa3-001-resin (Fmoc-MeAsp(O-Trt(2-Cl)resin)-pip) (100 mg, 0.455 mmol/g) prepared in Example 1-4 was provided as a starting raw material, and elongation was performed with Fmoc-MeLeu-OH (oxyma, 50°C, 10 hours), and then with Fmoc-Ile-OH (HOAt, 40°C, 2.5 hours) in accordance with synthesis method 1 to synthesize pd2-005-resin. The loading rate calculated by the Fmoc quantitation method using a dry resin (10.73 mg) was 0.337 mmol/g. (UVarea value at 294 nm: 3610.72 and UVarea value at 304 nm: 3254.78)
The recovery ratio is calculated from the following equation in accordance with the recovery ratio calculation method.

$$\text{Recovery ratio} = 0.337 \times (1 \div 0.455 - 436.51 \times 0.001 + 676.86 \times 0.001) = 82.2\%$$

Analysis of the isolating reaction liquid by LCMS showed that the purity of intended product pd2-005 was 97.7 area%, epimeric form pd2-005-a was observed at 0.1 area%, and excessively elongated form pd2-005-b was observed at 2.2 area%.

Analysis condition: SQDAA50long

[Table 26]

| | | Molecular weight | LCMS (ESI) m/z | Retention time (min) | LC area% |
|---|---|---|---|---|---|
| pd2-005 | Intended product | 676.86 | 677.6 (M+H)+ | 2.05 | 97.7 |
| pd2-005-a | Epimeric form | 676.86 | 677.6 (M+H)+ | 2.25 | 0.1 |
| pd2-005-b | Excessively elongated form | 873.11 | 873.9 (M+H)+ | 2.42 | 2.2 |

The table below collectively shows the above results.

[Table 27]

| | Recovery ratio (%) | Intended product pd2-005 (area%) | Epimeric form pd2-005-a (area%) | Excessively elongated form pd2-005-b (area%) | AA2-deficient form pd2-005-c (area%) |
|---|---|---|---|---|---|
| Condition 1 | 100.2 | 99.9 | 0.1 | - | - |
| Condition 2 | 90.0 | 98.3 | 0.1 | 1.6 | - |
| Condition 3 | 82.2 | 97.7 | 0.1 | 2.2 | - |

Under condition 1 where a dipeptide was supported, an intended product was obtained with a high recovery ratio and a high purity.
Under conditions 2 and 3 where a single amino acid was supported, generation of an excessively elongated form was observed in addition to a decrease in recovery ratio due to premature cleavage.

Synthesis of compound pd2-006-resin, (3S)-3-[[(2S)-2-cyclopentyl-2-[[(2S,3S)-2-(9H-fluoren-9-ylmethoxycarbonylamino)-3-methylpentanoyl]-methylamino]acetyl]-methylamino]-4-(dimethylamino)-4-oxobutanoic acid-2-chlorotrityl resin (Fmoc-Ile-MeGly(cPent)-MeAsp(O-Trt(2-Cl)resin)-NMe2)

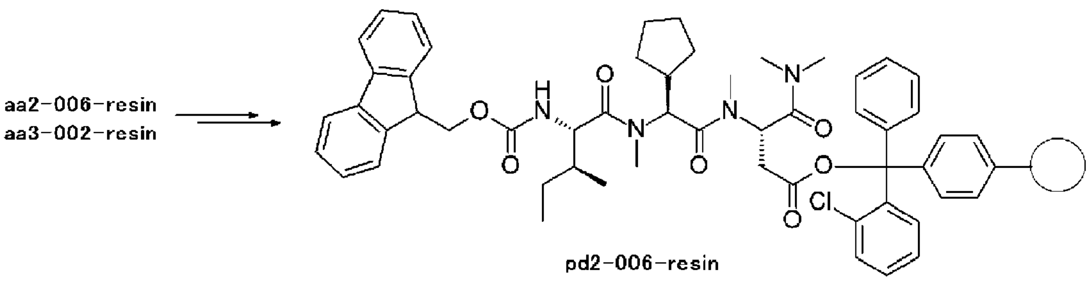

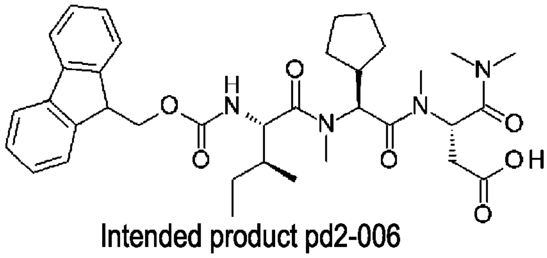

Intended product pd2-006

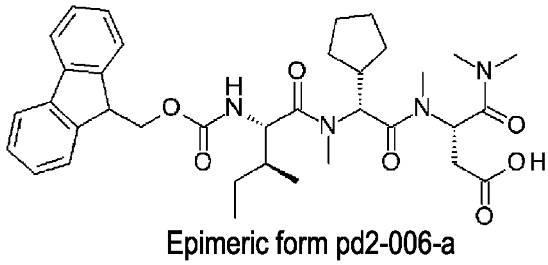

Epimeric form pd2-006-a

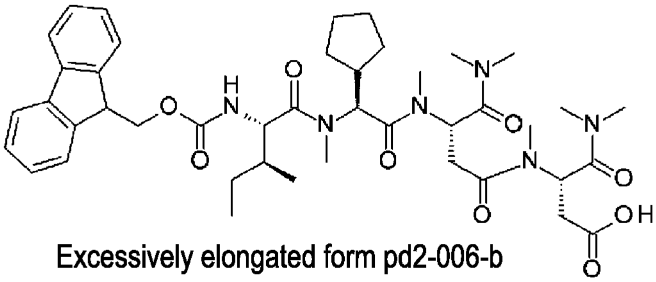

Excessively elongated form pd2-006-b

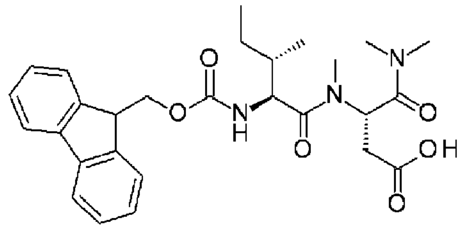

AA2-deficient form pd2-006-c

Synthesis of pd2-006-resin under condition 1

The dipeptide-supporting resin aa2-006-resin (Fmoc-MeGly(cPent)-MeAsp(O-Trt(2-Cl)resin)-NMe2) (100 mg, 0.345 mmol/g) prepared in Example 1-4 was provided as a starting raw material, and elongation was performed with Fmoc-Ile-OH (HOAt, 40°C, 2.5 hours) in accordance with synthesis method 1 to synthesize pd2-006-resin. The loading rate calculated by the Fmoc quantitation method using a dry resin (10.23 mg) was 0.349 mmol/g. (UVarea value at 294 nm: 3565.79 and UVarea value at 304 nm: 3223.59)
The recovery ratio is calculated from the following equation in accordance with the recovery ratio calculation method.

$$\text{Recovery ratio} = 0.349 \times (1 \div 0.345 - 535.64 \times 0.001 + 648.80 \times 0.001) = 105.1\%$$

Analysis of the isolating reaction liquid by LCMS showed that the purity of intended product pd2-006 was 100 area%.

Analysis condition: SQDFA05

[Table 28]

| | | Molecular weight | LCMS (ESI) m/z | Retention time (min) | LC area% |
|---|---|---|---|---|---|
| pd2-006 | Intended product | 648.80 | 649.6 (M+H)+ | 0.96 | 100 |
| pd2-006-a | Epimeric form | 648.80 | not detected | | |

Synthesis of pd2-006-resin under condition 2

The amino acid-supported resin aa3-002-resin (Fmoc-MeAsp(O-Trt(2-Cl)resin)-NMe2) (100 mg, 0.442 mmol/g) prepared in Example 1-4 was provided as a starting raw material, and elongation was performed with Fmoc-MeGly(cPent)-OH (HOAt, 40°C, 2.5 hours), and then with Fmoc-Ile-OH (HOAt, 40°C, 2.5 hours) in accordance with synthesis method 1 to synthesize pd2-006-resin. The loading rate calculated by the Fmoc quantitation method using a dry resin (10.73 mg) was 0.304 mmol/g. (UVarea value at 294 nm: 3263.10 and UVarea value at 304 nm: 2945.28)
The recovery ratio is calculated from the following equation in accordance with the recovery ratio calculation method.

$$\text{Recovery ratio} = 0.304 \times (1 \div 0.442 - 396.44 \times 0.001 + 648.80 \times 0.001) = 76.5\%$$

Analysis of the isolating reaction liquid by LCMS showed that the purity of intended product pd2-006 was 76.3 area%, epimeric form pd2-006-a was observed at 1.2 area%, excessively elongated form pd2-006-b was observed at 5.6 area%, and AA2-deficient form pd2-006-c was observed at 16.9 area%.

Analysis condition: SQDFA05

[Table 29]

| | | Molecular weight | LCMS (ESI) m/z | Retention time (min) | LC area% |
|---|---|---|---|---|---|
| pd2-006 | Intended product | 648.80 | 649.7 (M+H)+ | 0.97 | 76.3 |
| pd2-006-a | Epimeric form | 648.80 | 649.6 (M+H)+ | 1.03 | 1.2 |
| pd2-006-b | Excessively elongated form | 804.98 | 805.7 (M+H)+ | 0.93 | 5.6 |
| pd2-006-c | AA2-deficient form | 509.60 | 510.5 (M+H)+ | 0.88 | 16.9 |

Synthesis of pd2-006-resin under condition 3

The amino acid-supported resin aa3-002-resin (Fmoc-MeAsp(O-Trt(2-Cl)resin)-NMe2) (100 mg, 0.442 mmol/g) prepared in Example 1-4 was provided as a starting raw material, and elongation was performed with Fmoc-MeGly(cPent)-OH (oxyma, 50°C, 10 hours), and then with Fmoc-Ile-OH (HOAt, 40°C, 2.5 hours) in accordance with synthesis method 1 to synthesize pd2-006-resin. The loading rate calculated by the Fmoc quantitation method using a dry resin (10.60 mg) was 0.211 mmol/g. (UVarea value at 294 nm: 2239.56 and UVarea value at 304 nm: 2018.13)
The recovery ratio is calculated from the following equation in accordance with the recovery ratio calculation method.

$$\text{Recovery ratio} = 0.211 \times (1 \div 0.442 - 396.44 \times 0.001 + 648.80 \times 0.001) = 53.1\%$$

Analysis of the isolating reaction liquid by LCMS showed that the purity of intended product pd2-006 was 85.9 area%, and epimeric form pd2-006-b was observed at 14.1 area%.

Analysis condition: SQDFA05

[Table 30]

| | | Molecular weight | LCMS (ESI) m/z | Retention time (min) | LC area% |
|---|---|---|---|---|---|
| pd2-006 | Intended product | 648.80 | 649.6 (M+H)+ | 0.96 | 85.9 |
| pd2-006-a | Epimeric form | 648.80 | not detected | | |
| pd2-006-b | Excessively elongated form | 804.98 | 805.7 (M+H)+ | 0.92 | 14.1 |

The table below collectively shows the above results.

[Table 31]

| | Recovery ratio (%) | Intended product pd2-006 (area%) | Epimeric form pd2-006-a (area%) | Excessively elongated form pd2-006-b (area%) | AA2-deficient form pd2-006-c (area%) |
|---|---|---|---|---|---|
| Condition 1 | 105.1 | 100 | - | - | - |
| Condition 2 | 76.5 | 76.3 | 1.2 | 5.6 | 16.9 |

(continued)

| | Recovery ratio (%) | Intended product pd2-006 (area%) | Epimeric form pd2-006-a (area%) | Excessively elongated form pd2-006-b (area%) | AA2-deficient form pd2-006-c (area%) |
|---|---|---|---|---|---|
| Condition 3 | 53.1 | 85.9 | - | 14.1 | - |

Under condition 1 where a dipeptide was supported, an intended product was obtained with a high recovery ratio and a high purity.
When a single amino acid was supported, generation of an excessively elongated form and generation of an AA2-deficient form due to poor elongation of AA2 that is a site difficult to elongate were observed in addition to a decrease in recovery ratio due to premature cleavage under normal condition 2. Even under condition 3 that is adaptable to a sequence difficult to elongate, a decrease in recovery ratio due to premature cleavage was observed, and there was an increase in generation of an excessively elongated form although an AA2-deficient form was not generated.

Synthesis of compound pd2-007-resin, (3S)-4-(dimethylamino)-3-[[(2S)-2-[[(2S,3S)-2-(9H-fluoren-9-ylmethoxycarbonylamino)-3-methylpentanoyl]-methylamino]-4-methylpentanoyl]-methylamino]-4-oxobutanoic acid-2-chlorotrityl resin (Fmoc-Ile-MeLeu-MeAsp)(O-Trt(2-Cl)resin)-NMe2)

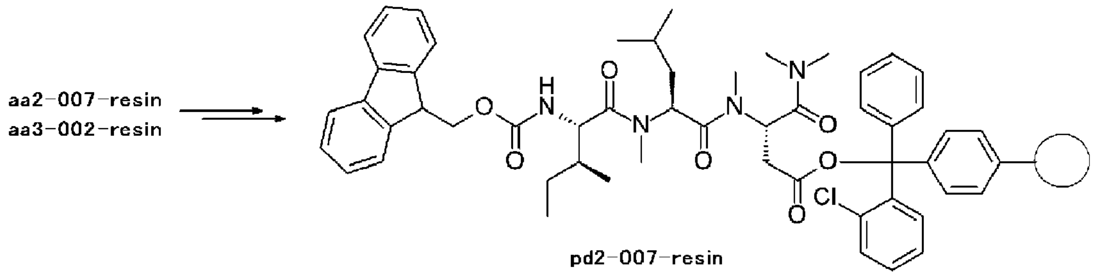


pd2-007-resin

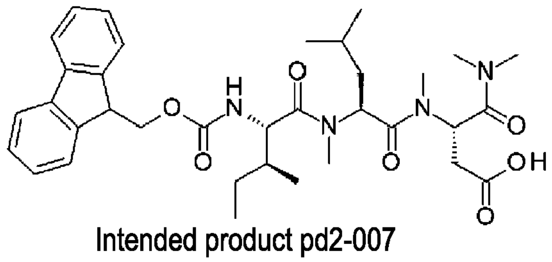

Intended product pd2-007

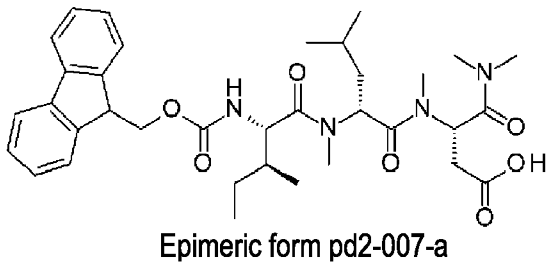

Epimeric form pd2-007-a

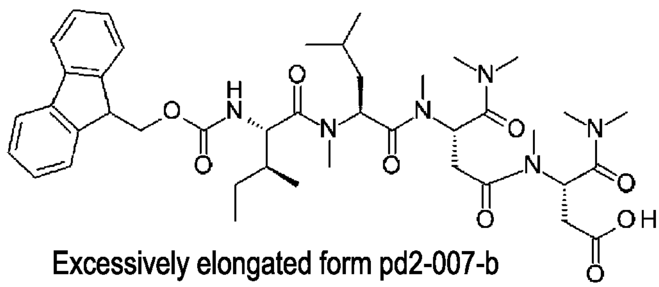

Excessively elongated form pd2-007-b

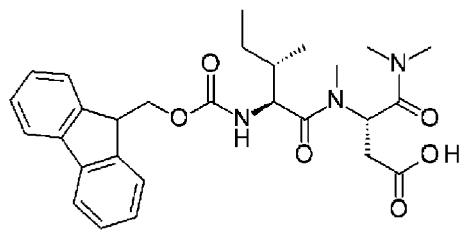

AA2-deficient form pd2-007-c

Synthesis of pd2-007-resin under condition 1

The dipeptide-supporting resin aa2-007-resin (Fmoc-MeLeu-MeAsp(O-Trt(2-Cl)resin)-NMe2) (100 mg, 0.384 mmol/g) prepared in Example 1-4 was provided as a starting raw material, and elongation was performed with Fmoc-Ile-OH (HOAt, 40°C, 2.5 hours) in accordance with synthesis method 1 to synthesize pd2-007-resin. The loading rate calculated by the Fmoc quantitation method using a dry resin (9.96 mg) was 0.367 mmol/g. (UVarea value at 294 nm: 3654.86 and UVarea value at 304 nm: 3298.78)
The recovery ratio is calculated from the following equation in accordance with the recovery ratio calculation method.

$$\text{Recovery ratio} = 0.367 \times (1 \div 0.384 - 523.63 \times 0.001 + 636.79 \times 0.001) = 99.7\%$$

Analysis of the isolating reaction liquid by LCMS showed that the purity of intended product pd2-007 was 100 area%.

Analysis condition: SQDFA05

[Table 32]

| | | Molecular weight | LCMS (ESI) m/z | Retention time (min) | LC area% |
|---|---|---|---|---|---|
| pd2-007 | Intended product | 636.79 | 637.6 (M+H)+ | 0.96 | 100 |
| pd2-007-a | Epimeric form | 636.79 | not detected | | |

Synthesis of pd2-007-resin under condition 2

The amino acid-supported resin prepared in Example 1-4 (aa3-002-resin (Fmoc-MeAsp(O-Trt(2-Cl)resin)-NMe2) (100 mg, 0.442 mmol/g) was provided as a starting raw material, and elongation was performed with Fmoc-MeLeu-OH (HOAt, 40°C, 2.5 hours), and then with Fmoc-Ile-OH (HOAt, 40°C, 2.5 hours) in accordance with synthesis method 1 to synthesize pd2-007-resin. The loading rate calculated by the Fmoc quantitation method using a dry resin (10.15 mg) was 0.371 mmol/g. (UVarea value at 294 nm: 3770.57 and UVarea value at 304 nm: 3391.62)
The recovery ratio is calculated from the following equation in accordance with the recovery ratio calculation method.

$$\text{Recovery ratio} = 0.371 \times (1 \div 0.442 - 396.44 \times 0.001 + 636.79 \times 0.001) = 92.9\%$$

Analysis of the isolating reaction liquid by LCMS showed that the purity of intended product pd2-007 was 97.9 area%, and an excessively elongated form pd2-007-b was observed at 2.1 area%. In this substrate, AA2-deficient form pd2-007-c was not observed even under condition 2.

Analysis condition: SQDFA05

[Table 33]

| | | Molecular weight | LCMS (ESI) m/z | Retention time (min) | LC area% |
|---|---|---|---|---|---|
| pd2-007 | Intended product | 636.79 | 637.7 (M+H)+ | 0.97 | 97.9 |
| pd2-007-a | Epimeric form | 636.79 | not detected | | |
| pd2-007-b | Excessively elongated form | 792.97 | 793.7 (M+H)+ | 0.92 | 2.1 |
| pd2-007-c | AA2-deficient form | 509.60 | not detected | | |

Synthesis of pd2-007-resin under condition 3

The amino acid-supported resin aa3-002-resin (Fmoc-MeAsp(O-Trt(2-Cl)resin)-NMe2) (100 mg, 0.442 mmol/g) prepared in Example 1-4 was provided as a starting raw material, and elongation was performed with Fmoc-MeLeu-OH (oxyma, 50°C, 10 hours), and then with Fmoc-Ile-OH (HOAt, 40°C, 2.5 hours) in accordance with synthesis method 1 to synthesize pd2-007-resin. The loading rate calculated by the Fmoc quantitation method using a dry resin (10.73 mg) was 0.334 mmol/g. (UVarea value at 294 nm: 3587.19 and UVarea value at 304 nm: 3234.07)
The recovery ratio is calculated from the following equation in accordance with the recovery ratio calculation method.

$$\text{Recovery ratio} = 0.334 \times (1 \div 0.442 - 396.44 \times 0.001 + 636.79 \times 0.001) = 83.6\%$$

Analysis of the isolating reaction liquid by LCMS showed that the purity of intended product pd2-007 was 96.6 area%, and an excessively elongated form pd2-007-b was observed at 3.4 area%.

Analysis condition: SQDFA05

[Table 34]

| | | Molecular weight | LCMS (ESI) m/z | Retention time (min) | LC area% |
|---|---|---|---|---|---|
| pd2-007 | Intended product | 636.79 | 637.6 (M+H)+ | 0.96 | 96.6 |
| pd2-007-a | Epimeric form | 636.79 | not detected | | |
| pd2-007-b | Excessively elongated form | 792.97 | 793.7 (M+H)+ | 0.92 | 3.4 |

The table below collectively shows the above results.

[Table 35]

| | Recovery ratio (%) | Intended product pd2-007 (area%) | Epimeric form pd2-007-a (area%) | Excessively elongated form pd2-007-b (area%) | AA2-deficient form pd2-007-c (area%) |
|---|---|---|---|---|---|
| Condition 1 | 99.7 | 100 | - | - | - |
| Condition 2 | 92.9 | 97.9 | - | 2.1 | - |
| Condition 3 | 83.6 | 96.6 | - | 3.4 | - |

Under condition 1 where a dipeptide was supported, an intended product was obtained with a high recovery ratio and a high purity.
Under conditions 2 and 3 where a single amino acid was supported, generation of an excessively elongated form was observed in addition to a decrease in recovery ratio due to premature cleavage.

Synthesis of compound pd2-008-resin, (3R)-3-[[(2S)-2-[[(2S,3S)-2-(9H-fluoren-9-ylmethoxycarbonylamino)-3-methylpentanoyl]-methylamino]-3-methylbutanoyl]-methylamino]butanoic acid-2-chlorotrityl resin (Fmoc-Ile-MeVal-D-3-MeAbu-O-Trt(2-Cl)resin)

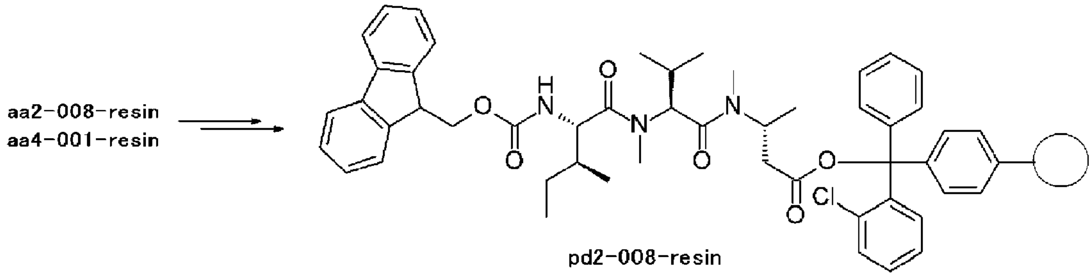


pd2-008-resin

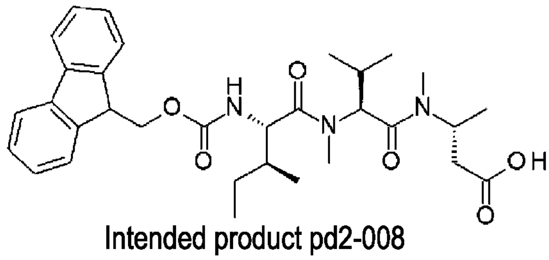

Intended product pd2-008

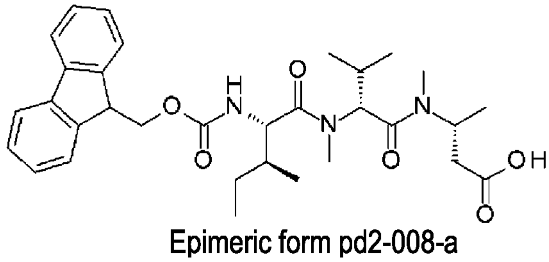

Epimeric form pd2-008-a

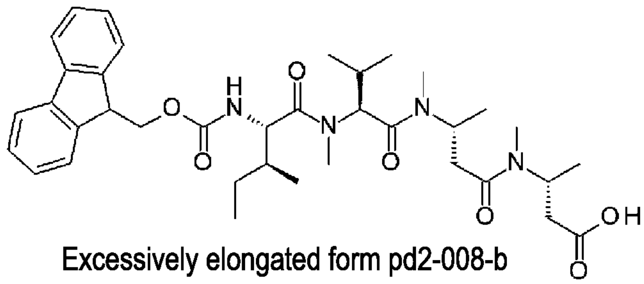

Excessively elongated form pd2-008-b

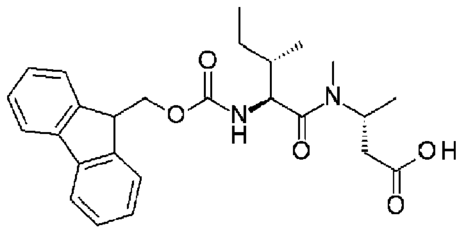

AA2-deficient form pd2-008-c

Synthesis of pd2-008-resin under condition 1

The dipeptide-supporting resin aa2-008-resin (Fmoc-MeVal-D-3-MeAbu-O-Trt(2-Cl)resin) (100 mg, 0.415 mmol/g) prepared in Example 1-4 was provided as a starting raw material, and elongation was performed with Fmoc-Ile-OH (HOAt, 40°C, 2.5 hours) in accordance with synthesis method 1 to synthesize pd2-008-resin. The loading rate calculated by the Fmoc quantitation method using a dry resin (10.74 mg) was 0.378 mmol/g. (UVarea value at 294 nm: 4060.03 and UVarea value at 304 nm: 3652.89)
The recovery ratio is calculated from the following equation in accordance with the recovery ratio calculation method.

$$\text{Recovery ratio} = 0.378 \times (1 \div 0.415 - 452.44 \times 0.001 + 565.71 \times 0.001) = 95.4\%$$

Analysis of the isolating reaction liquid by LCMS showed that the purity of intended product pd2-008 was 100 area%.

Analysis condition: SQDAA50long

[Table 36]

| | | Molecular weight | LCMS (ESI) m/z | Retention time (min) | LC area% |
|---|---|---|---|---|---|
| pd2-008 | Intended product | 565.71 | 566.5 (M+H)+ | 1.49 | 100 |
| pd2-008-a | Epimeric form | 565.71 | not detected | | |

Synthesis of pd2-008-resin under condition 2

The amino acid-supported resin aa4-001-resin (Fmoc-D-3-MeAbu-O-Trt(2-Cl)resin) (100 mg, 0.369 mmol/g) prepared in Example 1-4 was provided as a starting raw material, and elongation was performed with Fmoc-MeVal-OH (HOAt, 40°C, 2.5 hours), and then with Fmoc-Ile-OH (HOAt, 40°C, 2.5 hours) in accordance with synthesis method 1 to synthesize pd2-008-resin. The loading rate calculated by the Fmoc quantitation method using a dry resin (10.62 mg) was 0.295 mmol/g. (UVarea value at 294 nm: 3138.26 and UVarea value at 304 nm: 2821.05)
The recovery ratio is calculated from the following equation in accordance with the recovery ratio calculation method.

$$\text{Recovery ratio} = 0.295 \times (1 \div 0.369 - 339.39 \times 0.001 + 565.71 \times 0.001) = 86.6\%$$

Analysis of the isolating reaction liquid by LCMS showed that the purity of intended product pd2-008 was 99.1 area%, and an excessively elongated form pd2-008-b was observed at 1.0 area%. In this substrate, AA2-deficient form pd2-008-c was not observed even under condition 2.

Analysis condition: SQDAA50long

[Table 37]

| | | Molecular weight | LCMS (ESI) m/z | Retention time (min) | LC area% |
|---|---|---|---|---|---|
| pd2-008 | Intended product | 565.71 | 566.5 (M+H)+ | 1.50 | 99.1 |
| pd2-008-a | Epimeric form | 565.71 | not detected | | |
| pd2-008-b | Excessively elongated form | 664.84 | 665.6 (M+H)+ | 1.58 | 1.0 |
| pd2-008-c | AA2-deficient form | 452.55 | not detected | | |

Synthesis of pd2-008-resin under condition 3

The amino acid-supported resin aa4-001-resin (Fmoc-D-3-MeAbu-O-Trt(2-Cl)resin) (100 mg, 0.369 mmol/g) prepared in Example 1-4 was provided as a starting raw material, and elongation was performed with Fmoc-MeVal-OH (oxyma, 50°C, 10 hours), and then with Fmoc-Ile-OH (HOAt, 40°C, 2.5 hours) in accordance with synthesis method 1 to synthesize pd2-008-resin. The loading rate calculated by the Fmoc quantitation method using a dry resin (10.44 mg) was 0.284 mmol/g. (UVarea value at 294 nm: 2964.86 and UVarea value at 304 nm: 2667.78)
The recovery ratio is calculated from the following equation in accordance with the recovery ratio calculation method.

$$\text{Recovery ratio} = 0.284 \times (1 \div 0.369 - 339.39 \times 0.001 + 565.71 \times 0.001) = 83.4\%$$

Analysis of the isolating reaction liquid by LCMS showed that the purity of intended product pd2-008 was 98.1 area%, and an excessively elongated form pd2-008-b was observed at 1.9 area%.

Analysis condition: SQDAA50long

[Table 38]

| | | Molecular weight | LCMS (ESI) m/z | Retention time (min) | LC area% |
|---|---|---|---|---|---|
| pd2-008 | Intended product | 565.71 | 566.6 (M+H)+ | 1.51 | 98.1 |
| pd2-008-a | Epimeric form | 565.71 | not detected | | |
| pd2-008-b | Excessively elongated form | 664.84 | 665.6 (M+H)+ | 1.59 | 1.9 |

The table below collectively shows the above results.

[Table 39]

| | Recovery ratio (%) | Intended product pd2-008 (area%) | Epimeric form pd2-008-a (area%) | Excessively elongated form pd2-008-b (area%) | AA2-deficient form pd2-008-c (area%) |
|---|---|---|---|---|---|
| Condition 1 | 95.4 | 100 | - | - | - |
| Condition 2 | 86.6 | 99.1 | - | 1.0 | - |
| Condition 3 | 83.4 | 98.1 | - | 1.9 | - |

Under condition 1 where a dipeptide was supported, an intended product was obtained with a high recovery ratio and a high purity.

Under conditions 2 and 3 where a single amino acid was supported, generation of an excessively elongated form was observed in addition to a decrease in recovery ratio due to premature cleavage.

<u>Synthesis of compound pd2-009-resin, (3R)-3-[[(2S)-2-cyclohexyl-2-[[(2S,3S)-2-(9H-fluoren-9-ylmethoxycarbonylamino)-3-methylpentanoyl]-methylamino]acetyl]-methylamino]butanoic acid-2-chlorotrityl resin (Fmoc-Ile-MeChg-D-3-Me-Abu-O-Trt(2-Cl)resin)</u>

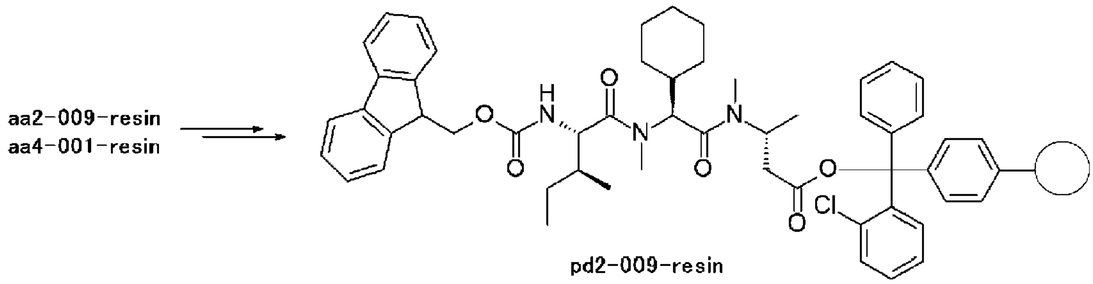


pd2-009-resin

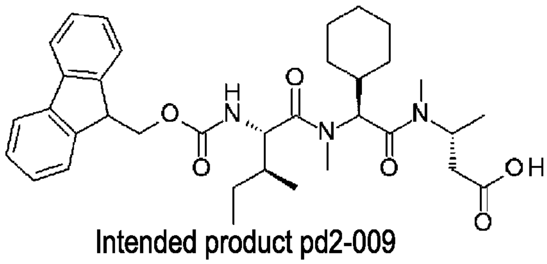

Intended product pd2-009

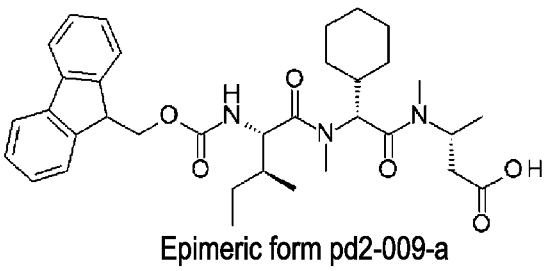

Epimeric form pd2-009-a

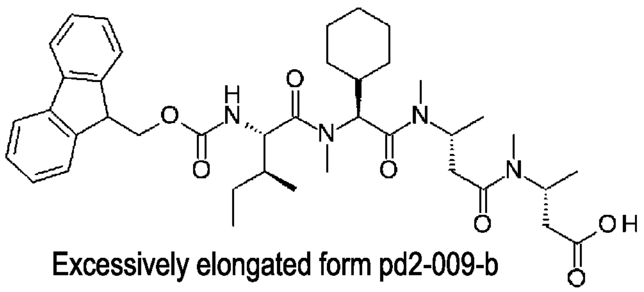

Excessively elongated form pd2-009-b

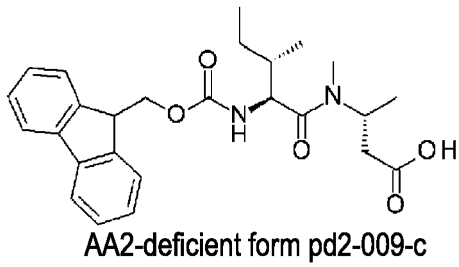

AA2-deficient form pd2-009-c

Synthesis of pd2-009-resin under condition 1

The dipeptide-supporting resin aa2-009-resin (Fmoc-MeChg-D-3-MeAbu-O-Trt(2-Cl)resin) (100 mg, 0.397 mmol/g) prepared in Example 1-4 was provided as a starting raw material, and elongation was performed with Fmoc-Ile-OH (HOAt, 40°C, 2.5 hours) in accordance with synthesis method 1 to synthesize pd2-009-resin. The loading rate calculated by the Fmoc quantitation method using a dry resin (11.10 mg) was 0.362 mmol/g. (UVarea value at 294 nm: 4014.43 and UVarea value at 304 nm: 3627.15)
The recovery ratio is calculated from the following equation in accordance with the recovery ratio calculation method.

$$\text{Recovery ratio} = 0.362 \times (1 \div 0.397 - 492.62 \times 0.001 + 605.78 \times 0.001) = 95.3\%$$

Analysis of the isolating reaction liquid by LCMS showed that the purity of intended product pd2-009 was 100 area%.

Analysis condition: SQDFA05long

[Table 40]

| | | Molecular weight | LCMS (ESI) m/z | Retention time (min) | LC area% |
|---|---|---|---|---|---|
| pd2-009 | Intended product | 605.78 | 606.5 (M+H)+ | 3.04 | 100 |
| pd2-009-a | Epimeric form | 605.78 | not detected | | |

The retention time of pd2-009 in this experiment (condition 1) is not completely consistent with the retention time of pd2-009 in condition 3, and this is ascribable to inter-measurement error.

Synthesis of pd2-009-resin under condition 2

The amino acid-supported resin aa4-001-resin (Fmoc-D-3-MeAbu-O-Trt(2-Cl)resin) (100 mg, 0.369 mmol/g) prepared in Example 1-4 was provided as a starting raw material, and elongation was performed with Fmoc-MeChg-OH (HOAt, 40°C, 2.5 hours), and then with Fmoc-Ile-OH (HOAt, 40°C, 2.5 hours) in accordance with synthesis method 1 to synthesize pd2-009-resin. The loading rate calculated by the Fmoc quantitation method using a dry resin (10.20 mg) was 0.293 mmol/g. (UVarea value at 294 nm: 2987.04 and UVarea value at 304 nm: 2692.68)
The recovery ratio is calculated from the following equation in accordance with the recovery ratio calculation method.

$$\text{Recovery ratio} = 0.293 \times (1 \div 0.369 - 339.39 \times 0.001 + 605.78 \times 0.001) = 87.2\%$$

Analysis of the isolating reaction liquid by LCMS showed that the purity of intended product pd2-009 was 92.8 area%, excessively elongated form pd2-009-b was observed at 3.4 area%, and AA2-deficient form pd2-009-c was observed at 3.8 area%.

Analysis condition: SQDFA05long

[Table 41]

| | | Molecular weight | LCMS (ESI) m/z | Retention time (min) | LC area% |
|---|---|---|---|---|---|
| pd2-009 | Intended product | 605.78 | 606.5 (M+H)+ | 3.03 | 92.8 |
| pd2-009-a | Epimeric form | 605.78 | not detected | | |
| pd2-009-b | Excessively elongated form | 704.91 | 705.6 (M+H)+ | 2.96 | 3.4 |
| pd2-009-c | AA2-deficient form | 452.56 | 453.5 (M+H)+ | 2.52 | 3.8 |

The retention times of pd2-009 and pd2-009-b in this experiment (condition 2) are not completely consistent with the retention times of pd2-009 and pd2-009-b in condition 3, and this is ascribable to inter-measurement error.

<u>Synthesis of pd2-009-resin under condition 3</u>

The amino acid-supported resin aa4-001-resin (Fmoc-D-3-MeAbu-O-Trt(2-Cl)resin) (100 mg, 0.369 mmol/g) prepared in Example 1-4 was provided as a starting raw material, and elongation was performed with Fmoc-MeChg-OH (oxyma, 50°C, 10 hours), and then with Fmoc-Ile-OH (HOAt, 40°C, 2.5 hours) in accordance with synthesis method 1 to synthesize pd2-009-resin. The loading rate calculated by the Fmoc quantitation method using a dry resin (9.56 mg) was 0.217 mmol/g. (UVarea value at 294 nm: 2072.81 and UVarea value at 304 nm: 1864.82)
The recovery ratio is calculated from the following equation in accordance with the recovery ratio calculation method.

$$\text{Recovery ratio} = 0.217 \times (1 \div 0.369 - 339.39 \times 0.001 + 605.78 \times 0.001) = 64.6\%$$

Analysis of the isolating reaction liquid by LCMS showed that the purity of intended product pd2-009 was 94.5 area%, and an excessively elongated form pd2-009-b was observed at 5.5 area%.

Analysis condition: SQDFA05long

[Table 42]

| | | Molecular weight | LCMS (ESI) m/z | Retention time (min) | LC area% |
|---|---|---|---|---|---|
| pd2-009 | Intended product | 605.78 | 606.6 (M+H)+ | 2.97 | 94.5 |
| pd2-009-a | Epimeric form | 605.78 | not detected | | |
| pd2-009-b | Excessively elongated form | 704.91 | 705.7 (M+H)+ | 2.92 | 5.5 |

The table below collectively shows the above results.

[Table 43]

| | Recovery ratio (%) | Intended product pd2-009 (area%) | Epimeric form pd2-009-a (area%) | Excessively elongated form pd2-009-b (area%) | AA2-deficient form pd2-009-c (area%) |
|---|---|---|---|---|---|
| Condition 1 | 95.3 | 100 | - | - | - |
| Condition 2 | 87.2 | 92.8 | - | 3.4 | 3.8 |

(continued)

| | Recovery ratio (%) | Intended product pd2-009 (area%) | Epimeric form pd2-009-a (area%) | Excessively elongated form pd2-009-b (area%) | AA2-deficient form pd2-009-c (area%) |
|---|---|---|---|---|---|
| Condition 3 | 64.6 | 94.5 | - | 5.5 | - |

Under condition 1 where a dipeptide was supported, an intended product was obtained with a high recovery ratio and a high purity.
When a single amino acid was supported, generation of an excessively elongated form and generation of an AA2-deficient form due to poor elongation of AA2 that is a site difficult to elongate were observed in addition to a decrease in recovery ratio due to premature cleavage under normal condition 2. Even under condition 3 that is adaptable to a sequence difficult to elongate, a decrease in recovery ratio due to premature cleavage was observed, and there was an increase in generation of an excessively elongated form although an AA2-deficient form was not generated.

Synthesis of compound pd2-010-resin, 2-[[(2S)-2-[[(2S,3S)-2-(9H-fluoren-9-ylmethoxycarbonylamino)-3-methylpentanoyl]-methylamino]-3-methylbutanoyl]-methylamino]acetic acid-2-chlorotrityl resin (Fmoc-Ile-MeVal-MeGly-O-Trt(2-Cl)resin)

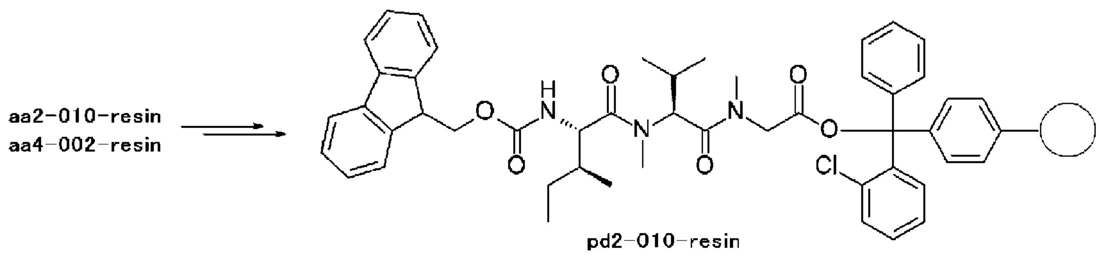


pd2-010-resin

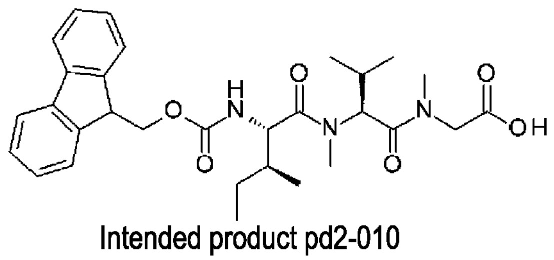

Intended product pd2-010

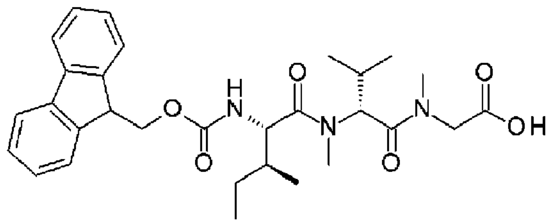

Epimeric form pd2-010-a

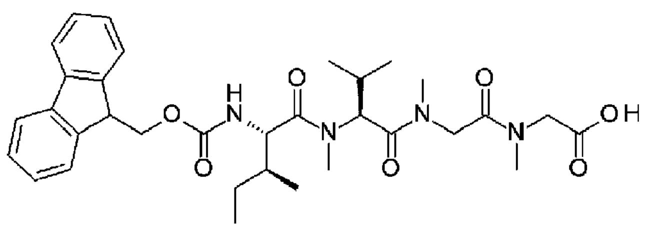

Excessively elongated form pd2-010-b

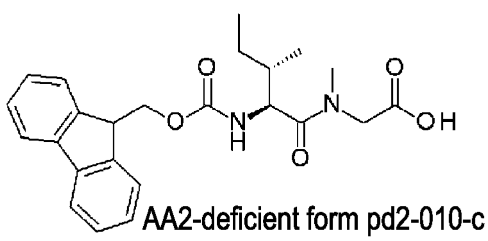

AA2-deficient form pd2-010-c

Synthesis of pd2-010-resin under condition 1

The dipeptide-supporting resin aa2-010-resin (Fmoc-MeVal-MeGly-O-Trt(2-Cl)resin) (100 mg, 0.374 mmol/g) prepared in Example 1-4 was provided as a starting raw material, and elongation was performed with Fmoc-Ile-OH

(HOAt, 40°C, 2.5 hours) in accordance with synthesis method 1 to synthesize pd2-010-resin. The loading rate calculated by the Fmoc quantitation method using a dry resin (10.86 mg) was 0.325 mmol/g. (UVarea value at 294 nm: 3525.31 and UVarea value at 304 nm: 3180.92)
The recovery ratio is calculated from the following equation in accordance with the recovery ratio calculation method.

$$\text{Recovery ratio} = 0.325 \times (1 \div 0.374 - 424.50 \times 0.001 + 537.66 \times 0.001) = 90.6\%$$

Analysis of the isolating reaction liquid by LCMS showed that the purity of intended product pd2-010 was 100 area%.

Analysis condition: SQDFA05

[Table 44]

| | | Molecular weight | LCMS (ESI) m/z | Retention time (min) | LC area% |
|---|---|---|---|---|---|
| pd2-010 | Intended product | 537.66 | 538.5 (M+H)+ | 0.94 | 100 |
| pd2-010-a | Epimeric form | 537.66 | not detected | | |

Synthesis of pd2-010-resin under condition 2

The amino acid-supported resin aa4-002-resin (Fmoc-MeGly-O-Trt(2-Cl)resin) (100 mg, 0.573 mmol/g) prepared in Example 1-4 was provided as a starting raw material, and elongation was performed with Fmoc-MeVal-OH (HOAt, 40°C, 2.5 hours), and then with Fmoc-Ile-OH (HOAt, 40°C, 2.5 hours) in accordance with synthesis method 1 to synthesize pd2-010-resin. The loading rate calculated by the Fmoc quantitation method using a dry resin (10.20 mg) was 0.326 mmol/g. (UVarea value at 294 nm: 3322.12 and UVarea value at 304 nm: 3002.34)
The recovery ratio is calculated from the following equation in accordance with the recovery ratio calculation method.

$$\text{Recovery ratio} = 0.326 \times (1 \div 0.573 - 311.34 \times 0.001 + 537.66 \times 0.001) = 64.3\%$$

Analysis of the isolating reaction liquid by LCMS showed that the purity of intended product pd2-010 was 95.6 area%, and an excessively elongated form pd2-010-b was observed at 4.4 area%. In this substrate, AA2-deficient form pd2-010-c was not observed even under condition 2.

Analysis condition: SQDFA05

[Table 45]

| | | Molecular weight | LCMS (ESI) m/z | Retention time (min) | LC area% |
|---|---|---|---|---|---|
| pd2-010 | Intended product | 537.66 | 538.5 (M+H)+ | 0.94 | 95.6 |
| pd2-010-a | Epimeric form | 537.66 | not detected | | |
| pd2-010-b | Excessively elongated form | 608.74 | 607.4 (M-H)- | 0.90 | 4.4 |
| pd2-010-c | AA2-deficient form | 424.50 | not detected | | |

Synthesis of pd2-010-resin under condition 3

The amino acid-supported resin aa4-002-resin (Fmoc-MeGly-O-Trt(2-Cl)resin) (100 mg, 0.573 mmol/g) prepared in Example 1-4 was provided as a starting raw material, and elongation was performed with Fmoc-MeVal-OH (oxyma, 50°C, 10 hours), and then with Fmoc-Ile-OH (HOAt, 40°C, 2.5 hours) in accordance with synthesis method 1 to synthesize pd2-010-resin. The loading rate calculated by the Fmoc quantitation method using a dry resin (10.69 mg)

was 0.331 mmol/g. (UVarea value at 294 nm: 3542.66 and UVarea value at 304 nm: 3189.92)
The recovery ratio is calculated from the following equation in accordance with the recovery ratio calculation method.

$$\text{Recovery ratio} = 0.331 \times (1 \div 0.573 - 311.34 \times 0.001 + 537.66 \times 0.001) = 65.3\%$$

Analysis of the isolating reaction liquid by LCMS showed that the purity of intended product pd2-010 was 93.9 area%, and an excessively elongated form pd2-010-b was observed at 6.1 area%.

Analysis condition: SQDFA05

[Table 46]

| | | Molecular weight | LCMS (ESI) m/z | Retention time (min) | LC area% |
|---|---|---|---|---|---|
| pd2-010 | Intended product | 537.66 | 538.5 (M+H)+ | 0.93 | 93.9 |
| pd2-010-a | Epimeric form | 537.66 | not detected | | |
| pd2-010-b | Excessively elongated form | 608.74 | 607.6 (M-H)- | 0.89 | 6.1 |

The table below collectively shows the above results.

[Table 47]

| | Recovery ratio (%) | Intended product pd2-010 (area%) | Epimeric form pd2-010-a (area%) | Excessively elongated form pd2-010-b (area%) | AA2-deficient form pd2-010-c (area%) |
|---|---|---|---|---|---|
| Condition 1 | 90.6 | 100 | - | - | - |
| Condition 2 | 64.3 | 95.6 | - | 4.4 | - |
| Condition 3 | 65.3 | 93.9 | - | 6.1 | - |

Under condition 1 where a dipeptide was supported, an intended product was obtained with a high recovery ratio and a high purity.
Under conditions 2 and 3 where a single amino acid was supported, generation of an excessively elongated form was observed in addition to a decrease in recovery ratio due to premature cleavage.

Synthesis of compound pd2-011-resin, (3S)-4-(dimethylamino)-3-[[(2S)-2-[[(2S,3S)-2-(9H-fluoren-9-ylmethoxycarbonylamino)-3-methylpentanoyl]-methylamino]-3-methylbutanoyl]amino]-4-oxobutanoic acid-2-chlorotrityl resin (Fmoc-Ile-MeVal-Asp(O-Trt(2-Cl)resin)-NMe2)

aa2-012-resin
aa3-003-resin

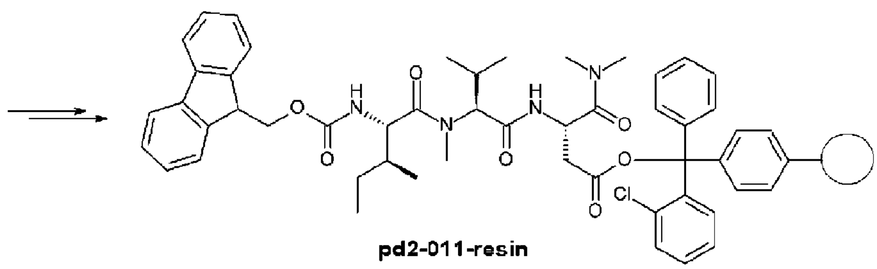

pd2-011-resin

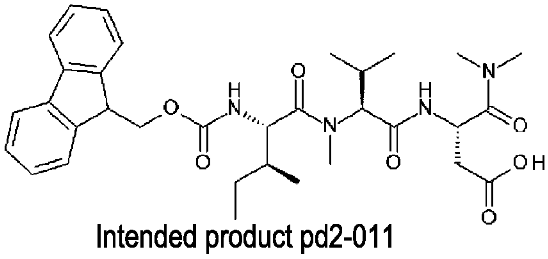

Intended product pd2-011

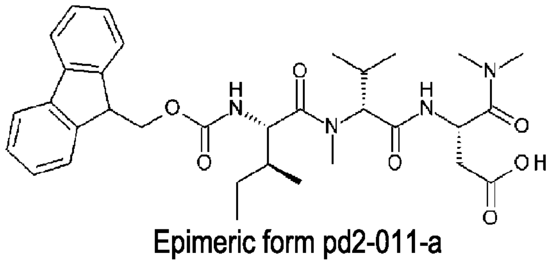

Epimeric form pd2-011-a

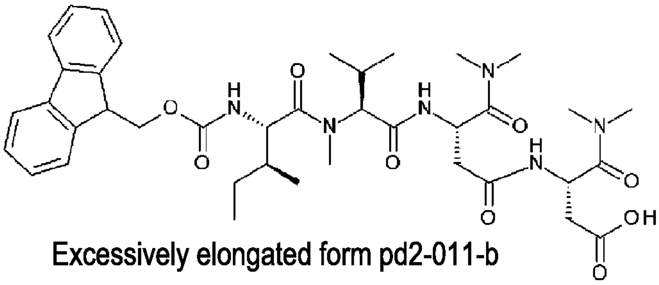

Excessively elongated form pd2-011-b

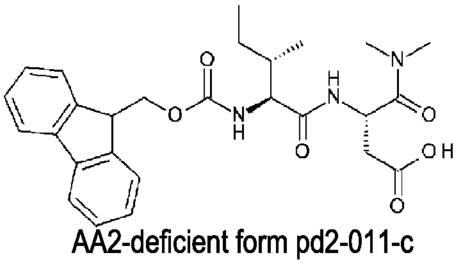

AA2-deficient form pd2-011-c

Synthesis of pd2-011-resin under condition 1

The dipeptide-supporting resin aa2-012-resin (Fmoc-MeVal-Asp(O-Trt(2-Cl)resin)-NMe2) (100 mg, 0.373 mmol/g) prepared in Example 1-4 was provided as a raw material, and elongation was performed with Fmoc-Ile-OH (HOAt, 40°C, 2.5 hours) in accordance with synthesis method 1 to synthesize pd2-011-resin. The loading rate calculated by the Fmoc quantitation method using a dry resin (9.87 mg) was 0.343 mmol/g. (UVarea value at 294 nm: 3397.67 and UVarea value at 304 nm: 3086.76)
The recovery ratio is calculated from the following equation in accordance with the recovery ratio calculation method.

$$\text{Recovery ratio} = 0.343 \times (1 \div 0.373 - 495.57 \times 0.001 + 608.73 \times 0.001) = 95.8\%$$

Analysis of the isolating reaction liquid by LCMS showed that the purity of intended product pd2-011 was 100 area%.

Analysis condition: SQDFA05long

[Table 48]

| | | Molecular weight | LCMS (ESI) m/z | Retention time (min) | LC area% |
|---|---|---|---|---|---|
| pd2-011 | Intended product | 608.73 | 609.6 (M+H)+ | 2.41 | 100 |
| pd2-01 1 -a | Epimeric form | 608.73 | not detected | | |

Synthesis of pd2-011-resin under condition 2

The amino acid-supported resin aa3-003-resin (Fmoc-Asp(O-Trt(2-Cl)resin)-NMe2) (100 mg, 0.469 mmol/g) prepared in Example 1-4 was provided as a raw material, and elongation was performed with Fmoc-MeVal-OH (HOAt, 40°C, 2.5 hours), and then with Fmoc-Ile-OH (HOAt, 40°C, 2.5 hours) in accordance with synthesis method 1 to synthesize pd2-011-resin. The loading rate calculated by the Fmoc quantitation method using a dry resin (10.54 mg) was 0.292 mmol/g. (UVarea value at 294 nm: 3084.36 and UVarea value at 304 nm: 2800.28)
The recovery ratio is calculated from the following equation in accordance with the recovery ratio calculation method.

$$\text{Recovery ratio} = 0.292 \times (1 \div 0.469 - 382.41 \times 0.001 + 608.73 \times 0.001) = 68.9\%$$

Analysis of the isolating reaction liquid by LCMS showed that the purity of intended product pd2-011 was 100 area%.

Analysis condition: SQDFA05long

[Table 49]

| | | Molecular weight | LCMS (ESI) m/z | Retention time (min) | LC area% |
|---|---|---|---|---|---|
| pd2-011 | Intended product | 608.73 | 609.6 (M+H)+ | 2.42 | 100 |
| pd2-011-a | Epimeric form | 608.73 | not detected | | |
| pd2-011-b | Excessively elongated form | 750.88 | not detected | | |
| pd2-011-c | AA2-deficient form | 495.57 | not detected | | |

Synthesis of pd2-011-resin under condition 3

The amino acid-supported resin aa3-003-resin (Fmoc-Asp(O-Trt(2-Cl)resin)-NMe2) (100 mg, 0.469 mmol/g) prepared in Example 1-4 was provided as a raw material, and elongation was performed with Fmoc-MeVal-OH (oxyma, 50°C, 10 hours), and then with Fmoc-Ile-OH (HOAt, 40°C, 2.5 hours) in accordance with synthesis method 1 to synthesize pd2-011-resin. The loading rate calculated by the Fmoc quantitation method using a dry resin (10.13 mg) was 0.320 mmol/g. (UVarea value at 294 nm: 3260.97 and UVarea value at 304 nm: 2943.08)
The recovery ratio is calculated from the following equation in accordance with the recovery ratio calculation method.

$$\text{Recovery ratio} = 0.320 \times (1 \div 0.469 - 382.41 \times 0.001 + 608.73 \times 0.001) = 75.5\%$$

Analysis of the isolating reaction liquid by LCMS showed that the purity of intended product pd2-011 was 100 area%.

Analysis condition: SQDFA05long

[Table 50]

| | | Molecular weight | LCMS (ESI) m/z | Retention time (min) | LC area% |
|---|---|---|---|---|---|
| pd2-011 | Intended product | 608.73 | 609.6 (M+H)+ | 2.41 | 100 |
| pd2-011-a | Epimeric form | 608.73 | not detected | | |
| pd2-011-b | Excessively elongated form | 750.88 | not detected | | |

The table below collectively shows the above results.

[Table 51]

| | Recovery ratio (%) | Intended product pd2-011 (area%) | Epimeric form pd2-011-a (area%) | Excessively elongated form pd2-011-b (area%) | AA2-deficient form pd2-011-c (area%) |
|---|---|---|---|---|---|
| Condition 1 | 95.8 | 100.0 | - | - | - |
| Condition 2 | 68.9 | 100.0 | - | - | - |

(continued)

| | Recovery ratio (%) | Intended product pd2-011 (area%) | Epimeric form pd2-011-a (area%) | Excessively elongated form pd2-011-b (area%) | AA2-deficient form pd2-011-c (area%) |
|---|---|---|---|---|---|
| Condition 3 | 75.5 | 100.0 | - | - | - |

Under condition 1 where a dipeptide was supported, an intended product was obtained with a high recovery ratio and a high purity.

Under conditions 2 and 3 where a single amino acid was supported, an intended product was obtained with a high purity, but there was a decrease in recovery ratio due to premature cleavage.

Synthesis of compound pd2-012-resin, (3S)-4-(dimethylamino)-3-[[2-[[(2S,3S)-2-(9H-fluoren-9-ylmethoxycarbonylamino)-3-methylpentanoyl]amino]acetyl]-methylamino]-4-oxobutanoic acid-2-chlorotrityl resin (Fmoc-Ile-Gly-MeAsp)(O-Trt(2-Cl)resin)-NMe2)

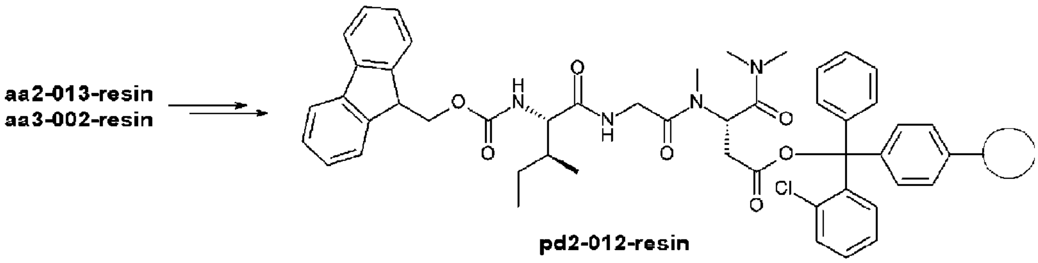


pd2-012-resin

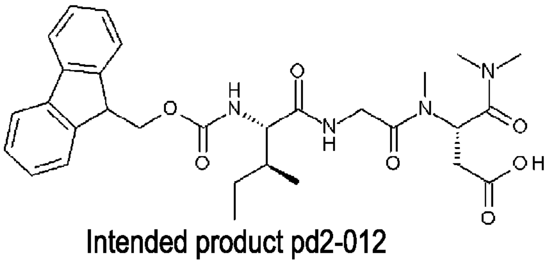

Intended product pd2-012

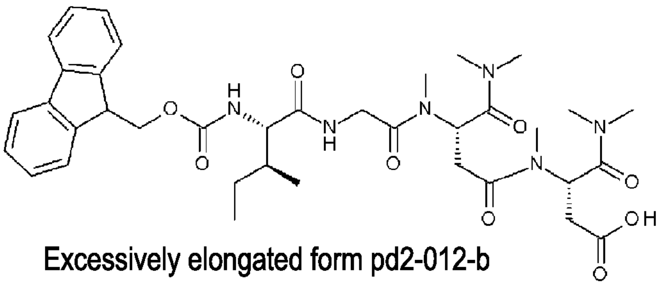

Excessively elongated form pd2-012-b

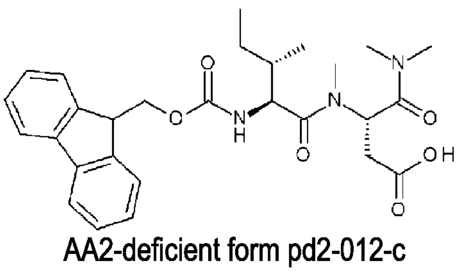

AA2-deficient form pd2-012-c

Synthesis of pd2-012-resin under condition 1

The dipeptide-supporting resin aa2-013-resin (Fmoc-Gly-MeAsp(O-Trt(2-Cl)resin)-NMe2) (100 mg, 0.386 mmol/g) prepared in Example 1-4 was provided as a raw material, and elongation was performed with Fmoc-Ile-OH (HOAt, 40°C, 2.5 hours) in accordance with synthesis method 1 to synthesize pd2-012-resin. The loading rate calculated by the Fmoc quantitation method using a dry resin (12.05 mg) was 0.364 mmol/g. (UVarea value at 294 nm: 4412.01

and UVarea value at 304 nm: 3987.67)
The recovery ratio is calculated from the following equation in accordance with the recovery ratio calculation method.

$$\text{Recovery ratio} = 0.364 \times (1 \div 0.386 - 453.49 \times 0.001 + 566.65 \times 0.001) = 98.4\%$$

Analysis of the isolating reaction liquid by LCMS showed that the purity of intended product pd2-012 was 100 area%.

Analysis condition: SQDFA05long

[Table 52]

| | | Molecular weight | LCMS (ESI) m/z | Retention time (min) | LC area% |
|---|---|---|---|---|---|
| pd2-012 | Intended product | 566.65 | 567.5 (M+H)+ | 2.14 | 100 |

Synthesis of pd2-012-resin under condition 2

The amino acid-supported resin aa3-002-resin (Fmoc-MeAsp(O-Trt(2-Cl)resin)-NMe2) (100 mg, 0.442 mmol/g) prepared in Example 1-4 was provided as a raw material, and elongation was performed with Fmoc-Gly-OH (HOAt, 40°C, 2.5 hours), and then with Fmoc-Ile-OH (HOAt, 40°C, 2.5 hours) in accordance with synthesis method 1 to synthesize pd2-012-resin. The loading rate calculated by the Fmoc quantitation method using a dry resin (10.16 mg) was 0.375 mmol/g. (UVarea value at 294 nm: 3841.95 and UVarea value at 304 nm: 3448.96)
The recovery ratio is calculated from the following equation in accordance with the recovery ratio calculation method.

$$\text{Recovery ratio} = 0.375 \times (1 \div 0.442 - 396.44 \times 0.001 + 566.65 \times 0.001) = 91.2\%$$

Analysis of the isolating reaction liquid by LCMS showed that the purity of intended product pd2-012 was 100 area%.

Analysis condition: SQDFA05long

[Table 53]

| | | Molecular weight | LCMS (ESI) m/z | Retention time (min) | LC area% |
|---|---|---|---|---|---|
| pd2-012 | Intended product | 566.65 | 567.5 (M+H)+ | 2.14 | 100 |
| pd2-012-b | Excessively elongated form | 722.83 | not detected | | |
| pd2-012-c | AA2-deficient form | 509.59 | not detected | | |

Synthesis of pd2-012-resin under condition 3

The amino acid-supported resin aa3-002-resin (Fmoc-MeAsp(O-Trt(2-Cl)resin)-NMe2) (100 mg, 0.442 mmol/g) prepared in Example 1-4 was provided as a raw material, and elongation was performed with Fmoc-Gly-OH (oxyma, 50°C, 10 hours), and then with Fmoc-Ile-OH (HOAt, 40°C, 2.5 hours) in accordance with synthesis method 1 to synthesize pd2-012-resin. The loading rate calculated by the Fmoc quantitation method using a dry resin (10.23 mg) was 0.346 mmol/g. (UVarea value at 294 nm: 3565.67 and UVarea value at 304 nm: 3211.93)
The recovery ratio is calculated from the following equation in accordance with the recovery ratio calculation method.

$$\text{Recovery ratio} = 0.346 \times (1 \div 0.442 - 396.44 \times 0.001 + 566.65 \times 0.001) = 84.2\%$$

Analysis of the isolating reaction liquid by LCMS showed that the purity of intended product pd2-011 was 100 area%.

Analysis condition: SQDFA05long

[Table 54]

| | | Molecular weight | LCMS (ESI) m/z | Retention time (min) | LC area% |
|---|---|---|---|---|---|
| pd2-012 | Intended product | 566.65 | 567.5 (M+H)+ | 2.14 | 100 |
| pd2-012-b | Excessively elongated form | 722.83 | not detected | | |

The table below collectively shows the above results.

[Table 55]

| | Recovery ratio (%) | Intended product pd2-012 (area%) | Excessively elongated form pd2-012-b (area%) | AA2-deficient form pd2-012-c (area%) |
|---|---|---|---|---|
| Condition 1 | 98.4 | 100.0 | - | - |
| Condition 2 | 91.2 | 100.0 | - | - |
| Condition 3 | 84.2 | 100.0 | - | - |

Under condition 1 where a dipeptide was supported, an intended product was obtained with a high recovery ratio and a high purity.

Under conditions 2 and 3 where a single amino acid was supported, an intended product was obtained with a high purity, but there was a decrease in recovery ratio due to premature cleavage.

<u>Synthesis of compound pd2-013-resin, (3S)-4-(dimethylamino)-3-[[2-[[(2S,3S)-2-(9H-fluoren-9-ylmethoxycarbonylamino)-3-methylpentanoyl]amino]-2-methylpropanoyl]methylamino]-4-oxobutanoic acid-2-chlorotrityl resin (Fmoc-Ile-Aib-MeAsp)(O-Trt(2-Cl)resin)-NMe2)</u>

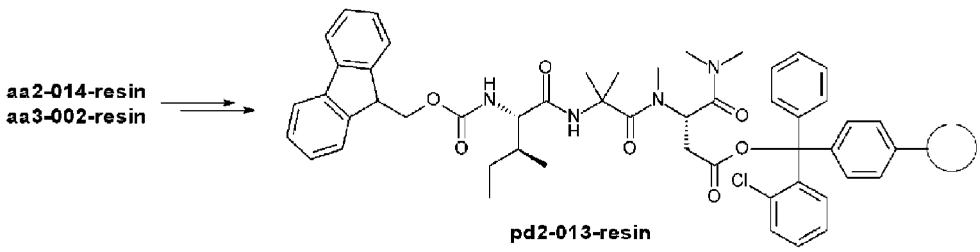


pd2-013-resin

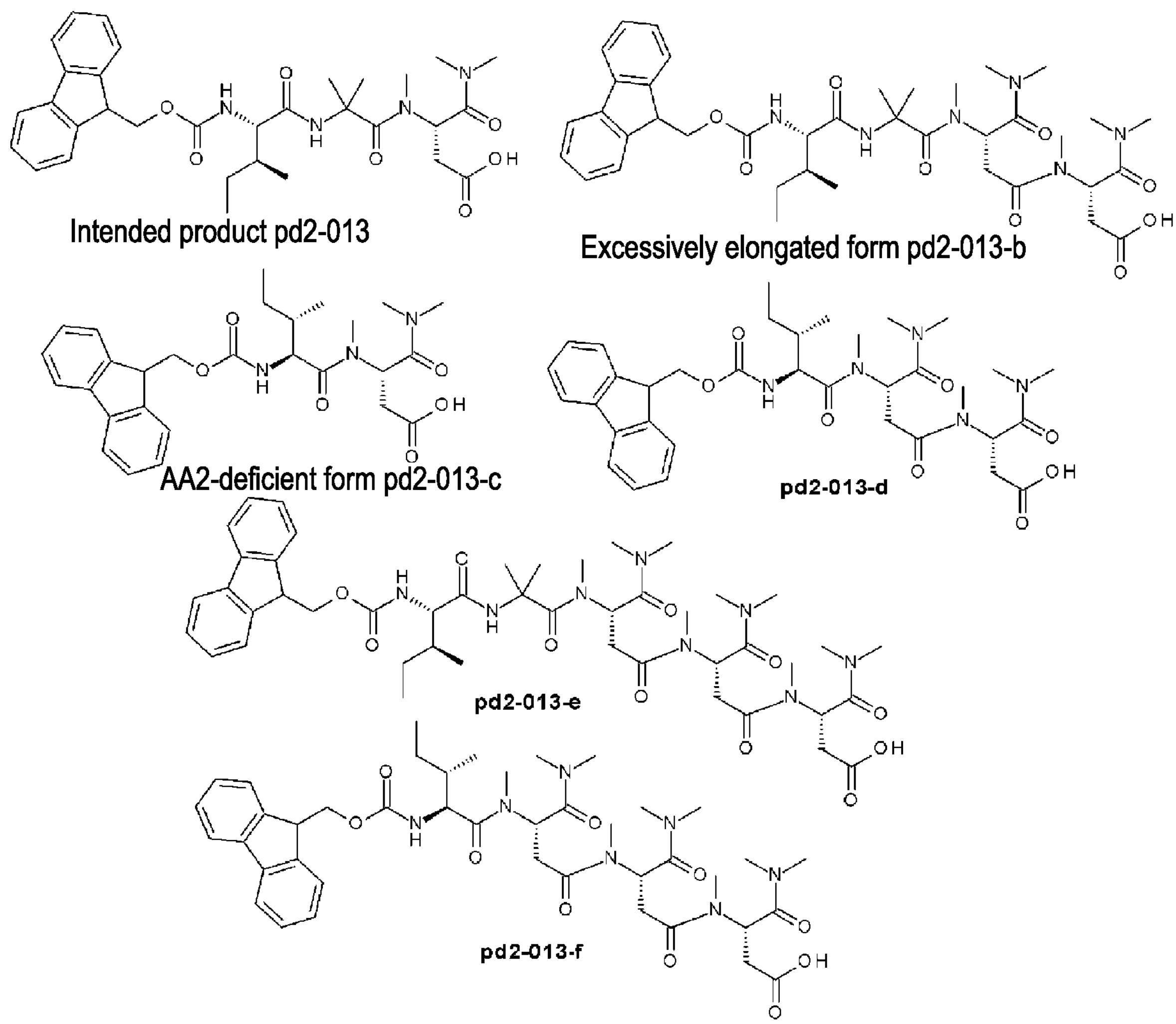

Intended product pd2-013

Excessively elongated form pd2-013-b

AA2-deficient form pd2-013-c

pd2-013-d

pd2-013-e

pd2-013-f

Synthesis of pd2-013-resin under condition 1

The dipeptide-supporting resin aa2-014-resin (Fmoc-Aib-MeAsp(O-Trt(2-Cl)resin)-NMe2) (100 mg, 0.413 mmol/g) prepared in Example 1-4 was provided as a raw material, and elongation was performed with Fmoc-Ile-OH (HOAt, 40°C, 2.5 hours) in accordance with synthesis method 1 to synthesize pd2-013-resin. The loading rate calculated by the Fmoc quantitation method using a dry resin (10.37 mg) was 0.305 mmol/g. (UVarea value at 294 nm: 3188.07 and UVarea value at 304 nm: 2860.10)
The recovery ratio is calculated from the following equation in accordance with the recovery ratio calculation method.

$$\text{Recovery ratio} = 0.305 \times (1 \div 0.413 - 481.54 \times 0.001 + 594.7 \times 0.001) = 77.3\%$$

Analysis of the isolating reaction liquid by LCMS showed that the purity of intended product pd2-013 was 100 area%.

Analysis condition: SQDFA05long

[Table 56]

| | | Molecular weight | LCMS (ESI) m/z | Retention time (min) | LC area% |
|---|---|---|---|---|---|
| pd2-013 | Intended product | 594.70 | 595.6 $(M+H)^+$ | 2.21 | 100 |

Synthesis of pd2-013-resin under condition 2

The amino acid-supported resin aa3-002-resin (Fmoc-MeAsp(O-Trt(2-Cl)resin)-NMe2) (100 mg, 0.442 mmol/g) prepared in Example 1-4 was provided as a raw material, and elongation was performed with Fmoc-Aib-OH (HOAt, 40°C, 2.5 hours), and then with Fmoc-Ile-OH (HOAt, 40°C, 2.5 hours) in accordance with synthesis method 1 to synthesize pd2-013-resin. The loading rate calculated by the Fmoc quantitation method using a dry resin (10.66 mg) was 0.360 mmol/g. (UVarea value at 294 nm: 3850.25 and UVarea value at 304 nm: 3502.10)
The recovery ratio is calculated from the following equation in accordance with the recovery ratio calculation method.

$$\text{Recovery ratio} = 0.360 \times (1 \div 0.442 - 396.44 \times 0.001 + 594.7 \times 0.001) = 88.6\%$$

Analysis of the isolating reaction liquid by LCMS showed that the purity of intended product pd2-013 was 2.6 area%, and AA2-deficient form pd2-013-c was obtained as a main product, where the amount of the AA2-deficient form was 93.0 area%. Excessively elongated form pd2-013-b was not observed, and pd3-013-d was observed as another by-product at 4.5 area%.

Analysis condition: SQDFA05long

[Table 57]

| | | Molecular weight | LCMS (ESI) m/z | Retention time (min) | LC area% |
|---|---|---|---|---|---|
| pd2-013 | Intended product | 594.70 | 595.6 $(M+H)^+$ | 2.21 | 2.6 |
| pd2-013-b | Excessively elongated form | 750.88 | not detected | | |
| pd2-013-c | AA2-deficient form | 509.59 | 510.5 $(M+H)^+$ | 2.33 | 93.0 |
| pd2-013-d | Other | 665.78 | 666.7 $(M+H)^+$ | 2.25 | 4.5 |

Synthesis of pd2-013-resin under condition 3

The amino acid-supported resin aa3-002-resin (Fmoc-MeAsp(O-Trt(2-Cl)resin)-NMe2) (100 mg, 0.442 mmol/g) prepared in Example 1-4 was provided as a raw material, and elongation was performed with Fmoc-Aib-OH (oxyma, 50°C, 10 hours), and then with Fmoc-Ile-OH (HOAt, 40°C, 2.5 hours) in accordance with synthesis method 1 to synthesize pd2-013-resin. The loading rate calculated by the Fmoc quantitation method using a dry resin (10.33 mg) was 0.195 mmol/g. (UVarea value at 294 nm: 2012.66 and UVarea value at 304 nm: 1833.56)
The recovery ratio is calculated from the following equation in accordance with the recovery ratio calculation method.

$$\text{Recovery ratio} = 0.195 \times (1 \div 0.442 - 396.44 \times 0.001 + 594.7 \times 0.001) = 48.0\%$$

Analysis of the isolating reaction liquid by LCMS showed that the purity of intended product pd2-013 was 21.6 area%, excessively elongated form pd2-013-b was observed at 27.3 area%, and AA2-deficient form pd2-013-c was obtained in an amount more than the amount of the intended product, where the amount of the AA2-deficient form was 36.5 area%. As other by-products, pd3-013-d was observed at 6.9 area%, pd3-013-e was observed at 5.6 area%, and pd3-013-f was observed at 2.1 area%.

Analysis condition: SQDFA05long

[Table 58]

| | | Molecular weight | LCMS (ESI) m/z | Retention time (min) | LC area% |
|---|---|---|---|---|---|
| pd2-013 | Intended product | 594.70 | 595.5 $(M+H)^+$ | 2.21 | 21.6 |

(continued)

| | | Molecular weight | LCMS (ESI) m/z | Retention time (min) | LC area% |
|---|---|---|---|---|---|
| pd2-013-b | Excessively elongated form | 750.88 | 751.7 $(M+H)^+$ | 2.12 | 27.3 |
| pd2-013-c | AA2-deficient form | 509.59 | 510.5 $(M+H)^+$ | 2.33 | 36.5 |
| pd2-013-d | Other | 665.78 | 666.7 $(M+H)^+$ | 2.25 | 6.9 |
| pd2-013-e | Other | 907.06 | 907.9 $(M+H)^+$ | 2.07 | 5.6 |
| pd2-013-f | Other | 821.96 | 822.7 $(M+H)^+$ | 2.18 | 2.1 |

The table below collectively shows the above results.

[Table 59]

| | Recovery ratio (%) | Intended product pd2-013 (area%) | Excessively elongated form pd2-013-b (area%) | AA2-deficient form pd2-013-c (area%) | Total amount of other by-products (area%) |
|---|---|---|---|---|---|
| Condition 1 | 77.3 | 100.0 | - | - | - |
| Condition 2 | 88.6 | 2.6 | - | 93.0 | 4.5 |
| Condition 3 | 48.0 | 21.6 | 27.3 | 36.5 | 14.6 |

Under condition 1 where a dipeptide was supported, an intended product was obtained with a high recovery ratio and a high purity.

Under conditions 2 and 3 where a single amino acid was supported, the recovery ratio in the reaction of elongation with Aib was poor, and an excessively elongated form and an AA2-deficient form were obtained as main products.

Synthesis of compound pd2-014-resin, (3R)-3-[[3-[[(2S,3S)-2-(9H-fluoren-9-ylmethoxycarbonylamino)-3-methylpentanoyl]amino]-2,2-dimethylpropanoyl]-methylamino]butanoic acid-2-chlorotrityl resin (Fmoc-Ile-bAla(2-Me2)-D-3-MeAbu-O-Trt(2-Cl)resin)

aa2-016-resin
aa4-001-resin

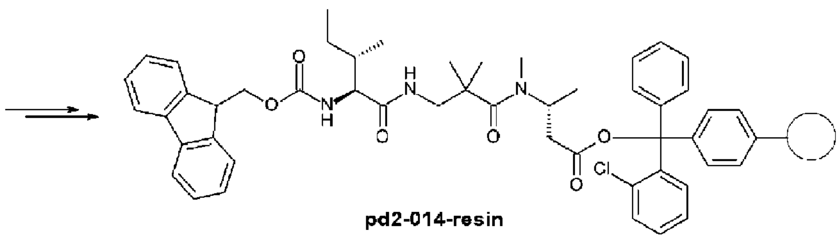

pd2-014-resin

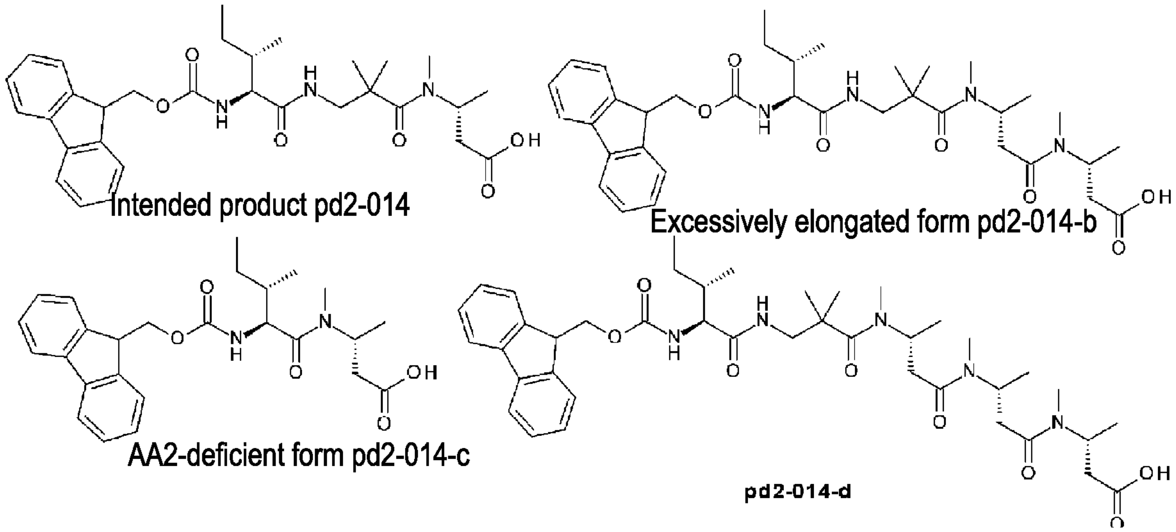

Intended product pd2-014

Excessively elongated form pd2-014-b

AA2-deficient form pd2-014-c

pd2-014-d

Synthesis of pd2-014-resin under condition 1

The dipeptide-supporting resin aa2-015-resin (Fmoc-bAla(2-Me2)-D-3-MeAbu-O-Trt(2-Cl)resin) (100 mg, 0.443 mmol/g) prepared in Example 1-4 was provided as a raw material, and elongation was performed with Fmoc-Ile-OH (HOAt, 40°C, 2.5 hours) in accordance with synthesis method 1 to synthesize pd2-014-resin. The loading rate calculated by the Fmoc quantitation method using a dry resin (11.21 mg) was 0.397 mmol/g. (UVarea value at 294 nm: 4470.95 and UVarea value at 304 nm: 4038.87)
The recovery ratio is calculated from the following equation in accordance with the recovery ratio calculation method.

$$\text{Recovery ratio} = 0.397 \times (1 \div 0.443 - 438.52 \times 0.001 + 551.67 \times 0.001) = 94.1\%$$

Analysis of the isolating reaction liquid by LCMS showed that the purity of intended product pd2-014 was 100 area%.

Analysis condition: SQDAA50long

[Table 60]

| | | Molecular weight | LCMS (ESI) m/z | Retention time (min) | LC area% |
|---|---|---|---|---|---|
| pd2-014 | Intended product | 551.67 | 552.5 $(M+H)^+$ | 1.21 | 100 |

Synthesis of pd2-014-resin under condition 2

The amino acid-supported resin aa4-001-resin (Fmoc-D-3-MeAbu-O-Trt(2-Cl)resin) (100 mg, 0.369 mmol/g) prepared in Example 1-4 was provided as a raw material, and elongation was performed with Fmoc-bAla(2-Me2)-OH (HOAt, 40°C, 2.5 hours), and then with Fmoc-Ile-OH (HOAt, 40°C, 2.5 hours) in accordance with synthesis method 1 to synthesize pd2-014-resin. The loading rate calculated by the Fmoc quantitation method using a dry resin (9.9 mg) was 0.239 mmol/g. (UVarea value at 294 nm: 2380.93 and UVarea value at 304 nm: 2140.88)
The recovery ratio is calculated from the following equation in accordance with the recovery ratio calculation method.

$$\text{Recovery ratio} = 0.239 \times (1 \div 0.369 - 339.38 \times 0.001 + 551.67 \times 0.001) = 69.8\%$$

Analysis of the isolating reaction liquid by LCMS showed that the purity of intended product pd2-014 was 68.1 area%, excessively elongated form pd2-014-b was observed at 4.8 area%, AA2-deficient form pd2-014-c was observed at 22.8 area%, and pd3-014-d was observed at 4.3% as another by-product.

Analysis condition: SQDAA50long

[Table 61]

| | | Molecular weight | LCMS (ESI) m/z | Retention time (min) | LC area% |
|---|---|---|---|---|---|
| pd2-014 | Intended product | 551.67 | 552.5 $(M+H)^+$ | 1.25 | 68.1 |
| pd2-014-b | Excessively elongated form | 650.81 | 651.7 $(M+H)^+$ | 1.36 | 4.8 |
| pd2-014-c | AA2-deficient form | 452.54 | 453.5 $(M+H)^+$ | 1.17 | 22.8 |
| pd2-014-d | Other | 749.94 | 750.7 $(M+H)^+$ | 1.45 | 4.3 |

Synthesis of pd2-014-resin under condition 3

The amino acid-supported resin aa4-001-resin (Fmoc-D-3-MeAbu-O-Trt(2-Cl)resin) (100 mg, 0.369 mmol/g) prepared in Example 1-4 was provided as a raw material, and elongation was performed with Fmoc-bAla(2-Me2)-OH (oxyma, 50°C, 10 hours), and then with Fmoc-Ile-OH (HOAt, 40°C, 2.5 hours) in accordance with synthesis method 1 to synthesize pd2-014-resin. The loading rate calculated by the Fmoc quantitation method using a dry resin (10.86 mg) was 0.227 mmol/g. (UVarea value at 294 nm: 2472.50 and UVarea value at 304 nm: 2237.05)
The recovery ratio is calculated from the following equation in accordance with the recovery ratio calculation method.

$$\text{Recovery ratio} = 0.227 \times (1 \div 0.369 - 339.38 \times 0.001 + 551.67 \times 0.001) = 66.3\%$$

Analysis of the isolating reaction liquid by LCMS showed that the purity of intended product pd2-014 was 73.9 area%, excessively elongated form pd2-014-b was observed at 22.4 area%, and pd3-014-d was observed at 3.7 area% as another by-product.

Analysis condition: SQDAA50long

[Table 62]

| | | Molecular weight | LCMS (ESI) m/z | Retention time (min) | LC area% |
|---|---|---|---|---|---|
| pd2-014 | Intended product | 551.67 | 552.5 $(M+H)^+$ | 1.25 | 73.9 |
| pd2-014-b | Excessively elongated form | 650.81 | 651.7 $(M+H)^+$ | 1.35 | 22.4 |
| pd2-014-d | Other | 749.94 | 750.8 $(M+H)^+$ | 1.44 | 3.7 |

The table below collectively shows the above results.

[Table 63]

| | Recovery ratio (%) | Intended product pd2-014 (area%) | Excessively elongated form pd2-014-b (area%) | AA2-deficient form pd2-014-c (area%) | Other by-product pd2-014-d (area%) |
|---|---|---|---|---|---|
| Condition 1 | 94.1 | 100.0 | - | - | - |
| Condition 2 | 69.8 | 68.1 | 4.8 | 22.8 | 4.3 |
| Condition 3 | 66.3 | 73.9 | 22.4 | - | 3.7 |

Under condition 1 where a dipeptide was supported, an intended product was obtained with a high recovery ratio and a high purity.

When a single amino acid was supported, generation of an excessively elongated form and generation of an AA2-deficient form due to poor elongation of AA2 that is a site difficult to elongate were observed in addition to a decrease in recovery ratio due to premature cleavage under normal condition 2. Even under condition 3 that is adaptable to a sequence difficult to elongate, a decrease in recovery ratio due to premature cleavage was observed, and there was an increase in generation of an excessively elongated form although an AA2-deficient form was not generated.

Synthesis of compound pd2-015-resin, (2S)-2-[[(2S)-2-[[(2S,3S)-2-(9H-fluoren-9-ylmethoxycarbonylamino)-3-methylpentanoyl]-methylamino]-4-methylpentanoyl]-methylamino]-3-methylbutanoic acid-2-chlorotrityl resin (Fmoc-Ile-MeLeu-MeVal-O-Trt(2-Cl)resin)

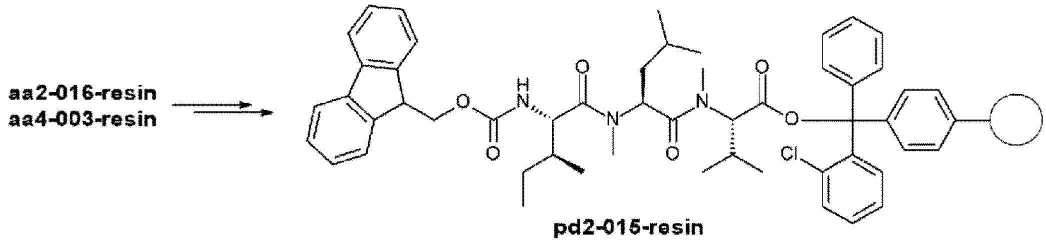


pd2-015-resin

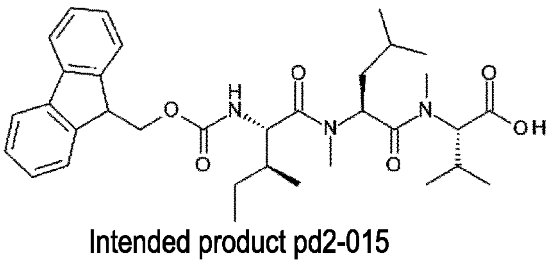

Intended product pd2-015

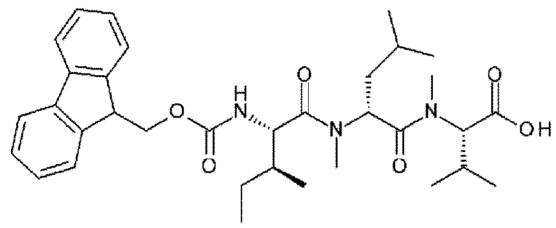

Epimeric form pd2-015-a

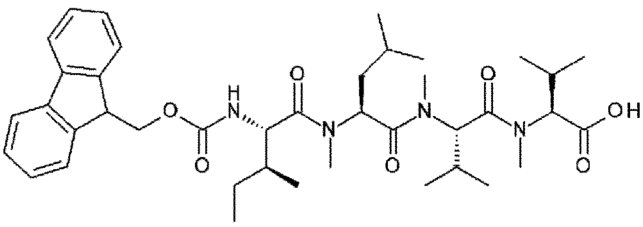

Excessively elongated form pd2-015-b

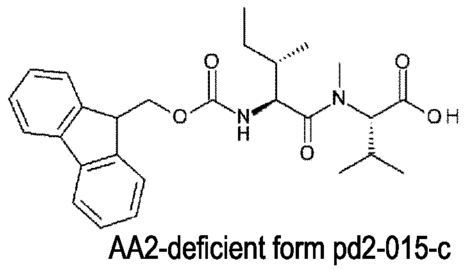

AA2-deficient form pd2-015-c

Synthesis of pd2-015-resin under condition 1

The dipeptide-supporting resin aa2-016-resin (Fmoc-MeLeu-MeVal-O-Trt(2-Cl)resin) (100 mg, 0.348 mmol/g) prepared in Example 1-4 was provided as a raw material, and elongation was performed with Fmoc-Ile-OH (HOAt, 40°C, 2.5 hours) in accordance with synthesis method 1 to synthesize pd2-015-resin. The loading rate calculated by the Fmoc quantitation method using a dry resin (10.89 mg) was 0.336 mmol/g. (UVarea value at 294 nm: 3689.12 and UVarea value at 304 nm: 3325.56)

The recovery ratio is calculated from the following equation in accordance with the recovery ratio calculation method.

$$\text{Recovery ratio} = 0.336 \times (1 \div 0.348 - 480.6 \times 0.001 + 593.75 \times 0.001) = 100.4\%$$

Analysis of the isolating reaction liquid by LCMS showed that the purity of intended product pd2-015 was 100 area%.

Analysis condition: SQDFA05long

[Table 64]

| | | Molecular weight | LCMS (ESI) m/z | Retention time (min) | LC area% |
|---|---|---|---|---|---|
| pd2-015 | Intended product | 593.75 | 594.6 $(M+H)^+$ | 3.10 | 100 |
| pd2-015-a | Epimeric form | 593.75 | not detected | | |

Synthesis of pd2-015-resin under condition 2

The amino acid-supported resin aa4-003-resin (Fmoc-MeVal-O-Trt(2-Cl)resin) (100 mg, 0.436 mmol/g) prepared in Example 1-4 was provided as a raw material, and elongation was performed with Fmoc-MeLeu-OH (HOAt, 40°C, 2.5 hours), and then with Fmoc-Ile-OH (HOAt, 40°C, 2.5 hours) in accordance with synthesis method 1 to synthesize pd2-015-resin. The loading rate calculated by the Fmoc quantitation method using a dry resin (10.26 mg) was 0.196 mmol/g. (UVarea value at 294 nm: 2024.53 and UVarea value at 304 nm: 1825.85)
The recovery ratio is calculated from the following equation in accordance with the recovery ratio calculation method.

$$\text{Recovery ratio} = 0.196 \times (1 \div 0.436 - 353.41 \times 0.001 + 593.75 \times 0.001) = 49.7\%$$

Analysis of the isolating reaction liquid by LCMS showed that the purity of intended product pd2-015 was 94.5 area%, and AA2-deficient form pd2-015-c was observed at 5.5 area%.

Analysis condition: SQDFA05long

[Table 65]

| | | Molecular weight | LCMS (ESI) m/z | Retention time (min) | LC area% |
|---|---|---|---|---|---|
| pd2-015 | Intended product | 593.75 | 594.6 $(M+H)^+$ | 3.10 | 94.5 |
| pd2-015-a | Epimeric form | 593.75 | not detected | | |
| pd2-015-b | Excessively elongated form | 706.91 | not detected | | |
| pd2-015-c | AA2-deficient form | 466.57 | 467.5 $(M+H)^+$ | 2.69 | 5.5 |

Synthesis of pd2-015-resin under condition 3

The amino acid-supported resin aa4-003-resin (Fmoc-MeVal-O-Trt(2-Cl)resin) (100 mg, 0.436 mmol/g) prepared in Example 1-4 was provided as a raw material, and elongation was performed with Fmoc-MeLeu-OH (oxyma, 50°C, 10 hours), and then with Fmoc-Ile-OH (HOAt, 40°C, 2.5 hours) in accordance with synthesis method 1 to synthesize pd2-015-resin. The loading rate calculated by the Fmoc quantitation method using a dry resin (11.7 mg) was 0.172 mmol/g. (UVarea value at 294 nm: 2031.91 and UVarea value at 304 nm: 1827.32)
The recovery ratio is calculated from the following equation in accordance with the recovery ratio calculation method.

$$\text{Recovery ratio} = 0.172 \times (1 \div 0.436 - 353.41 \times 0.001 + 593.75 \times 0.001) = 43.6\%$$

Analysis of the isolating reaction liquid by LCMS showed that the purity of intended product pd2-015 was 100 area%.

Analysis condition: SQDFA05long

[Table 66]

| | | Molecular weight | LCMS (ESI) m/z | Retention time (min) | LC area% |
|---|---|---|---|---|---|
| pd2-015 | Intended product | 593.75 | 594.6 $(M+H)^+$ | 3.10 | 100 |
| pd2-015-a | Epimeric form | 593.75 | not detected | | |
| pd2-015-b | Excessively elongated form | 706.91 | not detected | | |

The table below collectively shows the above results.

[Table 67]

| | Recovery ratio (%) | Intended product pd2-015 (area%) | Epimeric form pd2-015-a (area%) | Excessively elongated form pd2-015-b (area%) | AA2-deficient form pd2-015-c (area%) |
|---|---|---|---|---|---|
| Condition 1 | 100.4 | 100.0 | - | - | - |
| Condition 2 | 49.7 | 94.5 | - | - | 5.5 |
| Condition 3 | 43.6 | 100.0 | - | - | - |

Under condition 1 where a dipeptide was supported, an intended product was obtained with a high recovery ratio and a high purity.

When a single amino acid was supported, generation of an AA2-deficient form due to poor elongation of AA2 that is a site difficult to elongate was observed in addition to a decrease in recovery ratio due to premature cleavage under normal condition 2. Under condition 3 that is adaptable to a sequence difficult to elongate, an intended product was obtained with a high purity, but a decrease in recovery ratio due to premature cleavage was observed.

Synthesis of compound pd2-016-resin, (2S)-1-[(2S)-2-[[2S,3S)-2-(9H-fluoren-9-ylmethoxycarbonylamino)-3-methylpentanoyl]-methylamino]-3-methylbutanoyl]pyrrolidine-2-carboxylic acid-2-chlorotrityl resin (Fmoc-Ile-MeVal-Pro-O-Trt(2-Cl)resin)

aa2-017-resin
aa4-004-resin

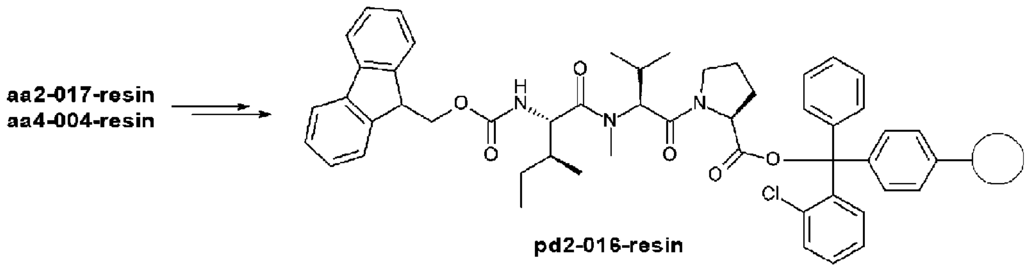

pd2-016-resin

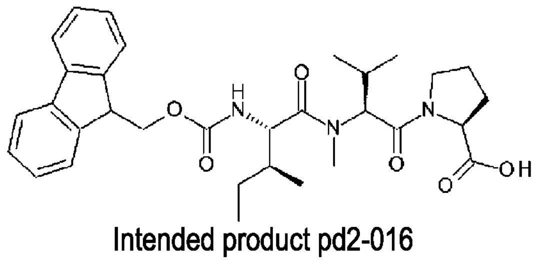

Intended product pd2-016

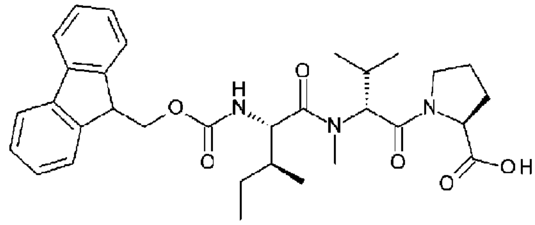

Epimeric form pd2-016-a

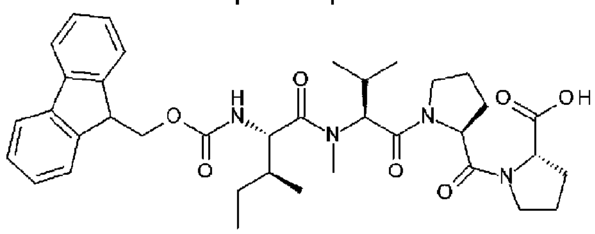

Excessively elongated form pd2-016-b

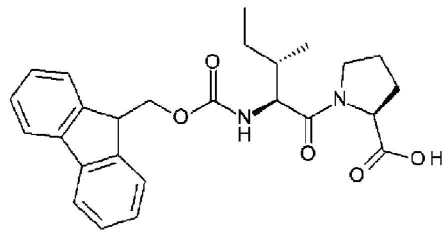

AA2-deficient form pd2-016-c

Synthesis of pd2-016-resin under condition 1

The dipeptide-supporting resin aa2-017-resin (Fmoc-MeVal-Pro-O-Trt(2-Cl)resin) (100 mg, 0.364 mmol/g) prepared in Example 1-4 was provided as a raw material, and elongation was performed with Fmoc-Ile-OH (HOAt, 40°C, 2.5 hours) in accordance with synthesis method 1 to synthesize pd2-016-resin. The loading rate calculated by the Fmoc quantitation method using a dry resin (10.32 mg) was 0.344 mmol/g. (UVarea value at 294 nm: 3574.16 and UVarea value at 304 nm: 3214.28)
The recovery ratio is calculated from the following equation in accordance with the recovery ratio calculation method.

$$\text{Recovery ratio} = 0.344 \times (1 \div 0.364 - 450.53 \times 0.001 + 563.68 \times 0.001) = 98.4\%$$

Analysis of the isolating reaction liquid by LCMS showed that the purity of intended product pd2-016 was 100 area%.

Analysis condition: SQDFA05long

[Table 68]

| | | Molecular weight | LCMS (ESI) m/z | Retention time (min) | LC area% |
|---|---|---|---|---|---|
| pd2-016 | Intended product | 563.68 | 564.5 $(M+H)^+$ | 2.62 | 100 |
| pd2-016-a | Epimeric form | 563.68 | not detected | | |

Synthesis of pd2-016-resin under condition 2

The amino acid-supported resin aa4-004-resin (Fmoc-Pro-O-Trt(2-Cl)resin) (100 mg, 0.432 mmol/g) prepared in Example 1-4 was provided as a raw material, and elongation was performed with Fmoc-MeVal-OH (HOAt, 40°C, 2.5 hours), and then with Fmoc-Ile-OH (HOAt, 40°C, 2.5 hours) in accordance with synthesis method 1 to synthesize pd2-016-resin. The loading rate calculated by the Fmoc quantitation method using a dry resin (10.58 mg) was 0.312 mmol/g. (UVarea value at 294 nm: 3327.96 and UVarea value at 304 nm: 2998.45)
The recovery ratio is calculated from the following equation in accordance with the recovery ratio calculation method.

$$\text{Recovery ratio} = 0.312 \times (1 \div 0.432 - 337.37 \times 0.001 + 563.68 \times 0.001) = 79.3\%$$

Analysis of the isolating reaction liquid by LCMS showed that the purity of intended product pd2-016 was 96.1 area%, and an excessively elongated form pd2-016-b was observed at 3.9 area%.

Analysis condition: SQDFA05long

[Table 69]

| | | Molecular weight | LCMS (ESI) m/z | Retention time (min) | LC area% |
|---|---|---|---|---|---|
| pd2-016 | Intended product | 563.68 | 564.6 $(M+H)^+$ | 2.62 | 96.1 |
| pd2-016-a | Epimeric form | 563.68 | not detected | | |
| pd2-016-b | Excessively elongated form | 660.80 | 661.7 $(M+H)^+$ | 2.54 | 3.9 |
| pd2-016-c | AA2-deficient form | 450.53 | not detected | | |

Synthesis of pd2-016-resin under condition 3

The amino acid-supported resin aa4-004-resin (Fmoc-Pro-O-Trt(2-Cl)resin) (100 mg, 0.432 mmol/g) prepared in Example 1-4 was provided as a raw material, and elongation was performed with Fmoc-MeVal-OH (oxyma, 50°C, 10 hours), and then with Fmoc-Ile-OH (HOAt, 40°C, 2.5 hours) in accordance with synthesis method 1 to synthesize pd2-016-resin. The loading rate calculated by the Fmoc quantitation method using a dry resin (11.71 mg) was 0.314 mmol/g. (UVarea value at 294 nm: 3704.39 and UVarea value at 304 nm: 3340.1)
The recovery ratio is calculated from the following equation in accordance with the recovery ratio calculation method.

$$\text{Recovery ratio} = 0.314 \times (1 \div 0.432 - 337.37 \times 0.001 + 563.68 \times 0.001) = 79.8\%$$

Analysis of the isolating reaction liquid by LCMS showed that the purity of intended product pd2-016 was 96.2 area%, and an excessively elongated form pd2-016-b was observed at 3.8 area%.

Analysis condition: SQDFA05long

[Table 70]

| | | Molecular weight | LCMS (ESI) m/z | Retention time (min) | LC area% |
|---|---|---|---|---|---|
| pd2-016 | Intended product | 563.68 | 564.5 $(M+H)^+$ | 2.62 | 96.2 |
| pd2-016-a | Epimeric form | 563.68 | not detected | | |
| pd2-016-b | Excessively elongated form | 660.80 | 661.8 $(M+H)^+$ | 2.54 | 3.8 |

The table below collectively shows the above results.

[Table 71]

| | Recovery ratio (%) | Intended product pd2-016 (area%) | Epimeric form pd2-016-a (area%) | Excessively elongated form pd2-016-b (area%) | AA2-deficient form pd2-016-c (area%) |
|---|---|---|---|---|---|
| Condition 1 | 98.4 | 100.0 | - | - | - |
| Condition 2 | 79.3 | 96.1 | - | 3.9 | - |

(continued)

| | Recovery ratio (%) | Intended product pd2-016 (area%) | Epimeric form pd2-016-a (area%) | Excessively elongated form pd2-016-b (area%) | AA2-deficient form pd2-016-c (area%) |
|---|---|---|---|---|---|
| Condition 3 | 79.8 | 96.2 | - | 3.8 | - |

Under condition 1 where a dipeptide was supported, an intended product was obtained with a high recovery ratio and a high purity.

Under conditions 2 and 3 where a single amino acid was supported, generation of an excessively elongated form was observed in addition to a decrease in recovery ratio due to premature cleavage.

Synthesis of compound pd2-017-resin, (2S)-1-[(2S)-2-[[(2S,3S)-2-(9H-fluoren-9-ylmethoxycarbonylamino)-3-methylpentanoyl]amino]-3-phenylpropanoyl]pyrrolidine-2-carboxylic acid-2-chlorotrityl resin (Fmoc-Ile-Phe-Pro-O-Trt(2-Cl)resin)

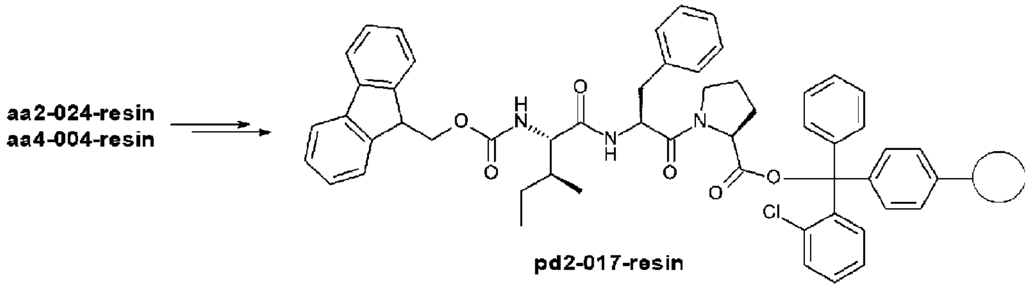

pd2-017-resin

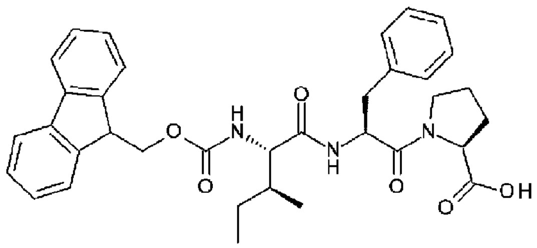
Intended product pd2-017

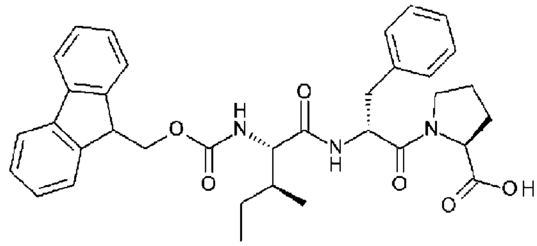
Epimeric form pd2-017-a

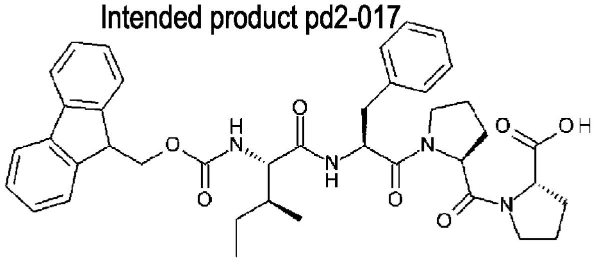
Excessively elongated form pd2-017-b

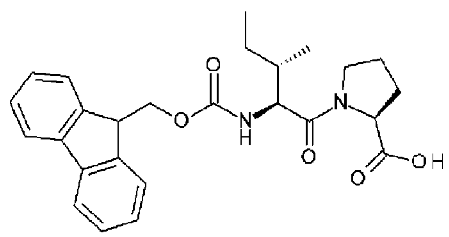
AA2-deficient form pd2-017-c

Synthesis of pd2-017-resin under condition 1

The dipeptide-supporting resin aa2-024-resin (Fmoc-Phe-Pro-O-Trt(2-Cl)resin) (100 mg, 0.383 mmol/g) prepared in Example 1-4 was provided as a raw material, and elongation was performed with Fmoc-Ile-OH (HOAt, 40°C, 2.5 hours) in accordance with synthesis method 1 to synthesize pd2-017-resin. The loading rate calculated by the Fmoc quantitation method using a dry resin (10.41 mg) was 0.356 mmol/g. (UVarea value at 294 nm: 3739.14 and UVarea

value at 304 nm: 3351.23)
The recovery ratio is calculated from the following equation in accordance with the recovery ratio calculation method.

$$\text{Recovery ratio} = 0.356 \times (1 \div 0.383 - 484.54 \times 0.001 + 597.7 \times 0.001) = 97.0\%$$

Analysis of the isolating reaction liquid by LCMS showed that the purity of intended product pd2-017 was 100 area%.

Analysis condition: SQDFA05long

[Table 72]

| | | Molecular weight | LCMS (ESI) m/z | Retention time (min) | LC area% |
|---|---|---|---|---|---|
| pd2-017 | Intended product | 597.70 | 598.5 $(M+H)^+$ | 2.68 | 100 |
| pd2-017-a | Epimeric form | 597.70 | not detected | | |

Synthesis of pd2-017-resin under condition 2

The amino acid-supported resin aa4-004-resin (Fmoc-Pro-O-Trt(2-Cl)resin) (100 mg, 0.432 mmol/g) prepared in Example 1-4 was provided as a raw material, and elongation was performed with Fmoc-Phe-OH (HOAt, 40°C, 2.5 hours), and then with Fmoc-Ile-OH (HOAt, 40°C, 2.5 hours) in accordance with synthesis method 1 to synthesize pd2-017-resin. The loading rate calculated by the Fmoc quantitation method using a dry resin (10.8 mg) was 0.339 mmol/g. (UVarea value at 294 nm: 3688.78 and UVarea value at 304 nm: 3317.01)
The recovery ratio is calculated from the following equation in accordance with the recovery ratio calculation method.

$$\text{Recovery ratio} = 0.339 \times (1 \div 0.432 - 337.37 \times 0.001 + 597.7 \times 0.001) = 87.3\%$$

Analysis of the isolating reaction liquid by LCMS showed that the purity of intended product pd2-017 was 97.0 area%, and an excessively elongated form pd2-017-b was observed at 3.0 area%.

Analysis condition: SQDFA05long

[Table 73]

| | | Molecular weight | LCMS (ESI) m/z | Retention time (min) | LC area% |
|---|---|---|---|---|---|
| pd2-017 | Intended product | 597.70 | 598.5 $(M+H)^+$ | 2.68 | 97.0 |
| pd2-017-a | Epimeric form | 597.70 | not detected | | |
| pd2-017-b | Excessively elongated form | 694.82 | 695.7 $(M+H)^+$ | 2.60 | 3.0 |
| pd2-017-c | AA2-deficient form | 450.53 | not detected | | |

Synthesis of pd2-017-resin under condition 3

The amino acid-supported resin aa4-004-resin (Fmoc-Pro-O-Trt(2-Cl)resin) (100 mg, 0.432 mmol/g) prepared in Example 1-4 was provided as a raw material, and elongation was performed with Fmoc-Phe-OH (oxyma, 50°C, 10 hours), and then with Fmoc-Ile-OH (HOAt, 40°C, 2.5 hours) in accordance with synthesis method 1 to synthesize pd2-017-resin. The loading rate calculated by the Fmoc quantitation method using a dry resin (10.85 mg) was 0.320 mmol/g. (UVarea value at 294 nm: 3510.64 and UVarea value at 304 nm: 3136.09)
The recovery ratio is calculated from the following equation in accordance with the recovery ratio calculation method.

$$\text{Recovery ratio} = 0.320 \times (1 \div 0.432 - 337.37 \times 0.001 + 597.7 \times 0.001) = 82.4\%$$

Analysis of the isolating reaction liquid by LCMS showed that the purity of intended product pd2-017 was 94.8 area%, and an excessively elongated form pd2-017-b was observed at 5.2 area%.

Analysis condition: SQDFA05long

[Table 74]

| | | Molecular weight | LCMS (ESI) m/z | Retention time (min) | LC area% |
|---|---|---|---|---|---|
| pd2-017 | Intended product | 597.70 | 598.6 $(M+H)^+$ | 2.68 | 94.8 |
| pd2-017-a | Epimeric form | 597.70 | not detected | | |
| pd2-017-b | Excessively elongated form | 694.82 | 695.6 $(M+H)^+$ | 2.60 | 5.2 |

The table below collectively shows the above results.

[Table 75]

| | Recovery ratio (%) | Intended product pd2-017 (area%) | Epimeric form pd2-017-a (area%) | Excessively elongated form pd2-017-b (area%) | AA2-deficient form pd2-017-c (area%) |
|---|---|---|---|---|---|
| Condition 1 | 97.0 | 100.0 | - | - | - |
| Condition 2 | 87.3 | 97.0 | - | 3.0 | - |
| Condition 3 | 82.4 | 94.8 | - | 5.2 | - |

Under condition 1 where a dipeptide was supported, an intended product was obtained with a high recovery ratio and a high purity.

Under conditions 2 and 3 where a single amino acid was supported, generation of an excessively elongated form was observed in addition to a decrease in recovery ratio due to premature cleavage.

Synthesis of compound aa2-018-resin, (2S)-1-[(2S)-2-[[(2S,3S)-2-(9H-fluoren-9-ylmethoxycarbonylamino)-3-methylpentanoyl]amino]-6-[(2-methylpropan-2-yl)oxycarbonylamino]hexanoyl]pyrrolidine-2-carboxylic acid-2-chlorotrityl resin (Fmoc-Ile-Lys(Boc)-Pro-O-Trt(2-Cl)resin)

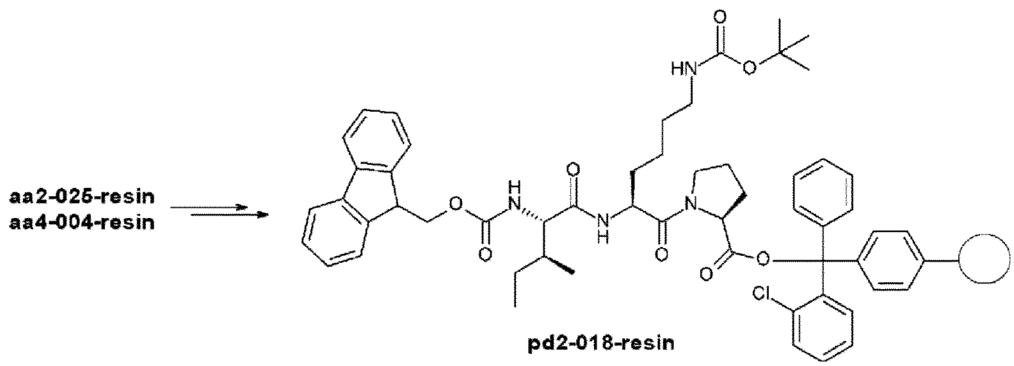


pd2-018-resin

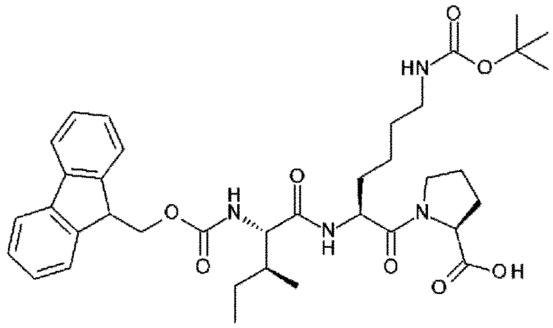

Intended product pd2-018

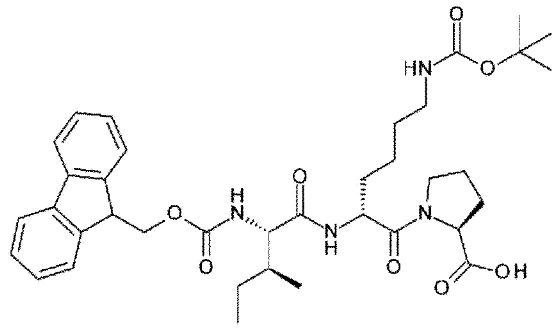

Epimeric form pd2-018-a

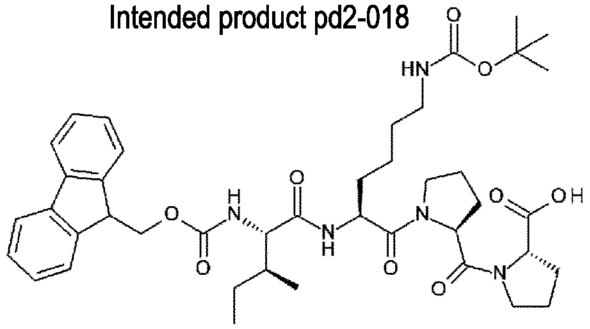

Excessively elongated form pd2-018-b

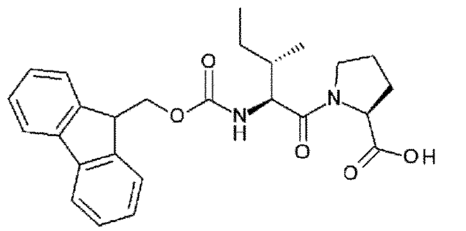

AA2-deficient form pd2-018-c

Synthesis of pd2-018-resin under condition 1

The dipeptide-supporting resin aa2-025-resin (Fmoc-Lys(Boc)-Pro-O-Trt(2-Cl)resin) (100 mg, 0.339 mmol/g) prepared in Example 1-4 was provided as a raw material, and elongation was performed with Fmoc-Ile-OH (HOAt, 40°C, 2.5 hours) in accordance with synthesis method 1 to synthesize pd2-018-resin. The loading rate calculated by the Fmoc quantitation method using a dry resin (10.97 mg) was 0.319 mmol/g. (UVarea value at 294 nm: 3517.51 and UVarea value at 304 nm: 3171.30)
The recovery ratio is calculated from the following equation in accordance with the recovery ratio calculation method.

$$\text{Recovery ratio} = 0.319 \times (1 \div 0.339 - 565.66 \times 0.001 + 678.81 \times 0.001) = 97.7\%$$

Analysis of the isolating reaction liquid by LCMS showed that the purity of intended product pd2-018 was 100 area%.

Analysis condition: SQDFA05long

[Table 76]

| | | Molecular weight | LCMS (ESI) m/z | Retention time (min) | LC area% |
|---|---|---|---|---|---|
| pd2-018 | Intended product | 678.81 | 679.7 $(M+H)^+$ | 2.74 | 100.0 |
| pd2-018-a | Epimeric form | 678.81 | not detected | | |

Synthesis of pd2-018-resin under condition 2

The amino acid-supported resin aa4-004-resin (Fmoc-Pro-O-Trt(2-Cl)resin) (100 mg, 0.432 mmol/g) prepared in Example 1-4 was provided as a raw material, and elongation was performed with Fmoc-Lys(Boc)-OH (HOAt, 40°C, 2.5 hours), and then with Fmoc-Ile-OH (HOAt, 40°C, 2.5 hours) in accordance with synthesis method 1 to synthesize pd2-018-resin. The loading rate calculated by the Fmoc quantitation method using a dry resin (10.72 mg) was 0.311 mmol/g. (UVarea value at 294 nm: 3353.43 and UVarea value at 304 nm: 3026.12)

The recovery ratio is calculated from the following equation in accordance with the recovery ratio calculation method.

$$\text{Recovery ratio} = 0.311 \times (1 \div 0.432 - 337.37 \times 0.001 + 678.81 \times 0.001) = 82.6\%$$

Analysis of the isolating reaction liquid by LCMS showed that the purity of intended product pd2-018 was 95.7 area%, and an excessively elongated form pd2-018-b was observed at 4.3 area%.

Analysis condition: SQDFA05long

[Table 77]

| | | Molecular weight | LCMS (ESI) m/z | Retention time (min) | LC area% |
|---|---|---|---|---|---|
| pd2-018 | Intended product | 678.81 | 679.7 $(M+H)^+$ | 2.74 | 95.7 |
| pd2-018-a | Epimeric form | 678.81 | not detected | | |
| pd2-018-b | Excessively elongated form | 775.93 | 776.8 $(M+H)^+$ | 2.67 | 4.3 |
| pd2-018-c | AA2-deficient form | 450.53 | not detected | | |

Synthesis of pd2-018-resin under condition 3

The amino acid-supported resin aa4-004-resin (Fmoc-Pro-O-Trt(2-Cl)resin) (100 mg, 0.432 mmol/g) prepared in Example 1-4 was provided as a raw material, and elongation was performed with Fmoc-Lys(Boc)-OH (oxyma, 50°C, 10 hours), and then with Fmoc-Ile-OH (HOAt, 40°C, 2.5 hours) in accordance with synthesis method 1 to synthesize pd2-018-resin. The loading rate calculated by the Fmoc quantitation method using a dry resin (9.90 mg) was 0.313 mmol/g. (UVarea value at 294 nm: 3111.54 and UVarea value at 304 nm: 2815.86)
The recovery ratio is calculated from the following equation in accordance with the recovery ratio calculation method.

$$\text{Recovery ratio} = 0.313 \times (1 \div 0.432 - 337.37 \times 0.001 + 678.81 \times 0.001) = 83.1\%$$

Analysis of the isolating reaction liquid by LCMS showed that the purity of intended product pd2-018 was 92.2 area%, and an excessively elongated form pd2-018-b was observed at 7.8 area%.

Analysis condition: SQDFA05long

[Table 78]

| | | Molecular weight | LCMS (ESI) m/z | Retention time (min) | LC area% |
|---|---|---|---|---|---|
| pd2-018 | Intended product | 678.81 | 679.7 $(M+H)^+$ | 2.74 | 92.2 |
| pd2-018-a | Epimeric form | 678.81 | not detected | | |
| pd2-018-b | Excessively elongated form | 775.93 | 776.7 $(M+H)^+$ | 2.68 | 7.8 |

The table below collectively shows the above results.

[Table 79]

| | Recovery (%) ratio (%) | Intended product pd2-018 (area%) | Epimeric form pd2-018-a (area%) | Excessively elongated form pd2-018-b (area%) | AA2-deficient form pd2-018-c (area%) |
|---|---|---|---|---|---|
| Condition 1 | 97.7 | 100.0 | - | - | - |
| Condition 2 | 82.6 | 95.7 | - | 4.3 | - |
| Condition 3 | 83.1 | 92.2 | - | 7.8 | - |

Under condition 1 where a dipeptide was supported, an intended product was obtained with a high recovery ratio and a high purity.

Under conditions 2 and 3 where a single amino acid was supported, generation of an excessively elongated form was observed in addition to a decrease in recovery ratio due to premature cleavage.

Synthesis of compound pd2-019-resin, 2-[[(2S)-2-[[(2S,3S)-2-(9H-fluoren-9-ylmethoxycarbonylamino)-3-methylpentanoyl]-methylamino]-4-methylpentanoyl]amino]-2-methylpropanoicacid-2-chlorotrityl resin (Fmoc-Ile-MeLeu-Aib-O-Trt(2-Cl)resin)

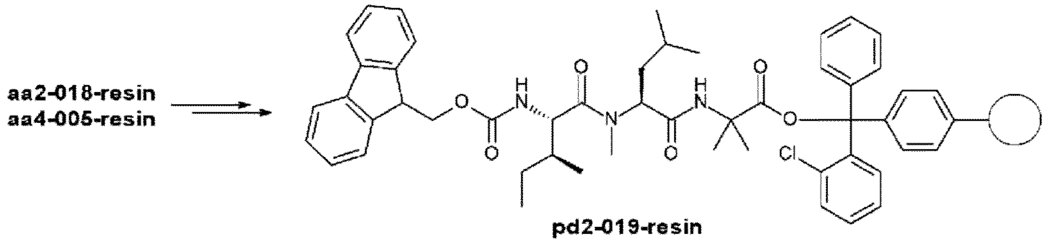


pd2-019-resin

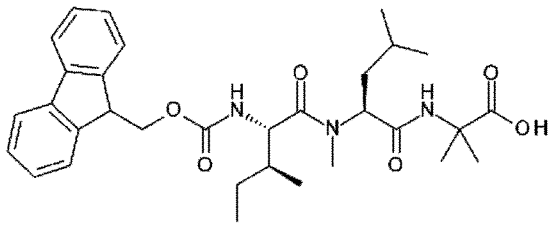

Intended product pd2-019

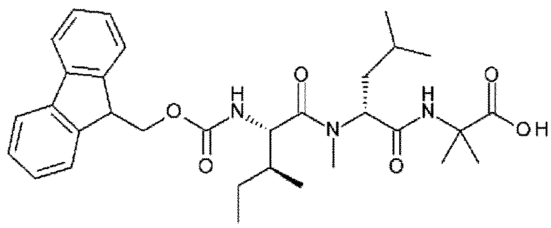

Epimeric form pd2-019-a

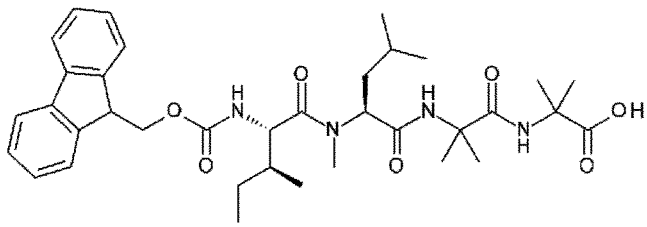

Excessively elongated form pd2-019-b

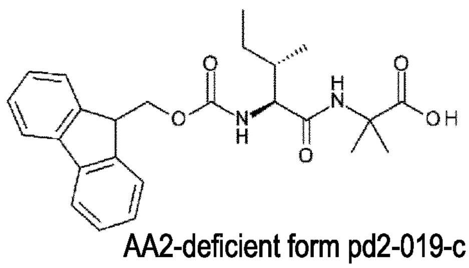

AA2-deficient form pd2-019-c

Synthesis of pd2-019-resin under condition 1

The dipeptide-supporting resin aa2-018-resin (Fmoc-MeLeu-Aib-O-Trt(2-Cl)resin) (100 mg, 0.300 mmol/g) prepared in Example 1-4 was provided as a raw material, and elongation was performed with Fmoc-Ile-OH (HOAt, 40°C, 2.5 hours) in accordance with synthesis method 1 to synthesize pd2-019-resin. The loading rate calculated by the Fmoc quantitation method using a dry resin (12.24 mg) was 0.292 mmol/g. (UVarea value at 294 nm: 3596.70 and UVarea value at 304 nm: 3242.23)
The recovery ratio is calculated from the following equation in accordance with the recovery ratio calculation method.

$$\text{Recovery ratio} = 0.292 \times (1 \div 0.300 - 452.54 \times 0.001 + 565.7 \times 0.001) = 100.6\%$$

Analysis of the isolating reaction liquid by LCMS showed that the purity of intended product pd2-019 was 100 area%.

Analysis condition: SQDFA05long

[Table 80]

| | | Molecular weight | LCMS (ESI) m/z | Retention time (min) | LC area% |
|---|---|---|---|---|---|
| pd2-019 | Intended product | 565.70 | 566.6 $(M+H)^+$ | 2.86 | 100 |
| pd2-019-a | Epimeric form | 565.70 | not detected | | |

Synthesis of pd2-019-resin under condition 2

The amino acid-supported resin aa4-005-resin (Fmoc-Aib-O-Trt(2-Cl)resin) (100 mg, 0.390 mmol/g) prepared in Example 1-4 was provided as a raw material, and elongation was performed with Fmoc-MeLeu-OH (HOAt, 40°C, 2.5 hours), and then with Fmoc-Ile-OH (HOAt, 40°C, 2.5 hours) in accordance with synthesis method 1 to synthesize pd2-019-resin. The loading rate calculated by the Fmoc quantitation method using a dry resin (9.87 mg) was 0.334 mmol/g. (UVarea value at 294 nm: 3325.50 and UVarea value at 304 nm: 2988.67)
The recovery ratio is calculated from the following equation in accordance with the recovery ratio calculation method.

$$\text{Recovery ratio} = 0.334 \times (1 \div 0.390 - 325.36 \times 0.001 + 565.7 \times 0.001) = 93.7\%$$

Analysis of the isolating reaction liquid by LCMS showed that the purity of intended product pd2-019 was 100 area%.

Analysis condition: SQDFA05long

[Table 81]

| | | Molecular weight | LCMS (ESI) m/z | Retention time (min) | LC area% |
|---|---|---|---|---|---|
| pd2-019 | Intended product | 565.70 | 566.6 $(M+H)^+$ | 2.86 | 100 |
| pd2-019-a | Epimeric form | 565.70 | not detected | | |
| pd2-019-b | Excessively elongated form | 650.81 | not detected | | |
| pd2-019-c | AA2-deficient form | 438.52 | not detected | | |

Synthesis of pd2-019-resin under condition 3

The amino acid-supported resin aa4-005-resin (Fmoc-Aib-O-Trt(2-Cl)resin) (100 mg, 0.390 mmol/g) prepared in Example 1-4 was provided as a raw material, and elongation was performed with Fmoc-MeLeu-OH (oxyma, 50°C, 10 hours), and then with Fmoc-Ile-OH (HOAt, 40°C, 2.5 hours) in accordance with synthesis method 1 to synthesize pd2-019-resin. The loading rate calculated by the Fmoc quantitation method using a dry resin (10.16 mg) was 0.294

mmol/g. (UVarea value at 294 nm: 3010.23 and UVarea value at 304 nm: 2713.79)
The recovery ratio is calculated from the following equation in accordance with the recovery ratio calculation method.

$$\text{Recovery ratio} = 0.294 \times (1 \div 0.390 - 325.36 \times 0.001 + 565.7 \times 0.001) = 82.5\%$$

Analysis of the isolating reaction liquid by LCMS showed that the purity of intended product pd2-019 was 100 area%.

Analysis condition: SQDFA05long

[Table 82]

| | | Molecular weight | LCMS (ESI) m/z | Retention time (min) | LC area% |
|---|---|---|---|---|---|
| pd2-019 | Intended product | 565.70 | 566.6 (M+H)+ | 2.85 | 100 |
| pd2-019-a | Epimeric form | 565.70 | not detected | | |
| pd2-019-b | Excessively elongated form | 650.81 | not detected | | |

The table below collectively shows the above results.

[Table 83]

| | Recovery ratio (%) | Intended product pd2-019 (area%) | Epimeric form pd2-019-a (area%) | Excessively elongated form pd2-019-b (area%) | AA2-deficient form pd2-019-c (area%) |
|---|---|---|---|---|---|
| Condition 1 | 100.6 | 100.0 | - | - | - |
| Condition 2 | 93.7 | 100.0 | - | - | - |
| Condition 3 | 82.5 | 100.0 | - | - | - |

Under condition 1 where a dipeptide was supported, an intended product was obtained with a high recovery ratio and a high purity.
Under conditions 2 and 3 where a single amino acid was supported, an intended product was obtained with a high purity, but there was a decrease in recovery ratio due to premature cleavage.

Synthesis of compound pd2-020-resin, 2-[[(2S)-2-[[(2S,3S)-2-(9H-fluoren-9-ylmethoxycarbonylamino)-3-methylpentanoyl]-methylamino]-3-methylbutanoyl]amino]aceticacid-2-chlorotrityl resin (Fmoc-Ile-MeVal-Gly-O-Trt(2-Cl)resin)

aa2-019-resin
aa4-006-resin

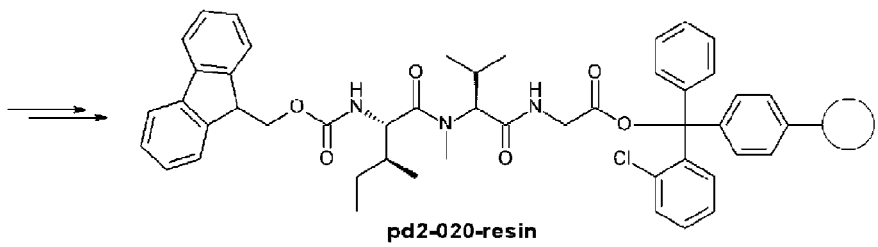

pd2-020-resin

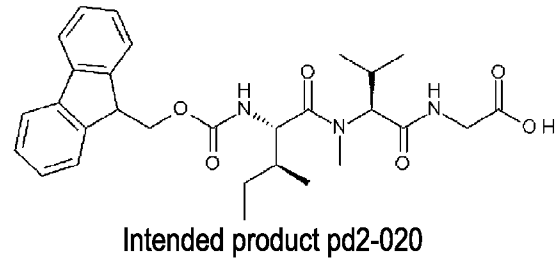

Intended product pd2-020

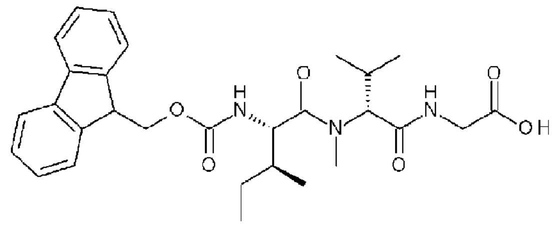

Epimeric form pd2-020-a

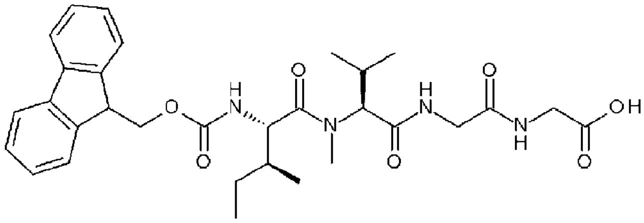

Excessively elongated form pd2-020-b

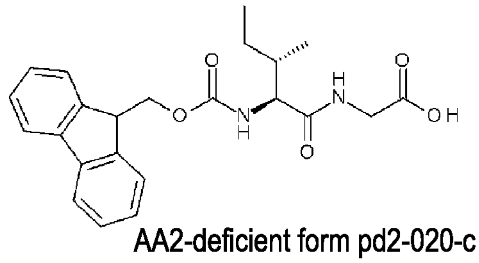

AA2-deficient form pd2-020-c

Synthesis of pd2-020-resin under condition 1

The dipeptide-supporting resin aa2-019-resin (Fmoc-MeVal-Gly-O-Trt(2-Cl)resin) (100 mg, 0.303 mmol/g) prepared in Example 1-4 was provided as a raw material, and elongation was performed with Fmoc-Ile-OH (HOAt, 40°C, 2.5 hours) in accordance with synthesis method 1 to synthesize pd2-020-resin. The loading rate calculated by the Fmoc quantitation method using a dry resin (10.5 mg) was 0.264 mmol/g. (UVarea value at 294 nm: 2792.18 and UVarea value at 304 nm: 2514.31)
The recovery ratio is calculated from the following equation in accordance with the recovery ratio calculation method.

$$\text{Recovery ratio} = 0.264 \times (1 \div 0.303 - 410.46 \times 0.001 + 523.62 \times 0.001) = 90.1\%$$

Analysis of the isolating reaction liquid by LCMS showed that the purity of intended product pd2-020 was 100 area%.

Analysis condition: SQDFA05long

[Table 84]

| | | Molecular weight | LCMS (ESI) m/z | Retention time (min) | LC area% |
|---|---|---|---|---|---|
| pd2-020 | Intended product | 523.62 | 524.5 $(M+H)^+$ | 2.45 | 100 |
| pd2-020-a | Epimeric form | 523.62 | not detected | | |

Synthesis of pd2-020-resin under condition 2

The amino acid-supported resin aa4-006-resin (Fmoc-Gly-O-Trt(2-Cl)resin) (100 mg, 0.250 mmol/g) prepared in Example 1-4 was provided as a raw material, and elongation was performed with Fmoc-MeVal-OH (HOAt, 40°C, 2.5 hours), and then with Fmoc-Ile-OH (HOAt, 40°C, 2.5 hours) in accordance with synthesis method 1 to synthesize pd2-020-resin. The loading rate calculated by the Fmoc quantitation method using a dry resin (10.18 mg) was 0.185 mmol/g. (UVarea value at 294 nm: 1889.34 and UVarea value at 304 nm: 1720.13)
The recovery ratio is calculated from the following equation in accordance with the recovery ratio calculation method.

$$\text{Recovery ratio} = 0.185 \times (1 \div 0.250 - 297.3 \times 0.001 + 523.62 \times 0.001) = 78.2\%$$

Analysis of the isolating reaction liquid by LCMS showed that the purity of intended product pd2-020 was 100

area%.

Analysis condition: SQDFA05long

[Table 85]

| | | Molecular weight | LCMS (ESI) m/z | Retention time (min) | LC area% |
|---|---|---|---|---|---|
| pd2-020 | Intended product | 523.62 | 524.5 $(M+H)^+$ | 2.45 | 100 |
| pd2-020-a | Epimeric form | 523.62 | not detected | | |
| pd2-020-b | Excessively elongated form | 580.67 | not detected | | |
| pd2-020-c | AA2-deficient form | 410.46 | not detected | | |

Synthesis of pd2-020-resin under condition 3

The amino acid-supported resin aa4-006-resin (Fmoc-Gly-O-Trt(2-Cl)resin) (100 mg, 0.250 mmol/g) prepared in Example 1-4 was provided as a raw material, and elongation was performed with Fmoc-MeVal-OH (oxyma, 50°C, 10 hours), and then with Fmoc-Ile-OH (HOAt, 40°C, 2.5 hours) in accordance with synthesis method 1 to synthesize pd2-011-resin. The loading rate calculated by the Fmoc quantitation method using a dry resin (10.93 mg) was 0.199 mmol/g. (UVarea value at 294 nm: 2175.24 and UVarea value at 304 nm: 1978.49)
The recovery ratio is calculated from the following equation in accordance with the recovery ratio calculation method.

$$\text{Recovery ratio} = 0.199 \times (1 \div 0.250 - 297.3 \times 0.001 + 523.62 \times 0.001) = 84.1\%$$

Analysis of the isolating reaction liquid by LCMS showed that the purity of intended product pd2-020 was 100 area%.

Analysis condition: SQDFA05long

[Table 86]

| | | Molecular weight | LCMS (ESI) m/z | Retention time (min) | LC area% |
|---|---|---|---|---|---|
| pd2-020 | Intended product | 523.62 | 524.5 $(M+H)^+$ | 2.45 | 100 |
| pd2-020-a | Epimeric form | 523.62 | not detected | | |
| pd2-020-b | Excessively elongated form | 580.67 | not detected | | |

The table below collectively shows the above results.

[Table 87]

| | Recovery ratio (%) | Intended product pd2-020 (area%) | Epimeric form pd2-020-a (area%) | Excessively elongated form pd2-020-b (area%) | AA2-deficient form pd2-020-c (area%) |
|---|---|---|---|---|---|
| Condition 1 | 90.1 | 100.0 | - | - | - |
| Condition 2 | 78.2 | 100.0 | - | - | - |
| Condition 3 | 84.1 | 100.0 | - | - | - |

Under condition 1 where a dipeptide was supported, an intended product was obtained with a high recovery ratio and a high purity.

Under conditions 2 and 3 where a single amino acid was supported, an intended product was obtained with a high purity, but there was a decrease in recovery ratio due to premature cleavage.

The results of Example 2 above show that a peptide can be prepared with a high recovery ratio and the purity thereof can be significantly improved by employing the method of the present invention, i.e. a method in which a dipeptide is prepared in advance, supported, and elongated with amino acids sequentially, rather than a common peptide synthesis method, i.e. a method in which a single amino acid is supported on a resin, and elongated with amino acids sequentially. It has been confirmed that an epimeric form, an excessively elongated form and an AA2-deficient form are factors of a decrease in purity in a common method, and by the method of the present invention, a decrease in purity by an excessively elongated form and an AA2-deficient form can be completely avoided. As described above, generation of an epimeric form can also be avoided or significantly reduced by performing precise purification before support on the resin, which is an advantageous point over a common peptide synthesis method.

## Example 3. Synthesis of various peptides under inventive conditions

In this Example, a resin supporting a peptide consisting of two or more amino acids was provided as a starting raw material, and a chain peptide with a chain length of 5 to 15 residues and a cyclic peptide were synthesized.

The chain peptide was synthesized in accordance with synthesis method 1 described in Example 2.

The cyclic peptide was synthesized in accordance with synthesis method 2 described below.

### Synthesis method 2

The amino acid elongation reaction using a peptide synthesizing machine was carried out in the same manner as in synthesis method 1. After completion of the peptide elongation, a DMF solution of DBU (2%v/v, 0.7 mL) was added into the solid phase reaction vessel containing the resin, the mixture was reacted at room temperature for 15 minutes to carry out a Fmoc group removal reaction, and the solution was then discharged from the frit. The obtained resin was washed four times with DMF (0.7 mL), then washed four times with DCM (0.7 mL).

To the obtained resin was added TFE/DCM (1/1, 2.0 mL) containing DIPEA at 0.75% (v/v), and mixture was reacted at room temperature for 2 hours to isolate the peptide chain from the resin. After the reaction, the solution in the tube was recovered from the frit. The operation of adding TFE/DCM (1/1, 1.0 mL) to the remaining resin and recovering the solution from the frit was carried out twice. All the obtained isolating solutions were mixed, DMF (4.0 mL) was mixed thereto, and the solvent was then removed by distillation under reduced pressure using High-Throughput Centrifugal Evaporator (HT-12) manufactured by Genevac Company.

The residue obtained by the above-described method was dissolved in a mixed liquid of DMF (4.0 mL) and DCM (4.0 mL), a solution of HATU in DMF (0.5 mol/L, 1.5 equivalents) and DIPEA (1.8 equivalents) were added, the mixture was stirred at room temperature for 2 hours to carry out a condensation cyclization reaction of an amino group at the N-terminal and a carboxylic acid serving as a site of binding to the resin, and the solvent was then removed by distillation under reduced pressure using High-Throughput Centrifugal Evaporator (HT-12) manufactured by Genevac Company. The number of equivalents was calculated with respect to a value obtained by multiplying the loading rate of the peptide on the resin used as a raw material (mmol/g) by the amount of the resin used.

DMSO was added to the residue obtained by the above-described method, insoluble substances were removed by filtration, and purification was then performed by preparative-HPLC to obtain an intended cyclic peptide. Waters Auto Purification System was used as a purification apparatus, YMC-Actus Triart C18 (internal diameter: 20 mm and length: 100 mm) was used as a column, and an aqueous methanol-ammonium acetate solution (50 mmol/L) was used as a mobile phase.

## Example 3-1. Synthesis of chain peptide using dipeptide-supporting resin

In this Example, the peptide-supporting resin prepared in Example 1-4 was provided as a raw material, and chain peptides shown in Table 88 below were synthesized by a solid phase reaction using a peptide synthesizing machine. The yields and purities of the peptides are described.

[Table 88]

| Compound No. | Abbreviation |
|---|---|
| pd3-001 | Fmoc-MeAla-MeLeu-Ala-MeVal-MeAsp-pip (SEQ ID NO: 1) |

(continued)

| Compound No. | Abbreviation |
|---|---|
| pd3-002 | Fmoc-MeAla-MeLeu-Gly-MeVal-Asp-NMe2 (SEQ ID NO: 2) |
| pd3-003 | Fmoc-MeAla-MeLeu-Gly-bAla(2-Me2)-D-3-MeAbu-OH (SEQ ID NO: 3) |
| pd3-004 | Fmoc-MeAla-Leu-MeAla-Phe-Pro-OH (SEQ ID NO: 4) |
| pd3-005 | Fmoc-MeAla-Leu-MeGly-MeAla-Ala-MeGly(cPent)-MeAsp-NMe2 (SEQ ID NO: 5) |
| pd3-006 | Fmoc-MeAla-Leu-MeLeu-MeAla-Gly-Aib-MeAsp-NMe2 (SEQ ID NO: 6) |
| pd3-007 | Fmoc-MeAla-Leu-MeGly-MeAla-Ala-MeVal-Gly-OH (SEQ ID NO: 7) |
| pd3-008 | Fmoc-MeAla-Leu-MeLeu-Ala-MeAla-Gly-Tyr(tBu)-OH (SEQ ID NO: 8) |
| pd3-009 | Fmoc-MeAla-Leu-MeGly-MeAla-Leu-MePhe-Ala-MeChg-D-3-MeAbu-OH (SEQ ID NO: 9) |
| pd3-010 | Fmoc-MeAla-Leu-MeLeu-Ala-MePhe-Ala-MeLeu-Gly-MeAsp-NMe2 (SEQ ID NO: 10) |
| pd3-011 | Fmoc-MeAla-Leu-MeGly-MeAla-Leu-MePhe-Ala-MeVal-Pro-OH (SEQ ID NO: 11) |
| pd3-012 | Fmoc-MeAla-Leu-MeLeu-Ala-MePhe-Gly-MeLeu-Ala-Ala-OH (SEQ ID NO: 12) |
| pd3-013 | Fmoc-MeAla-Leu-MeGly-Ala-Leu-MePhe-Ala-Pro-Ala-MeLeu-MeAsp-pip (SEQ ID NO: 13) |
| pd3-014 | Fmoc-MeAla-Leu-MeLeu-Ala-Gly-MePhe-MeAla-Leu-Gly-D-MeVal-MeAsp-NMe2 (SEQ ID NO: 14) |
| pd3-015 | Fmoc-MeAla-Leu-MeGly-Ala-Leu-MePhe-Ala-Pro-MeAla-Lys(Boc)-Pro-OH (SEQ ID NO: 15) |
| pd3-016 | Fmoc-MeAla-Leu-MeLeu-Ala-Gly-MePhe-MeAla-Leu-MeAla-Phe-Gly-OH (SEQ ID NO: 16) |
| pd3-017 | Fmoc-MeAla-Leu-MeGly-MeAla-Leu-MePhe-MeAla-Gly-Leu-MeLeu-Ala-MeChg-MeAsp-pip (SEQ ID NO: 17) |
| pd3-018 | Fmoc-MeAla-Leu-MeLeu-Ala-MePhe-Gly-MeAla-Leu-Ala-MeLeu-Gly-bAla(2-Me2)-MeAsp-NMe2 (SEQ ID NO: 18) |
| pd3-019 | Fmoc-MeAla-Leu-MeGly-MeAla-Leu-MePhe-MeAla-Gly-Leu-MeLeu-Ala-MeLeu-Aib-OH (SEQ ID NO: 19) |
| pd3-020 | Fmoc-MeAla-Leu-MeLeu-Ala-MePhe-Gly-MeAla-Leu-Ala-MeLeu-MeAla-Asn(Trt)-Gly-OH (SEQ ID NO: 20) |
| pd3-021 | Fmoc-MeAla-Leu-MeGly-Ala-Leu-MePhe-MeAla-Gly-MePhe-Pro-Ala-MeLeu-Gly-MeVal-D-MeAsp-NMe2 (SEQ ID NO: 21) |
| pd3-022 | Fmoc-MeAla-Leu-MeLeu-Ala-MePhe-Gly-MeAla-Leu-MePhe-MeAla-Ala-MeLeu-Ala-MeVal-MeGly-OH (SEQ ID NO: 22) |
| pd3-023 | Fmoc-MeAla-Leu-MeGly-Ala-Leu-MePhe-MeAla-Gly-MePhe-MeAla-Ala-MeLeu-MeAla-Gly-Val-OH (SEQ ID NO: 23) |
| pd3-024 | Fmoc-MeAla-Leu-MeLeu-Ala-MePhe-Gly-MeAla-Leu-MePhe-Pro-Ala-MeLeu-MeAla-Ser(tBu)-Gly-OH (SEQ ID NO: 24) |

Synthesis of compound pd3-001, (3S)-3-[[(2S)-2-[[(2S)-2-[[(2S)-2-[[(2S)-2-[9H-fluoren-9-ylmethoxycarbonyl(methyl)amino)propanoyl]-methylamino]-4-methylpentanoyl]amino]propanoyl]-methylamino]-3-methylbutanoyl]-methylamino]-4-oxo-4-piperidin-1-ylbutanoic acid (Fmoc-MeAla-MeLeu-Ala-MeVal-MeAsp-pip) (SEQ ID NO: 1)

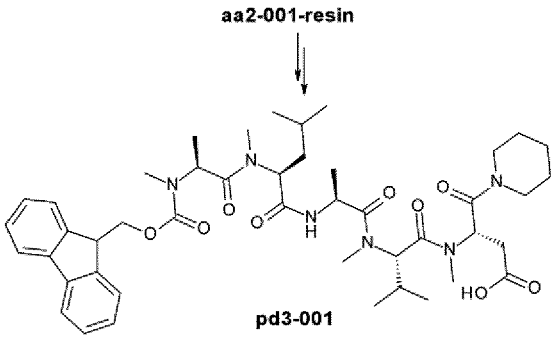

pd3-001

The dipeptide-supporting resin aa2-001-resin (Fmoc-MeVal-MeAsp(O-Trt(2-Cl)resin)-pip) (100.07 mg, 0.363 mmol/g, 0.0363 mmol) prepared in Example 1-4 was provided as a raw material, and reactions of elongation with corresponding amino acids were sequentially carried out in accordance with synthesis method 1 to synthesize pd3-001-resin (106.39 mg). The loading rate calculated by the Fmoc quantitation method using a dry resin (11.61 mg) was 0.290 mmol/g. (UVarea value at 294 nm: 3317.18 and UVarea value at 304 nm: 2984.87). Therefore, the amount of the obtained peptide was calculated to be 106.39 × 0.001 × 0.290 = 0.0309 mmol (yield: 84.9%).

Analysis of the isolating reaction liquid by LCMS showed that the purity of intended product pd3-001 was 96.9 area%.

LCMS (ESI) m/z = 833.9 $(M+H)^+$
Retention time: 0.98 min (analysis condition SQDFA05)

Synthesis of compound pd3-002, (3S)-4-(dimethylamino)-3-[[2-[[(2S)-2-[[(2S)-2-[[(2S)-2-[9H-fluoren-9-ylmethoxycarbonyl(methyl)amino)propanoyl]-methylamino]-4-methylpentanoyl]amino]acetyl]-methylamino]-3-methylbutanoyl]amino]-4-oxobutanoic acid (Fmoc-MeAla-MeLeu-Gly-MeVal-Asp-NMe2) (SEQ ID NO: 2)

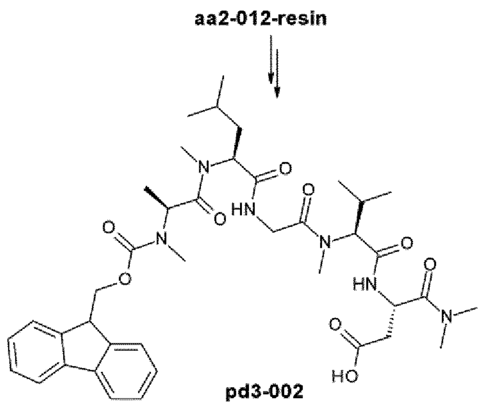

pd3-002

The dipeptide-supporting resin aa2-012-resin (Fmoc-MeVal-Asp(O-Trt(2-Cl)resin)-NMe2) (99.97 mg, 0.373 mmol/g, 0.0373 mmol) prepared in Example 1-4 was provided as a raw material, and reactions of elongation with corresponding amino acids were sequentially carried out in accordance with synthesis method 1 to synthesize pd3-002-resin (103.97 mg). The loading rate calculated by the Fmoc quantitation method using a dry resin (11.18 mg) was 0.282 mmol/g. (UVarea value at 294 nm: 3102.95 and UVarea value at 304 nm: 2794.81). Therefore, the amount of the obtained peptide was calculated to be 103.97 × 0.001 × 0.282 = 0.0293 mmol (yield: 78.6%).

Analysis of the isolating reaction liquid by LCMS showed that the purity of intended product pd3-002 was 94.7 area%.

LCMS (ESI) m/z = 765.7 $(M+H)^+$
Retention time: 0.85 min (analysis condition SQDFA05)

Synthesis of compound pd3-003, (3R)-3-[[3-[[2-[[(2S)-2-[[(2S)-2-[9H-fluoren-9-ylmethoxycarbonyl(methyl)amino)propanoyl]-methylamino]-4-methylpentanoyl]amino]acetyl]amino]-2,2-dimethylpropanoyll-methylaminolbutanoic acid (Fmoc-MeAla-MeLeu-Gly-bAla(2-Me2)-D-3-MeAbu-OH) (SEQ ID NO: 3)

aa2-015-resin

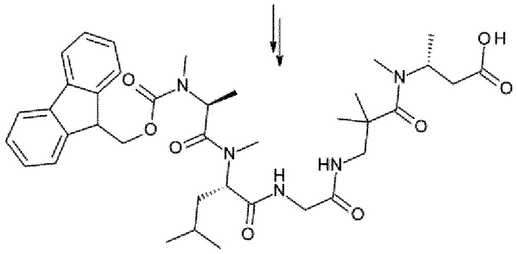

pd3-003

The dipeptide-supporting resin aa2-015-resin (Fmoc-bAla(2-Me2)-D-3-MeAbu-O-Trt(2-Cl)resin) (99.55 mg, 0.443 mmol/g, 0.0441 mmol) prepared in Example 1-4 was provided as a raw material, and reactions of elongation with corresponding amino acids were sequentially carried out in accordance with synthesis method 1 to synthesize pd3-003-resin (108.03 mg). The loading rate calculated by the Fmoc quantitation method using a dry resin (10.1 mg) was 0.284 mmol/g. (UVarea value at 294 nm: 2819.65 and UVarea value at 304 nm: 2543.21). Therefore, the amount of the obtained peptide was calculated to be 108.03 × 0.001 × 0.284 = 0.0307 mmol (yield: 69.6%).

Analysis of the isolating reaction liquid by LCMS showed that the purity of intended product pd3-003 was 98.3 area%.

LCMS (ESI) m/z = 708.7 $(M+H)^+$

Retention time: 0.85 min (analysis condition SQDFA05)

Synthesis of compound pd3-004, (2S)-1-[(2S)-2-[[(2S)-2-[[(2S)-2-[[(2S)-2-[9H-fluoren-9-ylmethoxycarbonyl(methyl)amino]propanoyl]amino]-4-methylpentanoyl]-methylamino]propanoyl]amino]-3-phenylpropanovl]pvrrolidine-2-carboxylic acid (Fmoc-MeAla-Leu-MeAla-Phe-Pro-OH) (SEQ ID NO: 4)

aa2-024-resin

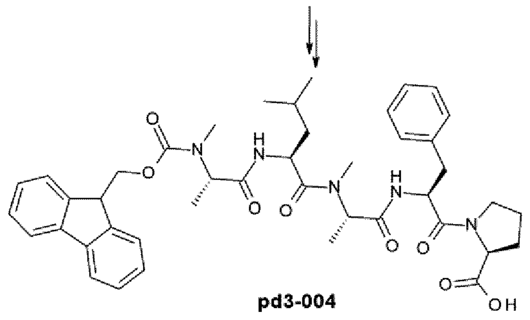

pd3-004

The dipeptide-supporting resin aa2-024-resin (Fmoc-Phe-Pro-O-Trt(2-Cl)resin) (100.43 mg, 0.383 mmol/g, 0.0385 mmol) prepared in Example 1-4 was provided as a raw material, and reactions of elongation with corresponding amino acids were sequentially carried out in accordance with synthesis method 1 to synthesize pd3-004-resin (107.41 mg). The loading rate calculated by the Fmoc quantitation method using a dry resin (10.43 mg) was 0.279 mmol/g. (UVarea value at 294 nm: 2859.19 and UVarea value at 304 nm: 2585.67). Therefore, the amount of the obtained peptide was calculated to be 107.41 × 0.001 × 0.279 = 0.0300 mmol (yield: 77.9%).

Analysis of the isolating reaction liquid by LCMS showed that the purity of intended product pd3-004 was 97.7 area%.

LCMS (ESI) m/z = 768.7 $(M+H)^+$

Retention time: 0.91 min (analysis condition SQDFA05)

Synthesis of compound pd3-005, (3S)-3-[[(2S)-2-cyclopentyl-2-[[(2S)-2-[[(2S)-2-[[2-[[(2S)-2-[[(2S)-2-[9H-fluoren-9-ylmethoxycarbonyl(methyl)aminol]propanoyl]amino]-4-methylpentanoyl]-methylamino]acelyl]-methylamino]propanoyl]amino]propanoyl]-methylamino]acetyl]-methylamino]-4-(dimethylamino)-4-oxobutanoic acid (Fmoc-MeAla-Leu-MeGly-MeAla-Ala-MeGly(cPent)-MeAsp-NMe2) (SEQ ID NO: 5)

aa2-006-resin

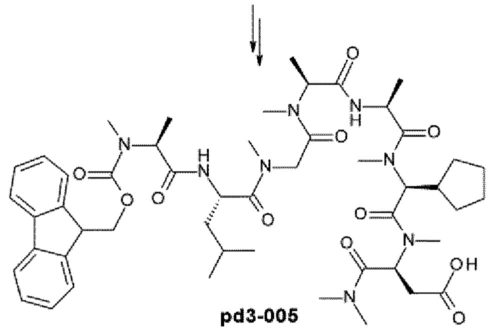

pd3-005

The dipeptide-supporting resin aa2-006-resin (Fmoc-MeGly(cPent)-MeAsp(O-Trt(2-Cl)resin)-NMe2) (100.13 mg, 0.345 mmol/g, 0.0345 mmol) prepared in Example 1-4 was provided as a raw material, and reactions of elongation with corresponding amino acids were sequentially carried out in accordance with synthesis method 1 to synthesize pd3-005-resin (109.69 mg). The loading rate calculated by the Fmoc quantitation method using a dry resin (12.17 mg) was 0.249 mmol/g. (UVarea value at 294 nm: 2972.89 and UVarea value at 304 nm: 2684.40). Therefore, the amount of the obtained peptide was calculated to be 109.69 $\times$ 0.001 $\times$ 0.249 = 0.0273 mmol (yield: 79. 1%).

Analysis of the isolating reaction liquid by LCMS showed that the purity of intended product pd3-005 was 98.5 area%.

LCMS (ESI) m/z = 962.0 $(M+H)^+$
Retention time: 0.84 min (analysis condition SQDFA05)

Synthesis of compound pd3-006, (3S)-4-(dimethylamino)-3-[[2-[[2-[[(2S)-2-[[(2S)-2-[[(2S)-2-[[(2S)-2-[9H-fluoren-9-ylmethoxycarbonyl(methyl)amino]propanoyl]amino]-4-methylpentanoyl]-methylamino]-4-methylpentanoyl]-methylamino]propanovl]amino]acetyl]amino]-2-methylpropanoyl]-methylamino]-4-oxobutanoic acid (Fmoc-MeAla-Leu-MeLeu-MeAla-Gly-Aib-MeAsp-NMe2) (SEQ ID NO: 6)

aa2-014-resin

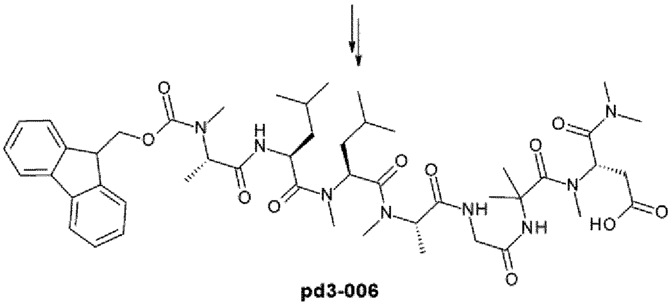

pd3-006

The dipeptide-supporting resin aa2-014-resin (Fmoc-Aib-MeAsp(O-Trt(2-Cl)resin)-NMe2) (101.05 mg, 0.413 mmol/g, 0.0417 mmol) prepared in Example 1-4 was provided as a raw material, and reactions of elongation with corresponding amino acids were sequentially carried out in accordance with synthesis method 1 to synthesize pd3-006-resin (117.09 mg). The loading rate calculated by the Fmoc quantitation method using a dry resin (9.53 mg) was 0.282 mmol/g. (UVarea value at 294 nm: 2641.40 and UVarea value at 304 nm: 2381.48). Therefore, the amount of the obtained

peptide was calculated to be 117.09 × 0.001 × 0.282 = 0.0330 mmol (yield: 79.1%).

Analysis of the isolating reaction liquid by LCMS showed that the purity of intended product pd3-006 was 97.2 area%.

LCMS (ESI) m/z = 949.9 $(M+H)^+$
Retention time: 0.85 min (analysis condition SQDFA05)

Synthesis of compound pd3-007, 2-[[(2S)-2-[[(2S)-2-[[(2S)-2-[[2-[[(2S)-2-[[(2S)-2-[9H-fluoren-9-ylmethoxycarbonyl(methyl)amino]propanoyl]amino]-4-methylpentanoyl]-methylamino]acetyl]-methylamino]propanoyl]amino]propanoyl]-methylamino]-3-methylbutanoyl]amino]acetic acid (Fmoc-MeAla-Leu-MeGly-MeAla-Ala-MeVal-Gly-OH) (SEQ ID NO: 7)

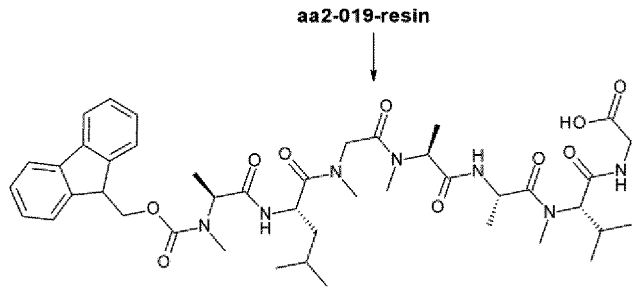

pd3-007

The dipeptide-supporting resin aa2-019-resin (Fmoc-MeVal-Gly-O-Trt(2-Cl)resin) (99.65 mg, 0.303 mmol/g, 0.0302 mmol) prepared in Example 1-4 was provided as a raw material, and reactions of elongation with corresponding amino acids were sequentially carried out in accordance with synthesis method 1 to synthesize pd3-007-resin (100.83 mg). The loading rate calculated by the Fmoc quantitation method using a dry resin (10.16 mg) was 0.150 mmol/g. (UVarea value at 294 nm: 1498.17 and UVarea value at 304 nm: 1349.88). Therefore, the amount of the obtained peptide was calculated to be 100.83 × 0.001 × 0.150 = 0.0151 mmol (yield: 50.1%).

Analysis of the isolating reaction liquid by LCMS showed that the purity of intended product pd3-007 was 100 area%.

LCMS (ESI) m/z = 836.8 $(M+H)^+$
Retention time: 0.81 min (analysis condition SQDFA05)

Synthesis of compound pd3-008, (2S)-2-[[2-[[(2S)-2-[[(2S)-2-[[(2S)-2-[[(2S)-2-[[(2S)-2-[9H-fluoren-9-ylmethoxycarbonyl(methyl)amino]propanoyl]amino]-4-methylpentanoyl]-methylamino]-4-methylpentanoyl]amino]propanoyl]-methylamino]propanoyl]amino]-3-[4-[(2-methylpropan-2-yl)oxy]phenyl]propanoic acid (Fmoc-MeAla-Leu-MeLeu-Ala-MeAla-Gly-Tyr(tBu)-OH) (SEQ ID NO: 8)

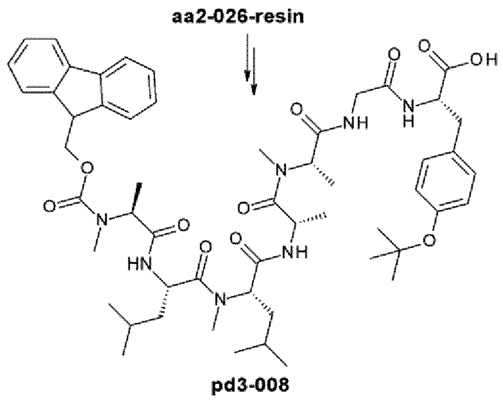

pd3-008

The dipeptide-supporting resin aa2-026-resin (Fmoc-Gly-Tyr(tBu)-O-Trt(2-Cl)resin) (99.51 mg, 0.244 mmol/g, 0.0243 mmol) prepared in Example 1-4 was provided as a raw material, and reactions of elongation with corresponding amino acids were sequentially carried out in accordance with synthesis method 1 to synthesize pd3-008-resin (103.75 mg). The loading rate calculated by the Fmoc quantitation method using a dry resin (10.03 mg) was 0.124 mmol/g. (UVarea value at 294 nm: 1220.62 and UVarea value at 304 nm: 1102.84). Therefore, the amount of the obtained peptide was calculated to be 103.75 × 0.001 × 0.124 = 0.0129 mmol (yield: 53.0%).

Analysis of the isolating reaction liquid by LCMS showed that the purity of intended product pd3-008 was 98.1 area%.

LCMS (ESI) m/z = 999.0 $(M+H)^+$
Retention time: 1.01 min (analysis condition SQDFA05)

Synthesis of compound pd3-009, (3R)-3-[[(2S)-2-cyclohexyl-2-[[(2S)-2-[[(2S)-2-[[(2S)-2-[[(2S)-2-[[2-[[(2S)-2-[[(2S)-2-[9H-fluoren-9-ylmethoxycarbonyl(methyl)amino]propanoyl]amino]-4-methylpentanoyl]-methylamino]acetyl]-methylamino]propanoyl]amino]-4-methylpentanoyl]-methylamino]-3-phenylpropanoyl]amino]propanoyl]-methylamino]acetyl]-methylamino]butanoic acid (Fmoc-MeAla-Leu-MeGly-MeAla-Leu-MePhe-Ala-MeChg-D-3-MeAbu-OH) (SEQ ID NO: 9)

aa2-009-resin

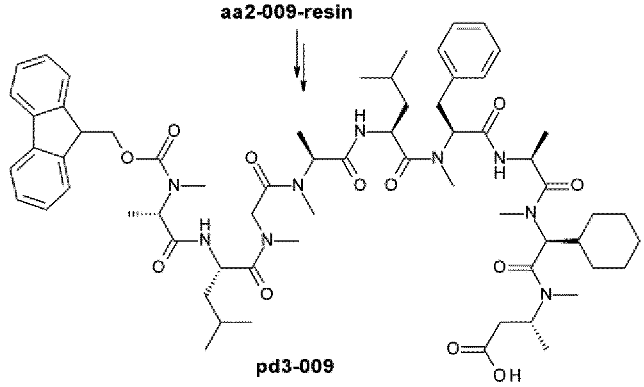

pd3-009

The dipeptide-supporting resin aa2-009-resin (Fmoc-MeChg-D-3-MeAbu-O-Trt(2-Cl)resin) (98.87 mg, 0.397 mmol/g, 0.0393 mmol) prepared in Example 1-4 was provided as a raw material, and reactions of elongation with corresponding amino acids were sequentially carried out in accordance with synthesis method 1 to synthesize pd3-009-resin (123.62 mg). The loading rate calculated by the Fmoc quantitation method using a dry resin (9.5 mg) was 0.261 mmol/g. (UVarea value at 294 nm: 2436.27 and UVarea value at 304 nm: 2197.47). Therefore, the amount of the obtained peptide was calculated to be 123.62 × 0.001 × 0.261 = 0.0323 mmol (yield: 82.2%).

Analysis of the isolating reaction liquid by LCMS showed that the purity of intended product pd3-009 was 100 area%.

LCMS (ESI) m/z = 1193.1 $(M+H)^+$
Retention time: 3.35 min (analysis condition SQDFA05long)

Synthesis of compound pd3-010, (3S)-4-(dimethylamino)-3-[[2-[[(2S)-2-[[(2S)-2-[[(2S)-2-[[(2S)-2-[[(2S)-2-[[(2S)-2-[[(2S)-2-[9H-fluoren-9-ylmethoxycarbonyl(methyl)amino]propanoyl]amino]-4-methylpentanoyl]-methylamino]-4-methylpentanoyl]amino]propanoyl]-methylamino]-3-phenylpropanoyl]amino]propanoyl]-methylamino]-4-methylpentanoyl]amino]acelyl]-methylamino]-4-oxobutanoic acid (Fmoc-MeAla-Leu-MeLeu-Ala-MePhe-Ala-MeLeu-Gly-MeAsp-NMe2) (SEQ ID NO: 10)

aa2-013-resin

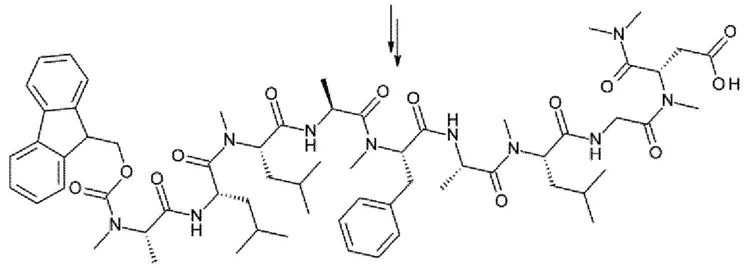

pd3-010

The dipeptide-supporting resin aa2-013-resin (Fmoc-Gly-MeAsp(O-Trt(2-Cl)resin)-NMe2) (102.05 mg, 0.386 mmol/g, 0.0394 mmol) prepared in Example 1-4 was provided as a raw material, and reactions of elongation with corresponding amino acids were sequentially carried out in accordance with synthesis method 1 to synthesize pd3-010-resin (124.68 mg). The loading rate calculated by the Fmoc quantitation method using a dry resin (10.93 mg) was 0.256 mmol/g. (UVarea value at 294 nm: 2749.59 and UVarea value at 304 nm: 2477.73). Therefore, the amount of the obtained peptide was calculated to be 124.68 × 0.001 × 0.256 = 0.0319 mmol (yield: 81.0%).

Analysis of the isolating reaction liquid by LCMS showed that the purity of intended product pd3-010 was 96.2 area%.

LCMS (ESI) m/z = 1210.1 $(M+H)^+$
Retention time: 3.08 min (analysis condition SQDFA05long)

<u>Synthesis of compound pd3-011, (2S)-1-[(2S)-2-[[(2S)-2-[[(2S)-2-[[(2S)-2-[[(2S)-2-[[2-[[(2S)-2-[[(2S)-2-[9H-fluoren-9-ylmethoxycarbonyl(methyl)amino]propanoyl]amino]-4-methylpentanoyl]-methylamino]acetyl]-methylamino]propanoyl]amino]-4-methylpentanoyl]-methylamino]-3-phenylpropanoyl]amino]propanoyl]-methylamino]-3-methylbutanoyl]pyrrolidine-2-carboxylic acid (Fmoc-MeAla-Leu-MeGly-MeAla-Leu-MePhe-Ala-MeVal-Pro-OH) (SEQ ID NO: 11)</u>

aa2-017-resin

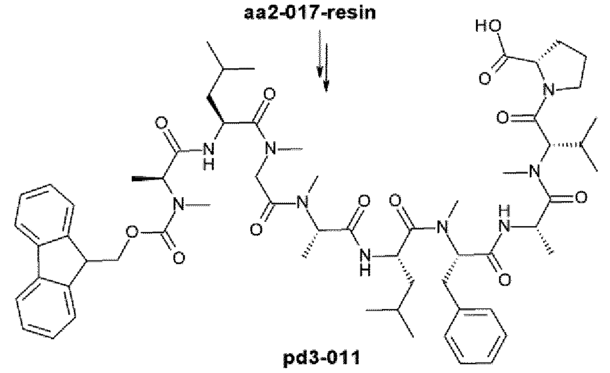

pd3-011

The dipeptide-supporting resin aa2-017-resin (Fmoc-MeVal-Pro-O-Trt(2-Cl)resin) (100.4 mg, 0.364 mmol/g, 0.0365 mmol) prepared in Example 1-4 was provided as a raw material, and reactions of elongation with corresponding amino acids were sequentially carried out in accordance with synthesis method 1 to synthesize pd3-011-resin (120.38 mg). The loading rate calculated by the Fmoc quantitation method using a dry resin (11.24 mg) was 0.251 mmol/g. (UVarea value at 294 nm: 2775.79 and UVarea value at 304 nm: 2505.11). Therefore, the amount of the obtained peptide was calculated to be 120.38 × 0.001 × 0.251 = 0.0302 mmol (yield: 82.7%).

Analysis of the isolating reaction liquid by LCMS showed that the purity of intended product pd3-011 was 100 area%.

LCMS (ESI) m/z = 1151.1 $(M+H)^+$
Retention time: 3.03 min (analysis condition SQDFA05)

Synthesis of compound pd3-012, (2S)-2-[[(2S)-2-[[(2S)-2-[[2-[[(2S)-2-[[(2S)-2-[[(2S)-2-[[(2S)-2-[[(2S)-2-[9H-fluoren-9-ylmethoxycarbonyl(methyl)amino]propanoyl]amino]-4-methylpentanoyl]-methylamino]-4-methylpentanoyl]amino]propanoyl]-methylamino]-3-phenylpropanoyl]amino]acetyl]-methylamino]-4-methylpentanoyl]amino]propanoyl]amino]propanoic acid (Fmoc-MeAla-Leu-MeLeu-Ala-MePhe-Gly-MeLeu-Ala-Ala-OH) (SEQ ID NO: 12)

aa2-027-resin

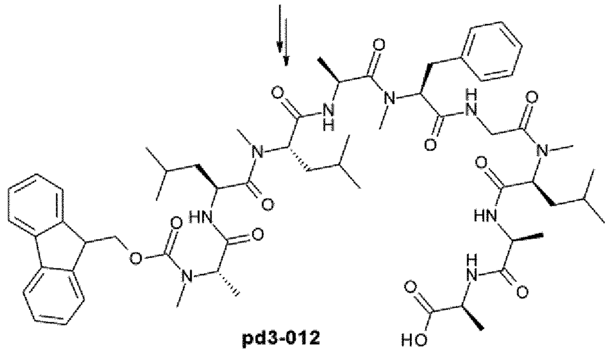

pd3-012

The dipeptide-supporting resin aa2-027-resin (Fmoc-Ala-Ala-O-Trt(2-Cl)resin) (100.08 mg, 0.334 mmol/g, 0.0334 mmol) prepared in Example 1-4 was provided as a raw material, and reactions of elongation with corresponding amino acids were sequentially carried out in accordance with synthesis method 1 to synthesize pd3-012-resin (112.35 mg). The loading rate calculated by the Fmoc quantitation method using a dry resin (11.29 mg) was 0.154 mmol/g. (UVarea value at 294 nm: 1713.25 and UVarea value at 304 nm: 1541.67). Therefore, the amount of the obtained peptide was calculated to be 112.35 × 0.001 × 0.154 = 0.0173 mmol (yield: 51.8%).

Analysis of the isolating reaction liquid by LCMS showed that the purity of intended product pd3-012 was 100 area%.

LCMS (ESI) m/z = 1125.1 $(M+H)^+$
Retention time: 3.09 min (analysis condition SQDFA05long)

Synthesis of compound pd3-013, (3S)-3-ΓΓ(2S)-2-ΓΓ(2S)-2-ΓΓ(2S)-1-Γ(2S)-2-ΓΓ(2S)-2-ΓΓ(2S)-2-ΓΓ(2S)-2-ΓΓ2-[[(2S)-2-[[(2S)-2-[9H-fluoren-9-ylmethoxycarbonyl(methyl)amino]propanoyl]amino]-4-methylpentanoyl]-methylamino]acetyl]amino]propanoyl]amino]-4-methylpentanoyl]-methylamino-3-phenylpropanoyl]amino]propanoyl]pyrrolidine-2-carbonyl]amino]propanoyl]-methylamino]-4-methylpentanoyl]-methylamino]-4-oxo-4-piperidin-]-ylbutanoic acid (Fmoc-MeAla-Leu-MeGly-Ala-Leu-MePhe-Ala-Pro-Ala-MeLeu-MeAsp-pip) (SEQ ID NO: 13

aa2-005-resin

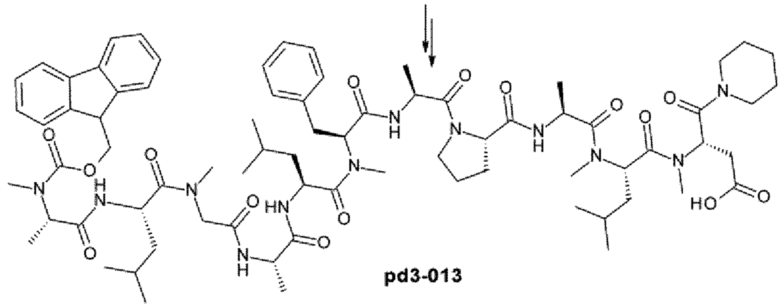

pd3-013

The dipeptide-supporting resin aa2-005-resin (Fmoc-MeLeu-MeAsp(O-Trt(2-Cl)resin)-pip) (98.77 mg, 0.355 mmol/g, 0.0351 mmol) prepared in Example 1-4 was provided as a raw material, and reactions of elongation with corresponding amino acids were sequentially carried out in accordance with synthesis method 1 to synthesize pd3-013-resin (122.47 mg). The loading rate calculated by the Fmoc quantitation method using a dry resin (12.12 mg) was 0.238

mmol/g. (UVarea value at 294 nm: 2831.35 and UVarea value at 304 nm: 2559.52). Therefore, the amount of the obtained peptide was calculated to be 122.47 × 0.001 ×0.238 = 0.0291 mmol (yield: 83.1%).

Analysis of the isolating reaction liquid by LCMS showed that the purity of intended product pd3-013 was 97.6 area%.

LCMS (ESI) m/z = 1418.3 $(M+H)^+$
Retention time: 3.06 min (analysis condition SQDFA05long)

Synthesis of compound pd3-014, (3S)-4-(dimethylamino)-3-[[(2R)-2-[[2-[[(2S)-2-[[(2S)-2-[[(2S)-2-[[2-[[(2S)-2-[[(2S)-2-[[(2S)-2-[[(2S)-2-[9H-fluoren-9-ylmethoxycarbonyl(methyl)amino]propanoyl]amino]-4-methylpentanoyl]-methylamino]-4-methylpentanoyl]amino]propanoyl]amino]acetyl]-methylamino]-3-phenylpropanoyl]-methylamino]propanoyl]amino]-4-methylpentanoyl]amino:acetyl]-methylamino]-3-methylbutanoyl]methylamino]-4-oxobutanoic acid (Fmoc-MeAla-Leu-MeLeu-Ala-Gly-MePhe-MeAla-Leu-Gly-D-MeVal-MeAsp-NMe2) (SEQ ID NO: 14)

aa2-020-resin

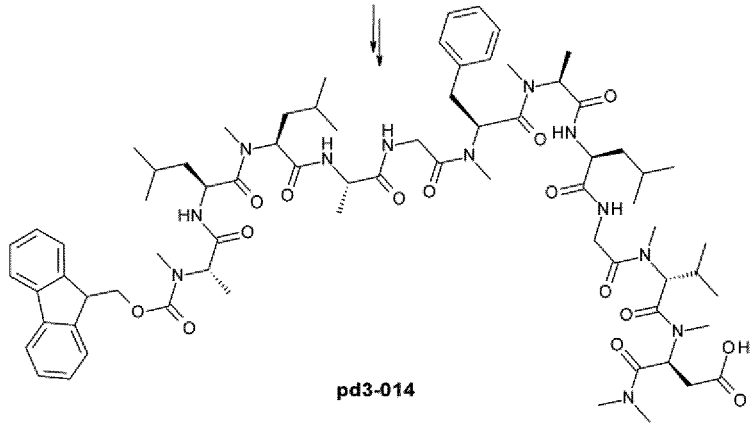

pd3-014

The dipeptide-supporting resin aa2-020-resin (Fmoc-D-MeVal-MeAsp(O-Trt(2-Cl)resin)-NMe2) (100.30 mg, 0.396 mmol/g, 0.0397 mmol) prepared in Example 1-4 was provided as a raw material, and reactions of elongation with corresponding amino acids were sequentially carried out in accordance with synthesis method 1 to synthesize pd3-014-resin (129.26 mg). The loading rate calculated by the Fmoc quantitation method using a dry resin (10.50 mg) was 0.240 mmol/g. (UVarea value at 294 nm: 2480.95 and UVarea value at 304 nm: 2240.17). Therefore, the amount of the obtained peptide was calculated to be 129.26 × 0.001 × 0.240 = 0.0310 mmol (yield: 78.1%).

Analysis of the isolating reaction liquid by LCMS showed that the purity of intended product pd3-014 was 97.8 area%.

LCMS (ESI) m/z = 1380.3 $(M+H)^+$
Retention time: 2.98 min (analysis condition SQDFA05long)

Synthesis of compound pd3-015, (2S)-1-Γ(2S)-2-ΓΓ(2S)-2-ΓΓ(2S)-1-Γ(2S)-2-ΓΓ(2S)-2-ΓΓ(2S)-2-ΓΓ(2S)-2-ΓΓ2-[[(2S)-2-[[(2S)-2-[9H-fluoren-9-ylmethoxycarbonyl(methyl)amino]propanoyl]amino]-4-methylpentanoyl]-methylamino]acetyl]amino]propanoyl]amino]-4-methylpentanoyl]-methylamino]-3-phenylpropanoyl]amino]propanoyl]pyrrolidine-2-carbonyl]-methylamino]propanoyl]amino]-6-[(2-methylpropan-2-yl)oxycarbonylamino1hexanoyl1pyrrolidine-2-carboxylic acid (Fmoc-MeAla-Leu-MeGly-Ala-Leu-MePhe-Ala-Pro-MeAla-Lys(Boc)-Pro-OH) (SEQ ID NO: 15)

aa2-025-resin

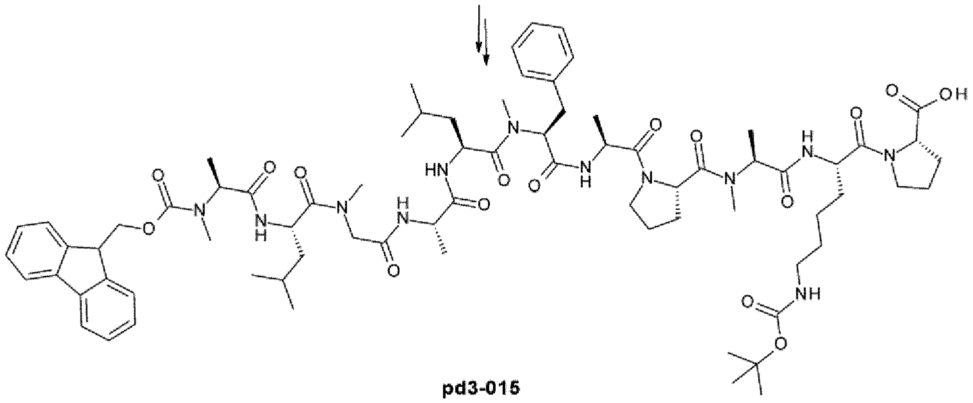

pd3-015

The dipeptide-supporting resin aa2-025-resin (Fmoc-Lys(Boc)-Pro-O-Trt(2-Cl)resin) (101.94 mg, 0.339 mmol/g, 0.0346 mmol) prepared in Example 1-4 was provided as a raw material, and reactions of elongation with corresponding amino acids were sequentially carried out in accordance with synthesis method 1 to synthesize pd3-015-resin (126.91 mg). The loading rate calculated by the Fmoc quantitation method using a dry resin (9.80 mg) was 0.207 mmol/g. (UVarea value at 294 nm: 1996.33 and UVarea value at 304 nm: 1798.34). Therefore, the amount of the obtained peptide was calculated to be 126.91 × 0.001 × 0.207 = 0.0263 mmol (yield: 76.0%).

Analysis of the isolating reaction liquid by LCMS showed that the purity of intended product pd3-015 was 100 area%.

LCMS (ESI) m/z = 1434.3 $(M+H)^+$
Retention time: 3.02 min (analysis condition SQDFA05long)

Synthesis of compound pd3-016, 2-[[(2S)-2-[[(2S)-2-[[(2S)-2-[[(2S)-2-[[(2S)-2-[[2-[[(2S)-2-[[(2S)-2-[[(2S)-2-[[(2S)-2-[9H-fluoren-9-ylmethoxycarbonyl(methyl)amino]propanoyl]amino]-4-methyl]pentanoyl]-methylamino]-4-methylpentanoyl]amino]propanoyl]amino]acetyl]-methylamino]-3-phenylpropanoyl]-methylamino]propanoyl]amino]-4-methylpentanoyl]-methylamino]propanoyl]amino]-3-phenylpropanoyl1amino 1aceti c acid (Fmoc-MeAla-Leu-MeLeu-Ala-Gly-MePhe-MeAla-Leu-MeAla-Phe-Gly-OH) (SEQ ID NO: 16)

aa2-028-resin

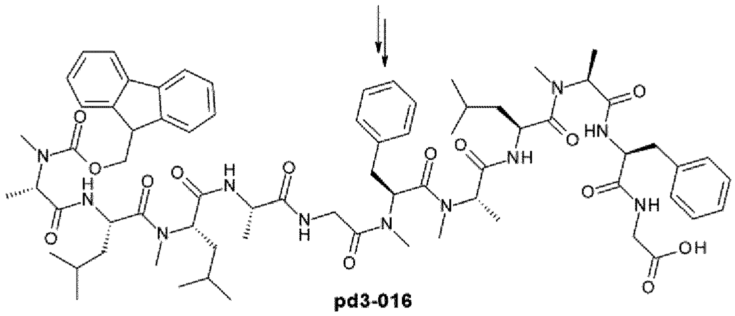

pd3-016

The dipeptide-supporting resin aa2-028-resin (Fmoc-Phe-Gly-O-Trt(2-Cl)resin) (100.91 mg, 0.248 mmol/g, 0.0250 mmol) prepared in Example 1-4 was provided as a raw material, and reactions of elongation with corresponding amino acids were sequentially carried out in accordance with synthesis method 1 to synthesize pd3-016-resin (111.48 mg). The loading rate calculated by the Fmoc quantitation method using a dry resin (11.35 mg) was 0.107 mmol/g. (UVarea value at 294 nm: 1190.73 and UVarea value at 304 nm: 1072.44). Therefore, the amount of the obtained peptide was calculated to be 111.48 × 0.001 × 0.107 = 0.0119 mmol (yield: 47.7%).

Analysis of the isolating reaction liquid by LCMS showed that the purity of intended product pd3-016 was 100 area%.

LCMS (ESI) m/z = 1343.2 $(M+H)^+$
Retention time: 3.17 min (analysis condition SQDFA05long)

Synthesis of compound pd3-017, (3S)-3-[[(2S)-2-cyclohexyl-2-[[(2S)-2-[[(2S)-2-[[(2S)-2-[[2-[[(2S)-2-[[(2S)-2-[[(2S)-2-[[(2S)-2-[[2-[[(2S)-2-[[(2S)-2-[9H-fluoren-9-ylmethoxycarbonyl(methyl)amino]propanoyl]amino]-4-methylpentanoyl]-methylamino]acetyl]methylamino]propanoyl]amino-4-methylpentanoyl]-methylamino]-3-phenylpropanoyl]-methylamino]propanoyl]amino]acetyl]amino]-4-methylpentanoyl]-methylamino]-4-methylpentanoyl]amino]propanoyl]-methylamino]acetyl]-methylamino]-4-oxo-4-piperidin-1-ylbutanoic acid (Fmoc-MeAla-Leu-MeGly-MeAla-Leu-MePhe-MeAla-Gly-Leu-MeLeu-Ala-MeChg-MeAsp-pip) (SEQ ID NO: 17)

aa2-004-resin

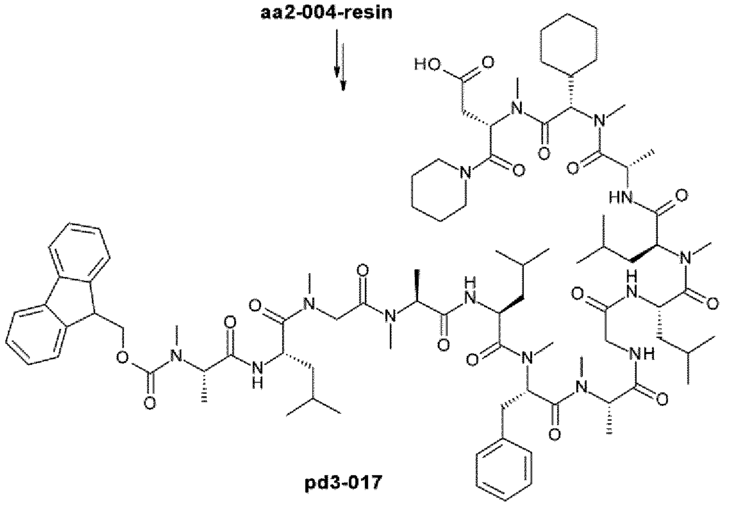

pd3-017

The dipeptide-supporting resin aa2-004-resin (Fmoc-MeChg-MeAsp(O-Trt(2-Cl)resin)-pip) (101.83 mg, 0.347 mmol/g, 0.0353 mmol) prepared in Example 1-4 was provided as a raw material, and reactions of elongation with corresponding amino acids were sequentially carried out in accordance with synthesis method 1 to synthesize pd3-017-resin (138.64 mg). The loading rate calculated by the Fmoc quantitation method using a dry resin (10.89 mg) was 0.217 mmol/g. (UVarea value at 294 nm: 2322.69 and UVarea value at 304 nm: 2091.98). Therefore, the amount of the obtained peptide was calculated to be 138.64 × 0.001 × 0.217 = 0.0301 mmol (yield: 85.1%).

Analysis of the isolating reaction liquid by LCMS showed that the purity of intended product pd3-017 was 97.9 area%.

LCMS (ESI) m/z = 1672.6 $(M+H)^+$
Retention time: 3.59 min (analysis condition SQDFA05long)

Synthesis of compound pd3-018, (3S)-4-(dimethylamino)-3-[[3-[[2-[[(2S)-2-[[(2S)-2-[[(2S)-2-[[(2S)-2-[[2-[[(2S)-2-[[(2S)-2-[[(2S)-2-[[(2S)-2-[[(2S)-2-[9H-fluoren-9-ylmethoxycarbonyl(methyl)amino]propanoyl]amino]-4-methylpentanoyl]-methylamino]-4-methylpentanoyl]amino]propanoyl]-methylamino]-3-phenylpropanoyl]amino]acetyl]-methylamino]propanoyl]amino]-4-methylpentanoyl]amino]propanoyl]-methylamino]-4-methylpentanoyl]amino]acetyl]amino]-2,2-dimethylpropanoyl]-methylamino]-4-oxobutanoic acid (Fmoc-MeAla-Leu- MeLeu- Ala- MePhe-Gl y- MeAl a- Leu - Ala-MeLeu -Gly -bAla(2- Me2)-MeAsp- NMe2) (SEQ ID NO: 18)

aa2-021-resin

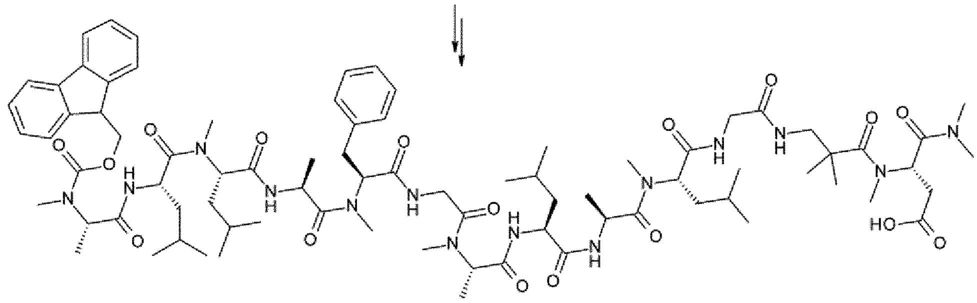

pd3-018

The dipeptide-supporting resin aa2-021-resin (Fmoc-bAla(2-Me2)-MeAsp(O-Trt(2-Cl)resin)-NMe2) (100.87 mg, 0.407 mmol/g, 0.0411 mmol) prepared in Example 1-4 was provided as a raw material, and reactions of elongation with corresponding amino acids were sequentially carried out in accordance with synthesis method 1 to synthesize pd3-018-resin (143.00 mg). The loading rate calculated by the Fmoc quantitation method using a dry resin (9.92 mg) was 0.234 mmol/g. (UVarea value at 294 nm: 2287.01 and UVarea value at 304 nm: 2057.57). Therefore, the amount of the obtained peptide was calculated to be 143.00 × 0.001 × 0.234 = 0.0335 mmol (yield: 81.5%).

Analysis of the isolating reaction liquid by LCMS showed that the purity of intended product pd3-018 was 97.9 area%.

LCMS (ESI) m/z = 1564.5 (M+H)+
Retention time: 3.05 min (analysis condition SQDFA05long)

Synthesis of compound pd3-019, 2-[[(2S)-2-[[(2S)-2-[[(2S)-2-[[(2S)-2-[[2-[[(2S)-2-[[(2S)-2-[[(2S)-2-[[(2S)-2-[[2-[[(2S)-2-[[(2S)-2-[9H-fluoren-9-ylmethoxycarbonyl(methyl)amino]proyanoyl]amino]-4-methyl pentanoyl1-methylamino 1acetyl1-methylamino 1propanoyl1amino 1-4-methyl pentanoyl1-methylamino 1-3-phenylpropanoyl]-methylamino]propanoyl]amino]acetyl]amino]-4-methylpentanoyl]-methylamino]-4-methylpentanoyl]amino]propanoyl]-methylamino]-4-methylpentanoyl]amino]-2-methylpropanoic acid (Fmoc-MeAla-Leu-MeGly-MeAla-Leu-MePhe-MeAla-Gly-Leu-MeLeu-Ala-MeLeu-Aib-OH)(SEQ ID NO: 19).

aa2-018-resin

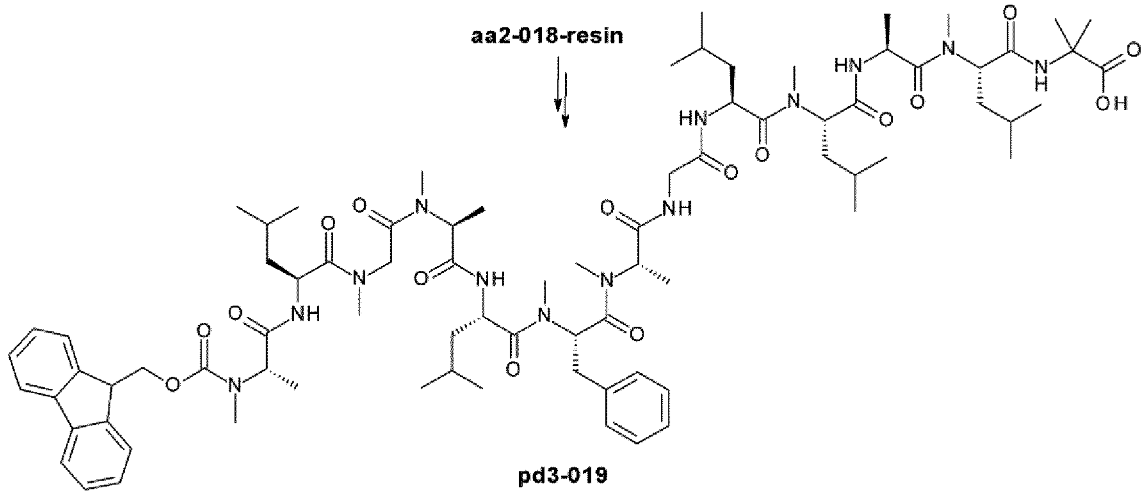

pd3-019

The dipeptide-supporting resin aa2-018-resin (Fmoc-MeLeu-Aib-O-Trt(2-Cl)resin) (99.74 mg, 0.300 mmol/g, 0.0299 mmol) prepared in Example 1-4 was provided as a raw material, and reactions of elongation with corresponding amino acids were sequentially carried out in accordance with synthesis method 1 to synthesize pd3-019-resin (125.92 mg). The loading rate calculated by the Fmoc quantitation method using a dry resin (9.72 mg) was 0.190 mmol/g. (UVarea value at 294 nm: 1817.28 and UVarea value at 304 nm: 1638.87). Therefore, the amount of the obtained peptide was calculated to be 125.92 ×0.001 × 0.190 = 0.0239 mmol (yield: 80.0%).

Analysis of the isolating reaction liquid by LCMS showed that the purity of intended product pd3-019 was 100

area%.

LCMS (ESI) m/z = 1535.5 $(M+H)^+$
Retention time: 3.35 min (analysis condition SQDFA05long)

Synthesis of compound pd3-020, 2-[[(2S)-2-[[(2S)-2-[[(2S)-2-[[(2S)-2-[[(2S)-2-[[(2S)-2-[[2-[[(2S)-2-[[(2S)-2-[[(2S)-2-[[(2S)-2-[[(2S)-2-[9H-fluoren-9-ylmethoxycarbonyl(methyl)amino]propanoyl]amino]-4-methylpentanoyl]-methylamino]-4-methylpentanoyl]amino]propanoyl]-methylamino]-3-phenylpropanoyl]amino]acetyl]-methylamino]propanoyl]amino]-4-methylpentanoyl]amino]propanoyl]-methylamino]-4-methylpentanoyl]-methylamino]propanoyl]amino]-4-oxo-4-(tritylamino)butanoyl]amino]acetic acid (Fmoc-MeAla-Leu-MeLeu-Ala-MePhe-Gly-MeAla-Leu-Ala-MeLeu-MeAla-Asn(Trt)-Gly-OH) (SEQ ID NO: 20)

aa2-029-resin

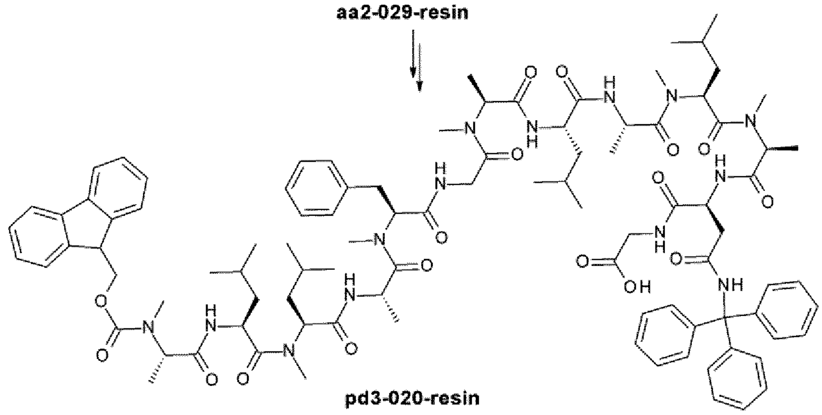

pd3-020-resin

The dipeptide-supporting resin aa2-029-resin (Fmoc-Asn(Trt)-Gly-O-Trt(2-Cl)resin) (99.73 mg, 0.259 mmol/g, 0.0258 mmol) prepared in Example 1-4 was provided as a raw material, and reactions of elongation with corresponding amino acids were sequentially carried out in accordance with synthesis method 1 to synthesize pd3-020-resin (119.40 mg). The loading rate calculated by the Fmoc quantitation method using a dry resin (9.67 mg) was 0.144 mmol/g. (UVarea value at 294 nm: 1373.79 and UVarea value at 304 nm: 1235.99). Therefore, the amount of the obtained peptide was calculated to be $119.4 \times 0.001 \times 0.144 = 0.0172$ mmol (yield: 66.6%).

Analysis of the isolating reaction liquid by LCMS showed that the purity of intended product pd3-020 was 100 area%.

LCMS (ESI) m/z = 1750.6 $(M+H)^+$
Retention time: 3.69 min (analysis condition SQDFA05long)

Synthesis of compound pd3-021, (3R)-4-(dimethylamino)-3-[[(2S)-2-[[2-[[(2S)-2-[[(2S)-2-[[(2S)-1-[(2S)-2-[[2-[[(2S)-2-[[(2S)-2-[[(2S)-2-[[(2S)-2-[[2-[[(2S)-2-[[(2S)-2-[9H-fluoren-9-ylmethoxycarbonyl(methyl)amino]propanoyl]amino]-4-methylpentanoyl]-methylamino]acetyl]amino]propanoyl]amino]-4-methylpentanoyl]-methylamino]-3-phenylpropanoyl]-methylamino]propanoyl]amino]acetyl]methylamino]-3-phenylpropanoyl]pyrrolidine-2-carbonyl]amino]propanoyl]-methylamino]-4-methylpentanoyl]amino]acetyl]-methylamino]-3-methylbutanoyl]-methylamino]-4-oxobutanoic acid (Fmoc-MeAla-Leu-MeGly-Ala-Leu-MePhe-MeAla-Gly-MePhe-Pro-Ala-MeLeu-Gly-MeVal-D-MeAsp-NMe2) (SEQ ID NO: 21)

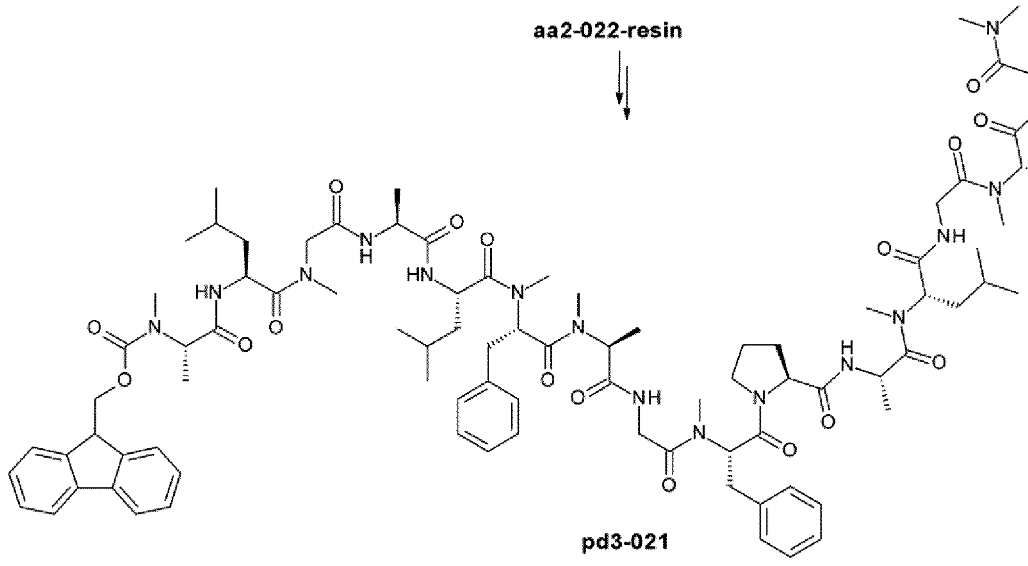


pd3-021

The dipeptide-supporting resin aa2-022-resin (Fmoc-MeVal-D-MeAsp(O-Trt(2-Cl)resin)-NMe2) (99.76 mg, 0.396 mmol/g, 0.0395 mmol) prepared in Example 1-4 was provided as a raw material, and reactions of elongation with corresponding amino acids were sequentially carried out in accordance with synthesis method 1 to synthesize pd3-021-resin (143.67 mg). The loading rate calculated by the Fmoc quantitation method using a dry resin (9.81 mg) was 0.230 mmol/g. (UVarea value at 294 nm: 2215.34 and UVarea value at 304 nm: 1997.01). Therefore, the amount of the obtained peptide was calculated to be 143.67 × 0.001 × 0.230 = 0.0330 mmol (yield: 83.6%). Analysis of the isolating reaction liquid by LCMS showed that the purity of intended product pd3-021 was 98.0 area%.

LCMS (ESI) m/z = 1780.6 $(M+H)^+$
Retention time: 2.91 min (analysis condition SQDFA05long)

Synthesis of compound pd3-022, 2-[[(2S)-2-[[(2S)-2-[[(2S)-2-[[(2S)-2-[[(2S)-2-[[(2S)-2-[[(2S)-2-[[(2S)-2-[[2-[[(2S)-2-[[(2S)-2-[[(2S)-2-[[(2S)-2-[[(2S)-2-[9H-fluoren-9-ylmethoxycarbonyl(methyl)amino]propanoyl]amino]-4-methylpentanoyl]-methylamino]-4-methylpentanoyl]amino]propanoyl]-methylamino]-3-phenylpropanoyl]amino]acetyl]-methylamino]propanoyl]amino]-4-methylpentanoyl]-methylamino]-3-phenylpropanoyl]-methylamino]propanoyl]amino]propanoyl]-methylamino]-4-methylpentanoyl]amino]propanoyl]-methylamino]-3-methylbutanoyl]-methylamino]acetic acid (Fmoc-MeAla-Leu-MeLeu-Ala-MePhe-Gly-MeAla-Leu-MePhe-MeAla-Ala-MeLeu-Ala-MeVal-MeGly-OH) (SEQ ID NO: 22)

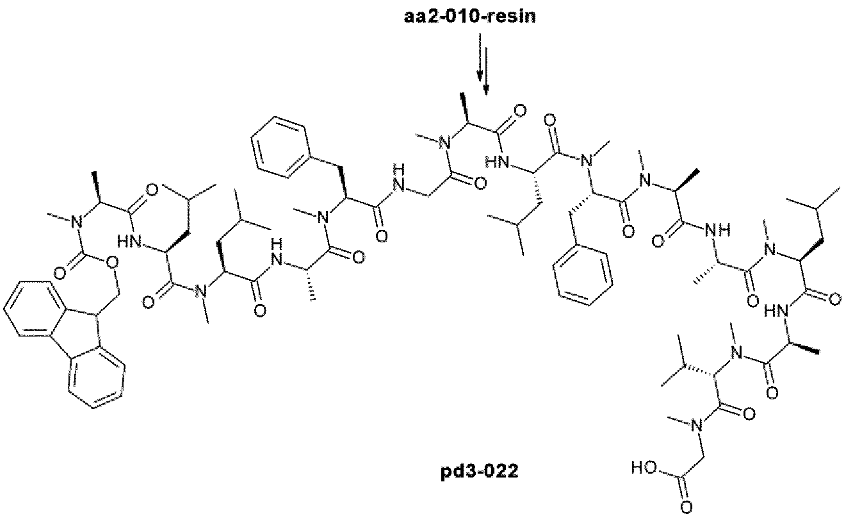


pd3-022

The dipeptide-supporting resin aa2-010-resin (Fmoc-MeVal-MeGly-O-Trt(2-Cl)resin) (99.21 mg, 0.374 mmol/g, 0.0371 mmol) prepared in Example 1-4 was provided as a raw material, and reactions of elongation with corresponding amino acids were sequentially carried out in accordance with synthesis method 1 to synthesize pd3-022-resin (130.94 mg). The loading rate calculated by the Fmoc quantitation method using a dry resin (10.65 mg) was 0.178 mmol/g. (UVarea value at 294 nm: 1866.70 and UVarea value at 304 nm: 1683.07). Therefore, the amount of the obtained peptide was calculated to be 130.94 × 0.001 × 0.178 = 0.0233 mmol (yield: 62.8%).

Analysis of the isolating reaction liquid by LCMS showed that the purity of intended product pd3-022 was 100 area%.

LCMS (ESI) m/z = 1753.6 $(M+H)^+$
Retention time: 3.46 min (analysis condition SQDFA05long)

Synthesis of compound pd3-023, (2S)-2-[[2-[[(2S)-2-[[(2S)-2-[[(2S)-2-[[(2S)-2-[[(2S)-2-[[2-[[(2S)-2-[[(2S)-2-[[(2S)-2-[[(2S)-2-[[2-[[(2S)-2-[[(2S)-2-[9H-fluoren-9-ylmethoxycarbonyl(methyl)amino]propanoyl]amino]-4-methylpentanoyl]-methylamino]acetyl]amino]propanoyl]amino]-4-methylpentanoyl]-methylamino]-3-phenylpropanoyl]-methylamino]propanoyl]amino]acetyl]methylamino]-3-phenylpropanoyl]-methylamino 1propanoyl1amino 1propanoyl1-methylamino 1-4-methylpentanoyl1-methylamino]propanoyl]amino]acetyl]amino]-3-methylbutanoic acid (Fmoc-MeAla-Leu-MeGly-Ala-Leu-MePhe-MeAla-Gly-MePhe-MeAla-Ala-MeLeu-MeAla-Gly-Val-OH) (SEQ ID NO: 23)

aa2-030-resin

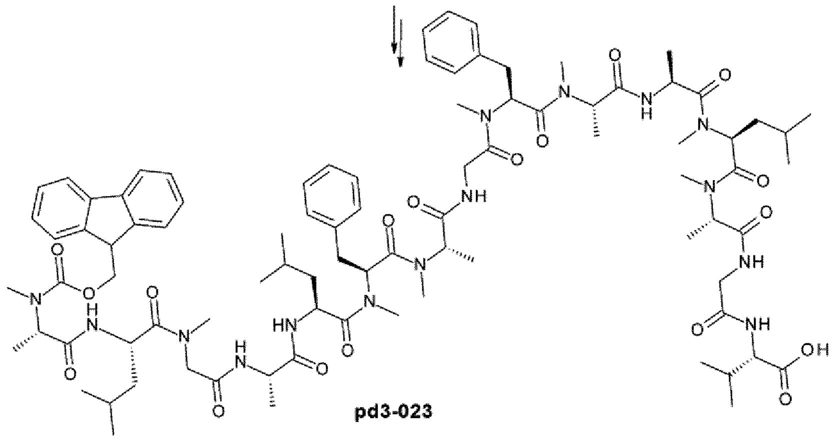

pd3-023

The dipeptide-supporting resin aa2-030-resin (Fmoc-Gly-Val-O-Trt(2-Cl)resin) (99.41 mg, 0.278 mmol/g, 0.0276 mmol) prepared in Example 1-4 was provided as a raw material, and reactions of elongation with corresponding amino acids were sequentially carried out in accordance with synthesis method 1 to synthesize pd3-023-resin (131.81 mg). The loading rate calculated by the Fmoc quantitation method using a dry resin (9.88 mg) was 0.162 mmol/g. (UVarea value at 294 nm: 1576.35 and UVarea value at 304 nm: 1424.24). Therefore, the amount of the obtained peptide was calculated to be 131.81 × 0.001 × 0.162 = 0.0214 mmol (yield: 77.3%).

Analysis of the isolating reaction liquid by LCMS showed that the purity of intended product pd3-023 was 99.6 area%.

LCMS (ESI) m/z = 1683.5 $(M+H)^+$
Retention time: 2.98 min (analysis condition SQDFA05long)

Synthesis of compound pd3-024, 2-ΓΓ(2S)-2-ΓΓ(2S)-2-ΓΓ(2S)-2-ΓΓ(2S)-2-ΓΓ(2S)-1-Γ(2S)-2-ΓΓ(2S)-2-ΓΓ(2S)-2-[[2-[[(2S)-2-[[(2S)-2-[[(2S)-2-[[(2S)-2-[[(2S)-2-[9H-fluoren-9-ylmethoxycarbonyl(methyl)amino]propanoyl]amino]-4-methylpentanoyl]-methylamino]-4-methylpentanoyl]amino]propanoyl]-methylamino]-3-phenylpropanoyl]amino]acetyl]-methylamino]propanoyl]amino]-4-methylpentanoyl]-methylamino]-3-phenylpropanoyl]pyrrolidine-2-carbonyl]amino]propanoyl]-methylamino]-4-methylpentanoyl]-methylamino]propanoyl]amino]-3-[(2-methylpropan-2-yl)oxy]propanoyl]amino]acetic acid (Fmoc-MeAla-Leu-MeLeu-Ala-MePhe-Gly-MeAla-Leu-MePhe-Pro-Ala-MeLeu-MeAla-Ser(tBu)-Gly-OH) (SEQ ID NO: 24)

aa2-031-resin

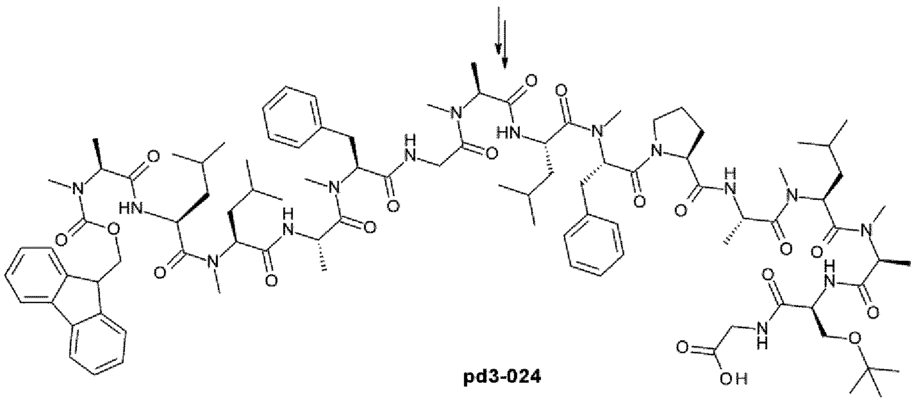

pd3-024

The dipeptide-supporting resin aa2-031-resin (Fmoc-Ser(tBu)-Gly-O-Trt(2-Cl)resin) (98.17 mg, 0.258 mmol/g, 0.0253 mmol) prepared in Example 1-4 was provided as a raw material, and reactions of elongation with corresponding amino acids were sequentially carried out in accordance with synthesis method 1 to synthesize pd3-024-resin (128.02 mg). The loading rate calculated by the Fmoc quantitation method using a dry resin (9.96 mg) was 0.139 mmol/g. (UVarea value at 294 nm: 1361.24 and UVarea value at 304 nm: 1223.95). Therefore, the amount of the obtained peptide was calculated to be 128.02 × 0.001 × 0.139 = 0.0178 mmol (yield: 70.3%).
Analysis of the isolating reaction liquid by LCMS showed that the purity of intended product pd3-024 was 98.8 area%.

LCMS (ESI) m/z = 1795.6 $(M+H)^+$
Retention time: 3.38 min (analysis condition SQDFA05long)

Example 3-2. Synthesis of chain peptide using tripeptide-supporting resin

In this Example, the tripeptide-supporting resin prepared in Example 1-4 was provided as a raw material, and chain peptides shown in Table 89 below were synthesized by a solid phase reaction using a peptide synthesizing machine. The yields and purities of the peptides are described.

[Table 89]

| Compound No. | Abbreviation |
|---|---|
| pd3-025 | Fmoc-MeA \| a-Leu-MeLeu-A \| a-A \| a-Pro-0H (SEQ ID NO: 25) |
| pd3-026 | Fmoc-MeA\|a-Leu-Me-Leu-A\|a-MeG\|y (cPent)-MeAsp-NMe2 (SEQ ID NO: 26) |
| pd3-027 | Fmoc-MeA\|a-MeLeu-A\|a-MeG\|y-MePhe-Leu-Me-Leu-A\|a-A\|a-Pro-0H (SEQ ID NO: 27) |
| pd3-028 | Fmoc-MeAla-MeLeu-Ala-MeLeu-MePhe-Leu-MeAla-Ala-MeGly(cPent)-MeAsp-NMe2 (SEQ ID NO: 28) |
| pd3-029 | Fmoc-MeAla-Leu-MeGly-MeAla-Leu-MePhe-MeAla-Gly-Leu-MeLeu-MeAla-Ala-Ala-Pro-OH (SEQ ID NO: 29) |
| pd3-030 | Fmoc-MeAla-Leu-MeGly-MeAla-Leu-MePhe-MeAla-Gly-Leu-MeLeu-MeAla-Ala-MeGly(cPent)-MeAsp-NMe2 (SEQ ID NO: 30) |

Synthesis of compound pd3-025, (2S)-1-Γ(2S)-2-ΓΓ(2S)-2-ΓΓ(2S)-2-ΓΓ(2S)-2-ΓΓ(2S)-2-Γ9H-fluoren-9-ylmethoxycarbonyl(methyl)amino]propanoyl]amino]-4-methylpentanoyl]-methylamino]-4-methylpentanoyl]amino]proyanoyl]amino]proyanoyl]pyrrolidine-2-carboxylic acid(Fmoc-MeAla-Leu-MeLeu-Ala-Ala-Pro-OH) (SEQ ID NO: 25)

aa2-032-resin

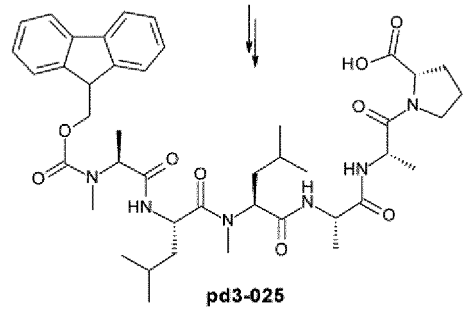

pd3-025

The tripeptide-supporting resin aa2-032-resin (Fmoc-Ala-Ala-Pro-O-Trt(2-Cl)resin) (99.58 mg, 0.393 mmol/g, 0.0391 mmol) prepared in Example 1-4 was provided as a raw material, and reactions of elongation with corresponding amino acids were sequentially carried out in accordance with synthesis method 1 to synthesize pd3-025-resin (106.62 mg). The loading rate calculated by the Fmoc quantitation method using a dry resin (11.42 mg) was 0.274 mmol/g. (UVarea value at 294 nm: 3085.85 and UVarea value at 304 nm: 2775.25). Therefore, the amount of the obtained peptide was calculated to be 106.62 × 0.001 × 0.274 = 0.0292 mmol (yield: 74.6%).

Analysis of the isolating reaction liquid by LCMS showed that the purity of intended product pd3-025 was 99.0 area%.

LCMS (ESI) m/z = 805.8 $(M+H)^+$
Retention time: 0.88 min (analysis condition SQDFA05)

Synthesis of compound pd3-026, (3S)-3-[[(2S)-2-cyclopentyl-2-[[(2S)-2-[[(2S)-2-[[(2S)-2-[[(2S)-2-[9H-fluoren-9-ylmethoxycarbonyl(methyl)amino]propanoyl]amino]-4-methylpentanoyl]-methylamino]-4-methylpentanoyl]amino]propanoyl]-methylamino]acetyl]-methylamino]-4-(dimethylamino)-4-oxobutanoic acid (Fmoc-MeAla-Leu-MeLeu-Ala-MeGly(cPent)-MeAsp-NMe2) (SEQ ID NO: 26)

aa2-023-resin

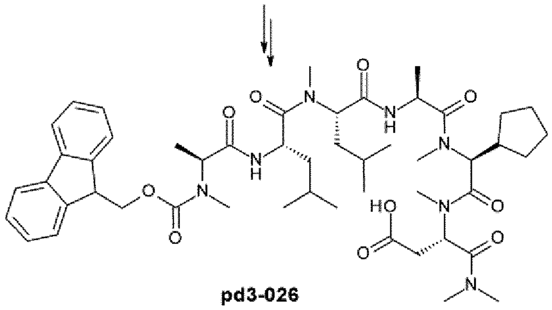

pd3-026

The tripeptide-supporting resin aa2-023-resin (Fmoc-Ala-MeGly(cPent)-MeAsp(O-Trt(2-Cl)resin)-NMe2) (99.54 mg, 0.378 mmol/g, 0.0376 mmol) prepared in Example 1-4 was provided as a raw material, and reactions of elongation with corresponding amino acids were sequentially carried out in accordance with synthesis method 1 to synthesize pd3-026-resin (105.54 mg). The loading rate calculated by the Fmoc quantitation method using a dry resin (9.85 mg) was 0.286 mmol/g. (UVarea value at 294 nm: 2767.26 and UVarea value at 304 nm: 2504.21). Therefore, the amount of the obtained peptide was calculated to be 105.54 × 0.001 × 0.286 = 0.0302 mmol (yield: 80.2%).

Analysis of the isolating reaction liquid by LCMS showed that the purity of intended product pd3-026 was 98.0 area%.

LCMS (ESI) m/z = 932.9 $(M+H)^+$
Retention time: 0.99 min (analysis condition SQDFA05)

Synthesis of compound pd3-027, (2S)-1-[(2S)-2-[[(2S)-2-[[(2S)-2-[[(2S)-2-[[(2S)-2-[[2-[[(2S)-2-[[(2S)-2-[[(2S)-2-[9H-fluoren-9-ylmethoxycarbonyl(methyl)amino]propanoyl]-methylamino]-4-methylpentanoyl]amino]propanoyl]-methylamino]acetyl]-methylamino]-3-phenylpropanoyl]amino]-4-methylpentanoyl]-methylamino]-4-methylpentanoyl]amino]propanoyl]amino]propanoyl]pyrrolidine-2-carboxylic acid (Fmoc-MeAla-MeLeu-Ala-MeGly-MePhe-Leu-MeLeu-Ala-Ala-Pro-OH) (SEQ ID NO: 27)

aa2-032-resin

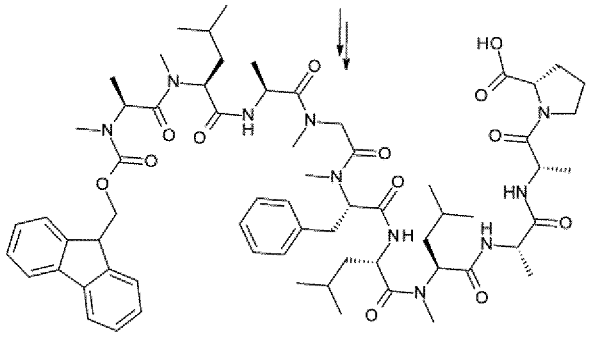

pd3-027

The tripeptide-supporting resin aa2-032-resin (Fmoc-Ala-Ala-Pro-O-Trt(2-Cl)resin) (98.96 mg, 0.393 mmol/g, 0.0389 mmol) prepared in Example 1-4 was provided as a raw material, and reactions of elongation with corresponding amino acids were sequentially carried out in accordance with synthesis method 1 to synthesize pd3-027-resin (115.73 mg). The loading rate calculated by the Fmoc quantitation method using a dry resin (9.65 mg) was 0.216 mmol/g. (UVarea value at 294 nm: 2050.67 and UVarea value at 304 nm: 1843.73). Therefore, the amount of the obtained peptide was calculated to be 115.73 × 0.001 × 0.216 = 0.0258 mmol (yield: 64.3%).

Analysis of the isolating reaction liquid by LCMS showed that the purity of intended product pd3-027 was 98.3 area%.

LCMS (ESI) m/z = 1236.2 $(M+H)^+$
Retention time: 3.04 min (analysis condition SQDFA05long)

Synthesis of compound pd3-028, (3S)-3-[[(2S)-2-cyclopentyl-2-[[(2S)-2-[[(2S)-2-[[(2S)-2-[[(2S)-2-[[(2S)-2-[[(2S)-2-[[(2S)-2-[[(2S)-2-[9H-fluoren-9-ylmethoxycarbonyl(methyl)amino]propanoyl]-methylamino]-4-methylpentanoyl]amino]propanoyl]-methylamino]-4-methylpentanoyl]-methylamino]-3-phenylpropanoyl]amino]-4-methylpentanoyl]-methylamino]propanoyl]amino]propanoyl]-methylamino]acetyl]-methylamino]-4-(dimethylamino)-4-oxobutanoic acid (Fmoc-MeAla-MeLeu-Ala-MeLeu-MePhe-Leu-MeAla-Ala-MeGly(cPent)-MeAsp-NMe2) (SEQ ID NO: 28)

aa2-023-resin

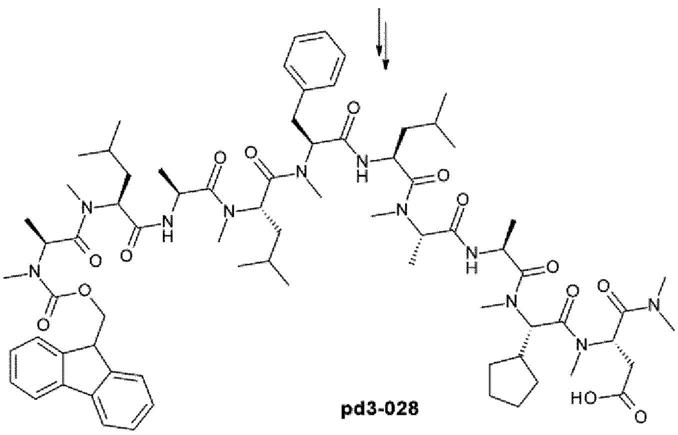

pd3-028

The tripeptide-supporting resin aa2-023-resin (Fmoc-Ala-MeGly(cPent)-MeAsp(O-Trt(2-Cl)resin)-NMe2) (98.56 mg, 0.378 mmol/g, 0.0373 mmol) prepared in Example 1-4 was provided as a raw material, and reactions of elongation with corresponding amino acids were sequentially carried out in accordance with synthesis method 1 to synthesize pd3-028-resin (117.53 mg). The loading rate calculated by the Fmoc quantitation method using a dry resin (9.53 mg) was 0.225 mmol/g. (UVarea value at 294 nm: 2107.24 and UVarea value at 304 nm: 1901.56). Therefore, the amount of the obtained peptide was calculated to be 117.53 × 0.001 × 0.225 = 0.0264 mmol (yield: 71.0%).

Analysis of the isolating reaction liquid by LCMS showed that the purity of intended product pd3-028 was 96.3 area%.

LCMS (ESI) m/z = 1377.3 $(M+H)^+$
Retention time: 3.44 min (analysis condition SQDFA05long)

Synthesis of compound pd3-029, (2S)-1-[(2S)-2-[[(2S)-2-[[(2S)-2-[[(2S)-2-[[(2S)-2-[[2-[[(2S)-2-[[(2S)-2-[[(2S)-2-[[(2S)-2-[[2-[[(2S)-2-[[(2S)-2-[9H-fluoren-9-ylmethoxycarbonyl(methyl)amino]propanoyl]amino]-4-methylpentanoyl]-methylamino]acetyl]-methyl]amino]propanoyl]amino]-4-methylpentanoyl]-methylamino]-3-phenylpropanoyl]-methylamino]propanoyl]amino]acetyl]amino]-4-methylpentanoyl]-methylamino]-4-methylpentanoyl]-methylamino]propanoyl]amino:propanoyl]amino]propanoyl]pyrrolidine-2-carboxylic acid (Fmoc-MeAla-Leu-MeGly-MeAla-Leu-MePhe-MeAla-Gly-Leu-MeLeu-MeAla-Ala-Ala-Pro-OH) (SEQ ID NO: 29)

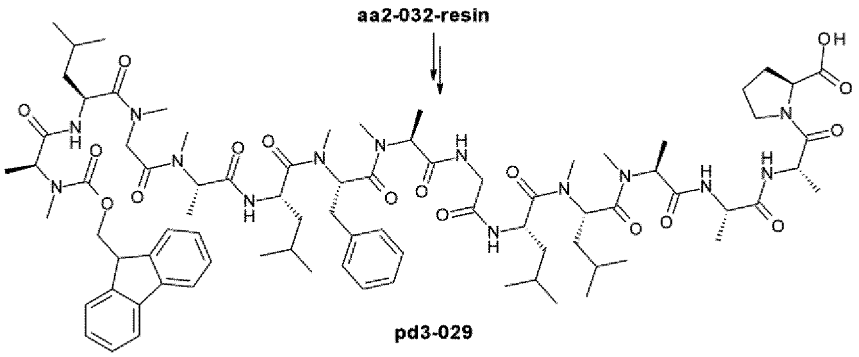


pd3-029

The tripeptide-supporting resin aa2-032-resin (Fmoc-Ala-Ala-Pro-O-Trt(2-Cl)resin) (99.49 mg, 0.393 mmol/g, 0.0391 mmol) prepared in Example 1-4 was provided as a raw material, and reactions of elongation with corresponding amino acids were sequentially carried out in accordance with synthesis method 1 to synthesize pd3-029-resin (135.39 mg). The loading rate calculated by the Fmoc quantitation method using a dry resin (10.35 mg) was 0.223 mmol/g. (UVarea value at 294 nm: 2277.17 and UVarea value at 304 nm: 2047.71). Therefore, the amount of the obtained peptide was calculated to be 135.39 × 0.001 × 0.223 = 0.0302 mmol (yield: 77.2%).

Analysis of the isolating reaction liquid by LCMS showed that the purity of intended product pd3-029 was 100 area%.

LCMS (ESI) m/z = 1576.4 (M+H)+
Retention time: 2.89 min (analysis condition SQDFA05long)

Synthesis of compound pd3-030, (3S)-3-[[(2S)-2-cyclopentyl-2-[[(2S)-2-[[(2S)-2-[[(2S)-2-[[(2S)-2-[[2-ΓΓ(2S)-2-ΓΓ(2S)-2-ΓΓ(2S)-2-ΓΓ(2S)-2-ΓΓ2-ΓΓ(2S)-2-ΓΓ(2S)-2-Γ9H-fluoren-9-ylmethoxycarbonyl(methyl)amino]propanoyl]amino]-4-methylpentanoyl]-methylamino]acetyl]-methylamino 1propanoyl1amino 1-4-methyl pentanoyl1-methylamino 1-3-phenyl propanoyl1-methylamino]propanoyl]amino]acetyl]amino]-4-methylpentanoyl]-methylamino]-4-methylpentanoyl]-methylamino]propanoyl]amino]propanoyl]-methylamino]acetyl]methylamino]-4-(dimethylamino)-4-oxobutanoic acid (Fmoc-MeAla-Leu-MeGly-MeAla-Leu-MePhe-MeAla-Gly-Leu-MeLeu-MeAla-Ala-MeGly(cPent)-MeAsp-NMe2) (SEQ ID NO: 30)

aa2-023-resin

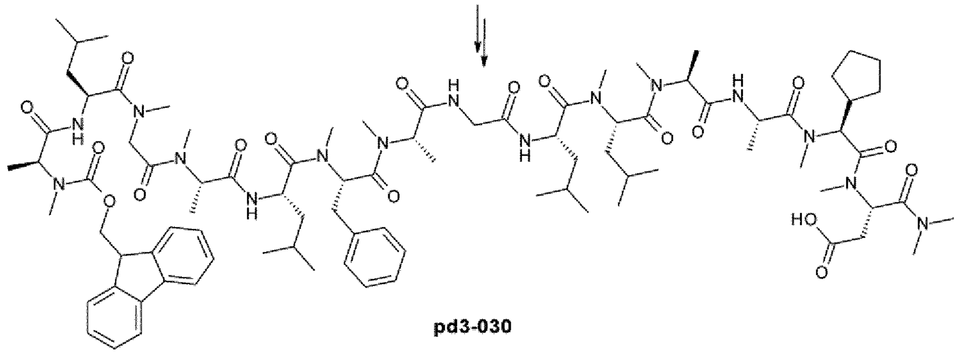

pd3-030

The tripeptide-supporting resin aa2-023-resin (Fmoc-Ala-MeGly(cPent)-MeAsp(O-Trt(2-Cl)resin)-NMe2) (100.21 mg, 0.378 mmol/g, 0.0379 mmol) prepared in Example 1-4 was provided as a raw material, and reactions of elongation with corresponding amino acids were sequentially carried out in accordance with synthesis method 1 to synthesize pd3-030-resin (135.50 mg). The loading rate calculated by the Fmoc quantitation method using a dry resin (9.36 mg) was 0.229 mmol/g. (UVarea value at 294 nm: 2106.02 and UVarea value at 304 nm: 1896.06). Therefore, the amount of the obtained peptide was calculated to be 135.50 × 0.001 × 0.229 = 0.0310 mmol (yield: 81.9%).

Analysis of the isolating reaction liquid by LCMS showed that the purity of intended product pd3-030 was 97.2 area%.

LCMS (ESI) m/z = 1703.6 $(M+H)^+$

Retention time: 3.12 min (analysis condition SQDFA05long)

Example 3-3. Synthesis of chain peptide using dipeptide-supporting Sieber resin

In this Example, the dipeptide-supporting Sieber resin prepared in Example 1-4 was provided as a raw material, and chain peptides shown in Table 90 below were synthesized by a solid phase reaction using a peptide synthesizing machine. The yields and purities of the peptides are described.

When Sieber resin was used, the elongation reaction of the peptide was carried out in accordance with synthesis method 1, a TFE/DCM/TFA (1/1/0.02, 1 mL) solution used in the peptide isolation reaction.

[Table 90]

| Compound No. | Abbreviation |
|---|---|
| pd4-001 | Fmoc-MeA \| a-Leu-A \| a-MeVa \| -MeAsn-pip (SEQ ID NO: 31) |
| pd4-002 | Fmoc-MeA \| a-Leu-MeG \| y-MeA \| a-A \| a-MeVa \| -MeAsn-pip (SEQ ID NO: 32) |
| pd4-003 | Fmoc-MeA\|a-Leu-MeG\|y-A\|a-Leu-MePhe-A\|a-Pro-A\|a-MeVa\|-MeAsn-pip (SEQ ID NO: 33) |

Synthesis of compound pd4-001, 9H-fluoren-9-ylmethyl N-Γ(2S)-1-ΓΓ(2S)-1-ΓΓ(2S)-1-ΓΓ(2S)-1-ΓΓ(2S)-4-amino-1,4-dioxo-1-piperidin-1-ylbutan-2-yl]-methylamino]-3-methyl-1-oxobutan-2-yl]methylamino]-1-oxopropan-2-yl]amino]-4-methyl-1-oxopentan-2-yl]amino]-1-oxopropan-2-yl]-N-methylcarbamate(Fmoc-MeAla-Leu-Ala-MeVal-MeAsn-pip) (SEQ ID NO: 31)

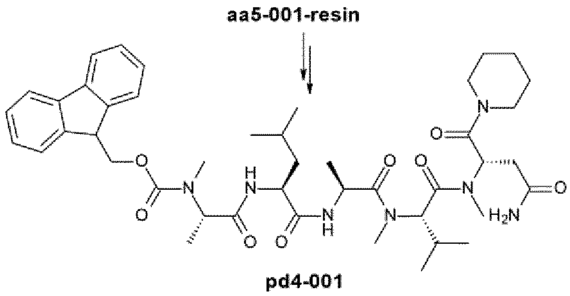


pd4-001

The dipeptide-supporting resin aa5-001-resin (Fmoc-MeVal-MeAsp(NH-Sieber resin)-pip) (99.51 mg, 0.538 mmol/g, 0.0535 mmol) prepared in Example 1-4 was provided as a raw material, and reactions of elongation with corresponding amino acids were sequentially carried out in accordance with synthesis method 1 to synthesize pd4-001-resin (114.04 mg). The loading rate calculated by the Fmoc quantitation method using a dry resin (11.54 mg) was 0.455 mmol/g. (UVarea value at 294 nm: 5167.27 and UVarea value at 304 nm: 4651.86). Therefore, the amount of the obtained peptide was calculated to be 114.04 × 0.001 × 0.455 = 0.0519 mmol (yield: 97.0%).

Analysis of the isolating reaction liquid by LCMS showed that the purity of intended product pd4-001 was 95.0 area%.

LCMS (ESI) m/z = 818.9 $(M+H)^+$
Retention time: 0.88 min (analysis condition SQDFA05)

Synthesis of compound pd4-002, 9H-fluoren-9-ylmethyl N-[(28)-1-[[(28)-1-[[2-[[(28)-1-[[(28)-1-[[(28)-1-[[(2S)-4-amino-1,4-dioxo-1-piperidin-1-ylbutan-2-yl]-methylamino]-3-methyl-1-oxobutan-2-yl]methylamino]-1-oxopropan-2-yl]amino]-1-oxopropan-2-yl]-methylamino]-2-oxoethyl]-methylamino]-4-methyl-1-oxopentan-2-yl]aminol-1-oxopropan-2-yl]-N-methylcarbamate (Fmoc-MeAla-Leu-MeGly-MeAla-Ala-MeVal-MeAsn-pip) (SEQ ID NO: 32)

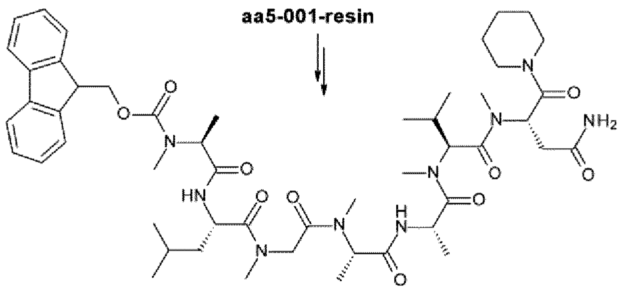


pd4-002

The dipeptide-supporting resin aa5-001-resin (Fmoc-MeVal-MeAsp(NH-Sieber resin)-pip) (97.92 mg, 0.538 mmol/g, 0.0527 mmol) prepared in Example 1-4 was provided as a raw material, and reactions of elongation with corresponding amino acids were sequentially carried out in accordance with synthesis method 1 to synthesize pd4-002-resin (123.73 mg). The loading rate calculated by the Fmoc quantitation method using a dry resin (11.08 mg) was 0.404 mmol/g. (UVarea value at 294 nm: 4405.15 and UVarea value at 304 nm: 3963.55). Therefore, the amount of the obtained peptide was calculated to be 123.73 × 0.001 ×0.404 = 0.0500 mmol (yield: 94.9%).

Analysis of the isolating reaction liquid by LCMS showed that the purity of intended product pd4-002 was 95.0 area%.

LCMS (ESI) m/z = 975.0 $(M+H)^+$
Retention time: 0.83 min (analysis condition SQDFA05)

Synthesis of compound pd4-003, 9H-fluoren-9-ylmethyl N-[(28)-1-[[(28)-1-[[2-[[(2S)-1-[[(28)-1-[[(28)-1-[[(2S)-1-[(2S)-2-[[(2S)-1-[[(2S)-1-[[(2S)-4-amino-1,4-dioxo-1-piperidin-1-ylbutan-2-yl]-methylamino]-3-methyl-1-oxobutan-2-yl]-methylamino]-1-oxopropan-2-yl]carbamoyl]pyrrolidin-1-yl]-1-oxopropan-2-yl]amino]-1-oxo-3-phenylpropan-2-yl]-methylamino]-4-methyl-1-oxopentan-2-yl]amino]-1-oxopropan-2-yl]amino]-2-oxoethyl]-methylamino]-4-methyl-1-oxopentan-2-yl]amino]-1-oxopropan-2-yl]-N-methylcarbamate (Fmoc-MeAla-Leu-MeGly-Ala-Leu-MePhe-Ala-Pro-Ala-MeVal-MeAsn-pip) (SEQ ID NO: 33)

aa5-001-resin

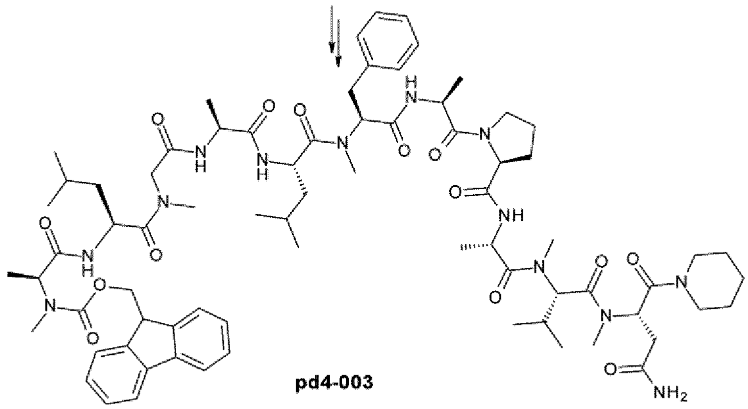

pd4-003

The dipeptide-supporting resin aa5-001-resin (Fmoc-MeVal-MeAsp(NH-Sieber resin)-pip) (97.73 mg, 0.538 mmol/g, 0.0526 mmol) prepared in Example 1-4 was provided as a raw material, and reactions of elongation with corresponding amino acids were sequentially carried out in accordance with synthesis method 1 to synthesize pd4-003-resin (146.90 mg). The loading rate calculated by the Fmoc quantitation method using a dry resin (9.45 mg) was 0.336 mmol/g. (UVarea value at 294 nm: 3126.02 and UVarea value at 304 nm: 2813.70). Therefore, the amount of the obtained peptide was calculated to be 146.90 $\times$ 0.001 $\times$ 0.336 = 0.0494 mmol (yield: 93.9%).

Analysis of the isolating reaction liquid by LCMS showed that the purity of intended product pd4-003 was 98.8 area%.

LCMS (ESI) m/z = 1403.3 $(M+H)^+$

Retention time: 2.85 min (analysis condition SQDFA05long)

Thus, as is shown in Example 3-3, the resin to which the present invention is applicable is not limited to 2-chlorotrityl resin.

Example 3-4. Synthesis of chain peptide using dipeptide-supporting resin

In this Example, the dipeptide-supporting resin prepared in Example 1-4 was provided as a raw material, and a solid phase reaction using a peptide synthesizing machine, isolation, cyclization and purification were carried out to synthesize a cyclic peptide.

Synthesis of compound pd5-001, (3S,9S,12S,15S,18S,21S,27S,30S,34S)-18-benzyl-1,4,12,13,19,21,25,30,31-nonamethyl-9,15,27-tris(2-methylpropyl)-34-(piperidine-1-carbonyl)-3-propan-2-yl-1,4,7,10,13,16,19,22,25,28,31-undecazacyclotetratriacontan-2,5,8,11,14,17,20,23,26,29,32-undecaone (cyclic compound having an amide bond formed by the N-terminal amino group and the side-chain carboxylic acid of MeAsp of H-MeAla-Leu-MeGly-Ala-MePhe-Leu-MeAla-Leu-Gly-MeVal-MeAsp-pip (SEQ ID NO: 34))

aa2-001-resin

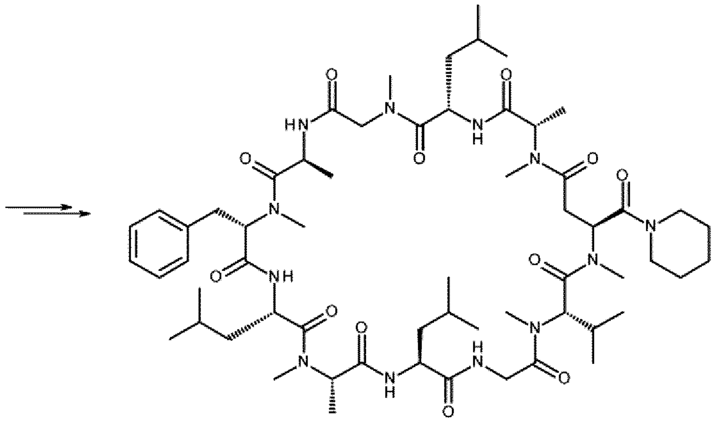

pd5-001

The dipeptide-supporting resin aa2-001-resin (100.78 mg, 0.363 mmol/g, 0.0366 mmol) prepared in Example 1-4 was provided as a raw material, and reactions of elongation with corresponding amino acids were sequentially carried out in accordance with synthesis method 2, and isolation, cyclization and purification were carried out to synthesize 28.6 mg of pd5-001 (yield: 66%).

LCMS (ESI) m/z = 1180.2 $(M+H)^+$
Retention time: 0.73 min (analysis condition SQDAA50)

Synthesis of compound pd5-002, (2S,5S,8S,11S,14S,20S,23S,26S,29S,32S)-14-benzyl-N,N,1,2,7,11,13,19,20,26,28-undecamethyl-5,8,23-tris(2-methylpropyl)-3,6,9,12,15,18,21,24,27,30,34-undecaoxo-29-propan-2-yl-1,4,7,10,13,16,1922252831-undecazacyclotetratriacontan-32-carboxamide (cyclic compound having an amide bond formed by the N-terminal amino group and the side-chain carboxylic acid of Asp of H-MeAla-Leu-MeLeu-Ala-MePhe-Gly-MeAla-Leu-Ala-MeVal-Asp-NMe2 (SEQ ID NO: 35))

aa2-012-resin

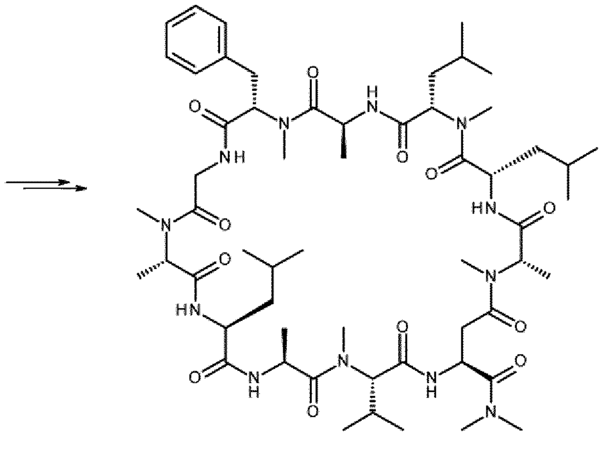

pd5-002

The dipeptide-supporting resin aa2-012-resin (99.83 mg, 0.373 mmol/g, 0.0372 mmol) prepared in Example 1-4 was provided as a raw material, and reactions of elongation with corresponding amino acids were sequentially carried out in accordance with synthesis method 2, and isolation, cyclization and purification were carried out to synthesize 27.1 mg of pd5-002 (yield: 64%).

LCMS (ESI) m/z = 1140.2 $(M+H)^+$
Retention time: 0.70 min (analysis condition SQDAA50)

Synthesis of compound pd5-003, (3S,6S,12S,15S,18S,21S,24S,27S,30S,33S)-27-benzyl-6,7,10,13,15,19,21,22,28,30-decamethyl-3,18,24-tris(2-methylpropyl)-12-propan-2-yl-1,4,7,10,13,16,1922252831-undecazabicyclor[31.3.0]hexatriacontan-2,5,8,11,14,172023262932-undecaone (cyclic compound having an amide bond formed by the N-terminal amino group and the C-terminal carboxylic acid of H-MeAla-Leu-Pro-Ala-MePhe-Leu-MeAla-MeLeu-Ala-MeVal-MeGly-OH (SEQ ID NO: 36))

aa2-010-resin

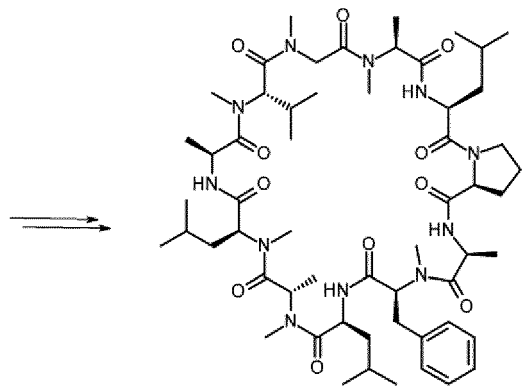

pd5-003

The dipeptide-supporting resin aa2-010-resin (101.36 mg, 0.374 mmol/g, 0.0379 mmol) prepared in Example 1-4 was provided as a raw material, and reactions of elongation with corresponding amino acids were sequentially carried out in accordance with synthesis method 2, and isolation, cyclization and purification were carried out to synthesize 7.07 mg of pd5-003 (yield: 17%).

LCMS (ESI) m/z = 1109.2 $(M+H)^+$

Retention time: 0.85 min (analysis condition SQDFA05_55deg)

Synthesis of compound pd5-004, (3S,9S,12S,18S,21S,24S,27S,30S,33S)-18,33-dibenzyl-1,3,4,10,12,13,21,22,28,30-decamethyl-9,24,27-tris(2-methylproyl)-1,4,7,10,13,16,19,22,25,28,31-undecazacyclotritriacontan-2,5,8,11,14,17,20,23,26,29,32-undecaone (cyclic compound having an amide bond formed by the N-terminal amino group and the side-chain carboxylic acid of H-MeAla-MeLeu-Gly-MeAla-MePhe-Ala-MeLeu-Leu-MeAla-Phe-Gly-OH (SEQ ID NO: 37))

aa2-028-resin

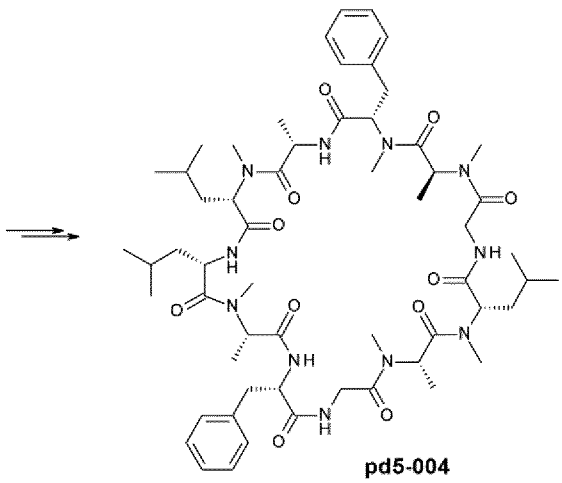

pd5-004

The dipeptide-supporting resin aa2-028-resin (100.88 mg, 0.248 mmol/g, 0.025 mmol) prepared in Example 1-4 was provided as a raw material, and reactions of elongation with corresponding amino acids were sequentially carried

out in accordance with synthesis method 2, and isolation, cyclization and purification were carried out to synthesize 7.03 mg of pd5-004 (yield: 25%).

LCMS (ESI) m/z = 1117.2 (M+H)+
Retention time: 0.75 min (analysis condition SQDAA50)

Example 3-5. Synthesis of cyclic peptide using tripeptide-supporting resin

In this Example, the tripeptide-supporting resin prepared in Example 1-4 was provided as a raw material, and a solid phase reaction using a peptide synthesizing machine, isolation, cyclization and purification were carried out to synthesize a cyclic peptide.

Synthesis of compound pd5-005, (3S,6S,9S,12S,15S,18S,21S,24S,30S,33S,36S)-21-benzyl-3,6,10,15,16,22,24,28,33,34-decamethyl-9,12,18,30-tetrakis(2-methylpropyl)-1,4,7,10,13,16,19,22,25,28,31,34-dodecazabicyclo[34.3.0]nonatriacontane-2,5,8,11,14,17,20,23,26,29,32,35-dodecaone (cyclic compound having an amide bond formed by the N-terminal amino group and the C-terminal carboxylic acid of H-MeAla-Leu-MeGly-Ala-MePhe-Leu-MeAla-Leu-MeLeu-Ala-Ala-Pro-OH (SEQ ID NO: 38))

aa2-032-resin

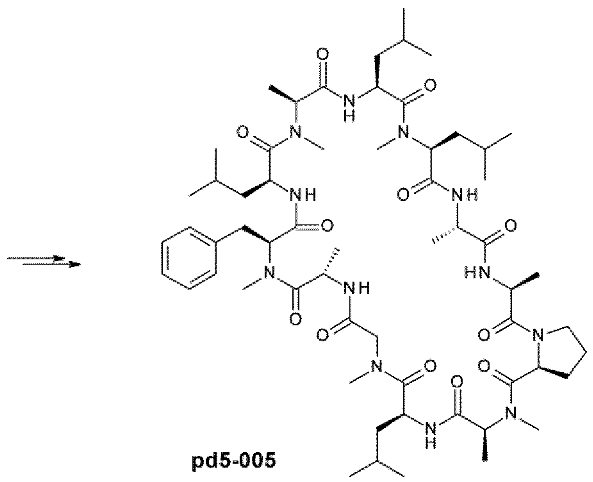

pd5-005

The tripeptide-supporting resin aa2-032-resin (Fmoc-Ala-Ala-Pro-O-Trt(2-Cl)resin) (99.1 mg, 0.393 mmol/g, 0.0389 mmol) prepared in Example 1-4 was provided as a raw material, and reactions of elongation with corresponding amino acids were sequentially carried out in accordance with synthesis method 2, and isolation, cyclization and purification were carried out to synthesize 23.8 mg of pd5-005 (yield: 52%).

LCMS (ESI) m/z = 1180.2 (M+H)+
Retention time: 0.73 min (analysis condition SQDAA50)

Synthesis of compound pd5-006, (2S,SS,8S,11S,14S,20S,23S,26S,29S,32S,35S)-14-benzvl-32-cvclopentvl-N,N,1,2,7,11,13,19,20,25,26,29,31,34-tetradecamethyl-5,8,23-tris(2-methylpropyl)-3,6,9,12,15,18,21,24,27,30,33,37-dodecaoxo-1,4,7,10,13,16,19,22,25,28,31,34-dodecazacycloheptatriacontane-35-carboxamide (cyclic compound having an amide bond formed by the N-terminal amino group and the side-chain carboxylic acid of MeAsp of H-MeAla-Leu-MeLeu-Ala-MePhe-Gly-MeAla-Leu-MeAla-Ala-MeGly(cPent)-MeAsp-NMe2 (SEQ ID NO: 39))

aa2-023-resin

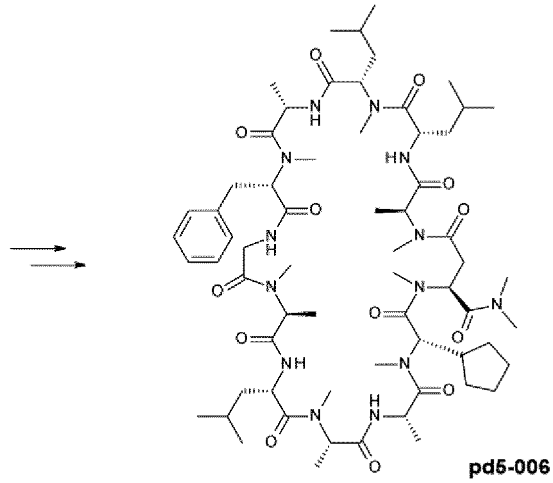

pd5-006

The tripeptide-supporting resin aa2-023-resin (Fmoc-Ala-MeGly(cPent)-MeAsp(O-Trt(2-Cl)resin)-NMe2) (99.12 mg, 0.378 mmol/g, 0.0375 mmol) prepared in Example 1-4 was provided as a raw material, and reactions of elongation with corresponding amino acids were sequentially carried out in accordance with synthesis method 2, isolation, cyclization and purification were carried out to synthesize 25.6 mg of pd5-006-resin (yield: 54%).

LCMS (ESI) m/z = 1265.3 $(M+H)^+$
Retention time: 0.73 min (analysis condition SQDAA50)

Industrial Applicability

The present invention provides a method for producing a peptide compound using a solid phase process. In addition, the present invention provides a method for improving the recovery ratio of the peptide compound, a method for suppressing generation of impurities and/or a method for suppressing premature cleavage in production of the peptide compound by a solid phase process.

## Claims

1. A method for producing a peptide compound containing at least one N-substituted amino acid residue, a salt thereof or a solvate thereof by a solid phase process, wherein a peptide is supported on a solid phase synthesis resin before a first elongation reaction in the solid phase process.

2. A method for producing a peptide compound containing at least one N-substituted amino acid residue, a salt thereof or a solvate thereof by a solid phase process, the method comprising a step of supporting a peptide on a solid phase synthesis resin.

3. The method according to claim 1 or 2, wherein the peptide is an oligopeptide containing two or more amino acid residues.

4. The method according to any one of claims 1 to 3, wherein an amino acid residue at the C-terminal of the peptide and/or an amino acid residue adjacent to the amino acid residue at the C-terminal are non-natural amino acid residues.

5. The method according to any one of claims 1 to 4, wherein the amino acid residue at the C-terminal of the peptide is a non-natural amino acid residue.

6. The method according to claim 5 or 6, wherein the non-natural amino acid residue is a N-substituted amino acid residue.

7. The method according to any one of claims 1 to 6, wherein the amino acid residue at the C-terminal of the peptide is supported on the solid phase synthesis resin by a carboxyl group bonded to a carbon atom at the β-position or a carbon atom at the γ-position on the amino group.

**8.** The method according to any one of claims 1 to 7, wherein an amino acid residue at the C-terminal of the peptide and/or an amino acid residue adjacent to the amino acid residue at the C-terminal have a bulky side chain.

**9.** The method according to claim 8, wherein the bulky side chain is an optionally substituted branched-chain alkyl group.

**10.** The method according to claim 9, wherein the branched-chain alkyl group is bonded to a carbon atom at the α-position on the carboxyl group.

**11.** The method according to claim 10, wherein the branched-chain alkyl group has a branch on a carbon atom at the β-position or a carbon atom at the γ-position on the carboxyl group.

**12.** The method according to any one of claims 1 to 11, wherein the solid phase synthesis resin is CTC resin, Wang resin, SASRIN resin, Trt resin, Mtt resin, or Mmt resin.

**13.** The method according to claim 12, wherein the solid phase synthesis resin is CTC resin.

**14.** The method according to any one of claims 1 and 3 to 13, comprising a step of supporting a peptide on the solid phase synthesis resin.

**15.** The method according to any one of claims 1 to 14, further comprising a step of elongating a peptide with one or more amino acids.

**16.** A method for producing a cyclic peptide, a salt thereof, or a solvate thereof, the method comprising the steps of:

obtaining a peptide compound containing at least one N-substituted amino acid residue, a salt thereof or a solvate thereof in accordance with the method according to any one of claims 1 to 15;
removing a solid phase synthesis resin; and
cyclizing a C-terminal-side group and a N-terminal-side group of the peptide compound, a salt thereof or a solvate thereof to form a cyclic portion.

**17.** A method for improving a recovery ratio of a peptide compound as compared to elongation with amino acid residues one by one, wherein a peptide is supported on a solid phase synthesis resin before a first elongation reaction in production of a peptide compound containing at least one N-substituted amino acid residue, a salt thereof or a solvate thereof by a solid phase process.

**18.** A method for suppressing generation of impurities as compared to elongation with amino acid residues one by one, wherein a peptide is supported on a solid phase synthesis resin before a first elongation reaction in production of a peptide compound containing at least one N-substituted amino acid residue, a salt thereof or a solvate thereof by a solid phase process.

**19.** A method for suppressing premature cleavage as compared to elongation with amino acid residues one by one, wherein a peptide is supported on a solid phase synthesis resin before a first elongation reaction in production of a peptide compound containing at least one N-substituted amino acid residue, a salt thereof or a solvate thereof by a solid phase process.

**INTERNATIONAL SEARCH REPORT**

International application No. **PCT/JP2021/048093**

**A. CLASSIFICATION OF SUBJECT MATTER**

***C07K 1/04***(2006.01)i; ***C07C 269/06***(2006.01)i; ***C07C 271/22***(2006.01)i; ***C07K 5/06***(2006.01)i; ***C07K 5/08***(2006.01)i; ***C07K 5/10***(2006.01)i; ***C07K 7/06***(2006.01)i; ***C07K 7/64***(2006.01)i
FI: C07K1/04 ZNA; C07C269/06; C07C271/22; C07K5/06; C07K5/08; C07K5/10; C07K7/06; C07K7/64

According to International Patent Classification (IPC) or to both national classification and IPC

**B. FIELDS SEARCHED**

Minimum documentation searched (classification system followed by classification symbols)

C07K1/04; C07C269/06; C07C271/22; C07K5/06; C07K5/08; C07K5/10; C07K7/06; C07K7/64

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2007-332042 A (NATIONAL INST. OF ADVANCED INDUSTRIAL & TECHNOLOGY) 27 December 2007 (2007-12-27)<br>claims, examples, paragraphs [0028]-[0030] | 1-6, 8-11, 14, 15, 17-19 |
| Y | claims, examples, paragraphs [0028]-[0030] | 1-19 |
| X | JP 2010-529957 A (ELI LILLY AND CO.) 02 September 2010 (2010-09-02)<br>example 88 | 1-6, 8, 9, 12-19 |
| Y | example 88 | 1-19 |
| Y | JP 2006-513980 A (APLAGEN GMBH) 27 April 2006 (2006-04-27)<br>claims, example 3 | 1-19 |
| Y | JP 2014-162722 A (CHEMICAL AND BIOPHARMACEUTICAL LABOLATORIES OF PATRA SA ) 08 September 2014 (2014-09-08)<br>claims, paragraphs [0040]-[0044], examples 22, 23 | 1-19 |
| Y | WO 2018/225851 A1 (CHUGAI SEIYAKU KK) 13 December 2018 (2018-12-13)<br>claims, examples | 1-19 |

☐ Further documents are listed in the continuation of Box C.   ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 January 2022** | **08 February 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2021/048093**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   - a. ☑ forming part of the international application as filed:
     - ☑ in the form of an Annex C/ST.25 text file.
     - ☐ on paper or in the form of an image file.
   - b. ☐ furnished together with the international application under PCT Rule 13*ter*.1(a) for the purposes of international search only in the form of an Annex C/ST.25 text file.
   - c. ☐ furnished subsequent to the international filing date for the purposes of international search only:
     - ☐ in the form of an Annex C/ST.25 text file (Rule 13*ter*.1(a)).
     - ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
**Information on patent family members**

International application No.
**PCT/JP2021/048093**

| Patent document cited in search report | Publication date (day/month/year) | Patent family member(s) | Publication date (day/month/year) |
|---|---|---|---|
| JP 2007-332042 A | 27 December 2007 | (Family: none) | |
| JP 2010-529957 A | 02 September 2010 | US 2008/0300177 A1<br>example 88<br>US 2010/0130409 A1<br>WO 2008/150689 A1<br>EP 2377579 A1<br>KR 10-2010-0003737 A<br>CN 101678213 A | |
| JP 2006-513980 A | 27 April 2006 | US 2007/0060508 A1<br>claims, example 3<br>WO 2004/014951 A2<br>EP 1394180 A1<br>KR 10-2005-0042146 A<br>CN 1675243 A | |
| JP 2014-162722 A | 08 September 2014 | (Family: none) | |
| WO 2018/225851 A1 | 13 December 2018 | US 2020/0277327 A1<br>claims, examples<br>EP 3636656 A1<br>CN 110799520 A<br>KR 10-2020-0016941 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013100132 A
- WO 2018115864 A
- WO 2020122182 A
- WO 2018225864 A
- WO 2018225851 A


### Non-patent literature cited in the description


- *Future Med. Chem.,* 2009, vol. 1, 1289-1310
- *Acc. Chem. Res.,* 2008, vol. 41, 1331-1342
- *Angew. Chem. Int. Ed.,* 2013, vol. 52, 254-269
- *Chem. Rev.,* 2019, vol. 119, 10360-10391
- *J. Peptide Res.,* 2005, vol. 65, 153-166
- *Amino Acids, Peptides and Proteins in Organic Chemistry: Building Blocks, Catalysis and Coupling Chemistry,* 2011, vol. 3
- *Amino Acids,* 2018, vol. 50, 39-68
- Solid phase peptide synthesis. Bachem, 06 November 2020
- *QSAR Comb. Sci.,* 2007, vol. 26, 1027-1035
- *Biopolymers,* 2012, vol. 98, 89-97
- *ACS Comb. Sci.,* 2013, vol. 15, 229-234
- Solid Phase Synthesis Handbook. Merck, 01 May 2002